(19) Europäisches Patentamt
European Patent Office
Office européen des brevets

(11) Veröffentlichungsnummer: **0 219 756 B1**

(12) **EUROPÄISCHE PATENTSCHRIFT**

(45) Veröffentlichungstag der Patentschrift: **05.01.94**

(51) Int. Cl.[5]: **C07D 295/18**, C07D 317/30, C07D 319/06, C07D 265/30, C07D 249/08, C07D 231/12, C07D 233/61, C07D 207/337, C07D 339/08, C07F 9/145, C07F 7/18

(21) Anmeldenummer: **86113835.2**

(22) Anmeldetag: **06.10.86**

Die Akte enthält technische Angaben, die nach dem Eingang der Anmeldung eingereicht wurden und die nicht in dieser Patentschrift enthalten sind.

# (54) Neue Acrylsäureamide.

(30) Priorität: **09.10.85 DE 3536029**
**26.11.85 DE 3541718**
**07.05.86 DE 3615447**

(43) Veröffentlichungstag der Anmeldung:
**29.04.87 Patentblatt 87/18**

(45) Bekanntmachung des Hinweises auf die Patenterteilung:
**05.01.94 Patentblatt 94/01**

(84) Benannte Vertragsstaaten:
**AT BE CH DE ES FR GB GR IT LI LU NL SE**

(56) Entgegenhaltungen:
**DE-A- 3 306 996**
**DE-A- 3 308 045**

(73) Patentinhaber: **SHELL INTERNATIONALE RESEARCH MAATSCHAPPIJ B.V.**
**Carel van Bylandtlaan 30**
**NL-2596 HR Den Haag(NL)**

(72) Erfinder: **Curtze, Jürgen, Dr.**
**Rheingaublick 6**
**D-6222 Geisenheim-Johannisberg(DE)**
Erfinder: **Pieper, Helmut, Dr. Dipl.-Chem.**
**Kapellenweg 5**
**D-7950 Biberach an der Riss/Riss 1(DE)**
Erfinder: **Nickl, Josef, Dr. Dipl.-Chem.**
**Silcherstrasse 8**
**D-7950 Biberach an der Riss 1(DE)**
Erfinder: **Becher, Heinz-Manfred, Dr. Chem.**
**Pfarrer-Heberer-Strasse 5**
**D-6530 Bingen am Rhein(DE)**
Erfinder: **Albert, Guido, Dr. Dipl.-Ing. Arg.**
**Volxheimer Strasse 4**
**D-6551 Hackenheim(DE)**
Erfinder: **Drandarevski, Christo, Dr.**
**Boehringer Strasse 8**
**D-6507 Ingelheim am Rhein(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.9/3.3.3)

Erfinder: **Lust, Sigmund, Dr.**
**Klappacher Strasse 2 F**
**D-6100 Darmstadt(DE)**
Erfinder: **Schröder, Ludwig, Dr. Dipl.-Chem.**
**Frankenstrasse 7**
**D-6507 Ingelheim am Rhein(DE)**

## Beschreibung

Die Erfindung betrifft neue Acrylsäureamide, ihre Herstellung und ihre Verwendung als mikrobizide Mittel.

EP-A-0120321 betrifft Acrylsäureamide der Formel

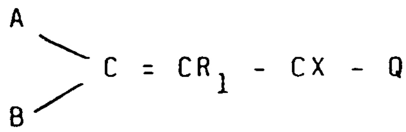

(I),

in der

A für die Gruppe

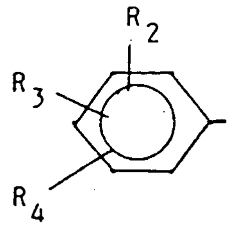

(II),

B für die Gruppe

$Y-(CR_5 = CR_6)_k-$ (III),

Q für eine der Gruppen

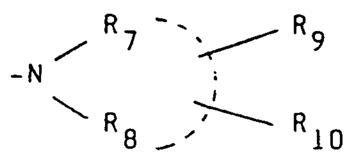

(IV)

und

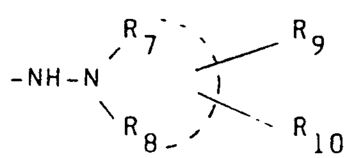

(V)

X für O, S oder NH,

Y für $C_3$-$C_{10}$-Alkyl sowie zusätzlich für $C_1$-$C_2$-Alkyl, wenn k gleich 1 oder 2 ist, oder für substituiertes $C_1$-$C_{10}$-Alkyl oder für einen gegebenenfalls substituierten Rest aus der Gruppe $C_3$-$C_7$-Alkenyl, $C_3$-$C_7$-Cycloalkyl, $C_5$-$C_7$-Cycloalkenyl, Pyridyl, Furyl, Thienyl, α-und β-Naphthyl; oder zusammen mit der Gruppe $CR_5$ für einen 5- bis 7-gliedrigen, vorzugsweise gesättigten, cycloaliphatischen Rest, an den ein Benzolring ankondensiert sein kann; oder für den Rest

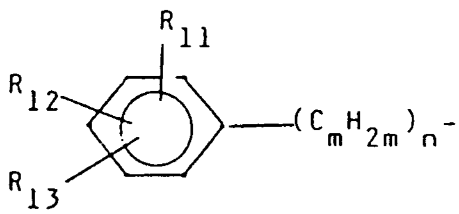

(VI)

steht, wobei

| | |
|---|---|
| k | die Zahlen 0, 1 oder 2, |
| m | die Zahlen 1, 2, 3 oder 4, |
| n | die Zahlen 0 oder 1, |
| $R_1$ | Wasserstoff, $C_1$-$C_4$-Alkyl, Cyano und, wenn k gleich O ist, auch Chlor, Brom oder Jod, |
| $R_2$ und $R_{11}$ | Wasserstoff, Halogen, Nitro, gegebenenfalls ein- oder mehrfach durch Fluor oder Chlor substituiertes $C_1$-$C_4$-Alkyl oder -Alkoxy, $C_3$-$C_4$-Alkinyloxy, Amino, NH($C_1$-$C_4$-Alkyl), N($C_1$-$C_4$-Alkyl)$_2$, Cyano, Phenyl, $C_3$-$C_6$-Cycloalkyl, $C_3$-$C_4$-Alkenyloxy, Hydroxy($C_1$-$C_4$-Alkyl), NH $COR_6$, $CO_2R_6$, $CONR_7R_8$, durch Sauerstoff unterbrochenes $C_2$-$C_8$-Alkyl oder |

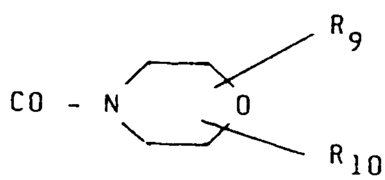

(VII),

| | |
|---|---|
| $R_3$, $R_4$, $R_{12}$ und $R_{13}$ | Wasserstoff, Halogen, $C_1$-$C_4$-Alkyl, $C_1$-$C_4$-Alkoxy, ($C_1$-$C_4$-Alkyl)-S(O)$_p$ (p = 0, 1 oder 2), Hydroxy oder ($C_1$-$C_4$-Acyl)oxy, $R_3/R_4$ und $R_{12}/R_{13}$ jeweils gemeinsam auch Methylendioxy oder Ethylendioxy, gebunden an zwei benachbarte Atome des Phenylrings, |
| $R_5$ und $R_6$ | Wasserstoff oder $C_1$-$C_4$-Alkyl, $R_5$ außerdem, zusammen mit dem C-Atom, an das es gebunden ist, und Y einen 5- bis 7-gliedrigen, vorzugsweise gesättigten, cycloaliphatischen Rest, an den ein Benzolring ankondensiert sein kann. |
| $R_7$ und $R_8$ | $C_1$-$C_4$-Alkyl, $C_3$-$C_7$-Cycloalkyl, Phenyl, Benzyl, Furfuryl, Tetrahydrofurfuryl oder $C_3$-$C_4$-Alkenyl, gemeinsam außerdem auch eine $C_3$-$C_5$-Alkylenkette, die durch O, $NR_6$, S(O)$_q$ (mit q = 0, 1 oder 2) unterbrochen sein kann, $R_7$ außerdem Wasserstoff, |
| $R_9$ und $R_{10}$, | die nur vorliegen, wenn $R_7$ und $R_8$ gemeinsam eine Kette darstellen, Wasserstoff oder $C_1$-$C_4$-Alkyl bedeuten, |

sowie gegebenenfalls die Säureadditionssalze der vorstehend definierten Verbindungen, mit der Einschränkung, dass A und B nicht beide für einen Rest aus der Gruppe Phenyl, Monohalogenphenyl, Mononitrophenyl und Monoaminophenyl stehen; und ihre Verwendung als Fungizide.

Die neuen Acrylsäureamide haben die Formel

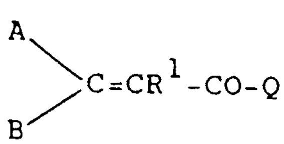

(I)

in der

A für

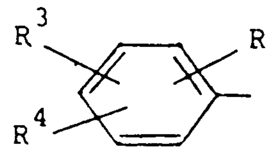

B für

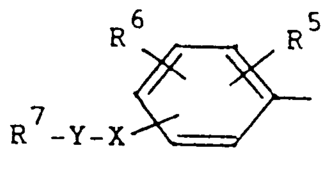 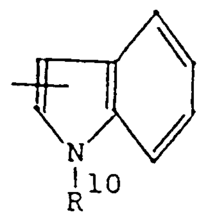 , 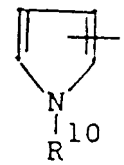

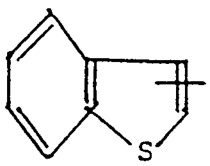 , 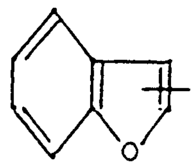 , 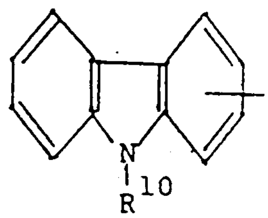

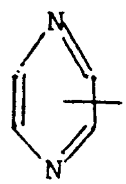 , 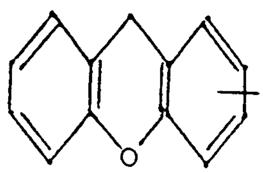 oder 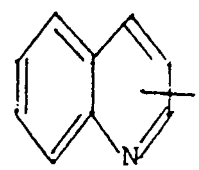

$R^1$ für Wasserstoff; Halogen, Cyano oder $C_1$-$C_4$-Alkyl gegebenenfalls ein oder mehrfach mit Hydroxy, Alkoxy, Mercapto, Halogen, Alkylthio, Nitro, Cyano, Amino oder Mono- oder Di-$C_1$-$C_4$-alkylamino substituiert, steht,

Q für

$NR^8R^9$ oder

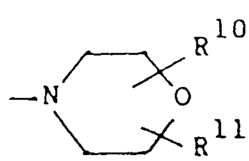

steht,

$R^2$, $R^3$, $R^4$, $R^5$ und $R^6$, die gleich oder verschieden sein können für Wasserstoff; Halogen, Nitro; Cyano; Carboxy; Hydroxy;

$C_1$-$C_4$-Alkoxycarbonyl; $CONR^{10}R^{11}$;

$NR^{10}R^{11}$; $NR^{10}$-CO-$R^{11}$;

oder für $C_1$-$C_6$-Alkyl, $C_1$-$C_4$-Alkoxy oder $C_1$-$C_4$-Alkyl-S(O)p (mit p = 0, 1, 2), gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl, Phenoxy oder Phenyl-S(O)p- (mit p = 0, 1, 2) gegebenenfalls bis zu dreifach durch Halogen, $C_{1-4}$ Alkyl,

Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^8$ und $R^9$, die gleich oder verschieden sein können für Wasserstoff; $C_1$-$C_4$-Alkyl, $C_3$-$C_7$-Alkenyl, $C_3$-$C_7$-Alkinyl oder Alkoxyalkyl gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl oder Benzyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^{10}$ und $R^{11}$, die gleich oder verschieden sein können, für Wasserstoff, Benzyl, Phenyl oder $C_1$-$C_4$-Alkyl, als Substituenten in Hetaryl auch für Halogen, Amino oder Mono- oder Di-$C_1$-$C_4$alkylamino stehen, oder gemeinsam für $-(CH_2)_4-$ oder $-(CH_2)_5-$,

X - Y für eine Einfachbindung; -O-; -S(O)p- (mit p = 0, 1, 2); -CONR$^{10}$-; -NR$^{10}$CO-; -NR$^{11}$CONR$^{10}$-; -N=N-; -CHR$^{10}$-S(O)p-(mit p = 0, 1, 2); -CHR$^{10}$O-; -SO$_2$NR$^{10}$-; -N=CH-; $-C_nH_{2n}-$ (mit n = 1 bis 10); -CH=CH-; -NR$^{1}$°CSNR$^{11}$-; -NR$^{10}$-; -R$^{10}$N-CHR$^{11}$-; -CHR$^{10}$NR$^{11}$-; -O-CHR$^{10}$-; -S(O)p-CHR$^{10}$- (mit p = 0, 1, 2); -NR$^{10}$SO$_2$- oder -OSO$_2$-; -O$_2$SO-; -O$_2$S-NR$^{10}$-; -C≡C-; -S-S-; -CHOH-; -CO-; >C=CH$_2$; >C=CH-COOH; -NH-NH-; -N(O)=N-;

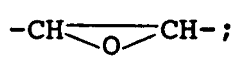

-N(R$^{10}$)-N=CH-; -N(R$^{10}$)-NH-CO-; -NH-CH=CH-; >CH-C$_6$H$_5$; -COCH$_2$O-; -CO-CH$_2$S-; -COCH$_2$NR$^{10}$-; -OCH$_2$CO; -SCH$_2$CO-; -CH=N-; -NR$^{10}$CH$_2$CO-; -CH=NO-; -CH$_2$O-CONR$^{10}$-; -CH$_2$O-CO-; -S-CH$_2$-; -CH$_2$CO-; -CO-O-; -O-CO-; -CO-S- oder -S-CO- steht;

$R^7$ für Wasserstoff; NR$^{10}$R$^{11}$; PO(OR$^{10}$)(OR$^{11}$); (CH$_2$)q-CO-O-R$^{10}$ (mit q = 0, 1, 2, 3); Cyano; CONR$^{10}$R$^{11}$; SO$_2$NR$^{10}$R$^{11}$; Tri-$C_1$-$C_4$alkyl-silyl; bis zu zweifach durch $R^{10}$ und $R^{11}$ substituiertes Hetaryl worin Hetaryl für Pyrazolyl-, Pyrazinyl-, Imidazolyl-,1,2,4-Triazolyl-, Morpholinyl-, Piperidinyl- Pyrrolyl-, Pyrrolidinyl-,2,5-Dioxopyrrolidinyl-, 1,3-Isoindoldionyl- und Pyridinylradikale, sowie von Indol, Benzofuran, Chinolin oder Benzothiophen abgeleitete Reste, steht oder

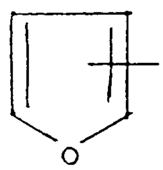 , 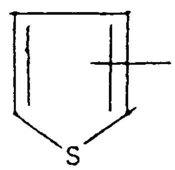 , 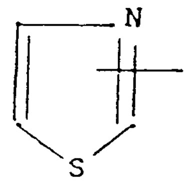 , 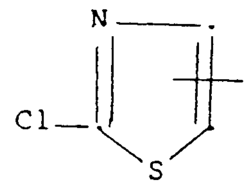

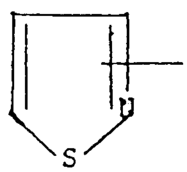 , 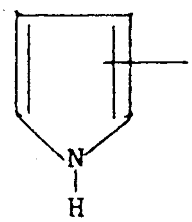 , 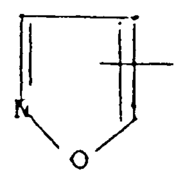 , 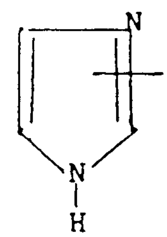

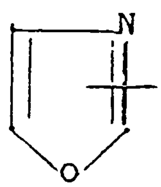 , 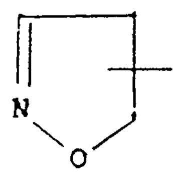 , 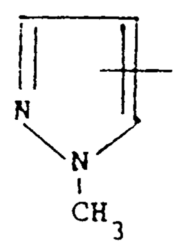 , 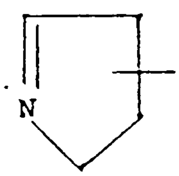

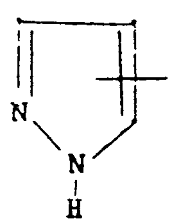 , 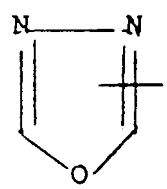 , 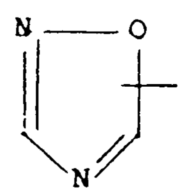 , 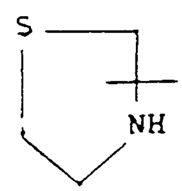

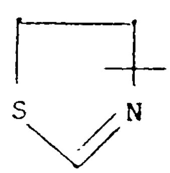 , 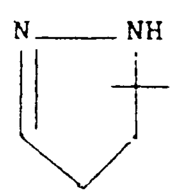 , 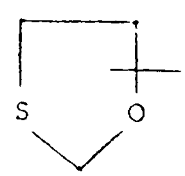 , 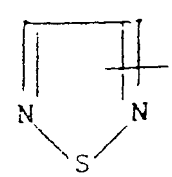

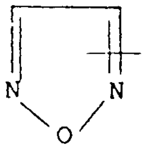 , 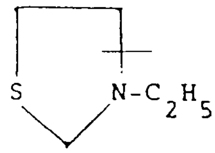 , 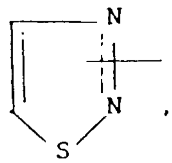 , 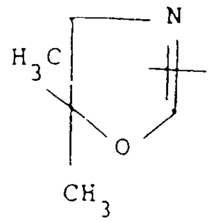

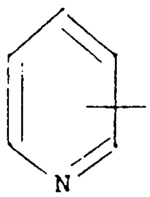 , 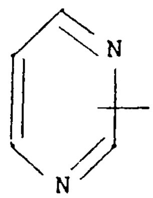 , 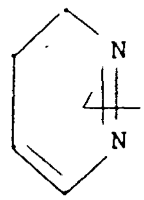 , 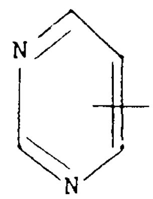

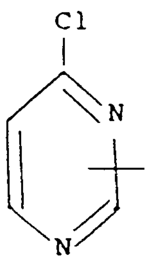 , 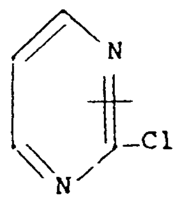 , 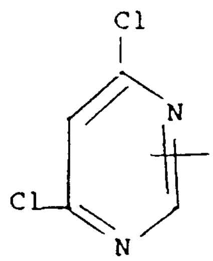 , 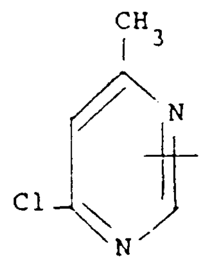

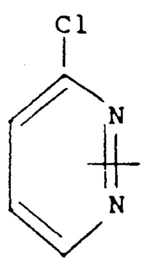 , 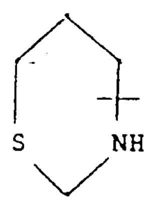 , 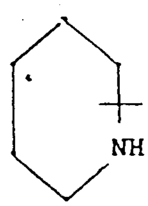 , 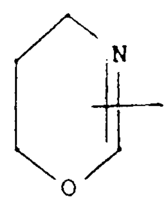

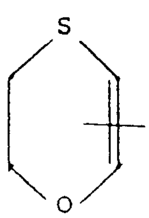 , 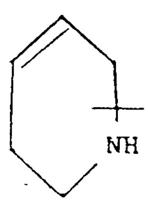 , 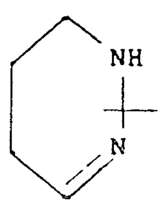 , 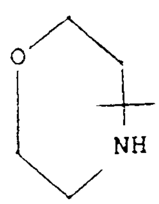

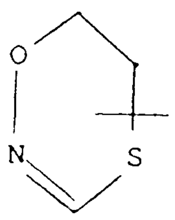 , 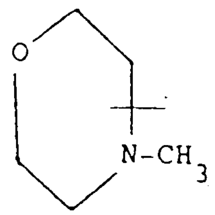 , 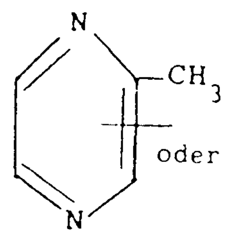 oder 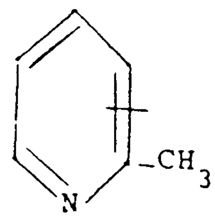

steht;

[(1-Formylamino-2,2,2-trichlor)ethyl]amino;

1-(3,4-Dimethoxyphenyl)-3-(morpholin-4-yl)-prop-1-en-3-on;

Phenyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert; Alkyl mit bis zu 12 C-Atomen gegebenenfalls durch Sauerstoff und/oder Schwefelatome unterbrochen und

gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, Cycloalkyl gegebenenfalls durch Sauerstoff und/oder Schwefelatome und/oder Stickstoff unterbrochen und gegebenenfalls bis zu dreifach durch Alkyl, Halogen, Hydroxy, Oxo oder Amino substituiert, phenylsubstituiertes $C_1$-$C_3$-Alkyl, $C_2$-$C_6$-Alkenyl, $C_2$-$C_6$-Alkinyl, $C_5$-$C_8$-Cycloalkenyl oder Naphthyl steht,

wenn X - Y für eine Einfachbindung steht, können $R^7$ and $R^6$ außerdem gemeinsam für eine vicinale Brücke der Formel

$-(CH_2)_n-E-$ (mit n = 2, 3), $-(CH=CH)-D$ oder

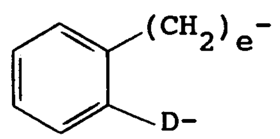

(mit e = 0 oder 1)

stehen;

wobei

E für $CH_2$; O; S oder $NR^{10}$

D für $CH_2$; O; S; $NR^{10}$; $-CH_2-CH_2-$ oder

$-CH=CH-$ steht, und gegebenenfalls ihre Salze; worin Alkyl $C_1$-$C_{12}$-C-Atome bedeutet;

und die Verbindungen:-3-(3,4-Dimethoxyphenyl)-3-[4-(4,5-dihydrooxazol-2-yl)phenyl]-acrylsäuremorpholid,

3-(3,4-Dimethoxyphenyl)-3-(4-benzimidazol-2-yl-phenyl)-acrylsäuremorpholid,

3-(3,4-Dimethoxyphenyl)-3-[4-(1,2-dichlorvinyloxy)-phenyl]-acrylsäuremorpholid,

3-(3,4-Dimethoxyphenyl)-3-(4-benzothiazol-2-yl-thiomethylphenyl)-acrylsäuremorpholid,

3-(3,4-Dimethoxyphenyl)-3-(4-benzothiazol-2-ylthiophenyl)-acrylsäuremorpholid,

3-(3,4-Dimethoxyphenyl)-3-[4-(3-p-toluol-sulfonylureido)-phenyl]-acrylsäuremorpholid,

3-(3,4-Dimethoxyphenyl)-3-[4-(4,6-dichlor-1,3,5-triazin-2-yloxy)-phenyl]-acrylsäuremorpholid,

3-(3,4-Dimethoxyphenyl)-3-[4-(2,5-dioxopyrrol-1-yl)-phenyl]-acrylsäuremorpholid,

3-(3,4-Dimethoxyphenyl)-3-[4-(6-chlor-2-ethylaminopyrimidin-4-yloxy)-phenyl]-acrylsäuremorpholid,

3-(3,4-Dimethoxyphenyl)-3-[4-(6-chlor-2-dimethylaminopyrimidin-4-yloxy)-phenyl]-acrylsäuremorpholid,

3-(3,4-Dimethoxyphenyl)-3-[4-(6-chlor-2-isopropylaminopyrimidin-4-yloxy)-phenyl]-acrylsäuremorpholid;

mit Ausnahme von dem Gegenstand der in EP-A-0120321 offenbart ist und mit Ausnahme von den Verbindungen der Formel I in der A und B beide für einen Rest aus der Gruppe Phenyl, Monohalogenphenyl, Mononitrophenyl und Monoaminophenyl Stehen.

Die Erfindung auch betrifft Verbindungen der Formel

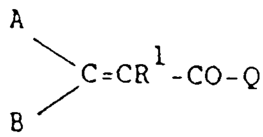

(I)

in der

A für

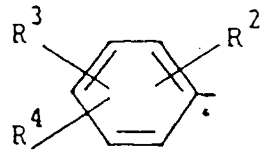

B für

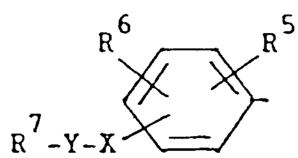,
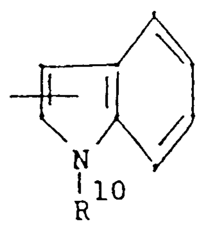,
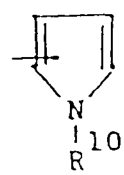,

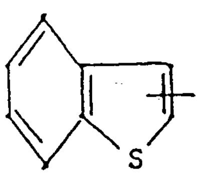,
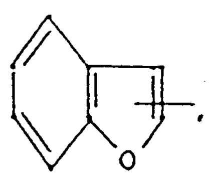,
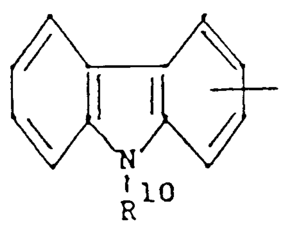

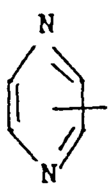,
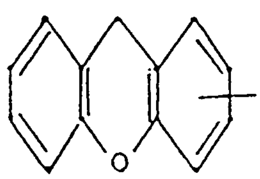
oder
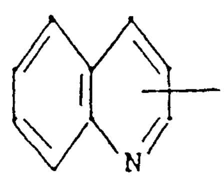

$R^1$ für Wasserstoff; Halogen, Cyano oder $C_1$-$C_4$-Alkyl gegebenenfalls ein oder mehrfach mit Hydroxy, Alkoxy, Mercapto, Halogen, Alkylthio, Nitro, Cyano, Amino oder Mono- oder Di-$C_1$-$C_4$-alkylamino substituiert, steht,

Q für

$NR^8R^9$ oder

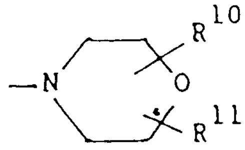

steht,

$R^2$, $R^3$, $R^4$, $R^5$ und $R^6$, die gleich oder verschieden sein können für Wasserstoff; Halogen, Nitro; Cyano; Carboxy; Hydroxy; $C_1$-$C_4$-Alkoxycarbonyl; $CONR^{10}R^{11}$; $NR^{10}R^{11}$; $NR^{10}$-CO-$R^{11}$; oder für $C_1$-$C_6$-Alkyl, $C_1$-$C_4$-Alkoxy oder $C_1$-$C_4$-Alkyl-S(O)p (mit p = 0, 1, 2), gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl, Phenoxy oder Phenyl-S(O)p- (mit p = 0, 1, 2) gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^8$ und $R^9$, die gleich oder verschieden sein können für Wasserstoff; $C_1$-$C_4$-Alkyl, $C_3$-$C_7$-Alkenyl, $C_3$-$C_7$-Alkinyl oder Alkoxyalkyl gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl oder Benzyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^{10}$ und $R^{11}$, die gleich oder verschieden sein können, für Wasserstoff, Benzyl, Phenyl oder $C_1$-$C_4$-Alkyl, als Substituenten in Hetaryl auch für Halogen, Amino oder Mono- oder Di-$C_1$-$C_4$-alkylamino stehen, oder gemeinsam für $-(CH_2)_4-$ oder $-(CH_2)_5-$,

X - Y für eine Einfachbndung; -O-; -S(O)p- (mit p = 0, 1, 2); $-CONR^{10}-$; $-NR^{10}CO-$; $-NR^{11}CONR^{10}-$; -N = N-; $-CHR^{10}$-S(O)p-(mit p = 0, 1, 2); $-CHR^{10}O-$; $-SO_2NR^{10}-$; -N = CH-; $-C_nH_{2n}-$ (mit n = 1 bis 10); -CH = CH-; $-NR^{10}CSNR^{11}-$; $-NR^{10}-$; $-R^{10}N-CHR^{11}-$; $-CHR^{10}NR^{11}-$; $-O-CHR^{10}-$; $-S(O)p-CHR^{10}-$ (mit p = 0, 1, 2); $-NR^{10}SO_2-$ oder $-OSO_2-$; $-O_2SO-$; $-O_2S-NR^{10}-$; -C≡C-; -S-S-; -CHOH-; -CO-; $>C = CH_2$; $>C$ = CH-COOH; -NH-NH-; -N(O) = N-;

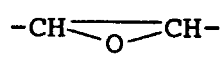 ;

$-N(R^{10})$-N = CH-; $-N(R^{10})$-NH-CO-; -NH-CH = CH-; $>CH-C_6H_5$; $-COCH_2O-$; $-CO-CH_2S-$; $-COCH_2NR^{10}-$; $-OCH_2CO$; $-SCH_2CO-$; -CH = N-; $-NR^{10}CH_2CO-$;-CH = NO-; $-CH_2O-CONR^{10}-$; $-CH_2O-CO-$; $-S-CH_2-$; $-CH_2CO-$; -CO-O-; -O-CO-; -CO-S- oder -S-CO- steht;

$R^7$ für Wasserstoff; $NR^{10}R^{11}$; $PO(OR^{10})(OR^{11})$; $(CH_2)q-CO-O-R^{10}$ (mit q = 0, 1, 2, 3); Cyano; $CONR^{10}R^{11}$; $SO_2NR^{10}R^{11}$; Tri-$C_1$-$C_4$-alkyl-silyl; bis zu zweifach durch $R^{10}$ und $R^{11}$ substituiertes Hetaryl worin Hetaryl für Pyrazolyl-, Pyrazinyl-, Imidazolyl-,1,2,4-Triazolyl-, Morpholinyl-, Piperidinyl- Pyrrolyl-, Pyrrolidinyl-,2,5-Dioxopyrrolidinyl-, 1,3-Isoindoldionyl- und Pyridinylradikale, sowie von Indol, Benzofuran, Chinolin oder Benzothiophen abgeleitete Reste, steht oder

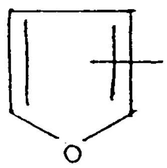 , 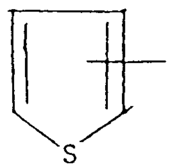 , 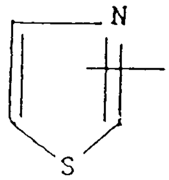 , 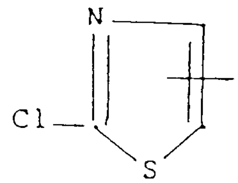

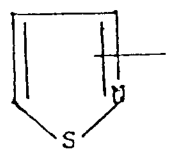, 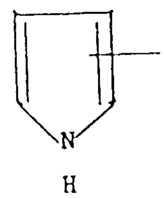, 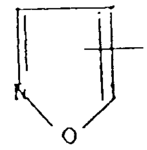, 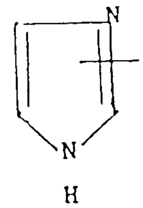

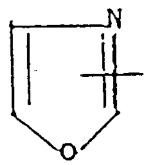, 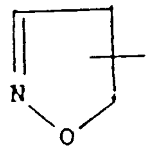, 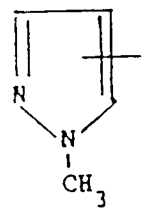, 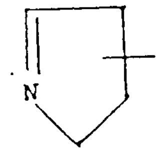

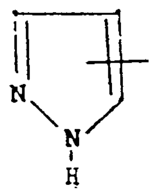, 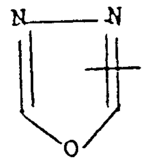, 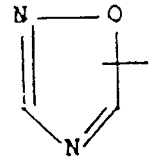, 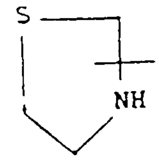

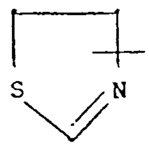, 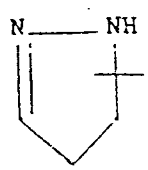, 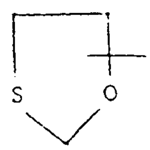, 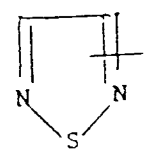

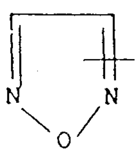, 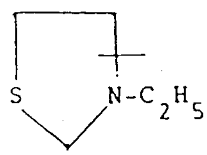, 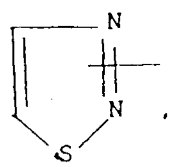, 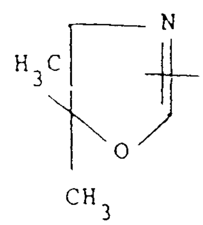

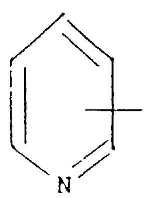, 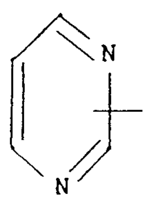, 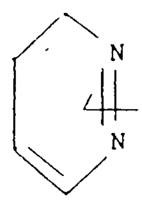, 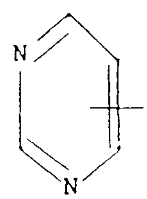

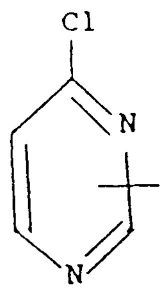 , 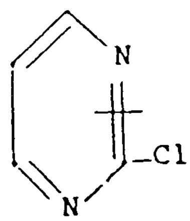 , 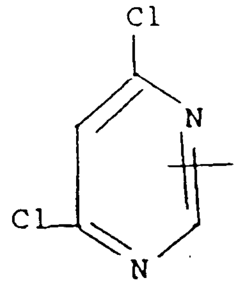 , 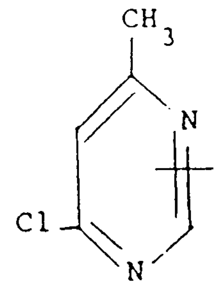

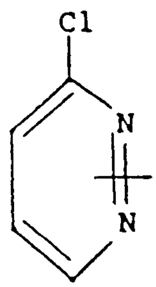 , 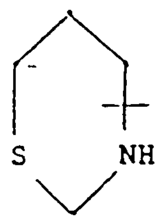 , 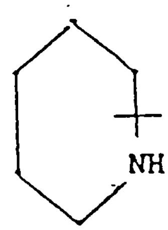 , 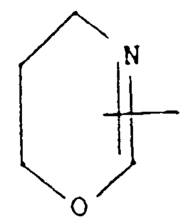

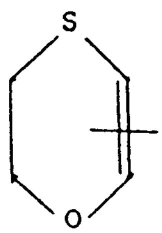 , 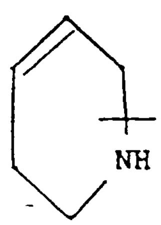 , 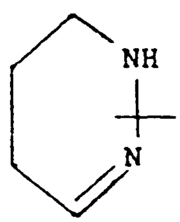 , 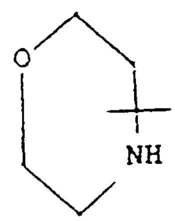

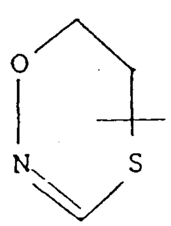 , 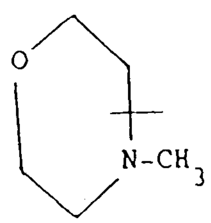 , 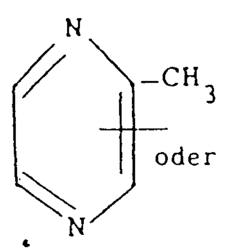 oder 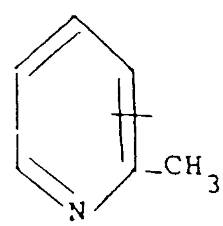

steht;

[(1-Formylamino-2,2,2-trichlor)ethyl]amino;

1-(3,4-Dimethoxyphenyl)-3-(morpholin-4-yl)-prop-1-en-3-on;

Phenyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert; Alkyl mit bis zu 12 C-Atomen gegebenenfalls durch Sauerstoff und/oder Schwefelatome unterbrochen und

gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, Cycloalkyl gegebenenfalls durch Sauerstoff und/oder Schwefelatome und/oder Stickstoff unterbrochen und gegebenenfalls bis zu dreifach durch Alkyl, Halogen, Hydroxy, Oxo oder Amino substituiert, phenylsubstituiertes $C_1$-$C_3$-Alkyl, $C_2$-$C_6$-Alkenyl, $C_2$-$C_6$-Alkinyl, $C_5$-$C_8$-Cycloalkenyl oder Naphthyl steht,

wenn X - Y für eine Einfachbindung steht, können $R^7$ and $R^6$ außerdem gemeinsam für eine vicinale Brücke der Formel

$-(CH_2)_n$-E- (mit n = 2, 3), -(CH=CH)-D oder

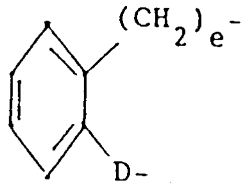

(mit e = O oder 1)

stehen;

wobei

E für $CH_2$; O; S oder $NR^{10}$

D für $CH_2$; O; S; $NR^{10}$; $-CH_2-CH_2-$ oder -CH=CH- steht, und gegebenenfalls ihre Salze; worin Alkyl $C_1$-$C_{12}$-C-Atome bedeutet;

und die Verbindungen:-

3-(3,4-Dimethoxyphenyl)-3-[4-(4,5-dihydrooxazol-2-yl)phenyl]-acrylsäuremorpholid,
3-(3,4-Dimethoxyphenyl)-3-(4-benzimidazol-2-yl-phenyl)-acrylsäuremorpholid,
3-(3,4-Dimethoxyphenyl)-3-[4-(1,2-dichlorvinyloxy)-phenyl]-acrylsäuremorpholid,
3-(3,4-Dimethoxyphenyl)-3-(4-benzothiazol-2-yl-thiomethylphenyl)-acrylsäuremorpholid,
3-(3,4-Dimethoxyphenyl)-3-(4-benzothiazol-2-ylthio-phenyl)-acrylsäuremorpholid,
3-(3,4-Dimethoxyphenyl)-3-[4-(3-p-toluol-sulfonylureido)-phenyl]-acrylsäuremorpholid,
3-(3,4-Dimethoxyphenyl)-3-[4-(4,6-dichlor-1,3,5-triazin-2-yloxy)-phenyl]-acrylsäuremorpholid,
3-(3,4-Dimethoxyphenyl)-3-[4-(2,5-dioxopyrrol-1-yl)-phenyl]-acrylsäuremorpholid,
3-(3,4-Dimethoxyphenyl)-3-[4-(6-chlor-2-ethylaminopyrimidin-4-yloxy)-phenyl]-acrylsäuremorpholid,
3-(3,4-Dimethoxyphenyl)-3-[4-(6-chlor-2-dimethylaminopyrimidin-4-yloxy)-phenyl]-acrylsäuremorpholid,
3-(3,4-Dimethoxyphenyl)-3-[4-(6-chlor-2-isopropylaminopyrimidin-4-yloxy)-phenyl]-acrylsäuremorpholid;

mit Ausnahme von den Verbindungen der Formel I in der

B für

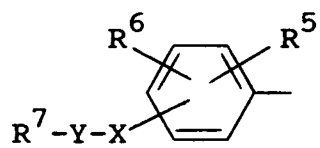

steht;

$R^1$ für Wasserstoff, $C_1$-$C_4$-Alkyl, Cyano, Chlor, Brom oder Jod steht;

$R^2$, $R^3$ und $R^4$, die gleich oder verschieden sein können, für Wasserstoff, Halogen, Nitro, gegebenenfalls ein- oder mehrfach durch Fluor oder Chlor substituiertes $C_1$-$C_4$-Alkyl oder -Alkoxy, Amino, $NH(C_1$-$C_4$-Alkyl), $N(C_1$-$C_4$-Alkyl$)_2$, Cyano, Phenyl, $C_3$-$C_6$-Cycloalkyl, Hydroxy ($C_1$-$C_4$-Alkyl), NHCOH, $NHCO(C_1$-$C_4$ Alkyl), $CO_2H$, $CO_2(C_1$-$C_4$-Alkyl), $CONR^{10}R^{11}$ in der $R^{10}$ und $R^{11}$, die gleich oder verschieden sein können, für $C_1$-$C_4$-Alkyl, Phenyl oder Benzyl, gemeinsam außerdem für $-(CH_2)_4$- oder $-(CH_2)_5$-, und $R^{10}$ außerdem für Wasserstoff, stehen, ($C_1$-$C_4$-Alkyl)-$S(O)_p$ (mit p=0,1,2) oder Hydroxy steht;

$R^5$ und $R^6$, die gleich oder verschieden sein können, für Wasserstoff, Halogen, $C_1$-$C_4$-Alkyl, $C_1$-$C_4$-Alkoxy, ($C_1$-$C_4$-Alkyl)-$S(O)_p$ (mit p=0,1,2) oder Hydroxy stehen, und $R^5$ außerdem für Nitro, ein- oder mehrfach durch Fluor oder Chlor substituiertes $C_1$-$C_4$-Alkyl oder -Alkoxy, NHCOH oder $NHCO(C_1$-$C_4$-Alkyl) steht;

X-Y für eine Einfachbindung steht;

$R^7$ für Wasserstoff, gegebenenfalls ein- oder mehrfach durch Fluor oder Chlor substituiertes $C_1$-$C_4$-Alkyl, Amino, $NH(C_1$-$C_4$-Alkyl), $N(C_1$-$C_4$-Alkyl$)_2$, Cyano, Phenyl, $C_3$-$C_6$-Cycloalkyl, Hydroxy ($C_1$-$C_4$-Alkyl), $CO_2H$, $CO_2(C_1$-$C_4$-Alkyl), $CONR^{10}R^{11}$ in der $R^{10}$ und $R^{11}$, die gleich oder verschieden sein können, für $C_1$-$C_4$-Alkyl, Phenyl oder

Benzyl, gemeinsam außerdem für $-(CH_2)_4-$ oder $-(CH_2)_5-$, und $R^{10}$ außerdem für Wasserstoff, stehen, durch Sauerstoff unterbrochenes $C_2$-$C_8$-Alkyl steht; oder $R^7$ und $R^6$ außerdem gemeinsam für eine vicinale Brücke der Formel -(CH = CH)-D stehen können, wobei D für -CH = CH- steht;

Q für $NR^8R^9$ oder

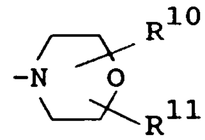

steht, in der

$R^8$ und $R^9$, die gleich oder verschieden sein können, für $C_1$-$C_4$-Alkyl, $C_3$-$C_7$-Cycloalkyl, Phenyl, Benzyl oder $C_3$-$C_4$-Alkenyl, und $R^8$ außerdem Wasserstoff, stehen, und

$R^{10}$ und $R^{11}$, die gleich oder verschieden sein können, für Wasserstoff oder $C_1$-$C_4$-Alkyl stehen;

und mit Ausnahme von den Verbindungen der Formel I in der A und B beide für einen Rest aus der Gruppe Phenyl, Monohalogenphenyl, Mononitrophenyl und Monoaminophenyl stehen.

Bevorzugt sind Verbindungen der Formel

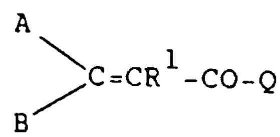

(I)

in der

A für

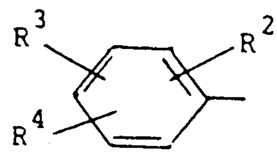

B für

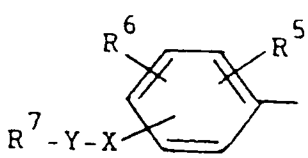

oder gegebenenfalls substituiertes p-Biphenylyl,

$R^1$ für Wasserstoff; ferner auch für Halogen, Cyano oder gegebenenfalls substituiertes $C_1$-$C_4$-Alkyl steht,

Q für

$NR^8R^9$ oder

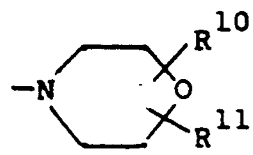

steht, wobei $R^8$ und $R^9$ insbesondere für $C_1$-$C_4$-Alkyl stehen, vor allem für $R^8$ gleich Methyl und $R^9$ gleich $C_1$-$C_4$-Alkyl, etwa Methyl, Ethyl, Propyl, und $R^{10}$ und $R^{11}$ vor allem für Wasserstoff stehen,

$R^2$, $R^3$, $R^4$, $R^5$ und $R^6$, die gleich oder verschieden sein können, für Wasserstoff; Halogen; Nitro, Cyano; Carboxy; Hydroxy;
$C_1$-$C_4$-Alkoxycarbonyl; $CONR^{10}R^{11}$;
$NR^{10}R^{11}$; $NR^{10}COR^{11}$
oder die gegebenenfalls substituierten Reste:
$C_1$-$C_4$-Alkyl, $C_1$-$C_4$-Alkoxy,
$C_1$-$C_4$-Alkyl-S(O)p; (mit p = 0, 1, 2);
$C_3$-$C_7$-Cycloalkyl, Phenyl, Phenoxy oder
Phenyl-(SO)p-(mit p = 0, 1, 2) stehen,

$R^8$ und $R^9$, die gleich oder verschieden sein können für Wasserstoff;
die gegebenenfalls substituierten Reste:
$C_1$-$C_4$-Alkyl, $C_3$-$C_7$-Cycloalkyl; Phenyl;
Benzyl, $C_3$-$C_4$-Alkenyl, Propargyl oder
Alkoxyalkyl stehen,

$R^{10}$ und $R^{11}$, die gleich oder verschieden sein können für Wasserstoff oder $C_1$-$C_4$-Alkyl stehen.

Die bevorzugten Bedeutungskombinationen für X-Y und $R^7$ sind:

a) wenn X - Y für eine Einfachbindung steht, ist
$R^7$ gegebenenfalls substituiertes $C_4$-$C_{12}$-Alkyl; gegebenenfalls substituiertes Phenyl oder $C_3$-$C_7$-Cycloalkyl; gegebenenfalls substituiertes Naphthyl oder bis zu zweifach durch $R^{10}$ und $R^{11}$ substituiertes Hetaryl oder $PO(OR^{10})(OR^{11})$;

b) wenn X - Y für O oder S(O)p (mit p = 0, 1, 2) steht, ist
$R^7$ Wasserstoff oder substituiertes Alkyl mit bis zu 12 C-Atomen; gegebenenfalls substituiertes Phenyl, $C_3$-$C_7$-Cycloalkyl, Naphthyl; $PO(OR^{10})(OR^{11})$; $COOR^{10}$ (mit $R^{10} \neq H$) gegebenenfalls substituiertes Pyridyl oder Tri-niederalkylsilyl;

c) wenn X - Y für $NR^{10}CO$ steht, ist
$R^7$ gegebenenfalls substituiertes Phenyl, Benzyl, Naphthyl oder Cycloalkyl; durch $R^{10}$ und $R^{11}$ bis zu zweifach substituiertes Hetaryl oder Hetaryloxy oder $NR^{10}R^{11}$;

d) wenn X - Y für $NR^{10}CSNR^{11}$ oder $NR^{10}CONR^{11}$ steht, ist
$R^7$ Wasserstoff, gegebenenfalls substituiertes Alkyl, Phenyl, Benzyl, Naphthyl oder $C_3$-$C_7$-Cycloalkyl oder $NR^{10}R^{11}$; bedeutet

e) wenn X - Y für N=N steht, ist
$R^7$ gegebenenfalls substituiertes Phenyl oder Naphthyl;

f) wenn X - Y für $CHR^{10}$-$NR^{11}$; -$CHR^{10}$-O oder -$CHR^{10}$S- steht, ist
$R^7$ Wasserstoff, gegebenenfalls substituiertes $C_1$ -$C_{12}$-Alkyl, Phenyl, Benzyl, $C_3$-$C_7$-Cycloalkyl oder Naphthyl; $PO(OR^{10})(OR^{11})$ oder $COOR^{10}$ (mit $R^{10} \neq H$);

g) wenn X - Y für $SO_2NR^{10}$ steht, ist
$R^7$ Wasserstoff; $NR^{10}R^{11}$; gegebenenfalls substituiertes $C_1$-$C_{12}$-Alkyl, Phenyl, Benzyl, Naphthyl oder $C_3$-$C_7$-Cycloalkyl;

h) wenn X - Y für N=CH steht, ist
$R^7$ gegebenenfalls substituiertes Phenyl, Benzyl oder Naphthyl;

i) wenn X - Y für $(CH_2)_n$ (mit n = 1, 2, 3, 4) steht, ist
$R^7$ Cyano; $COOR^{10}$; $PO(OR^{10})(OR^{11})$; $NR^{10}R^{11}$); gegebenenfalls substituiertes Phenyl, $C_3$-$C_7$-Cycloalkyl oder Naphthyl oder bis zu zweifach durch $R^{10}$ und $R^{11}$ substituiertes Hetaryl;

j) wenn X - Y für CH=CH steht, ist
$R^7$ gegebenenfalls substituiertes $C_1$-$C_{12}$-Alkyl, Phenyl, Benzyl oder Naphthyl;

k) wenn X - Y für $NR^{10}$ steht, ist

$R^7$ $PO(OR^{10})(OR^{11})$; $COOR^{11}$ (mit $R^{11} \neq H$) gegebenenfalls substituiertes Phenyl, Benzyl, Naphthyl oder $C_3$-$C_7$-Cycloalkyl;

l) wenn X - Y für $R^{10}NCHR^{11}$; O-$CHR^{10}$ oder S(O)p$CHR^{10}$ (mit p = 0, 1, 2) steht, ist

$R^7$ substituiertes $C_1$-$C_4$-Alkyl; $PO(OR^{10})(OR^{11})$ Tri-niederalkylsilyl; gegebenenfalls substituiertes $C_5$-$C_{12}$-Alkyl, Phenyl, Benzyl, $C_3$-$C_7$-Cycloalkyl, Naphthyl oder bis zu zweifach durch $R^{10}$ und $R^{11}$ substituiertes Hetaryl oder $(CH_2)$q $COOR^{10}$ (mit q = 0, 1, 2, 3);

m) wenn X - Y für $NR^{10}SO_2$ oder O-$SO_2$ steht, ist

$R^7$ gegebenenfalls substituiertes $C_1$-$C_{12}$-Alkyl, Phenyl, Benzyl, Naphthyl, $C_3$-$C_7$-Cycloalkyl; $NR^{10}R^{11}$; bis zu zweifach durch $R^{10}$ und $R^{11}$ substituiertes Hetaryl oder $(CH_2)$q-$COOR^{10}$ (mit q = 0, 1, 2, 3);

n) wenn X - Y für O-CO oder S-CO steht, ist

$R^7$ Wasserstoff; $NR^{10}R^{11}$; gegebenenfalls substituiertes Alkyl mit bis zu 12 C-Atomen, Phenyl, Benzyl, Cycloalkyl oder Naphthyl;

o) wenn X - Y für CO-O oder CO-S, oder $CONR^{10}$ steht, ist

$R^7$ substituiertes $C_1$-$C_4$-Alkyl; gegebenenfalls substituiertes $C_5$-$C_{12}$-Alkyl, Phenyl, Benzyl, Cycloalkyl oder Naphthyl.

In den vorstehenden Definitionen können die Reste und Gruppen jeweils gleich oder verschieden sein, d.h. wenn einer der zuvor genannten Substituenten in einem bestimmten Molekül mehrfach vorkommt, kann die jeweilige Bedeutung im Rahmen der Definitionsbreite frei gewählt werden.

Von den Resten in A ($R^2$, $R^3$ und $R^4$) bzw. von den Resten B ($R^5$ und $R^6$) bedeutet in der Regel nur einer Carboxy, $C_1$-$C_4$-Alkoxycarbonyl, $CONR^{10}R^{11}$, $NR^{10}R^{11}$, $NR^{10}$-$COR^{11}$, $C_3$-$C_7$-Cycloalkyl, Phenyl, Phenoxy oder Phenyl-S(O)p.

Mit Alkyl sind geradkettige oder verzweigte $C_1$-$C_{12}$-Alkylradikale oder aber als Niederalkyl die $C_1$-$C_4$-Alkylradikale gemeint und umfassen die Methyl-, Ethyl-, n-Propyl-, iso-Propyl-, n-Butyl-, sek-Butyl-, tert-Butyl-, iso-Butyl-, sowie die isomeren Pentyl-, Hexyl-, Heptyl-, Octyl-, Nonyl-, Decyl-, Undecyl- und Dodecylradikale.

Die vorstehende Definition gilt auch, wenn das Alkylradikal substituiert und/oder Bestandteil einer Alkoxyalkyl-, Alkoxycarbonyl-, Carbamoyl-, Alkoxy-, Alkylthio-, Alkylsulfinyl-, Alkylsulfonyl-, Monoalkylamino-, Aralkyl-, Alkylthiomethyl-, Dialkylaminogruppe ist oder das Alkylradikal als Substituent an ein aromatisches, heterocyclisches oder carbocyclisches System gebunden ist.

Unter substituiertem Alkyl sind Alkylradikale zu verstehen, die ein oder mehrfach mit Hydroxy, Alkoxy, Mercapto, Halogen, Alkylthio, Nitro, Cyano oder Amino, Mono- oder Di-niederalkylamino substituiert sind. Bevorzugt sind Halogen, Hydroxy, Alkoxy, Cyano; hervorzuheben ist die Trifluormethyl und die Trichlormethylgruppe, ferner -CCl = CHCl.

Halogene sind Fluor, Chlor, Brom und Jod, vorzugsweise Fluor, Chlor und Brom und in zweiter Linie Jod.

$C_3$-$C_7$-Cycloalkyl sind Cyclopropan, Cyclobutan, Cyclopentan, Cyclohexan und Cycloheptan. Bevorzugt ist Cyclopropan, Cyclopentan und Cyclohexan.

Die Cycloalkylgruppen sind vorzugsweise unsubstituiert oder bis zu dreifach durch Alkyl, Halogen (bevorzugt Fluor, Chlor), Hydroxy, Oxo oder Amino substituiert. Durch Sauerstoffatome unterbrochene Cycloalkylgruppen sind z.B.

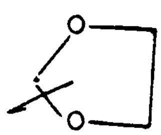 , 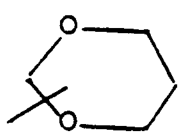 , 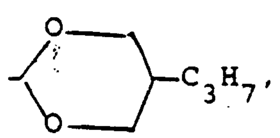 , 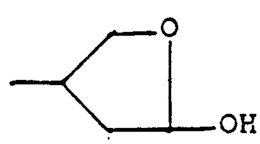

Substituiertes Phenyl oder substituiertes Naphthyl sind vorzugsweise bis zu dreifach durch Halogen, $C_1$-$C_4$-Alkyl, Alkylthio oder Alkoxy, Nitro, Cyano, Amino, Monoalkylamino, Dialkylamino, Halogenalkyl, Halogenalkylthio, Halogenalkoxy, Carboxy oder Alkoxycarbonyl substituierte Systeme.

Mit "Hetaryl"-Gruppen sind vor allem N-Heterocyclen gemeint, die über ein Ringstickstoffatom oder auch über ein C-Atom an das Verbindungsglied X - Y gebunden sind. Hetaryl umfaßt neben aromatischen auch ganz oder teilweise hydrierte Verbindungen, ferner kondensierte Systeme, die Benzolringe enthalten, z.B. Pyrazolyl-, Pyrazinyl-, Imidazolyl-, 1,2,4-Triazolyl-, Morpholinyl-, Piperidinyl-, Pyrrolyl-, Pyrrolidinyl-, 2,5-Dioxopyrrolidinyl-, 1,3-Isoindoldionyl- und Pyridinylradikale sowie von Indol, Benzofuran, Chinolin oder

Benzothiophen abgeleitete Reste.

Weitere Hetaryl-Gruppen sind beispielhaft durch die nachstehenden Formeln dargestellt:

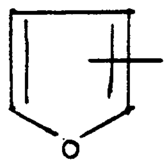 , 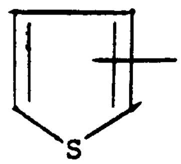 , 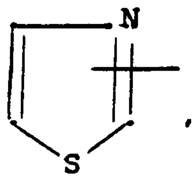 , 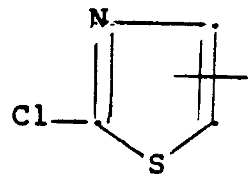

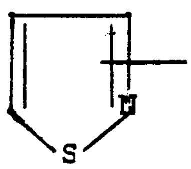 , 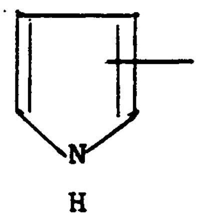 , 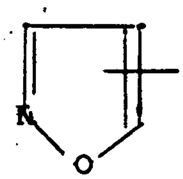 , 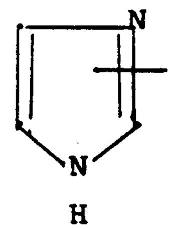

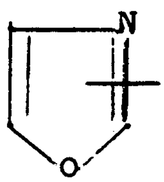 , 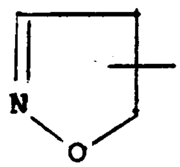 , 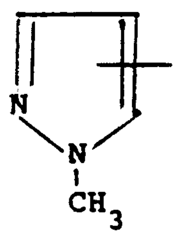 , 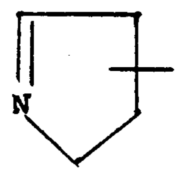

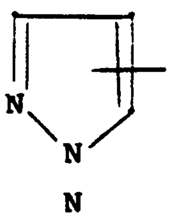 , 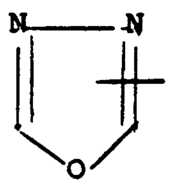 , 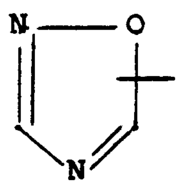 , 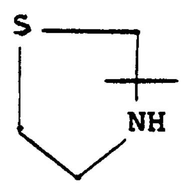

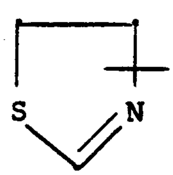 , 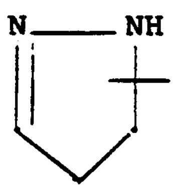 , 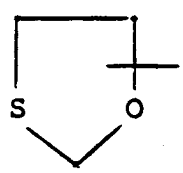 , 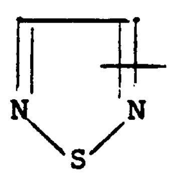

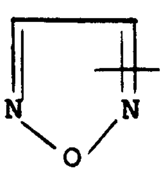 , 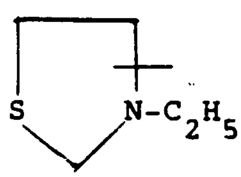 , 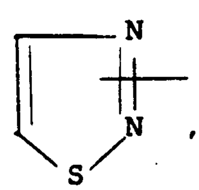 , 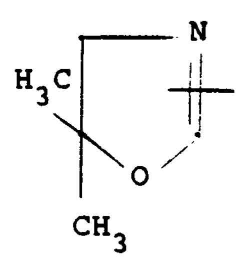

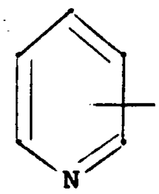 , 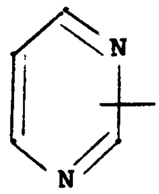 , 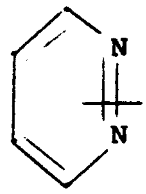 , 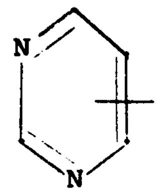

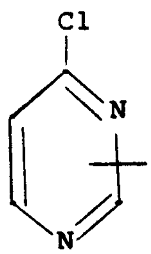 , 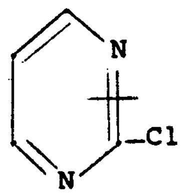 , 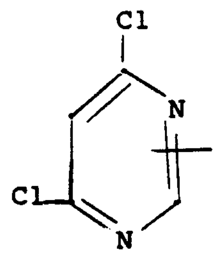 , 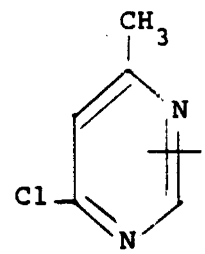

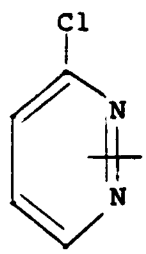 , 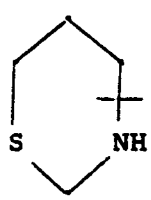 , 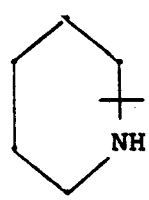 , 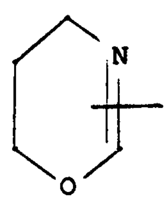

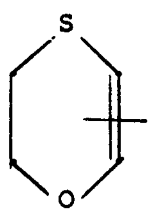 , 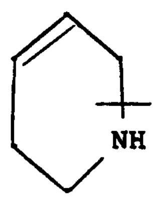 , 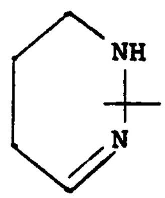 , 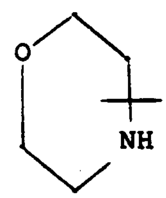

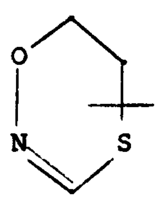 , 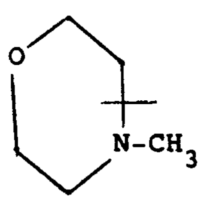 , 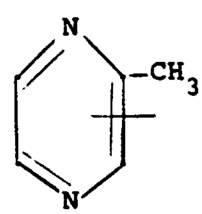 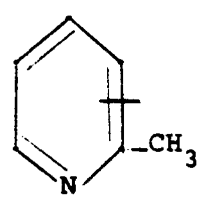

Die an die Phenylradikale A und B gebundenen Substituenten sind vorzugsweise (mit Ausnahme der an das Brückenglied X - Y gebundenen Substituenten) Halogen, Nitro, Amino, Methylthiomethyl, Methoxymethyl, Methylthio, Methoxy, Cyanomethyl, Ethoxy, Ethylthio, $C_1$-$C_4$-Alkyl, Acetamido, Methylamino und Dimethylamino.

Die Substituenten sind vorzugsweise in meta oder para-Stellung an die beiden Phenylringe A und B gebunden. Als bevorzugtes Substitutionsmuster für den Ring A seien neben dem unsubstituierten System genannt: 3,4-Dimethoxy, 3-Ethoxy-4-methoxy, 3-Chlor-4-methoxy, 3,5-Dichlor-4-amino. 3-Brom-4-methoxy, 3-Methyl-4-methoxy, 3-Ethyl-4-methoxy, 3-Propyl-4-methoxy, 3-Brom-4-dimethylamino, 3,4-Dimethyl, 3-Amino-4-methoxy, 3-Acetamido, 3-Acetamido-4-methoxy, 3-Acetamido-4-chlor, 3,5-Dimethyl-4-methoxy, 4-Methoxy, 4-Ethoxy, 3-Methoxy-4-methyl und 3-Brom-4-amino.

Vorzugsweise, A für 3,4-Dimethoxyphenyl, 3-Ethoxy-4-methoxyphenyl, 3-Chlor-4-methoxyphenyl, 3-Methyl-4-methoxyphenyl, 3-Brom-4-methoxyphenyl, 3-Ethyl-4-methoxyphenyl, 3,4-Dimethylphenyl, 3,5-Dichlor-4-aminophenyl steht:
Vorzugsweise, $R^1$ für Wasserstoff bedeutet.

Der Rest Q leitet sich bevorzugt von folgenden Aminen ab: Dimethylamin, Diethylamin, Methylethylamin, Methylpropylamin, Methylbutylamin, Morpholin, 2-Methyl-morpholin, 2,6-Di-methylmorpholin, N-(2-Hydroxyethyl)-N-Methylamin, N-(2-Hydroxyethyl)-N-ethylamin. Vorzugsweise, Q für $NR^8R^9$, worin $R^8$ und $R^9$ $C_1$-$C_4$-Alkyl ist, oder für die Morpholinogruppe steht.

Vorzugsweise, B für

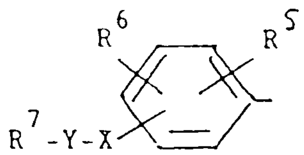

steht, worin -X-Y-eine Einfachbindung, Sauerstoff, Schwefel, $-CH_2O-$, $-OCH_2-$ oder -N=N- bedeutet. Vorzugsweise, $R^5$ und $R^6$ Wasserstoff bedeuten, -X-Y- eine Einfachbindung, Sauerstoff, Schwefel oder -N=N- darstellt und $R^7$ für ein gegebenenfalls substituiertes Phenyl, gegebenenfalls substituiertes $C_1$-$C_{12}$-Alkyl, gegebenenfalls durch Sauerstoff und/oder Schwefel und/oder $NR^{10}$ ein- oder mehrfach unterbrochenes gegebenenfalls hydroxy- oder $C_1$-$C_4$-alkylsubstituiertes $C_3$-$C_7$-Cycloalkyl oder bis zu zweifach durch $R^{10}$ und $R^{11}$ substituiertes Hetaryl steht.

Bevorzugten Verbindungen sind:-
3-[4-(4-Chlorphenoxy)-phenyl]-3-(3,4-dimethoxyphenyl)-acrylsäuremorpholid,
3-(4-n-Butylphenyl)-phenyl)-3-(3,4-dimethoxyphenyl)-acrylsäuremorpholid,
3-[4-(4-Chlorophenyl)-phenyl]-3-(3,4-dimethoxyphenyl)-acrylsäure-methyl-ethylamid.

Sind A und B in der Formel I verschieden, so können die Verbindungen der Formel I als cis-/trans-Isomere vorliegen. Die Formel I umfaßt in diesem Fall sowohl die einzelnen Isomeren als auch Gemische der cis- und der trans-Verbindung.

Des weiteren können die Reste A bzw. B in ihrer freien Drehbarkeit um die Achse der Einfachbindung aufgrund sterischer oder sonstiger Sekundärwechselwirkungen beeinträchtigt sein; derartige Effekte können Atropisomerie hervorrufen. Die Erfindung umfaßt somit auch die atropisomeren Strukturen von (I).

Einige typische Verbindungen gemäß der Erfindung sind in den nachstehenden Tabellen aufgeführt.

## TABELLE A

Verbindungen der Formel

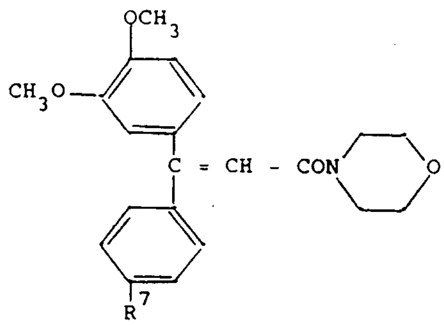

(Formel I, XY gleich Einfachbindung)

| Nr. | $R^7$ |
|---|---|
| 1 | $-n-C_5H_{11}$ |
| 2 | $-n-C_6H_{13}$ |
| 3 | 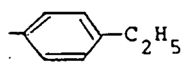 |
| 4 | 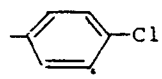 |
| 5 | 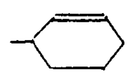 |
| 6 | 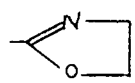 |
| 7 | 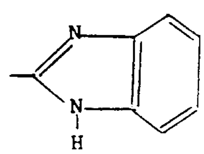 |

## TABELLE B

Verbindungen der Formel

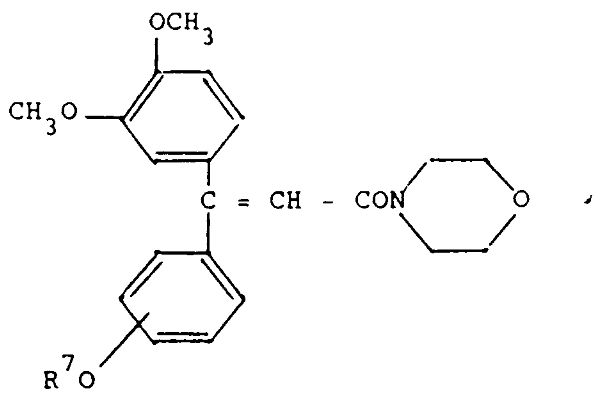

(Formel I, XY gleich -O-)

| Nr. | $R_7$ | Position von $OR^7$ |
|---|---|---|
| 1 | $-n-C_5H_{11}$ | 4 |
| 2 | -CCl=CHCl | 4 |
| 3 | 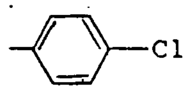 | 4 |
| 4 | 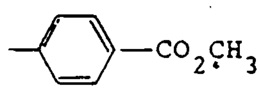 | 4 |
| 5 | 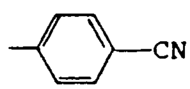 | 4 |
| 6 | 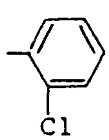 | 4 |
| 7 | 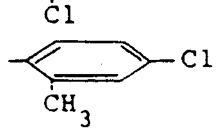 | 4 |

| Nr. | $R_7$ | Position von $OR^7$ |
|---|---|---|
| 8 | 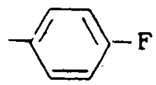 | 4 |
| 9 | $-C_6H_5$ | 3 |
| 10 | 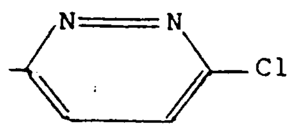 | 4 |
| 11 | 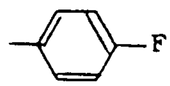 | 3 |

## TABELLE C

Verbindungen der Formel

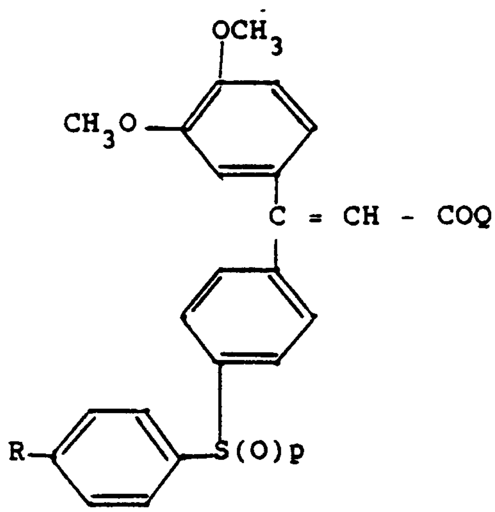

| Nr. | R | p | Q |
|---|---|---|---|
| 1 | Cl | 0 | 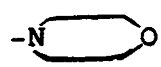 |
| 2 | Br | 0 | 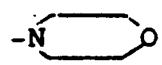 |
| 3 | Cl | 0 | 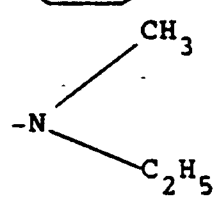 |
| 4 | Cl | 1 | " |
| 5 | Cl | 2 | " |
| 6 | Cl | 1 | 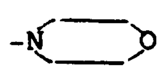 |
| 7 | Cl | 2 | 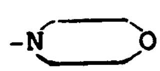 |

## TABELLE D

Verbindungen der Formel

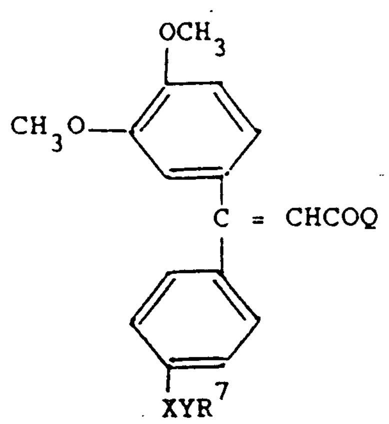

| Nr. | $R^7$-YX- | Q |
|---|---|---|
| 1 | $C_6H_5$-N=N- | 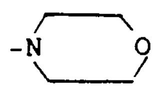 |
| 2 | $C_6H_5$-$CH_2$O- | " |
| 3 | $C_6H_5$-$OCH_2$- | " |
| 4 | $C_6H_5$-N=N- | 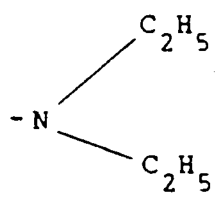 |
| 5 | $C_6H_5$-$CH_2$O- | 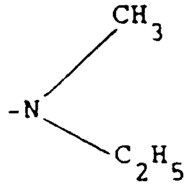 |

| Nr. | $R^7$-YX- | Q |
|---|---|---|
| 6 | 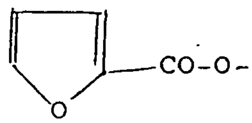 | 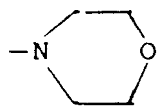 |
| 7 | 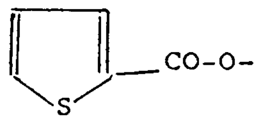 | |
| 8 | 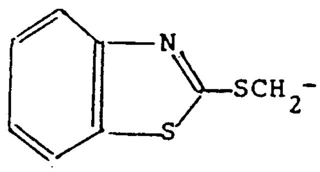 | |
| 9 | 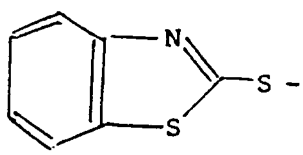 | 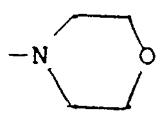 |

Allgemein zeigt sich, daß die Verbindungen der Formel

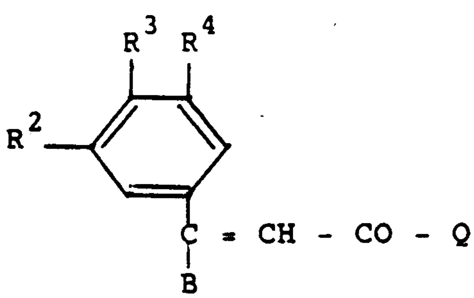

,

insbesondere mit Q gleich Morpholino oder Methyl-ethylamino und $R^2$, $R^3$ gleich Methoxy, $R^4$ gleich Wasserstoff, hinsichtlich des Restes B sehr stark variiert werden können, ohne daß die fungizide Wirkung sich verliert. Ähnliches gilt insbesondere von den Substituentenkombinationen $R^2/R^3/R^4$ gleich $C_2H_5O/CH_3O/H$; $Cl/CH_3O/H$; $Br/CH_3O/H$; $CH_3/CH_3O/H$; $Cl/NH_2/Cl$; $CH_3O/Cl/H$.

Man erhält die neuen Verbindungen nach an sich bekannten Verfahren:

1. Umsetzung einer Acrylsäure der Formel

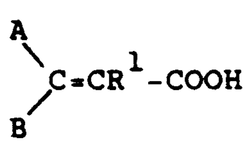 (II)

worin A, B und $R^1$ wie zuvor definiert sind oder durch Umsetzung eines gegebenenfalls in situ hergestellten reaktionsfähigen Derivates von (II) mit einer Verbindung der Formel

HQ (III),

in der Q wie zuvor definiert ist.

Das Verfahren stellt somit die Acylierung einer Verbindung der Formel III mit einer Carbonsäure der Formel II dar, wobei die Umsetzung vorteilhaft in Gegenwart eines die Säure II aktivierenden oder eines wasserentziehenden Mittels oder aber mit reaktiven Derivaten der Carbonsäure II oder des Edukts III gearbeitet wird.

Als gegebenenfalls im Reaktionsgemisch hergestellte reaktive Derivate einer Carbonsäure der Formel II kommen bespielsweise ihre Alkyl-, Aryl-, Aralkylester oder -thioester wie der Methyl-, Ethyl-, Phenyl-, oder Benzylester, ihre Imidazolide, ihre Säurehalogenide wie das Säurechlorid oder -bromid, ihre Anhydride, ihre gemischten Anhydride mit aliphatischen oder aromatischen Carbon-, Sulfen-, Sulfin-, Sulfonsäuren oder mit Kohlensäureestern, z.B. mit der Essigsäure, der Propinsäure, der p-Toluolsulfonsäure oder der O-Ethylkohlensäure, oder ihre N-Hydroxyimidester in Betracht. Als gegebenenfalls im Reaktionsgemisch hergestellte reaktive Derivate eines Amins der Formel III eignen sich z.B. ihre "Phosphorazoderivate".

Als säureaktivierende und/oder wasserentziehende Mittel kommen beispielsweise Chlorameisensäureester wie Chlorameisensäureäthylester, Phosphorpentoxid, N,N-Dicyclohexylcarbodiimid, N,N'-Carbonyldiimidazol oder N,N'-Thionyldiimidazol in Betracht.

Die Umsetzung wird zweckmäßigerweise in einem Lösungsmittel oder Lösungsmittelgemisch wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart einer anorganischen Base wie Natriumcarbonat oder einer tertären organischen Base wie Triethylamin oder Pyridin, welche gleichzeitig als Lösungsmittel dienen kann, und gegebenenfalls in Gegenwart eines säureaktivierenden Mittels bei Temperaturen zwischen -25 °C und 150 °C, vorzugsweise jedoch bei Temperaturen zwischen -10 °C und der Siedetemperatur des Reaktionsgemisches, durchgeführt. Hierbei braucht ein gegebenenfalls im Reaktionsgemisch entstandenes reaktionsfähiges Derivat einer Verbindung der allgemeinen Formeln II oder III nicht isoliert zu werden, ferner kann die Umsetzung auch in einem Überschuß der eingesetzten Verbindung der allgemeinen Formel III als Lösungsmittel durchgeführt werden.

Erfindungsgemäß erhaltene cis-/trans-Isomerengemische können gewünschtenfalls anschließend nach üblichen Methoden in die entsprechenden cis- und trans-Isomeren aufgetrennt werden. Das gleiche gilt für eventuelle Atropisomere.

2. Verbindungen der Formel (I) sind auch darstellbar durch Umsetzung eines Ketons der Formel (IV) mit einem Phosphonoessigsäurederivat der Formel (XIV), in der R' vorzugsweise für eine $C_1$-$C_4$-Alkylgruppe steht, nach dem Verfahren von Wittig und Horner

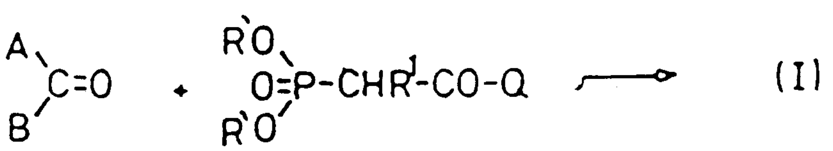

(IV) (V)

Die Isomerentrennung erfolgt vorzugsweise durch fraktionierte Kirstallisation, beispielsweise auf Methanol, Ethanol, Isopropanol, Methanol/Wasser oder Ethanol/Petrolether.

Verbindungen der Formel I mit basischen Gruppen können gewünschtenfalls in Säureadditionssalze übergeführt werden, vorzugsweise in Salze von Mineralsäuren wie Salzsäure, Bromwasserstoffsäure, Schwefelsäure, Phosphorsäure.

Die Acrylsäurederivate der Formel II sind neu. Sie können nach bekannten Verfahren hergestellt werden. Ausgangsstoffe der Formel II, in denen die Reste A und B der Definition von Anspruch 1 entsprechen, können ausgehend vom Keton der Formel IV nach zahlreichen literaturbekannten Verfahren hergestellt werden.

Herstellung von Verbindungen der Formel II:

(A) Durch Umsetzung von IV mit α-Halogencarbonsäureester VI und anschließende Verseifung:

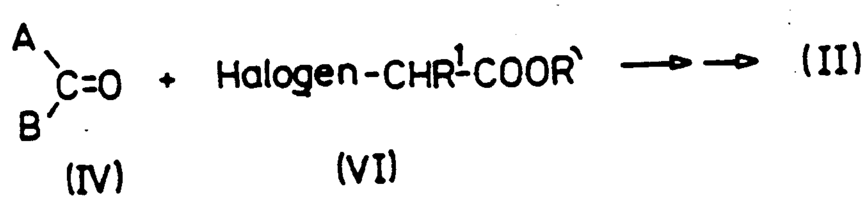

(B) Durch Umsetzung von IV mit CH-aciden Komponenten nach Knoevenagel, hier erläutert anhand der Umsetzung von IV mit einem Nitril VII mit anschließender Verseifung des Acrylnitrils VIII zur Carbonsäure II:

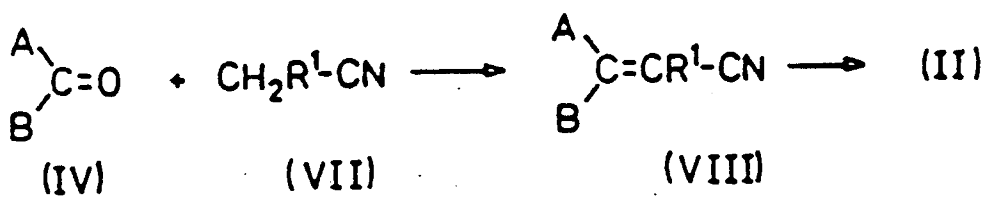

(C) Acrylsäuren der Formel II können auch nach Wittig-Horner ausgehend von Keton IV durch Umsetzung mit einer Phosphonessigsäureverbindung der Formel IX und anschließende Verseifung des Esters X hergestellt werden:

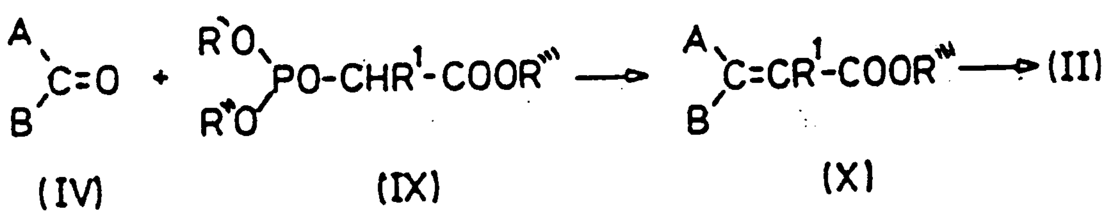

(R', R'' und R''' sind gleiche oder verschiedene niedere Alkylreste)
3. Aufbau bzw. Einführung des Restes -XY-$R^7$ in Verbindungen der Formel

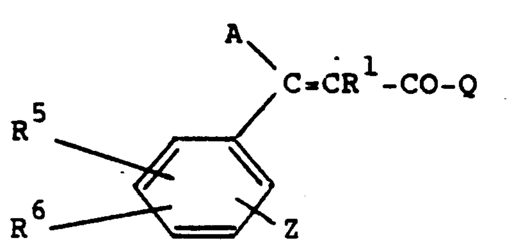

(XI),

worin A, Q, $R^1$, $R^5$ und $R^6$ die obige Bedeutung haben und Z für ein Wasserstoffatom oder einen gegen -XY-$R^7$ (worin X, Y und $R^7$ die obige Bedeutung haben) austauschbaren oder in -XY-$R^7$ umwandelbaren Substituenten steht.

Die Reaktionen zum Aufbau oder zur Einführung von -XY-$R^7$ können sehr unterschiedlicher Art sein. Zu nennen sind insbesondere Substitutionsreaktionen, Additionsreaktionen, Veresterungs- und Amidierungsreaktionen, Oxidations- und Reduktionsreaktionen.

(3a) Substituenten der Formel X-Y-$R^7$, welche über ein Stickstoffatom an dem Phenylrest B gebunden sind, (wie etwa -$NR^{10}$-CO etc) sind ausgehend von Aminoverbindungen nach allgemein bekannten Verfahren herstellbar. Die Aminoverbindungen sind ihrerseits etwa durch Reduktion der entsprechenden aromatischen Nitroverbindungen gut zugänglich.

(3b) Phenolische OH-Gruppen am Molekülteil B können nach üblichen Verfahren abgewandelt werden, um Verbindungen mit -XY- gleich -O- herzustellen oder solche Verbindungen, in denen die Gruppe -XY-$R^7$ über ein Sauerstoffatom an den Benzolring gebunden ist.

(3c) Substituenten der Formel -X-Y-$R^7$, in denen -XY-gleich S(O)p ist, können z.B. aus den entsprechenden Mercaptoverbindungen (XI, Z gleich -SH) durch Substitution nach üblichen Methoden (p = O) und gegebenenfalls Oxidation zu den Sulfoxiden (p = 1) oder Sulfonen (p = 2) erhalten werden.

(3d) Wenn das Brückenglied -XY- eine an den Benzolring gebundene $CH_2$-Gruppe aufweist, kann eine entsprechende Verbindung XI mit Z gleich -$CH_2$Hal (Hal = Cl, Br, J) durch nucleophile Substitutionsreaktionen, z.B. mit Phenolat oder Thiophenolat, oder mit anderen Reaktionspartnern, die unter Halogenwasserstoffabspaltung mit der $CH_2$-Gruppe verknüpft werden können, substituiert werden.

(3e) Wenn -XY-$R^7$ einen über -CO- an den Benzolring gebundenen Rest darstellt, können entsprechende Verbindungen XI mit Z = COOH funktionalisiert werden.

(3f) In Verbindungen XI, in denen Z eine leaving group, z.B. ein reaktionsfähiges Halogen darstellt, kann das Halogenatom durch Umsetzung mit entsprechenden nucleophilen Verbindungen H-XY-$R^7$ unter Halogenwasserstoffabspaltung funktionalisiert werden.

(3g) Eine wichtige Methode zur Funktionalisierung von Verbindungen der Formel XI, in denen Z für Jod oder Brom, $R^1$ für Wasserstoff steht und die Verbindung XI keine weiteren Brom- oder Jodatome enthält, besteht in der Umsetzung der entsprechenden Verbindungen XI mit einem entsprechenden Alken nach der Methode von Heck (Einzelheiten sind bei Verfahren 4 beschrieben). Dabei wird unter Halogenwasserstoffabspaltung eine Verknüpfung zwischen dem Benzolring und dem Alken geschaffen, gegebenenfalls unter Verschiebung der Doppelbindung, etwa bei der Umsetzung mit Cyclohexen. Durch Verwendung von carboxy- oder alkoxycarbonylsubstituierten Alkenen, z.B. Acrylsäure oder Zimtsäure oder ihren Niederalkylestern, gegebenenfalls Hydrolyse und Decarboxylierung, kann die Verknüpfungsposition gesteuert werden, Schema:

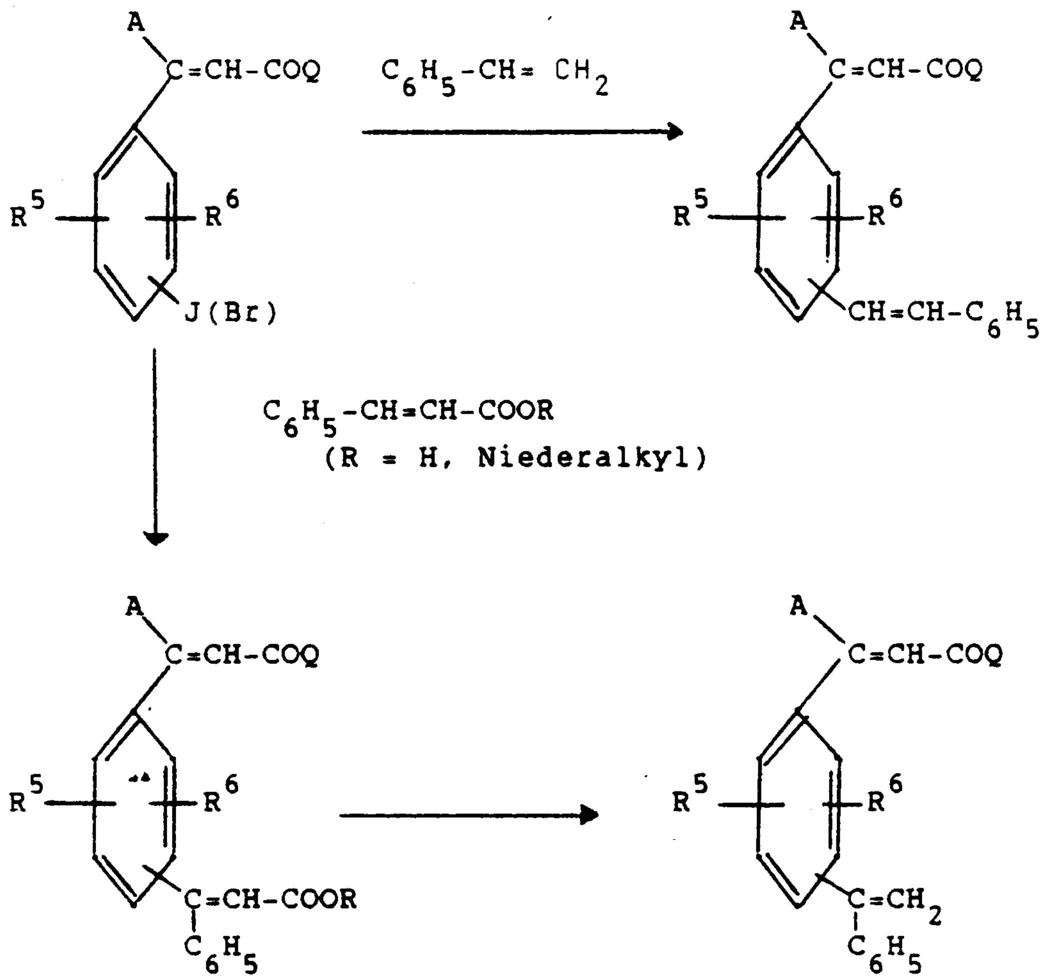


Die vorstehenden Reaktionswege zur Herstellung von Acrylsäureamiden der Formel I sind in nachstehendem Syntheseschema zusammengefaßt.

Viele Ausgangsstoffe für die erfindungsgemäßen Verbindungen sind bekannt. Anderenfalls können sie nach bekannten Methoden z.T. auch nach den vorstehend beschriebenen Methoden erhalten werden.

So können die Umsetzungen, die bei Verfahren 3 für die Verbindungen XI angegeben wurden, auch bereits auf Vorstufen angewendet werden, z.B. auf die Benzophenone

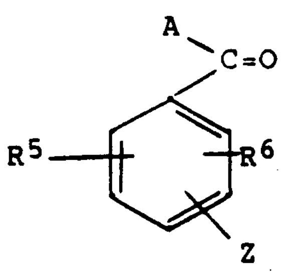

(XII),

worin A, $R^5$, $R^6$ und Z die obige Bedeutung haben, oder auf Acrylsäuren oder Acrylsäureester der Formel

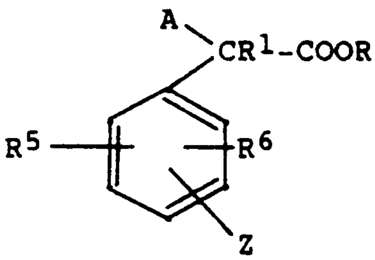

(XIII),

worin A, $R^1$, $R^5$, $R^6$ und Z die obige Bedeutung haben und R = H oder Niederalkyl ist. Verbindungen XII und XIII mit Z gleich OH können u.a. durch Etherspaltung nach üblichen Methoden aus entsprechenden Verbindungen mit Z gleich Alkoxy, z.B. $CH_3O$, erhalten werden.

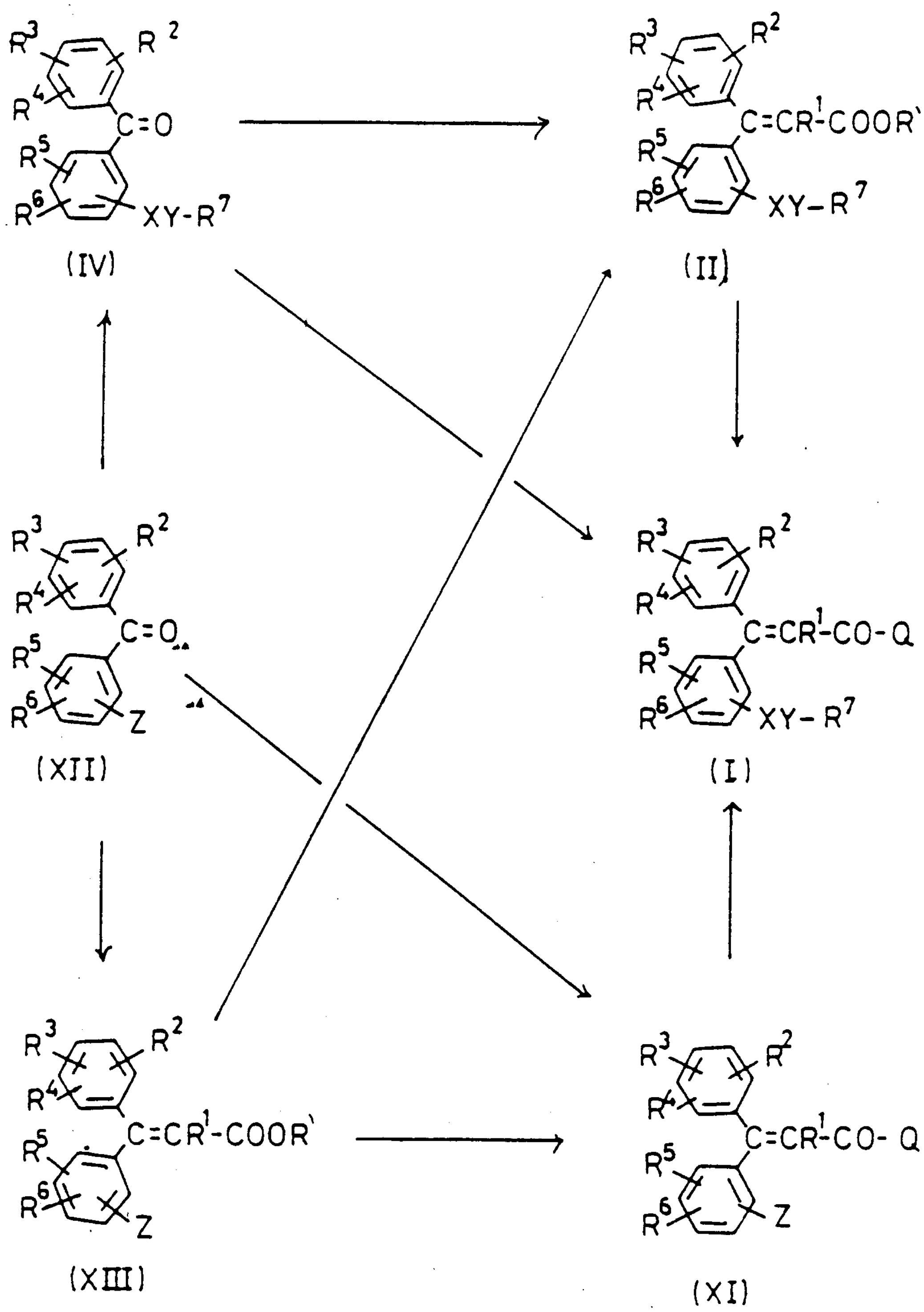

(IV) (II) (XII) (I) (XIII) (XI)

Schema 1: Darstellung der Reaktionswege zur Synthese von I

4. Zur Herstellung von Verbindungen der Formel

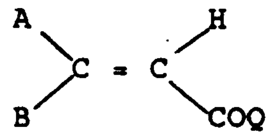

(Ia)

oder

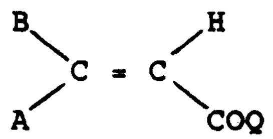

(Ib)

setzt man eine Verbindung

B - CH = CH - COQ (XIVa)

oder

A - CH = CH - COQ (XIVb)

in Gegenwart von Palladium-Katalysatoren mit entsprechenden Verbindungen der Formel

A - Hal bzw. B - Hal

(XVa) (XVb)

um, in der Hal für Chlor, Brom oder Jod steht und A und B die oben angegebene Bedeutung haben.

Die Reaktions - eine Palladium (O) - katalysierte Vinylilierung von Halogenbenzolen ist als Heck-Reaktion bekannt geworden (R.F. Heck, Organic Reactions, Vol 27, 345 H).

Als Halogenbenzole besonders geeignet sind entsprechend substituierte Jodbenzole und Brombenzole der Formel XVa/XVb.

Die Reaktion kann mit und ohne Lösungsmittel durchgeführt werden. Geeignet sind polare und unpolare Lösungsmittel, z.B. Nitrile, wie Acetonitril, Propionitril; Alkohole, wie Methanol, Ethanol, Propanol, Isopropanol; Ketone, wie Aceton, Methylehtylketon; Ether, wie Diethylether, Tetrahydrofuran, Diisopropylether, Glyme, Diglyme etc.; Amide, wie Dimethylformamid oder N-Methylpyrrolidon oder Hexamethylphosphorsäuretriamid; Aromaten, wie Benzol, Toluol, Xylol, Chlorbenzol.

Die Anwesenheit geringer Mengen an Wasser ist im allgemeinen nicht störend.

Die Reaktion kann bei Temperaturen zwischen Raumtemperatur und der Siedetemperatur des Lösungsmittels durchgeführt werden. In geschlossenen Apparaturen kann auch bei noch höheren Temperaturen unter Druck gearbeitet werden. Bevorzugt ist der Temperaturbereich von 30 bis 160 °C zu nennen.

Bei einer Reaktionstemperatur von etwa 100 °C ist eine Menge von 1 Mol-% des Palladium-Katalysators (bezogen auf die Menge an Halogenbenzol XV) ausreichend. Höhere Katalysatormengen können die Reaktionsgeschwindigkeit verbessern oder erlauben eine Verminderung der Reaktionstemperatur.

Im Reaktionsverlauf wird Halogenwasserstoffsäure freigesetzt. zu deren Neutralisation Basen zugesetzt werden. Sofern als Edukte Carbonsäuren eingesetzt werden, ist es vorteilhaft, eine der Carboxyfunktion äquivalente Menge an Base zusätzlich einzusetzen.

Als Basen sind zu nennen:
anorganische Basen, wie Soda, Natriumacetat, Natriumbicarbonat etc.
Amine, wie Triethylamin DABCO u.a. Heterocyclen, wie Pyridin, Chinolin etc.

Als Palladium-Katalysator können sowohl die laborüblichen Pd-C-Katalysatoren oder aber Palladium (II) -salze, wie Palladiumacetat oder Palladiumhalogenide verwendet werden. Die Salze werden unter den Reaktionsbedingungen zu Palladium-(O) reduziert. Oft ist es von Vorteil - insbesondere bei Verwendung von Palladium-II-salzen - komplexierende Mittel, wie Triarylphosphine, zuzusetzen.

Es hat sich gezeigt, daß die neu eintretende Gruppe A bzw. B weit überwiegend in trans-Stellung zu der Gruppe COQ eintritt. Daher kann durch entsprechende Wahl der Ausgangsprodukte das gewünschte Isomere hergestellt werden.

Die Benennung der Isomeren wird nach der E/Z-Nomenklatur vorgenommen, wobei die vier Substituenten dem Cahn-Ingold-Prelog-System gemäß in der Reihenfolge sinkender Priorität angeordnet werden (Angewandte Chemie 78 (1966) 413; J. Chem. Soc. 1951, 612; Experimenta 12 (1956), 81; Bayer/Walter, Lehrbuch der organischen Chemie, 19. Aufl. S. Hirzel Verlag Stuttgart, S. 69; Pure-Appl. Chem. 45, 11-30 (1976); J. Org. Chem. 1970, 2849).

Des weiteren wurde gefunden, daß die E/Z-Isomeren durch Lichteinwirkung durch Lewissäure-Katalyse, thermisch oder durch Basenkatalyse bei erhöhter Temperatur in einem Gleichgewicht stehen.

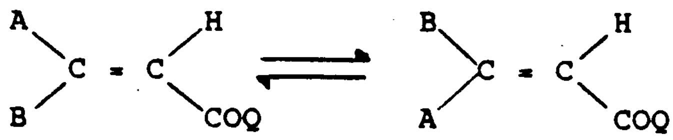

(Ia) (Ib).

Die Verbindungen der Formel Ia sind daher als wertvolle Vorstufen für die eigentlich fungizid wirksame Form Ib anzusehen. Insbesondere unter Freilandbedingungen können die per se unwirksamen Verbindungen Ia über das Gleichgewicht Ia / Ib die wirksame Form Ib zur Verfügung stellen.

Die als Ausgangsstoffe benötigten Verbindungen XIV können nach dem erfindungsgemäßen Verfahren B aus den Aldehyden

B-CHO bzw. A-CHO

(XVIa) (XVIb)

mit geeigneten Phosphonessigsäurederivaten nach Wittig-Horner erhalten werden.

Ferner können die Verbindungen XIV durch Heck-Reaktion mit Pd(O)-Katalyse aus entsprechenden Acrylsäurederivaten der Formel

$CH_2$ = CH - COQ (XVI)

und einer Verbindung XVb oder XVa erhalten werden.

Die Umsetzung nach dem obigen Verfahren 4 kann auch mit der vorstehenden Herstellung des Ausgangsstoffes kombiniert, d.h. im Sinne einer "Eintopfreaktion" durchgeführt werden. Dazu wird zunächst ein Halogenbenzol der Formel XVa bzw. XVb mit einer Acrylsäure der Formel XVI unter den Bedingungen der Heck-Reaktion zu einem Zimtsäurederivat der Formel XIVa bzw. XIVb umgesetzt, wobei das Halogenbenzol vollständig verbraucht wird. Danach wird das Halogenbenzol XVb bzw. XVa zugesetzt, welches in der zweiten Stufe mit XIVa bzw. XIV b zum Produkt Ib bzw. Ia reagiert. Die bei dem "Eintopfverfahren" eingesetzten Äquivalente an Base sind so bemessen, daß sie die gesamte bei der Reaktion freiwerdenden Halogenstoffsäure zu binden vermögen.

Die erfindungsgemäßen Verbindungen zeigen eine starke Wirkung besonders gegen phytopathogene Pilze, vor allem gegen echten Mehltau, falschen Mehltau (etwa Plasmopara und Phytophthora), Schorf,

Grauschimmel, Rostpilze. Wegen ihrer nur sehr geringen Phytotoxizität können die neuen Verbindungen in praktisch allen Nutz- und Zierpflanzenkulturen eingesetzt werden, beispielsweise in Getreide, etwa Mais, Weizen, Roggen, Hafer, in Reis, in Tomaten, Gurken, Bohnen, Kartoffeln, Rüben, im Wein- und Obstbau, in Rosen, Nelken und Chrysanthemen.

Die neuen Verbindungen zeigen Blattwirkung und systemische wirkung. So wird mit zahlreichen erfindungsgemäßen Verbindungen bei der Blattbehandlung gegen Plasmopora mit einer Wirkstoffkonzentration zwischen 20 und 100 ppm eine vollständige Abtötung der Pilze erreicht.

Bei der Bekämpfung von Phytophthora genügen im allgemeinen Wirkstoffkonzentrationen von 100 ppm, zum Teil weniger, für eine ausreichende Wirkung.

In manchen Fällen ist es günstig, die erfindungsgemäßen Verbindungen mit bekannten fungiziden Wirkstoffen zu kombinieren. Dabei geht die Wirkung der Kombinationen z.T. deutlich über die rein additive Wirkung hinaus.

Kombinationspartner

Manganethylenbisdithiocarbamat (Maneb)
Mangan-Zinkethylenbisdithiocarbamat (Mancozeb)
Zinkethylenbisdithiocarbamat (Zineb)
N-Trichlormethylhtio-tetrahydrophthalimid (Captan)
N-Trichclormethylthiophthalimid (Folpet)
N-(1,1,2,2-Tetrachlorethylthio)tetrahydrophthalimid (Captafol)
2,3-Dicyano-1,4-dithiaanthrachinon (Dithianon)
Zink-(N,N'-propylen-bisdithiocarbamat (Propineb)
Kupferoxychlorid
Natrium-4-dimethylaminobenzoldiazoldiazosulfonat (Fenaminosulf)
Triphenylzinnacetat (Fentinacetat)
Triphenylzinnhydroxid (Fentinhydroxyd)
Eisendimethyldithiocarbamat (Ferbam)
N-(2-Furoyl)-N-(2,6-xylyl)-DL-alanin (Furalaxyl)
3-(Dimethylamino)propylcarbamat (Propamocarb)
N-Ethyl-N-(3-dimethylamino)thiocarbamat (Prothiocarb)
Tetramethylthiuramidsulfid (Thiram)
N-Dichlorfluormethylthio-N,N'-dimethyl-N-p-tolylsulfamid (Tolylfluamid)
N-(2-Methoxyacetyl)-N-(2,6-xylyl)alanin (Metalaxyl)
Zinkdimethylthiocarbamat (Ziram)
N-Dichlorfluormethylthio-N,N'-dimethyl-N-phenylsulfamid (Dichlorfluanid)
3-Trichlormethyl-5-ethoxy-1,2,4-thiadizol (Etridazol)
Tri[aminzink-ethylenbis(dithiocarbamat)]tetrahydro-1,2,4,7-dithiadiazicin-3,8-dithion polymer (Metiram)
Aluminotris-(0-ethylphosphat) (Phosethyl)
2-Cyano-N-(ethylcarbamoyl)-2-methyloximino)-acetamid (Cymocanil)
N-(3-Chlorphenyl)-N-(tetrahydrofuran-2-on-3-yl)-cyclopropancarbonamit (Cyprofuran)
Tetrachlor-isophthalodinitril (Chlorothalonil)
6-Methyl-2-oxo-1,3-dithio[4,5-b]-chinoxalin (Chinomethionat)
4-Cyclododecyl-2,6-dimethylmorpholin (Dodemorph)
1-Dodecylguanidiniumacetat (Dodin)
Diisopropyl-5-nitroisophthalat (Nitrothal-isopropyl)
2,4-Dichlor-$\alpha$-(pyrimidin-5-yl)benhydrylalkohol (Fenarimol)
1-($\beta$-Allyloxy-2,4-dichlorphenethyl)imidazol (Imazalil)
3-(3,5-Dichlorphenyl)-N-isopropyl)-2,4-dioxoimidazolidin-1-carboxamid(Iprodion)
Schwefel
2,3-Dihydro-6-methyl-5-phenylcarbamoyl-1,4-oxythiin-4,4-dioxid (Oxycarboxin)
N-(3,5-Dichlorphenyl)-1,2-dimethylcyclopropan-1,2-dicarboximid (Procymidon)
6-Ethoxycarbonyl-5-methylpyrazolo[1,5-]pyrimidin-2-yl-0,0-diethylphosphorothioat (Pyrazophos)
2-(Thiazol-4-yl-benzimidazol (Thiabendazol)
1-(4-Chlorphenoxy-3,3-dimethyl-1-(1,2,4-triazol-1-yl-2-butanon (Triadimefon)
1-(4-Chlorphenoxy)3,3-dimethyl-1-(1,2,4-triazol-1-yl-butanol (Triadimenol)
3-(3,5-dichlorphenyl)-5-methyl-5-vinyloxyzolidin-2,4-dion (Vinclozolin)
Methylbenzimidazol-2-ylcarbamat (Carbendazin)
2,4,5-Trimethyl-N-phenyl-3-furancarboxamid (Methfuroxam)
$\beta$-[1,1-Biphenyl]-4-yl-oxy-$\alpha$-(1,1-dimethylethyl)-1H-1,2,4-triazol-1-ethanol (Bitertanol)

2-(2-Furyl)benzimidazol (Fuberidazol)
5-Butyl-2-ethylamino-6-methylpyrimidin-4-ol (Ethirimol)
2-Methyl-3-furanilid (Fenfuram)
Bis-(8-guanidino-octyl)amin (Guazatin)
N-Cyclohexyl-N-methoxy-2,5-dimethylfuran-3-carbonsäureamid (Furmecyclox)
2-Chlor-4'-fluor-α-(pyrimidin-5-yl)benzhydrylalkohol (Nuarimol)
Phosphorige Säure und deren Salze
Methyl-1-(butylcarbamoyl)benzimidazolcarbamat (Benomyl)
0,0-Diethylphthalimidaophosphonathioat (Dithalin)
7-Brom-5-chlorchinolin-8-acrylat (Halacrimat)
1-[2-(2,4-Dichlorphenyl)4-propyl-1,3-dioxolan-2-yl-methyl]1H-1,2,4-triazol (Propiconazol)
Dimethyl-4,4'-α-(o-phenylen) bis (3-thioallophanat) (Thiophanatmethyl)
1,4-Bis(2,2,2-trichlor-1-flormamidoethyl)piperazin (Triforine)
2,2-Dimethyl-4-tridecylmorpholin (Tridemorph)
4-[3-]4-(1,1-Dimethyl-ethyl)phenyl[2-methyl]-propyl-2,6-(cis-dimethylmorpholin (Fenpropemorph)
1-[2-(2,4-Dichlorphenyl)-4-ethyl-1,3-dioxolan-2-yl-methyl]1H-1,2,4-trizol (Etaconazol)
1-[1-(2,4-Chlorphenyl)-4,4-dimethyl-3-hydroxy-2-pentyl]1,2,4-triazol (Diclobutrazol)
2,4-Dichlor-6-(2-chloranilino-1,3,5-triazin (Anilazin)
2-Jodo-N-phenylbenzamid (Benodanil)
2-sec.-butyl-4,6-dinitrophenyl-3-methylcrotonate (Binapacryl)
5-Butyl-2-(ethylamino)-6-methyl-4-pyrimidinyl-dimethyl-sulfonat (Buprimat)
2,4-Dinitro-6-octylphenylcrotinat (Dinocap)
5,6-Dihydro-2-methyl-1,4-oxathiin-3-carbanilid (Carboxin)
N-Propyl-N-[(2,4,6-trichlorphenoxy)-2-ethyl]-imidazol-1-carbonamid (Prochloraz)

Für die Anwendung im Pflanzenschutz werden die neuen Verbindungen in üblicher Weise mit Hilfs- und/oder Trägerstoffen zu gebräuchlichen Formen von Schädlingsbekämpfungsmitteln verarbeitet, z.B. zu Lösungen, Emulsions- bzw. Lösungskonzentraten, Suspensionspulvern, Stäuben. Soweit Kombinationen mit anderen Wirkstoffen zur Anwendung gelangen sollen, kann dies in Form gemeinsamer Formulierungen oder z.B. in Form von Tankmischungen geschehen.

Die Konzentrate werden vor der Anwendung gegebenenfalls mit Wasser verdünnt, so daß Spritzbrühen mit einem Wirkstoffgehalt zwischen etwa 0,001 und 1 Gewichtsprozent erhalten werden. Bei der Anwendung als Low-volume oder Ultra-Low-volume-Formulierung kann der Wirkstoffgehalt auch erheblich höher sein (bis ca. 20 bzw. bis ca. 90 Gewichtsprozent).

Beispiele für erfindungsgemäße Formulierungen:

1. Suspensionspulver

20 Gew.-Teile einer Verbindung der Formel I
20 Gew.-Teile Kaolin
5 Gew.-Teile Natriumsulfat
2 Gew.-Teile Schlämmkreide
9 Gew.-Teile Calciumligninsulfonat
1 Gew.-Teile Diisobutylnaphthalinnatriumsulfonat
43 Gew.-Teile Kieselkreide

Die Bestandteile werden vermahlen. Das Mittel wird für die Anwendung in so viel Wasser suspendiert, daß die Wirkstoffkonzentration etwa 0,001 bis 0,5 Gewichtsprozent beträgt.

2. Emulsionskonzentrat

15 Gew.-Teile einer Verbindung der Formel I
10 Gew.-Teile Dodecylbenzolsulfonsäuretriethylaminsalz
75 Gew.-Teile Dimethylformamid

Die nachstehenden Beispiele sollen die erfindungsgemäßen Herstellungsverfahren näher erläutern.

Nach den erfindungsgemäßen Verfahren können die in nachstehender Tabelle angegebenen Verbindungen der Formel

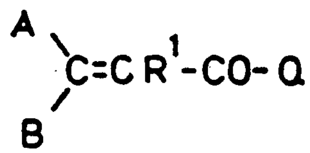

(I)

synthetisiert werden.

Die als Öle oder Harze anfallenden Verbindungen der Formel I werden durch den Rf-Wert charakterisiert. Der Rf-Wert wird dünnschichtchromatographisch an Kieselgelplatten mit folgenden Laufmittelgemischen bestimmt:

1) Toluol/Aceton 7:3
2) Toluol/Aceton 3:7
3) Essigester

## Beispiele

Zahlreiche Acrylsäureamide der Formel I können nur als Öl, als Harz oder als erstarrendes Harz isoliert werden. Zur Charakterisierung dieser Verbindung wird deshalb der Rf-Wert angegeben, der dünnschichtchromatographisch mit DC-Platten des Typs Polygram SIL G/UV 254 der Firma Macherey-Nagen bestimmt wird.

### Beispiel 1:

1,4-Bis-[1-(3,4-dimethoxyphenyl)-2-(morpholinocarbonyl)-1-vinylen]-benzol

a) 1,4-Bis-(3,4-dimethoxybenzoyl)-benzol

Zu Aluminiumchlorid (16 g; 0,12 Mol) in Dichlorethan (50 ml) wird unter Rühren, Feuchtigkeitsausschluß und Eiskühlung zuerst Terephthalsäuredichlorid (10,15 g; 0,05 Mol) und dann Veratrol (13,8 g; 0,1 Mol) zugetropft. Dann wird noch 1 Tag bei Raum- temperatur gerührt.

Das Reaktionsgemisch wird dann mit Wasser/HCl zersetzt und mit Chloroform (100 ml) extrahiert. Nach Trocknen des organischen Extrakts wird eingeengt, es verbleibt ein langsam kristallisierendes Öl, welches mit wenig Toluol/Methanol verrieben wird.

Nach einstündigem Stehen wird vom Produkt abgesaugt.

Ausbeute: 9,0 g der Titelverbindung vom Schmp. 173-188 ° C (Substanz beginnt ab 140 ° zu sintern).

b) 1,4-Bis-[1-(3,4-dimethoxyphenyl)-2-carboxy-1-vinylen]-benzol

Zu einer Suspension von Natriumhydrid (80 %ig; 1,5 g; 0,05 Mol) in Dimethoxyethan (50 ml) wird unter Rühren und Einkühlung Triethylphosphonoacetat (11,2 g; 0,05 Mol) getropft. Danach wird 1,4-Bis(3,4-dimethoxybenzoyl)-benzol (8,9 g; 0,022 Mol) zugegeben und 5 Stunden auf 100 ° C erhitzt. Nach dem Einengen wird mit Toluol/ Wasser geschüttelt und die organische Phase wird getrocknet und eingeengt.

Man isoliert 11,0 g eines Rückstandes, der mit einem KOH, MethanolWassergemisch /8,4 g KOH, 100 ml Methanol, 5 ml Wasser) versetzt und 2 Stunden zum Sieden erhitzt wird.

Der nach dem Einengen erhaltene Rückstand wird in Wasser aufgenommen, mit Toluol ausgeschüttet und dann mit Salzsäure im Harz ausgefällt, welches aus Toluol umkristallisiert wird.

Ausbeute: 8,0 g der Titelverbindung als langsam kristallisierendes Harz, welches ohne weitere Reinigung in die nachfolgende Reaktion eingesetzt wird.

c) 1,4-Bis-[1-(3,4-dimethoxyphenyl)-2-(morpholinocarbonyl)-1-vinylen]benzol

Zu einer Aufschlämmung von 1,4-Bis-[1-(3,4-dimethoxyphenyl)-2-carboxy-1-vinylen]-benzol (8,1 g; 0,0165 Mol) in Tetrahydrofuran (50 ml) gibt man rasch unter Rühren bei Raumtemperatur Carbonyldiimidazol (5,8 g; 0,036 Mol) zu. Unter starker $CO_2$-Abspaltung löst sich die Aufschlämmung. Es wird mit Morpholin (3,1 g; 0,036 Mol) versetzt und 1 Stunde zum Sieden erhitzt. Nach Abdestillieren des Lösungsmittels wird der Rückstand in Toluol/Essigester (1:1) aufgenommen, mit Wasser gewaschen und an Kieselgel mit Toluol/Aceton (9:1) gereinigt.

Ausbeute: 2 g der gereinigten Titelverbindung mit einem Rf-Wert von 0,783 (in Toluol/Aceton = 3:7).

**Beispiel 2**

1,3-Bis-[1-(3,4-dimethoxyphenyl)-2-(morpholinocarbonyl)-1-vinylen]benzol

Analog zu Beispiel 1 erhält man die Titelverbindung als Harz mit Rf: 0,27 (Kieselgel, Toluol/Aceton 7:3) ausgehend von Isophthalsäuredichlorid und Veratrol.

**Beispiel 3**

3-[3-(4-Chlorphenoxymethyl)-4-methoxyphenyl]-3-phenylacrylsäuremorpholid

a) 3-Brommethyl-4-methoxybenzophenon
3-Methyl-4-methoxybenzophenon (34 g) wird in einer Mischung aus Tetrachlorkohlenstoff (100 ml) und Schwefelkohlenstoff (5 ml) unter Rühren zum Sieden erhitzt und mit einer UV-Lampe bestrahlt.

Zu dieser Lösung wird innerhalb von 3 Stunden Brom (24,0 g) in Tetrachlorkohlenstoff (10 ml) getropft und noch eine weitere Stunde belichtet und erhitzt.

Anschließend wird mit Wasser ausgeschüttelt getrocknet, eingeengt und aus Methanol umkristallisiert.

Ausbeute: 32,4 g der Titelverbindung als Kristalle vom Schmp. 98-100 °C.

b) 3-(4-Chlorphenoxymethyl)-4-methoxybenzophenon
3-Brommethyl-4-methoxybenzophenon (15,25 g) und Natrium-4-chlorphenolat (7,6 g) werden in Acetonitril (70 ml) unter Rühren für 3 Stunden zum Sieden erhitzt.

Nach Abdampfen des Lösungsmittels wird in Toluol aufgenommen, mit Wasser gewaschen erneut eingeengt und aus Methanol umkristallisiert.

Ausbeute: 15 g der Titelverbindung als Kristalle vom Schmp. 103 °C.

c) 3-[3-(4-Chlorphenoxymethyl)-4-methoxyphenyl]-3-phenylacrylsäuremorpholid
Zu einer Lösung von Natriumhydrid (80 %ig), 1,2 g) in Tetrahydrofuran (50 ml) wird unter Rühren Diethylphosphonoessigsäuremorpholid (8,8 g) getropft und 30 Min. bei Raumtemperatur gerührt und dann 3-(4-Chlorphenoxymethyl)-4-methoxybenzophenon (10 g) zugegeben und 2 Stunden zum Sieden erhitzt.

Nach Einengen des Reaktionsgemisches wird mit Wasser/Methylenchlorid geschüttelt, die organische Phase abgetrennt, getrocknet und eingeengt. Man isoliert die Titelverbindung als Harz.

Ausbeute: 8,8 g der Titelverbindung als Harz mit einem Rf-Wert von 0,79 (Kieselgel, Toluol/Aceton 3:7)

**Beispiel 4**

3-[4-(4-Chlorphenylmercapto)-phenyl]-3-(3,4-dimethoxyphenyl)-acrylsäuremorpholid

a) 3,4-Dimethoxy-4'-fluor-benzophenon
In eine auf 0 °C gekühlten Mischung aus Aluminiumchlorid (34 g) und 4-Fluorbenzoylchlorid (35 g) wird unter Rühren Veratrol (29,3 g) gegeben und 12 Stunden bei Raumtemperatur belassen. Danach wird 1 Stunde zum Sieden erhitzt. Das Reaktionsgemisch wird danach mit Wasser/HCl zersetzt und mit Chloroform extrahiert.

Die organische Phase wird getrocknet und eingeengt. Der Rückstand wird mit Methanolkristallin getrieben.

Ausbeute: 46 g der Titelverbindung als Kristalle vom Schmp. 112-115 °C.

b) 3,4-Dimethoxy-4'-(4-Chlorphenylthio)-benzophenon
Aus p-Chlorthiophenol (4,3 g) in Methanol (10 ml) und Natriummethylat (5,4 g einer 30 %igen Natriummethylatlösung) wird durch Einengen am Rotationsverdampfer Natrium-p-chlorthiophenolat hergestellt. Das so erhaltene Salz wird zusammen mit 3,4-Dimethoxy-4'-fluorbenzophenone (7,8 g) in Dimethylformamid (30 ml) gelöst und 3 Stunden auf 100 °C erhitzt.

Dann wird das Reaktionsgemisch in Wasser eingegossen, die dabei ausgefallenen Kristalle werden aus Ethanol umkristallisiert.

Ausbeute: 7,4 g der Titelverbindung als glänzende Kristalle vom Schmp. 118-120 °C.

c) 3-[4-(4-Chlorphenylmercapto)-phenyl]-3-(3,4-dimethoxyphenyl)acrylsäuremorpholid
Zu einer Suspension von Natriumhydrid (80 %ig, 0,75 g) in Tetrahydrofuran (40 ml) wird unter Rühren Diethylphosphonoessigsäuremorpholid (6,6 g) gegeben und noch 1 Stunde weitergerührt.

Danach wird 3,4-Dimethoxy-4'-(4-chlor-phenylthio)-benzophenon (7,3 g) zugegeben und 2 Stunden zum Sieden erhitzt, das Lösungsmittel abdestilliert und der Rückstand mit Wasser/Essigester extrahiert. Das aus der organischen Phase isolierte Harz wird an Kieselgel mit Toluol/Aceton (9:1) gereinigt.

Ausbeute: 5 g der Titelverbindung als Harz mit Rf: 0,43 (Kieselgel; Toluol/Aceton 7:3)

**Beispiel 5**

3-(3,4-Dimethoxyphenyl)-3-(4-imidazol-1-yl-phenyl)-acrylsäuremorpholid

a) 3,4-Dimethoxy-4'-(imidazol-1-yl)-benzophenon

3,4-Dimethoxy-4'-fluorbenzophenon (5,2 g) und Imidazol-Natrium (1,8 g) werden in Dimethylformamid (20 ml) 4 Stunden auf 100 ° C erhitzt und danach in Wasser eingegossen. Das ausfallende Öl wird abgetrennt und mit Wasser kristallin gerieben. Das Produkt wird aus Ethanol umkristallisiert.

Ausbeute: 4,3 g der Titelverbindung als Kristalle vom Schmp. 149-151 ° C.

b) 3-(3,4-Dimethoxyphenyl)-3-(4-imidazol-1-yl-phenyl)-acrylsäuremorpholid

Zu einer Suspension von Natriumhydrid (80 %ig, 0,5 g) in Tetrahydrofuran (30 ml) wird unter Rühren Diethylphosphonoessigsäuremorpholid (4,5 g) gegeben und noch eine Stunde Raumtemperatur nachgerührt, mit 3,4-Dimethoxy-4'-(imidazol-1-yl)-benzophenon (4,0 g) versetzt und 2 Stunden zum Sieden erhitzt.

Nach Abdestillieren des Lösungsmittels wird der Rückstand mit Wasser/Essigester ausgeschüttelt, die organische Phase wird abgetrennt, eingeengt, und an Kieselgel mit Toluol/Aceton (7:3) gereinigt.

Ausbeute: 3,0 g der Titelverbindung als farbloses hartes Harz mit Rf: 0,18 (Kieselgel, Toluol/Aceton 7:3)

**Beispiel 6**

3-(3,4-Dimethoxyphenyl)-3-[4-(3-phenylureido)-phenyl]-acrylsäuremorpholid

3-(3,4-Dimethoxyphenyl)-3-(4-aminophenyl)-acrylsäuremorpholid (2,2 g) werden zusammen mit Phenylisocyanat (0,72 g) in Toluol (40 ml) 2 Stunden zum Sieden erhitzt.

Die Titelverbindung wird durch Zusatz von Benzin aus dem Reaktionsgemisch ausgefällt und aus Ethanol umkristallisiert.

Ausbeute: 2,0 g der Titelverbindung als farblose Kristalle vom Schmp. 215-220 ° C. (Zers.)

**Beispiel 7**

3-(3,4-Dimethoxyphenyl)-3-(4-phenylazophenyl)-acrylsäuremorpholid

3-(3,4-Dimethoxyphenyl)-3-(4-aminophenyl)-acrylsäuremorpholid (2,2 g) werden in Eisessig (30 ml) unter leichtem Erwärmen gelöst. Zu dieser Lösung wird bei Raumtemperatur Nitrosobenzol (0,7 zugetropft und noch eine Stunde weitergerührt. Danach wird 3 Stunden zum Sieden erhitzt, in Wasser eingegossen und mit Essigester extrahiert.

Die organische Phase wird eingeengt und an Kieselgel mit Toluol/Aceton (95:5) gereinigt.

Ausbeute: 1,4 g der Titelverbindung als intensiver orangefarbenes Harz Rf: 0,55 (Kieselgel, Cyclohexan/Aceton 1:1)

**Beispiel 8**

3-(3,4-Dimethoxyphenyl)-3-[4-(4-chlorbenzoylamino)-phenyl]-acrylsäuremorpholid

Zu einer Aufschlämmung von 3-(3,4-Dimethoxyphenyl)-3-(4-aminophenyl)-acrylsäuremorpholid (7,4 g) in Dichlorethan (30 ml) und Pyridin (1,6 g) wird 4-Chlorbenzoylchlorid (3,5 g) zugetropft. Es wird 12 Stunden bei Raumtemperatur belassen und dann noch 1 Stunde zum Sieden erhitzt. Nach dem Einengen wird zweimal mit Wasser versetzt und der Überstand dekantiert. Danach wird mit Methanol kristallin gerieben.

Ausbeute: 9,0 g der Titelverbindung als Kristalle vom Schmp. 197-207 ° C.

**Beispiel 9**

3-(3,4-Dimethoxyphenyl)-3-[4-(2,5-dimethyl-pyrryl-1-yl)-phenyl]-acrylsäuremorpholid

3-(3,4-Dimethoxyphenyl)-3-(4-aminophenyl)-acrylsäuremorpholid (5,0 g) werden mit 2,5-Hexandion (10 ml) 3 Stunden zum Sieden erhitzt.

Aus der klaren braunen Lösung wird durch Zusatz von Benzin eine schmierige Masse ausgefällt, die beim Verreiben mit Diisopropylether kristallisiert.
Das Produkt wird dann durch Umkristallisieren aus Diisopropylether gereinigt.

Ausbeute: 3,3 g der Titelverbindung als beige Kristalle vom Schmp. 143-145 °C.

**Beispiel 10**

3-(3,4-Dimethoxyphenyl)-3-[4-(3-p-toluol-sulfonylureido)-phenyl]-arylsäuremorpholid

3-(3,4-Dimethoxyphenyl)-3-(4-aminophenyl)-acrylsäuremorpholid (3,7 g) werden zusammen mit p-Toluolsulfonylisocyanat (2,0 g) in Toluol (40 ml) 40 min. zum Sieden erhitzt.
Nach dem Erhalten wird von dem Produkt abgesaugt und aus Methanol umkristallisiert.

Ausbeute: 2,3 g der Titelverbindung als farblose Kristalle vom Schmp. 220-225 °C.

**Beispiel 11**

3-(4-Ethoxycarbonylmethoxy-3-methyl-phenyl)-3-phenyl-acrylsäuremorpholid

Zu einer Lösung von Natriumethanolat in Ethanol (hergestellt aus 0,7 g Natrium und 60 ml Ethanol) wir 3-(4-Hydroxy-3-methylphenyl)3-phenylacrylsäuremorpholid (8,1 g) gegeben und dann wird Chloressigsäureethylester (3,8 g) zugetropft und danach 2 Stunden zum Sieden erhitzt. Nach Abdampfen des Lösungsmittels wird der Rückstand mit Toluol/Natronlauge ausgeschüttelt.

Aus der organischen Phase wird das Produkt nach Abdampfen des Lösungsmittels als Harz isoliert.

Ausbeute: 5,8 g der Titelverbindung als Harz mit Rf: 0,475 (Kieselgel, Toluol/Aceton 7:3)

**Beispiel 12**

3-(3-Methyl-4-methylaminocarbonyloxyphenyl)-3-phenylacrylsäuremorpholid

Zu einer auf 40 °C erwärmten Lösung von 3-(4-Hydroxy-3-methylphenyl)-3-phenylacrylsäuremorpholid (9,7 g) in Toluol (50 ml), Triethylamin (1 ml) wird Methylisocyanat (2,0 g) gegeben. Nach 1 Stunde wird mit Wasser und Natronlauge ausgeschüttelt und die organische Phase eingeengt.
Der Rückstand wird an Kieselgel mit Toluol/Aceton (7:3) gereinigt.

Ausbeute: 6 g der Titelverbindung als gelbes Harz mit Rf: 0,3 (Kieselgel, Toluol/Aceton 7:3)

**Beispiel 13**

3-(3-Methyl-4-dimethylaminocarbonyloxyphenyl)-3-phenylacrylsäuremorpholid

Analog zu Beispiel 11 erhält man die Titelverbindung als Harz mit Rf: 0,4 (Kieselgel, Toluon/Aceton 7:3) ausgehend von 3-(3-Methyl-4-hydroxyphenyl)-3-phenylacrylsäuremorpholid und N,N-Dimethylcarbonylchlorid.

**Beispiel 14**

3-(3-Methyl-4-dimethylaminosulfonyloxyphenyl)-3-phenylacrylsäuremorpholid

Analog zu Beispiel 11 erhält man die Titelverbindung als Kristalle vom Schmp. 108 °C.ausgehend von 3-(3-Methyl-4-hydroxyphenyl)-3-phenylacrylsäuremorpholid und N,N-Dimethylamidosulfonsäurechlorid.

**Beispiel 15**

0,0-Diethyl-0-[3-methyl-4-(2-morpholinocarbonyl)-1-phenyl-vinylen)-phenyl]-phosphorsäureester

Analog zu Beispiel 11 erhält man die Titelverbindung als Öl mit Rf: 0,24 (Kieselgel, Toluol/Aceton 7:3) ausgehend von 3-(3-Methyl-4-hydroxyphenyl)-3-phenyl-acrylsäuremorpholid und 0,0-Diethylphosphorsäurechlorid.

**Beispiel 16**

3-(4-Benzyloxy-3-methylphenyl)-3-phenylacrylsäuremorpholid

Analog zu Beispiel 11 erhält man die Titelverbindung als bräunliches Harz vom Rf: 0,56 (Kieselgel, Toluol/Aceton 7:3) ausgehend von 3-(4-Hydroxy-3-methylphenyl)-3-phenylacrylsäuremorpholid.

**Beispiel 17**

3-(3-Methyl-4-trimethylsilyloxyphenyl)-3-phenylacrylsäuremorpholid

Analog zu Beispiel 11 erhält man die Titelverbindung als Harz vom Rf: 0,59 (Kieselgel, Toluol/Aceton 7:3) ausgehend von Trimethylchlorsilan und 3-(3-Methyl-4-hydroxyphenyl)-3-phenylacrylsäuremorpholid.

**Beispiel 18**

3-(3-Methyl-4-methylsulfonyloxyphenyl)-3-phenylacrylsäuremorpholid

Analog zu Beispiel 11 erhält man die Titelverbindung als Harz mit Rf 0,73 (Kieselgel, Toluol/Aceton 3/7) ausgehend von Methansulfonsäurechlorid und 3-(3-Methyl-4-hydroxyphenyl)-3-phenyl-acrylsäuremorpholid.

**Beispiel 19**

3-[4-(4-Chlorphenylsulfinyl)-phenyl]-3-(3,4-dimethoxyphenyl)-acrylsäuremorpholid

Zu einer Suspension von Natriumhydrid (80 %ig, 0,45 g) in Tetrahydrofuran (20 ml) wird unter Rühren Diethylphosphonoessigsäuremorpholid (3,98 g) gegeben und noch eine Stunde bei Raumtemperatur nachgerührt. Zu der dann klaren Lösung gibt man 4-(4-Chlorphenylsulfinyl)-3',4'-dimethoxybenzophenon (4,8 g) gelöst in Tetrahydrofuran (10 ml) und erhitzt 1 Stunde zum Sieden.

Danach wird das Reaktionsgemisch eingedampft und der Rückstand mit Toluol/Wasser ausgeschüttelt.

Die organische Phase wird eingeengt und an Kieslgel mit Toluol/Aceton gereinigt.

Ausbeute: 4,2 g der Titelverbindung als Öl mit Rf 0,28 (Kieselgel, Toluol/Aceton 7:3)

Beispiel 20

Z-3-(4-Benzoylphenyl)-3-(3,4-dimethoxyphenyl)-acrylsäuremorpholid

5,55 g = 20 m Mol 3,4-Dimethoxyzimtsäuremorpholid, 5,7 g = 22 m Mol 4-Brombenzophenon, 45 mg = 0,2 m Mol Palladium-II-acetat, 122 mg = 0,4 m Mol Tri-o-tolylphosphin werden mit 10 ml Triethylamin und 10 ml Dimethylformamid 15 Stunden unter Rückfluß gekocht. Nach dem Abkühlen wird mit Toluol/Wasser geschüttelt, die org. Phase nochmals mit Wasser gewaschen, getrocknet und über eine Säule mit 60 g Kieselgel gereinigt. Eluiert wird mit Toluol, Toluol-Aceton-Gemisch 90:10. Die Fraktionen mit der Substanz vom Rf = 0,34 (Toluol-Aceton 70:30) werden im Vakuum eingedampft.
Ausbeute: 4,0 g (= 44 %) der Titelverbindung mit einem E/Z-Verhältnis von 10/90.

Beispiel 21

E-3-(4-Benzoylphenyl)-3-(3,4-dimethoxyphenyl)-acrylsäuremorpholid

Analog zu Beispiel 20 kann die Titelverbindung durch Umsetzung von 4-Benzoylzimtsäuremorpholid und 4-Bromveratrol erhalten werden.

Beispiel 22

Z-3-(4-Cyanomethylphenyl)-3-(3,4-dimethoxyphenyl)-acrylsäuremorpholid

Analog zu den Beispielen kann die Titelverbindung (Rf = 0,32, Kieselgel, Toluol/Aceton = 7/3) aus 3,4-Dimethoxyzimtsäuremorpholid und 4-Bromphenylacetonitril hergestellt werden.

Beispiel 23

E-3-(4-Cyanomethylphenyl)-3-(3,4-dimethoxyphenyl)-acrylsäuremorpholid

Analog zu den Beispielen kann die Titelverbindung aus 4-Cyanomethylzimtsäuremorpholid und 4-Bromveratrol hergestellt werden.

Beispiel 24

Z-3-(4-Carboxymethylphenyl)-3-(3,4-dimethoxyphenyl)-acrylsäuremorolid

Analog zu den Beispielen erhält man die Titelverbindung als Kristalle vom Schmp. 192-194 °C aus 3,4-Dimethoxyzimtsäuremorpholid und (4-Bromphenyl)-essigsäure.

Beispiel 25

Z-3-(3,4-Dimethoxyphenyl)-3-(4-formylphenyl)-acrylsäuremorpholid

Analog zu den Beispielen erhält man die Titelverbindung aus 3,4-Di-methoxyzimtsäuremorpholid und 4-Brombenzaldehyd.

Beispiel 26

Z-3-(3,4-Dimethoxyphenyl)-3-[4-(1,3-dioxolan-2-yl)-phenyl]-acrylsäure-morpholid

Analog zu den Beispielen erhält man die Titelverbindung aus 3,4-Dimethoxy-zimtsäuremorpholid und 2-(4-Bromphenyl)-1,3-dioxolan.

Beispiel 27

E-3-(3,4-Dimethoxyphenyl)-3-[4-(4-n-propyl-1,3-dioxan-2-yl)-phenyl]-acrylsäuremorpholid

Analog zu den Beispielen erhält man die Titelverbindung aus 4-(4-n-Propyl-1,3-dioxolan-2-yl)-zimtsäuremorpholid und 4-Bromveratrol

Beispiel 28

Z-3-(3,4-Dimethoxyphenyl-3-[4-(4-n-propyl-1,3-dioxan-2-yl)-phenyl]-acrysäuremorpholid

Analog zu den Beispielen erhält man die Titelverbindung aus 2-(4-Bromphenyl)-4-n-propyl-1,3-dioxolan und 3,4-Dimethoxyzimtsäuremorpholid

Beispiel 29

E-3-(3,4-Dimethoxyphenyl)-3-[4-(1,3-dithian-2-yl)-phenyl]-acrylsäure-morpholid

Analog zu den Beispielen erhält man die Titelverbindung aus 4-(1,3-dithioxan-2-yl)-zimtsäuremorpholid und 4-Bromveratrol

Beispiel 30

3-(3,4-Dimethoxyphenyl)-3-[4-(1,2-dichlorvinyloxy]-phenyl)-acrylsäuremorpholid

5,5 g 3-(3,4-Dimethoxyphenyl)-3-(4-hydroxyphenyl)-acrylsäuremorpholid werden in 40 ml einer äquivalenten Natriumalkoholatlösung gelöst, auf Rückstand eingedampft und der Rückstand in 40 ml Dimethylformamid gelöst. Zu dieser Lösung werden bei 60°C 2,2 g Trichloräthylen in 5 ml Dimethylformamid zugetropft. Die Mischung wird 6 Stunden bei 80°C gerührt. Im Vakuum wird auf Rückstand eingedampft. Der Rückstand wird in Toluol/Wasser aufgenommen und geschüttelt. Nach Trocknen der Toluolphase und Eindampfen werden 5,2 g der Titelverbindung erhalten. Rf-Wert 0.72 (Toluol/Aceton 3:7).

Beispiel 31

3-(3,4-Dimethoxyphenyl)-3-[4-(4,6-dichlor-1,3,5-triazin-2-yloxy)-phenyl]-acrylsäure-morpholid

Zu einer Lösung von 1,9 g (0,01 Mol) Cyanurchlorid in 12 ml Chloroform tropft man bei Raumtemperatur eine Phenolatlösung, hergestellt aus 4,1 g des nach dem vorhergehenden Beispiel erhaltenen Phenols (0.011 Mol),

0.5 g 85 proz. Natriumhydroxid und 5 ml Wasser. Man rührt 1,5 Stunden bei 50°C, verdünnt mit 50 ml Chloroform, trennt die organische Phase ab, wäscht sie mit kalter verdünnter Natronlauge und mit Kochsalzlösung, trocknet mit Natriumsulfat und engt im Vakuum bei 60°C ein. Das Rohprodukt (6,5 g) wird über eine Kieselgel-Säule gereinigt (Toluol/Aceton 1:1). Man erhält die Titelverbindung mit einer Ausbeute von 3,1 g (60 % der Theorie), Fp. 90-100°C, Rf = 0.60 (Toluol/Aceton 1:1).

Beispiel 32

3-(3,4-Dimethoxyphenyl)-3-[4-(2-styryl)-phenyl]-acrylsäuremorpholid

13 g 3-(3,4-Dimethoxyphenyl)-3-(4-bromphenyl)acrylsäuremorpholid (0,03 Mol) werden in 60 ml Dimethylformamid/Triethylamin 1:1 gelöst, mit 4,2 g Styrol (0,04 Mol) sowie mit 68 mg Palladium (II)-acetat (0,3 mMol) und 183 mg Tri-(o-tolyl)-phosphin (0,6 mMol) versetzt und 12 Stunden unter Rückfluß gekocht. Nach Abkühlen auf Raumtemperatur wird mit 100 ml Toluol versetzt, abgesaugt und die Lösung mti Wasser, verdünnter Salzsäure, nochmals mit Wasser und schließlich gesättigter Kochsalzlösung gewaschen. Man trocknet die organische Phase über Natriumsulfat und engt bei 60°C im Vakuum ein. Das Rohprodukt (15 g) wird chromatographisch gereinigt (Kieselgelsäule, Elutionsmittel Toluol/Aceton 1:1). Durch Einengen der Lösung im Vakuum bei 60°C erhält man 10,8 g eines orange gefärbten Öls, 79 % der Theorie.
Rf = 0.31 (Toluol/Aceton 7:3).

Beispiel 33

3-(3,4-Dimethoxyphenyl)-3-[4-(1-carboxy-1-styryl)-phenyl]-acrylsäuremorpholid

4,3 g 3-(3,4-Dimethoxyphenyl)-3-(4-bromphenyl)-acrylsäuremorpholid(0,01 Mol) und 1,95 g Zimtsäure (0,013 Mol) werden in 20 ml Triethylamin und 10 ml Dimethylformamid gelöst, mit 23 mg Palladium-(II)-acetat (0,1 mMol) sowie 61 mg Tri-(o-tolyl)-phosphin (0,2 mMol) versetzt und 16 Stunden unter Rückfluß gekocht. Nach Abkühlen werden die Lösungsmittel im Vakuum bei 60°C entfernt. der Rückstand wird in 200 ml Ethylacetat gelöst, mit 2N Salzsäure angesäuert und abgesaugt. Die organische Phase wird mit Natriumsulfat getrocknet, im Vakuum eingedampft, der Rückstand über eine Kieselgelsäule, Aceton/Ethanol 1:1 chromatographisch gereinigt. Aus der Lösung erhält man 1,8 g (36 % der Theorie) einer erstarrenden Verbindung.
Rf = 0,23 (Toluol/Aceton 7:3).

Beispiel 34

3-(3,4-Dimethoxyphenyl)-3-[4-(1-styryl)-phenyl]-acrylsäuremorpholid

4.6 g der nach Beispiel 40 erhaltenen Carbonsäure (9,2 mMol) werden zur Decarboxylierung in einer Mischung aus 30 ml Dioxan und 30 ml konz. Salzsäure 5 Stunden unter Rückfluß gerührt. Die Lösung wird im Vakuum bei 60 °C eingeengt, in Toluol aufgenommen und die Lösung zur Trockne eingeengt. Man reinigt chromatographisch über eine Kieselgelsäule mit Toluol/Aceton 7:3. Aus der Lösung erhält man ein Öl, das langsam kristallisiert. Ausbeute 3,3 g /79 % der Theorie), Fp. 115-124 °C (aus Diisopropylether, Rf = 0,45 (Toluol/Aceton 7:3).

Nach den erfindungsgemäßen Verfahren können die in nachstehender Tabelle angegebenen Verbindungen der Formel

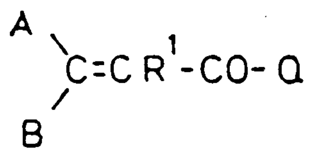

(I)

synthetisiert werden.

Die als Öle oder Harze anfallenden Verbindungen der Formel I werden durch den Rf-Wert charakterisiert. Der Rf-Wert wird dünnschichtchromatographisch an Kieselgelplatten mit folgenden Laufmittelgemischen bestimmt:

1) Toluol/Aceton 7:3
2) Toluol/Aceton 3:7

3) Essigester

TABELLE I

| No. | $R^1$ | A | B | Q | Physik. Daten |
|---|---|---|---|---|---|
| 1 | H | $3-CH_3-4-CH_3O-C_6H_3$ | $4-C_6H_5-O-C_6H_4$ | 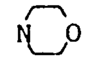 | Rf: 0,54 1) |
| 2 | H | $C_6H_5$ | $3-CH_3-4-(C_2H_5O-CO-CH(CH_3)-O)-C_6H_3$ | 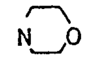 | Rf: 0,52 1) |
| 3 | H | $C_6H_5$ | $3-CH_3-4-(C_2H_5-O-CO-CH_2-O)-C_6H_3$ | 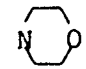 | Rf: 0,48 1) |
| 4 | H | $3,4-(CH_3O)_2-C_6H_3$ | $4-(n-C_6H_{13})-C_6H_4$ | 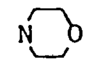 | Rf: 0,45 1) |
| 5 | H | $3,4-(CH_3O)_2-C_6H_3$ | $4-(n-C_5H_{11})-C_6H_4$ | 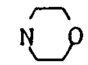 | |
| 6 | H | $3,4-(CH_3O)_2-C_6H_3$ | $4-(n-C_{12}H_{25})-C_6H_4$ | 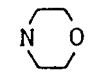 | Rf: 0,48 1) |
| 7 | H | $3,4-(CH_3O)_2-C_6H_3$ | $4-(4-NO_2-C_6H_4)-C_6H_4$ | 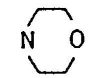 | Rf: 0,36 1) |
| 8 | H | $C_6H_5$ | $4-CH_3O-3-[(EtO)_2POCH_2]-C_6H_3$ | 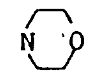 | Rf: 0,11 1) |
| 9 | H | $C_6H_5$ | $4-CH_3O-3-(NC-CH_2)-C_6H_3$ | 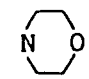 | Rf: 0,42 1) |
| 10 | H | $C_6H_5$ | $4-CH_3O-3-$(Imidazol-1-yl-methyl)$C_6H_3$ | 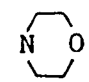 | Rf: 0,13 2) |
| 11 | H | $C_6H_5$ | $4-CH_3-O-3-$(1,2,4-Triazol-1-yl-methyl)-$C_6H_3$ | 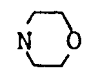 | |
| 12 | H | $C_6H_5$ | $4-CH_3O-3-CH_3SCH_2-C_6H_3$ | 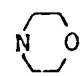 | Fp: 105-120° |

| No. | $R^1$ | A | B | Q | Physik. Daten |
|---|---|---|---|---|---|
| 13 | H | $C_6H_5$ · | $4\text{-}NO_2\text{-}C_6H_4\text{-}O\text{-}C_6H_4$ | 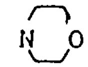 | |
| 14 | H | $3,4\text{-}(CH_3O)_2\text{-}C_6H_3$ | $4\text{-}C_5H_{11}\text{-}O\text{-}C_6H_4$ | 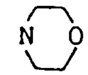 | Rf: 0,06 3) |
| 15 | H | $C_6H_5$ | $4\text{-}CH_3O\text{-}3\text{-}[(CH_3)_2NCH_2]\text{-}C_6H_3$ | 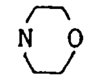 | Fp: 123-125° |
| 16 | H | $C_6H_5$ | $4\text{-}CH_3O\text{-}3\text{-}(C_6H_5\text{-}O\text{-}CH_2)\text{-}C_6H_3$ | 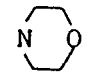 | Rf: 0,53 1) |
| 17 | H | $3,4\text{-}(CH_3O)_2\text{-}C_6H_3$ | $4\text{-}[(CH_3)_2NSO_2]\text{-}C_6H_4$ | 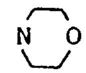 | Rf: 0,26 1) |
| 18 | H | $3,4\text{-}(CH_3O)_2\text{-}C_6H_3$ | 4-(1,2,4-Triazol-1-yl)-$C_6H_4$ | 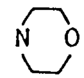 | Fp: 145-146° |
| 19 | H | $3,4\text{-}(CH_3O)_2\text{-}C_6H_3$ | $4\text{-}(4\text{-}Cl\text{-}C_6H_4\text{-}SO_2)\text{-}C_6H_4$ | 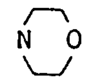 | Fp: 173-175° |
| 20 | H | $3,4\text{-}(CH_3O)_2\text{-}C_6H_3$ | $4\text{-}(4\text{-}Cl\text{-}C_6H_4O)C_6H_4$ | 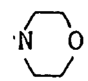 | Rf: 0,35 1) |
| 21 | H | $3,4\text{-}(CH_3O)_2\text{-}C_6H_3$ | $4\text{-}(C_6H_5\text{-}O\text{-}CH_2)C_6H_4$ | 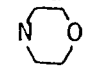 | Rf: 0,42 1) |
| 22 | H | $3,4\text{-}(CH_3O)_2\text{-}C_6H_3$ | $4\text{-}(C_6H_5\text{-}CH_2\text{-}O)C_6H_4$ | 4-Morpholinyl | Rf: 0,42 1) |
| 23 | H | $3,4\text{-}(CH_3O)_2\text{-}C_6H_3$ | $4\text{-}(4\text{-}CH_3\text{-}C_6H_4\text{-}O)C_6H_4$ | 4-Morpholinyl | Rf: 0,45 1) |
| 24 | H | $3,4\text{-}(CH_3O)_2\text{-}C_6H_3$ | $4\text{-}(C_6H_5\text{-}NH\text{-}CO\text{-}NH)\text{-}C_6H_4$ | 4-Morpholinyl | Fp: 215-220° |
| 25 | H | $3,4\text{-}(CH_3O)_2\text{-}C_6H_3$ | $4\text{-}(4\text{-}C_2H_5\text{-}C_6H_4)\text{-}C_6H_4$ | 4-Morpholinyl | Fp: 65-67° |

| No. | $R^1$ | A | B | Q | Physik. Daten |
|---|---|---|---|---|---|
| 26 | H | $3,4-(CH_3O)_2-C_6H_3$ | $4-(HOCH_2CH_2NHCO)-C_6H_4$ | 4-Morpholinyl | Rf: 0,15 1) |
| 27 | H | $C_6H_5$ | $4-CH_3O-3-H_2NCH_2-C_6H_3$ | 4-Morpholinyl | Rf: 0,06 2) |
| 28 | H | $3,4-(CH_2O)_2-C_6H_3$ | 1,2,3,4-Tetrahydronaphthalin-6-yl | 4-Morpholinyl | Rf: 0,41 1) |
| 29 | H | $3,4-(CH_2O)_2-C_6H_3$ | 4-Pyrazol-1-yl-$C_6H_4$ | 4-Morpholinyl | Rf: 0,28 1) |
| 30 | H | $C_6H_5$ | $4-CH_3O-3-$(Morpholin-4-yl-methyl)$C_6H_3$ | 4-Morpholinyl | Rf: 0,14 1) |
| 31 | H | $C_6H_5$ | $4-CH_3O-3-$(2,6-dimethyl-morpholin-4-yl-methyl)-$C_6H_3$ | 4-Morpholinyl | Rf: 0,28 1) |
| 32 | H | $3,4-(CH_3O)_2-C_6H_3$ | Indan-5-yl | 4-Morpholinyl | Rf: 0,40 1) |
| 33 | H | $3,4-(CH_3O)_2-C_6H_3$ | 4-(Morpholin-4-yl)-$C_6H_4$ | 4-Morpholinyl | Rf: 0,25 1) |
| 34 | H | $4-Cl-C_6H_4$ | $4-(CH_3SO_2-O)-C_6H_4$ | 4-Morpholinyl | Rf: 0,35 1) |
| 35 | H | $C_6H_5$ | $4-CH_3O-3-(4-Cl-C_6H_4-S-CH_2)C_6H_3$ | 4-Morpholinyl | Rf: 0,56 1) |
| 36 | H | $C_6H_5$ | $3-CH_3-4-[(CH_3)_3SiCH_2O]-C_6H_3$ | 4-Morpholinyl | Rf: 0,59 1) |
| 37 | H | $3,4-(CH_3O)_2C_6H_3$ | $4-C_6H_5O-C_6H_4$ | 4-Morpholinyl | Rf: 0,39 3) |

| No. | $R^1$ | A | B | Q | Physik. Daten |
|---|---|---|---|---|---|
| 38 | H | $3,4-(CH_3O)_2C_6H_3$ | Dibenzofuran-2-yl | 4-Morpholinyl | |
| 39 | H | $3,4-(CH_3O)_2C_6H_3$ | Fluoren-2-yl | 4-Morpholinyl | Rf: 0,44 [1)] |
| 40 | H | $3-Cl-4-CH_3O-C_6H_3$ | $3-C_6H_5O-C_6H_4$ | Morpho-4-yl | |
| 41 | H | $3-CH_3-4-CH_3O-C_6H_3$ | $4-(2-Cl-C_6H_4O)-C_6H_4$ | $N(CH_3)_2$ | |
| 42 | H | $3-CH_3O-4-CH_3-C_6H_3$ | $4-(2-C_2H_5O_2C-C_6H_4O)C_6H_4$ | Morpho-4-yl | |
| 43 | H | $3-C_2H_5-4-CH_3O-C_6H_3$ | $4-(3-Cl-C_6H_4O)C_6H_4$ | Morpholin-4-yl | |
| 44 | H | $3-Br-4-N(CH_3)_2-C_6H_3$ | $4-(4-CH_3O-C_6H_4O)C_6H_4$ | Morpholin-4-yl | |
| 45 | H | $3-Br-4-N(CH_3)_2-C_6H_3$ | $4-(4-CH_3O-C_6H_4O)C_6H_4$ | Morpholin-4-yl | |

| No. | $R^1$ | A | B | Q | Physik. Daten |
|---|---|---|---|---|---|
| 46 | H | $3,5\text{-}Cl_2\text{-}4\text{-}NH_2C_6H_2$ | $4\text{-}(2\text{-}CH_3O\text{-}C_6H_4O)C_6H_4$ | Morpholin-4-yl | |
| 47 | $CH_3$ | $3,5\text{-}(CH_3)_2\text{-}4\text{-}CH_3OC_6H_2$ | $4\text{-}(4\text{-}Br\text{-}C_6H_4)\text{-}C_6H_4$ | Morpholin-4-yl | |
| 48 | H | $3\text{-}Br\text{-}4\text{-}CH_3OC_6H_3$ | $3\text{-}Cl\text{-}4\text{-}(3\text{-}CH_3C_6H_4S)C_6H_3$ | Morpholin-4-yl | |
| 49 | H | $3,4\text{-}(CH_3)_2\text{-}C_6H_3$ | $4\text{-}(4\text{-}CH_3\text{-}C_6H_4)\text{-}C_6H_4$ | Morpholin-4-yl | |
| 50 | H | $3\text{-}NH_2\text{-}4\text{-}CH_3OC_6H_3$ | $4\text{-}(4\text{-}F\text{-}C_6H_5O)C_6H_4$ | Morpholin-4-yl | |
| 51 | H | $3\text{-}NH_2\text{-}4\text{-}CH_3\text{-}C_6H_3$ | $4\text{-}C_6H_5S\text{-}C_6H_4$ | Morpholin-4-yl | |
| 52 | Br | $3\text{-}CH_3\text{-}4\text{-}C_2H_5OC_6H_3$ | $4\text{-}(4\text{-}NH_2\text{-}C_6H_4)C_6H_4$ | Morpholin-4-yl | |
| 53 | H | $3\text{-}Cl\text{-}4\text{-}CH_3OC_6H_3$ | $4\text{-}(4\text{-}C_3H_7\text{-}C_6H_4)C_6H_4$ | Morpholin-4-yl | |
| 54 | H | $3\text{-}CH_3\text{-}4\text{-}CH_3OC_6H_5$ | $4\text{-}(4\text{-}C_4H_9OC_6H_4O)\text{-}C_6H_4$ | Morpholin-4-yl | |
| 55 | H | $3\text{-}CH_3O\text{-}4\text{-}CH_3C_6H_3$ | $4\text{-}C_6H_5O\text{-}C_6H_4$ | Morpholin-4-yl | |
| 56 | H | $3\text{-}Br\text{-}4\text{-}NHCH_3C_6H_3$ | $4\text{-}(2\text{-}Cl\text{-}C_6H_4S)C_6H_4$ | Morpholin-4-yl | |

| No. | $R^1$ | A | B | Q | Physikal. Daten |
|---|---|---|---|---|---|
| 57 | H | $3,5-Cl_2-4-NH_2-C_6H_2$ | $4-(3,4-(CH_3)_2-C_6H_3O)C_6H_4$ | Morpholin-4-yl | |
| 58 | H | $3-C_2H_5-4-CH_3OC_6H_3$ | $4-(2-ClC_6H_4)-C_6H_4$ | Morpholin-4-yl | |
| 59 | H | $3-Cl-4-CH_3OC_6H_3$ | $4-(2,3-Cl_2C_6H_3)-C_6H_4$ | Morpholin-4-yl | |
| 60 | H | $3-CH_3-4-CH_3O-C_6H_3$ | $4-(3-Cl-C_6H_4)-C_6H_4$ | Morpholin-4-yl | |
| 61 | H | $3-Br-4-CH_3O-C_6H_3$ | $4-(4-C_2H_5OC_6H_4)C_6H_4$ | Morpholin-4-yl | |
| 62 | H | $3,4-(CH_3O)_2-C_6H_3$ | $4-(4-CH_3OCO-C_6H_4)C_6H_4$ | Morpholin-4-yl | |
| 63 | H | $3,4-(CH_3)_2-C_6H_3$ | $4-(4-C_2H_5O-CO-C_6H_4)C_6H_4$ | Morpholin-4-yl | |
| 64 | H | $3,4-(CH_3O)_2-C_6H_3$ | $4-(1-Pyrryl)-C_6H_4$ | Morpholin-4-yl | Fp: 164-166° |
| 65 | H | $3,4-(CH_3O)_2-C_6H_3$ | $4-(4-Cl-C_6H_4)-C_6H_4$ | Morpholin-4-yl | Rf: 0,45 1) |
| 66 | H | $3,4-(CH_3O)_2-C_6H_3$ | $4-(2,5-Dioxopyrrol-1-yl)-C_6H_4$ | Morpholin-4-yl | |
| 67 | H | $3-C_2H_5O-4-CH_3O-C_6H_4$ | $4-(2,4-Cl_2-C_6H_3S)-C_6H_4$ | $N(CH_3)C_2H_5$ | |
| 68 | $CH_3$ | $3-Cl-4-CH_3O-C_6H_3$ | $4-(4-Br-C_6H_4O)-C_6H_4$ | $N(CH_3)C_3H_7$ | |

| No. | $R^1$ | A | B | Q | Physik. Daten |
|---|---|---|---|---|---|
| 69 | H | $3-Br-4-CH_3O-C_6H_3$ | $3,4-Cl_2-C_6H_3C_6H_4$ | 2-Methyl-morpholin-4-yl | |
| 70 | CN | $3-CH_3-4-CH_3O-C_6H_3$ | 1-Naphthoxy-$C_6H_4$ | Morpholin-4-yl | |
| 71 | H | $3-CH_3O-4-CH_3C_6H_3$ | 2-Naphthoxy-$C_6H_4$ | Morpholin-4-yl | |
| 72 | H | $3,5-Cl_2-4-NH_2-C_6H_2$ | $4-(4-CH_3-C_6H_4-S)-C_6H_4$ | Morpholin-4-yl | |
| 73 | Cl | $3-C_2H_5-4-CH_3O-C_6H_3$ | $4-C_6H_5NH-C_6H_4$ | Morpholin-4-yl | |
| 74 | Br | $3-C_3H_7-4-CH_3OC_6H_3$ | 4-(1-Naphthylthio)-$C_6H_4$ | Morpholin-4-yl | |
| 75 | H | $3-Br-4-(CH_3)_2N-C_6H_3$ | $4-(4-ClC_6H_4CH_2O)-C_6H_4$ | $N(CH_2CH_3)_2$ | |
| 76 | H | $3,4-(CH_3)_2C_6H_3$ | $4-(4-NO_2-C_6H_4CH_2O)-C_6H_4$ | Morpholin-4-yl | |
| 77 | H | $3-NH_2-4-CH_3OC_6H_3$ | $4-(2,4-Cl_2-C_6H_3O)-C_6H_4$ | Morpholin-4-yl | |
| 78 | H | $3-NHCOCH_3-4-CH_3O-C_6H_3$ | $4-C_6H_5-CH_2S-C_6H_4$ | Morpholin-4-yl | |
| 79 | H | $3-NHCOCH_3-4-Cl-C_6H_3$ | $4-(4-Cl-C_6H_4)-C_6H_4$ | Morpholin-4-yl | |

| No. | $R^1$ | A | B | Q | Physik. Daten |
|---|---|---|---|---|---|
| 80 | H | $3,5-(CH_3)_2-4-CH_3O-C_6H_3$ | $3-Cl-4-(2,4-(CH_3)_2-C_6H_3O)C_6H_3$ | Morpholin-4-yl | |
| 81 | H | $4-CH_3O-C_6H_4$ | $4-(C_6H_5CH_2)-C_6H_4$ | Morpholin-4-yl | |
| 82 | H | $4-C_2H_5O-C_6H_4$ | $4-C_6H_5-CH=CH-C_6H_4$ | Morpholin-4-yl | |
| 83 | $CH_3$ | $3-C_2H_5O-4-CH_3O-C_6H_3$ | $4-(4-CN-C_6H_4O)-C_6H_4$ | $N(CH_3)(C_4H_9)$ | |
| 84 | H | $3-Br-4-CH_3O-C_6H_3$ | $4-(3-NO_2-C_6H_4O)-C_6H_4$ | Morpholin-4-yl | |

## TABELLE II

Verbindungen der Formel

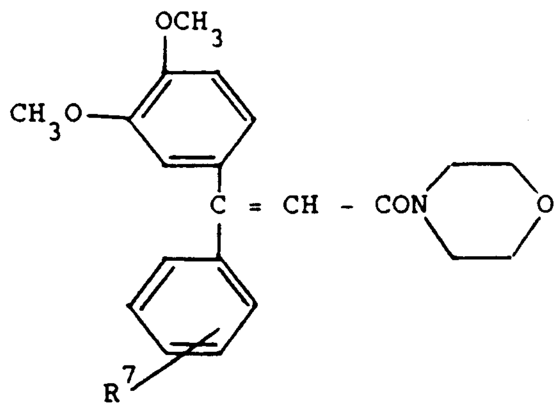

| Nr. | $-XYR^7$ | physik. Daten |
|---|---|---|
| 1 | 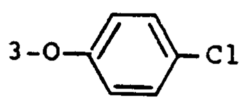 | Rf = 0,43 [1)] |
| 2 | $4-n-C_5H_{11}$ | Rf = 0,22 [1)] |
| 3 | 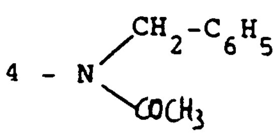 | Rf = 0,21 [1)] |
| 4 | 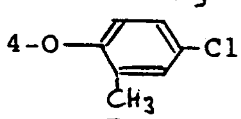 | Rf = 0,38 [1)] |
| 5 | 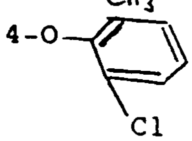 | Rf = 0,41 [1)] |
| 6 | 4-OCCl=CHCl | Rf = 0,72 [2)] |

| Nr. | $-XYR^7$ | physik.Daten |
|---|---|---|
| 7 | 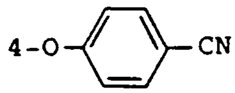 | Rf = 0,34 [1)] |
| 8 | 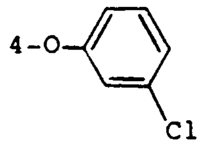 | Rf = 0,40 [1)] |
| 9 | $4-OSO_2N(CH_3)_2$ | Rf = 0,68 [2)] |
| 10 | $4-O(CH_2)_3-OH$ | Rf = 0,51 [2)] |
| 11 | $4-NH-CO-(CH_2)_3-C_6H_5$ | Rf = 0,38 (in Methylenchlorid/ Acetonitril 1:1) |
| 12 | 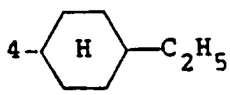 | Rf = 0,52 [1)] |
| 13 | 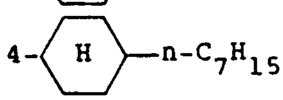 | Rf = 0,55 [1)] |
| 14 | 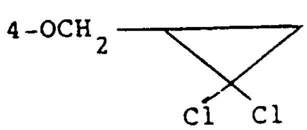 | Rf = 0,47 (Toluol/Aceton 1:1) |
| 15 | $4-COCH_3$ | Rf = 0,31 [1)] |
| 16 | 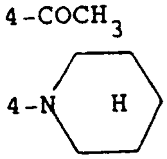 | Rf = 0,37 [1)] |
| 17 | $2-CO-C_6H_5$ | Rf = 0,43 [1)] |

| Nr. | $-XYR^7$ | physik.Daten |
|---|---|---|
| 18 | 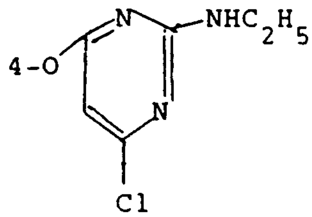 | Rf = 0,40 [1)] |
| 19 | $4-SO_2NH-C_6H_5$ | Rf = 0,29 [1)] |
| 20 | $4-CH_2-COOH$ | Fp. 192-194°C |
| 21 | $4-n-C_6H_{13}$ | Rf = 0,55 [1)] |
| 22 | $4-n-C_7H_{15}$ | Rf = 0,58 [1)] |
| 23 | 4-CH=CH-COOH | Rf = 0,48 (Toluol/Ethanol 80 : 20) |
| 24 | 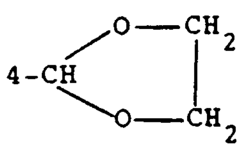 | Rf = 0,23 [1)] |
| 25 | $4-CH=CH-C_6H_5$ | Rf = 0,31 [1)] |
| 26 | 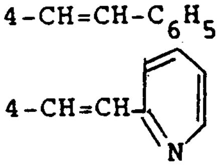 | Rf 0,33 [1)] |
| 27 | 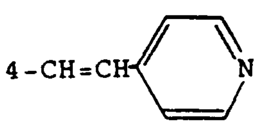 | Rf = 0,33 (Toluol/Aceton 1:1) |

| Nr. | $-XYR^7$ | physik.Daten |
|---|---|---|
| 28 | 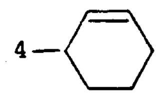 | Rf = 0,38 [1)] |
| 29 | 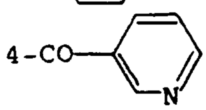 | Rf = 0,16 [1)] |
| 30 | 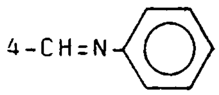 | Rf = 0,31 [1)] |
| 31 | 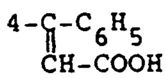 | Rf = 0,23 [1)] |
| 32 | 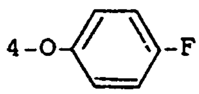 | Rf = 0,36 [1)] |
| 33 | 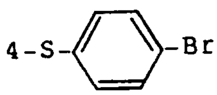 | Rf = 0,46 [1)] |
| 34 | 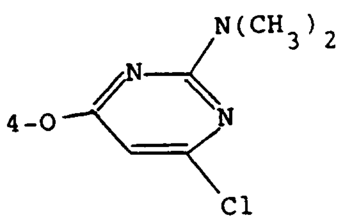 | Rf = 0,44 [1)] |
| 35 | 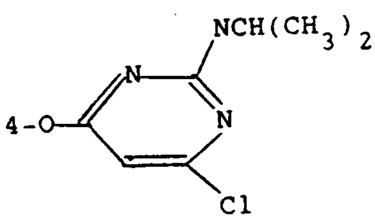 | $Rf_1$ = 0,44 [1)]<br>$Rf_2$ = 0,56<br>(E/Z-Gemisch) |
| 36 | $4-CH_2O-CONH-C_6H_5$ | Rf = 0,34 [1)] |

| Nr. | $-XYR^7$ | physik.Daten |
|---|---|---|
| 37 | 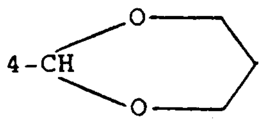 | Rf = 0,36 [1)] |
| 38 | 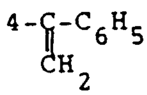 | Rf = 0,45 [1)] |
| 39 | 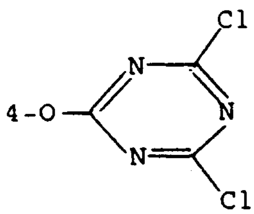 | Rf = 0,60 [1)] (Toluol/Aceton 1:1) |
| 40 | $4-CH_2CO_2C_2H_5$ | Rf = 0,33 [1)] |
| 41 | $4-CH=CH-CO_2C_2H_5$ | Rf = 0,37 [1)] |
| 42 | 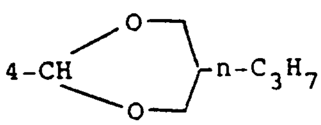 | Rf = 0,40 [1)] |
| 43 | $4-NHCONHCH_3$ | Rf = 0,20 (Toluol/Aceton 1:1) |

| Nr. | $-XYR^7$ | physik. Daten |
|---|---|---|
| 44 | 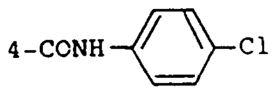 | Fp. 228-230°C |
| 45 | 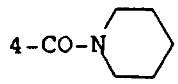 | Rf = 0,23 [1)] |
| 46 | $4-CH=CH-n-C_3H_7$ | Rf = 0,67<br>(Toluol/Aceton 1:1) |
| 47 | 4-CH=CH-CN | Rf = 0,39 [1)] |
| 48 | 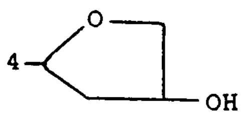 | $Rf_1$ = 0,33<br>$Rf_2$ = 0,39<br>(Toluol/Aceton 1:1) |
| 49 | $-CHOH-C_6H_5$ | Rf = 0,34 [1)] |

## TABELLE III

Verbindungen der Formel

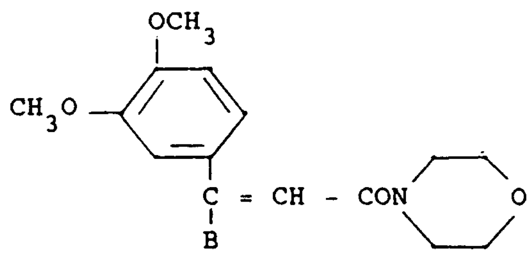

| Nr. | B | physik.Daten |
|---|---|---|
| 1 | 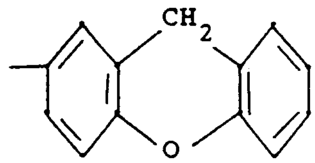 | Rf = 0,43 < |
| 2 | 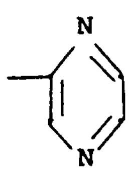 | Rf = 0,50 (Toluol/Ethanol 8:2) |
| 3 | 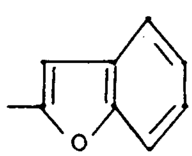 | Fp. 147°C |
| 4 | 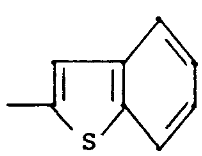 | Fp. 150°C |

| Nr. | B | physik.Daten |
|---|---|---|
| 5 | 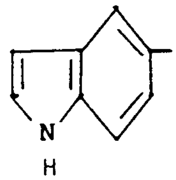 | Rf = 0,32 [1)] |
| 6 | 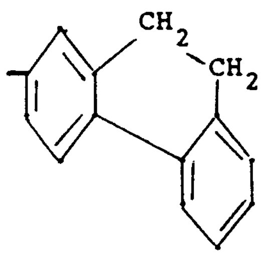 | Rf = 0,37 [1)] |
| 7 | 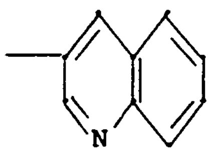 | Rf = 0,18 [1)] |
| 8 | 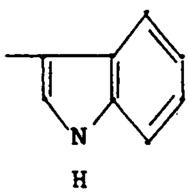 | Rf = 0,30 [1)] |
| 9 | 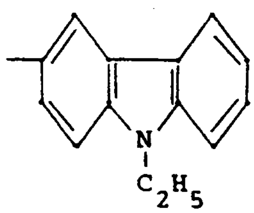 | Rf = 0,43 [1)] |
| 10 | 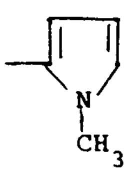 | Rf = 0,36 [1)] |

## Patentansprüche

## Patentansprüche für folgende Vertragsstaaten : BE, CH, DE, FR, GB, GR, IT, LI, LU, NL, SE

**1.** Verbindungen der Formel

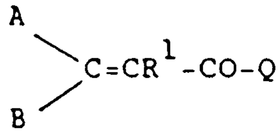
(I)

in der

A für

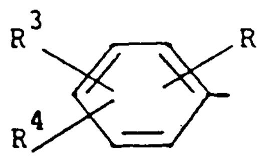

B für

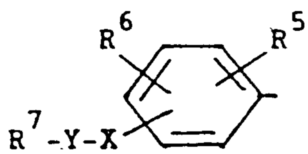 , 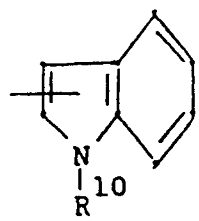 , 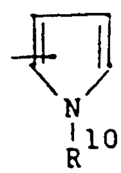

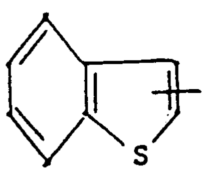 , 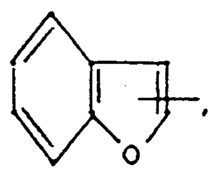 , 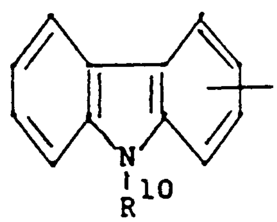

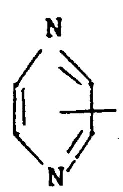 , 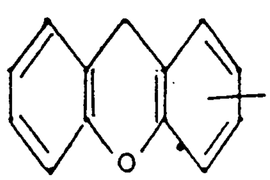 oder 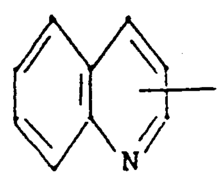

$R^1$ für Wasserstoff; Halogen, Cyano oder $C_1$-$C_4$-Alkyl gegebenenfalls ein oder mehrfach mit Hydroxy, Alkoxy, Mercapto, Halogen, Alkylthio, Nitro, Cyano, Amino oder Mono- oder Di-$C_1$-$C_4$-alkylamino substituiert, steht,

Q für

$NR^8R^9$ oder

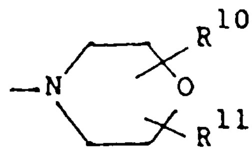

steht,

$R^2$, $R^3$, $R^4$, $R^5$ und $R^6$, die gleich oder verschieden sein können für Wasserstoff; Halogen, Nitro; Cyano; Carboxy; Hydroxy;
$C_1$-$C_4$-Alkoxycarbonyl: $CONR^{10}R^{11}$;
$NR^{10}R^{11}$; $NR^{10}$-CO-$R^{11}$;
oder für $C_1$-$C_6$-Alkyl, $C_1$-$C_4$-Alkoxy oder $C_1$-$C_4$-Alkyl-S(O)p (mit p = 0, 1, 2), gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl, Phenoxy oder Phenyl-S(O)p-(mit p = 0, 1, 2) gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^8$ und $R^9$, die gleich oder verschieden sein können für Wasserstoff;
$C_1$-$C_4$-Alkyl, $C_3$-$C_7$-Alkenyl, $C_3$-$C_7$-Alkinyl oder Alkoxyalkyl gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl oder Benzyl gegebenenfalls bis Zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^{10}$ und $R^{11}$, die gleich oder verschieden sein können, für Wasserstoff, Benzyl, Phenyl oder $C_1$-$C_4$-Alkyl, als Substituenten in Hetaryl auch für Halogen, Amino oder Mono- oder Di-$C_1$-$C_4$alkylamino stehen, oder gemeinsam für $-(CH_2)_4$- oder $-(CH_2)_5$-,

X - Y für eine Einfachbndung;
-O-; -S(O)p- (mit p = 0, 1, 2); -$CONR^{10}$-; -$NR^{10}CO$-; -$NR^{11}CONR^{10}$-; -N=N-; -$CHR^{10}$-S(O)p-(mit p = 0, 1, 2); -$CHR^{10}O$-; -$SO_2NR^{10}$-; -N=CH-; -$C_nH_{2n}$- (mit n = 1 bis 10); -CH=CH-; -$NR^{10}CSNR^{11}$-; -$NR^{10}$-; -$R^{10}N$-$CHR^{11}$-; -$CHR^{10}NR^{11}$-; -O-$CHR^{10}$-; -S(O)p-$CHR^{10}$- (mit p = 0, 1, 2); -$NR^{10}SO_2$- oder -$OSO_2$-; -$O_2SO$-; -$O_2S$-$NR^{10}$-; -C≡C-; -S-S-; -CHOH-; -CO-;
$>C=CH_2$; $>C$=CH-COOH; -NH-NH-; -N(O)=N-;

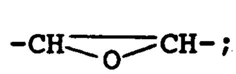

-N($R^{10}$)-N=CH-; -N($R^{10}$)-NH-CO-; -NH-CH=CH-; $>CH$-$C_6H_5$; -$COCH_2O$-; -CO-$CH_2S$-; -$COCH_2NR^{10}$-; -$OCH_2CO$; -$SCH_2CO$-; -CH=N-; -$NR^{10}CH_2CO$-; -CH=NO-; -$CH_2O$-$CONR^{10}$-; -$CH_2O$-CO-; -S-$CH_2$-; -$CH_2CO$-; -CO-O-; -O-CO-; -CO-S- oder -S-CO- steht;

$R^7$ für Wasserstoff; $NR^{10}R^{11}$; $PO(OR^{10})(OR^{11})$; $(CH_2)q$-CO-O-$R^{10}$ (mit q = 0, 1, 2, 3); Cyano; $CONR^{10}R^{11}$; $SO_2NR^{10}R^{11}$;
Tri-$C_1$-$C_4$alkyl-silyl; bis zu zweifach durch $R^{10}$ und $R^{11}$ substituiertes Hetaryl worin Hetaryl für Pyrazolyl-, Pyrazinyl-, Imidazolyl-,1,2,4-Triazolyl-, Morpholinyl-, Piperidinyl- Pyrrolyl-, Pyrrolidinyl-,2,5-Dioxopyrrolidinyl-, 1,3-Isoindoldionyl- und Pyridinylradikale, sowie von Indol, Benzofuran, Chinolin oder Benzothiophen abgeleitete Reste, steht oder

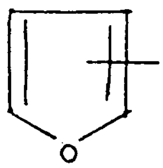 , 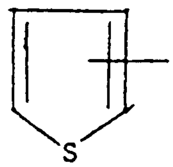 , 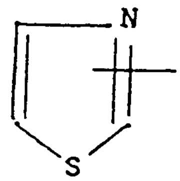 , 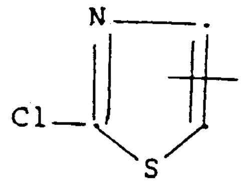

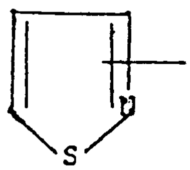 , 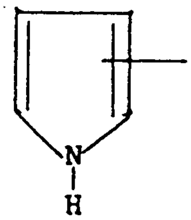 , 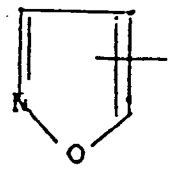 , 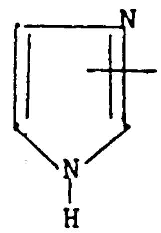

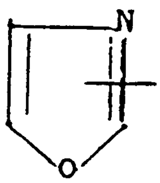 , 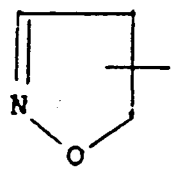 , 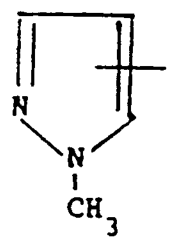 , 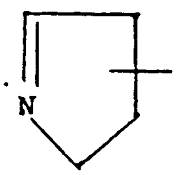

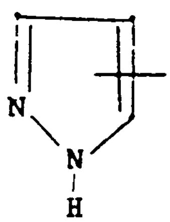 , 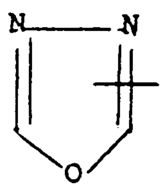 , 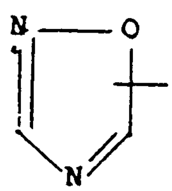 , 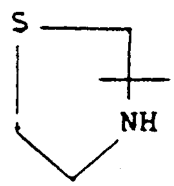

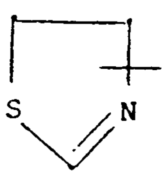 , 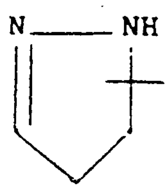 , 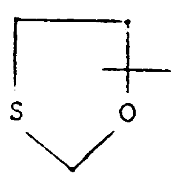 , 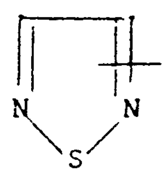

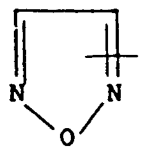 , 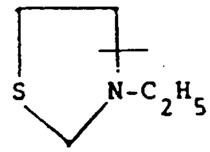 , 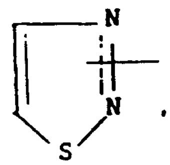 , 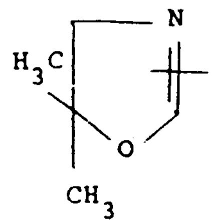

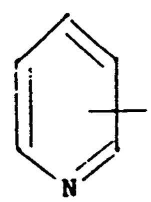 , 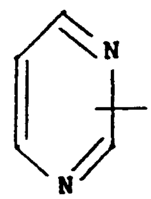 , 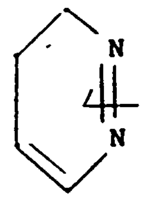 , 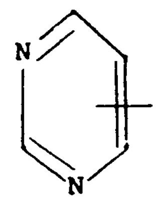

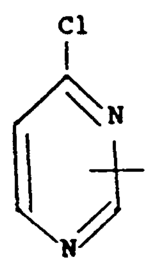 , 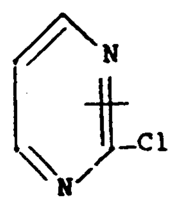 , 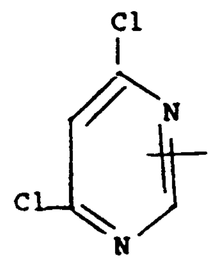 , 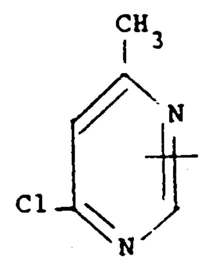

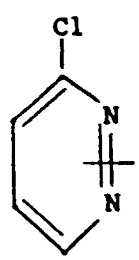 , 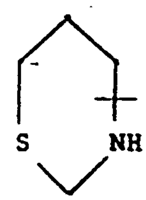 , 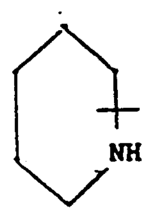 , 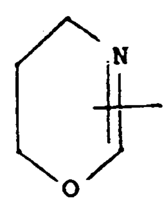

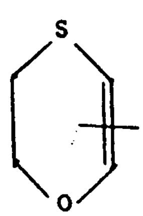 , 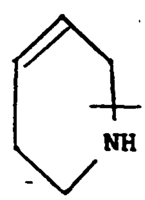 , 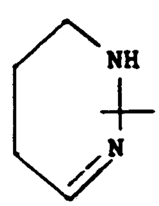 , 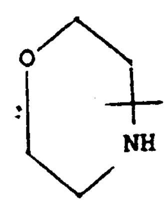

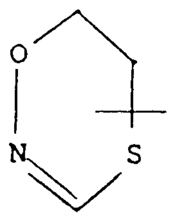 , 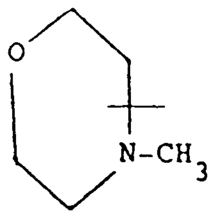 , 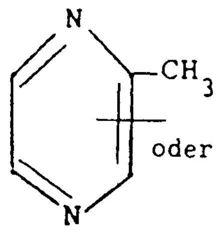 oder 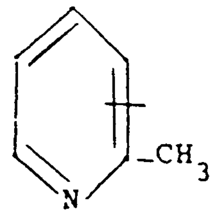

steht;

[(1-Formylamino-2,2,2-trichlor)ethyl]amino;

1-(3,4-Dimethoxyphenyl)-3-(morpholin-4-yl)-prop-1-en-3-on;

Phenyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert; Alkyl mit bis zu 12 C-Atomen gegebenenfalls durch Sauerstoff und/oder Schwefelatome unterbrochen und

gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, Cycloalkyl gegebenenfalls durch Sauerstoff und/oder Schwefelatome und/oder Stickstoff unterbrochen und gegebenenfalls bis zu dreifach durch Alkyl, Halogen, Hydroxy, Oxo oder Amino substituiert, phenylsubstituiertes $C_1$-$C_3$-Alkyl, $C_2$-$C_6$-Alkenyl, $C_2$-$C_6$-Alkinyl, $C_5$-$C_8$-Cycloalkenyl oder Naphthyl steht,

wenn X - Y für eine Einfachbindung steht, können $R^7$ and $R^6$ außerdem gemeinsam für eine vicinale Brücke der Formel

$-(CH_2)_n$-E- (mit n = 2, 3), -(CH=CH)-D oder

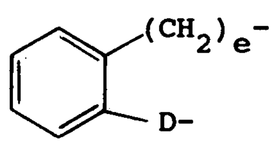

(mit e = 0 oder 1)

stehen;

wobei

E für $CH_2$; O; S oder $NR^{10}$

D für $CH_2$; O; S; $NR^{10}$; $-CH_2$-$CH_2$- oder

-CH=CH- steht, und gegebenenfalls ihre Salze; worin Alkyl $C_1$-$C_{12}$-C-Atome bedeutet;

und die Verbindungen:-

3-(3,4-Dimethoxyphenyl)-3-[4-(4,5-dihydrooxazol-2-yl)phenyl]-acrylsäuremorpholid,

3-(3,4-Dimethoxyphenyl)-3-(4-benzimidazol-2-yl-phenyl)-acrylsäuremorpholid,

3-(3,4-Dimethoxyphenyl)-3-[4-(1,2-dichlorvinyloxy)-phenyl]-acrylsäuremorpholid,

3-(3,4-Dimethoxyphenyl)-3-(4-benzothiazol-2-ylthiomethylphenyl)-acrylsäuremorpholid,

3-(3,4-Dimethoxyphenyl)-3-(4-benzothiazol-2-ylthiophenyl)-acrylsäuremorpholid,

3-(3,4-Dimethoxyphenyl)-3-[4-(3-p-toluol-sulfonylureido)-phenyl]-acrylsäuremorpholid,

3-(3,4-Dimethoxyphenyl)-3-[4-(4,6-dichlor-1,3,5-triazin-2-yloxy)-phenyl]-acrylsäuremorpholid,

3-(3,4-Dimethoxyphenyl)-3-[4-(2,5-dioxopyrrol-1-yl)-phenyl]-acrylsäuremorpholid,

3-(3,4-Dimethoxyphenyl)-3-[4-(6-chlor-2-ethylaminopyrimidin-4-yloxy)-phenyl]-acrylsäuremorpholid,

3-(3,4-Dimethoxyphenyl)-3-[4-(6-chlor-2-dimethylaminopyrimidin-4-yloxy)-phenyl]-acrylsäuremorpholid,

3-(3,4-Dimethoxyphenyl)-3-[4-(6-chlor-2-isopropylaminopyrimidin-4-yloxy)-phenyl]-acrylsäuremorpholid;

mit Ausnahme von dem Gegenstand der in EP-A-0120321 offenbart ist und mit Ausnahme von den Verbindungen der Formel I in der A und B beide für einen Rest aus der Gruppe Phenyl, Monohalogenphenyl, Mononitrophenyl und Monoaminophenyl stehen.

**2.** Verbindungen der Formel I nach Anspruch 1

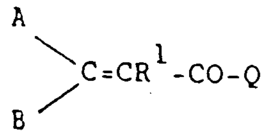

(I)

in der

A für

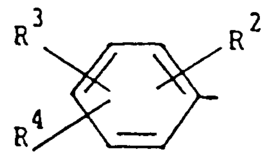

B für

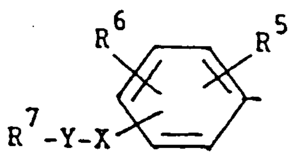
,
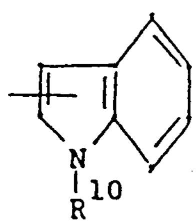
,
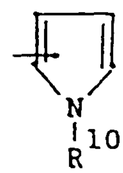
,
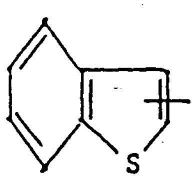
,
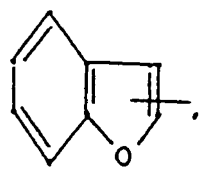
,
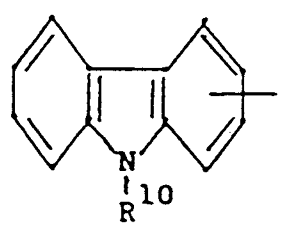

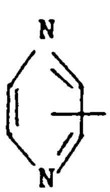
,
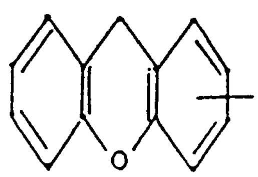
oder
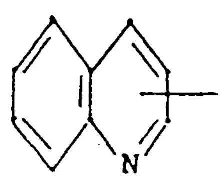

$R^1$ für Wasserstoff; Halogen, Cyano oder $C_1$-$C_4$-Alkyl gegebenenfalls ein oder mehrfach mit Hydroxy, Alkoxy, Mercapto, Halogen, Alkylthio, Nitro, Cyano, Amino oder Mono- oder Di-$C_1$-$C_4$-alkylamino substituiert, steht,

Q für

$NR^8R^9$ oder

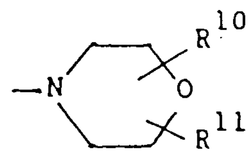

steht,

$R^2$, $R^3$, $R^4$, $R^5$ und $R^6$, die gleich oder verschieden sein können für Wasserstoff; Halogen, Nitro; Cyano; Carboxy; Hydroxy; $C_1$-$C_4$-Alkoxycarbonyl; $CONR^{10}R^{11}$; $NR^{10}R^{11}$; $NR^{10}$-$CO$-$R^{11}$; oder für $C_1$-$C_6$-Alkyl, $C_1$-$C_4$-Alkoxy oder $C_1$-$C_4$-Alkyl-S(O)p (mit p = 0, 1, 2), gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl, Phenoxy oder Phenyl-S(O)p-(mit p = 0, 1, 2) gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^8$ und $R^9$, die gleich oder verschieden sein können für Wasserstoff; $C_1$-$C_4$-Alkyl, $C_3$-$C_7$-Alkenyl, $C_3$-$C_7$-Alkinyl oder Alkoxyalkyl gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl oder Benzyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^{10}$ und $R^{11}$, die gleich oder verschieden sein können, für Wasserstoff, Benzyl, Phenyl oder $C_1$-$C_4$-Alkyl, als Substituenten in Hetaryl auch für Halogen, Amino oder Mono- oder Di-$C_1$-$C_4$-alkylamino stehen, oder gemeinsam für $-(CH_2)_4-$ oder $-(CH_2)_5-$,

X - Y für eine Einfachbndung; -O-; -S(O)p- (mit p = 0, 1, 2); $-CONR^{10}-$; $-NR^{10}CO-$; $-NR^{11}CONR^{10}-$; -N=N-; $-CHR^{10}$-S(O)p-(mit p = 0, 1, 2); $-CHR^{10}O-$; $-SO_2NR^{10}-$; -N=CH-; $-C_nH_{2n}-$ (mit n = 1 bis 10); -CH=CH-; $-NR^{10}CSNR^{11}-$; $-NR^{10}-$; $-R^{10}N$-$CHR^{11}-$; $-CHR^{10}NR^{11}-$; $-O-CHR^{10}-$; $-S(O)p-CHR^{10}-$ (mit p = 0, 1, 2); $-NR^{10}SO_2-$ oder $-OSO_2-$; $-O_2SO-$; $-O_2S-NR^{10}-$; -C≡C-; -S-S-; -CHOH-; -CO-;
$>C=CH_2$; $>C=CH-COOH$; -NH-NH-; -N(O)=N-;

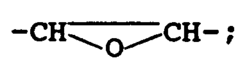

$-N(R^{10})-N=CH-$; $-N(R^{10})-NH-CO-$; -NH-CH=CH-; $>CH-C_6H_5$; $-COCH_2O-$; $-CO-CH_2S-$; $-COCH_2NR^{10}-$; $-OCH_2CO$; $-SCH_2CO-$; -CH=N-; $-NR^{10}CH_2CO-$; -CH=NO-; $-CH_2O-CONR^{10}-$; $-CH_2O-CO-$; $-S-CH_2-$; $-CH_2CO-$; -CO-O-; -O-CO-; -CO-S- oder -S-CO- steht;

$R^7$ für Wasserstoff; $NR^{10}R^{11}$; $PO(OR^{10})(OR^{11})$; $(CH_2)q-CO-O-R^{10}$ (mit q = 0, 1, 2, 3); Cyano; $CONR^{10}R^{11}$; $SO_2NR^{10}R^{11}$; Tri-$C_1$-$C_4$-alkyl-silyl; bis zu zweifach durch $R^{10}$ und $R^{11}$ substituiertes Hetaryl worin Hetaryl für Pyrazolyl-, Pyrazinyl-, Imidazolyl-,1,2,4-Triazolyl-, Morpholinyl-, Piperidinyl- Pyrrolyl-, Pyrrolidinyl-2,5-Dioxopyrrolidinyl-, 1,3-Isoindoldionyl- und Pyridinylradikale, sowie von Indol, Benzofuran, Chinolin oder Benzothiophen abgeleitete Reste, steht oder

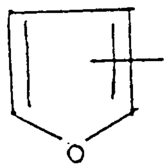 , 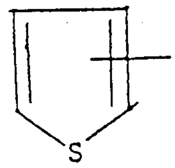 , 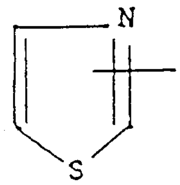 , 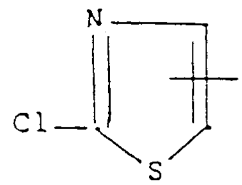

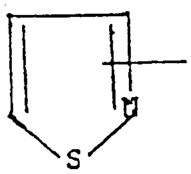 , 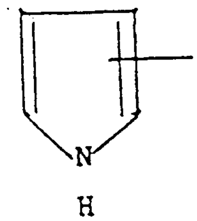 , 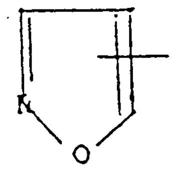 , 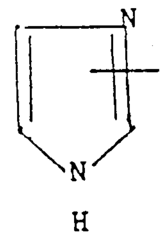

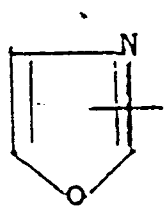 , 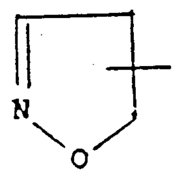 , 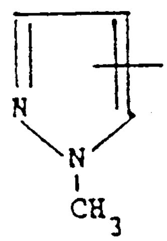 , 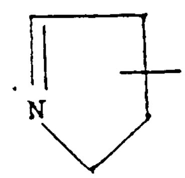

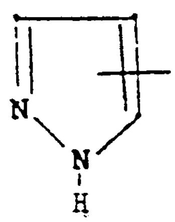 , 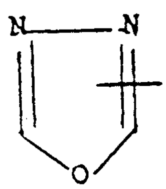 , 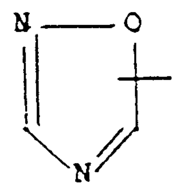 , 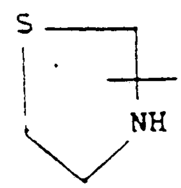

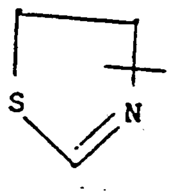 , 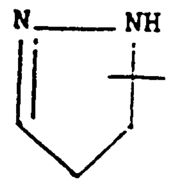 , 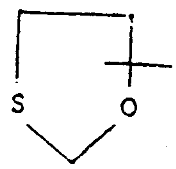 , 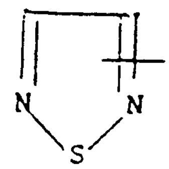

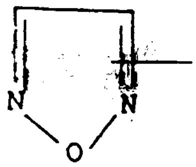 , 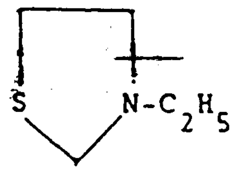 . 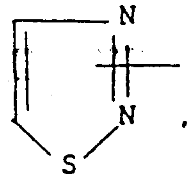 , 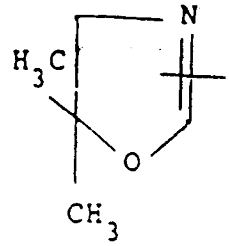

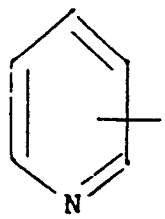 , 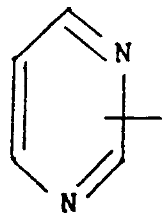 , 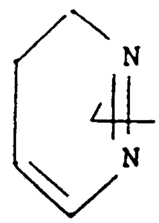 , 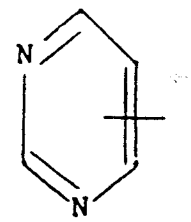

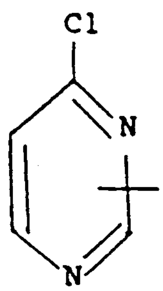 , 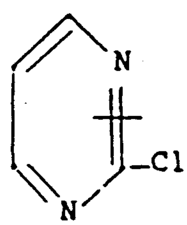 , 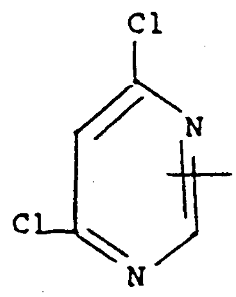 , 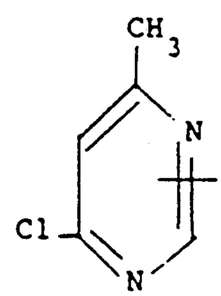

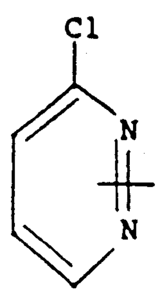 , 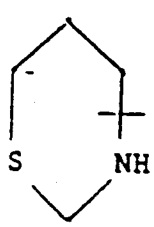 , 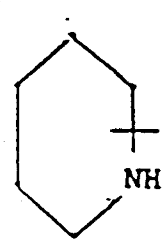 , 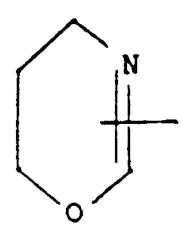

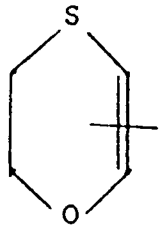 , 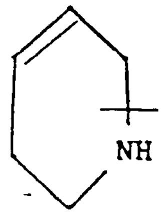 , 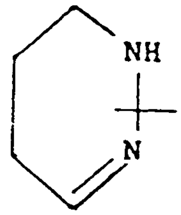 , 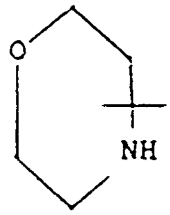

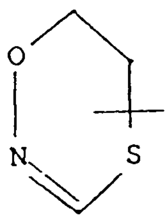 , 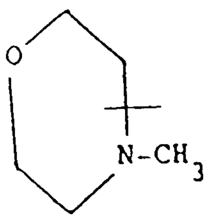 , 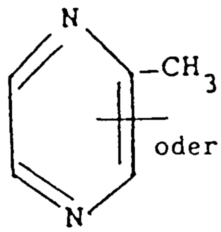 oder 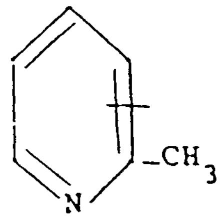

steht;

[(1-Formylamino-2,2,2-trichlor)ethyl]amino;

1-(3,4-Dimethoxyphenyl)-3-(morpholin-4-yl)-prop-1-en-3-on;

Phenyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert; Alkyl mit bis zu 12 C-Atomen gegebenenfalls durch Sauerstoff und/oder Schwefelatome unterbrochen und

gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, Cycloalkyl gegebenenfalls durch Sauerstoff und/oder Schwefelatome und/oder Stickstoff unterbrochen und gegebenenfalls bis zu dreifach durch Alkyl, Halogen, Hydroxy, Oxo oder Amino substituiert, phenylsubstituiertes $C_1$-$C_3$-Alkyl, $C_2$-$C_6$-Alkenyl, $C_2$-$C_6$-Alkinyl, $C_5$-$C_8$-Cycloalkenyl oder Naphthyl steht,

wenn X - Y für eine Einfachbindung steht, können $R^7$ and $R^6$ außerdem gemeinsam für eine vicinale Brücke der Formel

$-(CH_2)_n$-E- (mit n = 2, 3), -(CH = CH)-D oder

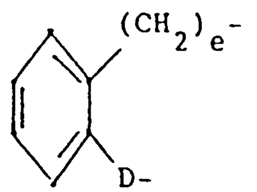

(mit e = O oder 1)

stehen;

wobei

E für $CH_2$; O; S oder $NR^{10}$

D für $CH_2$; O; S; $NR^{10}$; $-CH_2-CH_2-$ oder

-CH = CH- steht, und gegebenenfalls ihre Salze; worin Alkyl $C_1$-$C_{12}$-C-Atome bedeutet;

und die Verbindungen:-

3-(3,4-Dimethoxyphenyl)-3-[4-(4,5-dihydrooxazol-2-yl)phenyl]-acrylsäuremorpholid,

3-(3,4-Dimethoxyphenyl)-3-(4-benzimidazol-2-yl-phenyl)-acrylsäuremorpholid,

3-(3,4-Dimethoxyphenyl)-3-[4-(1,2-dichlorvinyloxy)-phenyl]-acrylsäuremorpholid,

3-(3,4-Dimethoxyphenyl)-3-(4-benzothiazol-2-ylthiomethylphenyl)-acrylsäuremorpholid,

3-(3,4-Dimethoxyphenyl)-3-(4-benzothiazol-2-ylthiophenyl)-acrylsäuremorpholid,

3-(3,4-Dimethoxyphenyl)-3-[4-(3-p-toluol-sulfonylureido)-phenyl]-acrylsäuremorpholid,

3-(3,4-Dimethoxyphenyl)-3-[4-(4,6-dichlor-1,3,5-triazin-2-yloxy)-phenyl]-acrylsäuremorpholid,

3-(3,4-Dimethoxyphenyl)-3-[4-(2,5-dioxopyrrol-1-yl)-phenyl]-acrylsäuremorpholid,

3-(3,4-Dimethoxyphenyl)-3-[4-(6-chlor-2-ethylaminopyrimidin-4-yloxy)-phenyl]-acrylsäuremorpholid,

3-(3,4-Dimethoxyphenyl)-3-[4-(6-chlor-2-dimethylaminopyrimidin-4-yloxy)-phenyl]-acrylsäuremorpholid,

3-(3,4-Dimethoxyphenyl)-3-[4-(6-chlor-2-isopropylaminopyrimidin-4-yloxy)-phenyl]-acrylsäuremorpholid;

mit Ausnahme von den Verbindungen der Formel I in der

B für

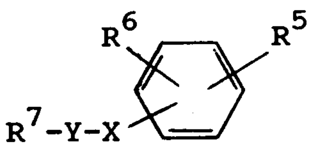

steht;

$R^1$ für Wasserstoff, $C_1$-$C_4$-Alkyl, Cyano, Chlor, Brom oder Jod steht;

$R^2$, $R^3$ und $R^4$, die gleich oder verschieden sein können, für Wasserstoff, Halogen, Nitro, gegebenenfalls ein- oder mehrfach durch Fluor oder Chlor substituiertes $C_1$-$C_4$-Alkyl oder -Alkoxy, Amino, NH($C_1$-$C_4$-Alkyl), N($C_1$-$C_4$-Alkyl)$_2$, Cyano, Phenyl, $C_3$-$C_6$-Cycloalkyl, Hydroxy ($C_1$-$C_4$-Alkyl), NHCOH, NHCO($C_1$-$C_4$ Alkyl), $CO_2H$, $CO_2$($C_1$-$C_4$-Alkyl), $CONR^{10}R^{11}$ in der $R^{10}$ und $R^{11}$, die gleich oder verschieden sein können, für $C_1$-$C_4$-Alkyl, Phenyl oder Benzyl, gemeinsam außerdem für $-(CH_2)_4-$ oder $-(CH_2)_5-$, und $R^{10}$ außerdem für Wasserstoff, stehen, ($C_1$-$C_4$-Alkyl)-S(O)$_p$ (mit p = 0,1,2) oder Hydroxy steht;

$R^5$ und $R^6$, die gleich oder verschieden sein können, für Wasserstoff, Halogen, $C_1$-$C_4$-Alkyl, $C_1$-$C_4$-Alkoxy, ($C_1$-$C_4$-Alkyl)-S(O)$_p$ (mit p = 0,1,2) oder Hydroxy stehen, und $R^5$ außerdem für Nitro, ein- oder mehrfach durch Fluor oder Chlor substituiertes $C_1$-$C_4$-Alkyl oder -Alkoxy, NHCOH oder NHCO($C_1$-$C_4$-Alkyl) steht;

X-Y für eine Einfachbindung steht;

$R^7$ für Wasserstoff, gegebenenfalls ein- oder mehrfach durch Fluor oder Chlor substituiertes $C_1$-$C_4$-Alkyl, Amino, NH($C_1$-$C_4$-Alkyl), N($C_1$-$C_4$-Alkyl)$_2$, Cyano, Phenyl, $C_3$-$C_6$-Cycloalkyl, Hydroxy($C_1$-$C_4$-Alkyl), $CO_2H$, $CO_2$($C_1$-$C_4$-Alkyl), $CONR^{10}R^{11}$ in der $R^{10}$ und $R^{11}$, die gleich oder verschieden sein können, für $C_1$-$C_4$-Alkyl, Phenyl oder Benzyl, gemeinsam außerdem für $-(CH_2)_4-$ oder $-(CH_2)_5-$, und $R^{10}$ außerdem für Wasserstoff, stehen, durch Sauerstoff unterbrochenes $C_2$-$C_8$-Alkyl steht; oder $R^7$ und $R^6$ außerdem gemeinsam für eine vicinale Brücke der Formel -(CH = CH)-D stehen können, wobei D für -CH = CH- steht;

Q für $NR^8R^9$ oder

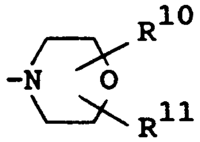

steht, in der

$R^8$ und $R^9$, die gleich oder verschieden sein können, für $C_1$-$C_4$-Alkyl, $C_3$-$C_7$-Cycloalkyl, Phenyl, Benzyl oder $C_3$-$C_4$-Alkenyl, und $R^8$ außerdem Wasserstoff, stehen, und

$R^{10}$ und $R^{11}$, die gleich oder verschieden sein können, für Wasserstoff oder $C_1$-$C_4$-Alkyl stehen;

und mit Ausnahme von den Verbindungen der Formel I in der A und B beide für einen Rest aus der Gruppe Phenyl, Monohalogenphenyl, Mononitrophenyl und Monoaminophenyl stehen.

**3.** Verbindungen der Formel I, nach Auspruch 1 oder 2 worin A für 3,4-Dimethoxyphenyl, 3-Ethoxy-4-methoxyphenyl, 3-Chlor-4-methoxyphenyl, 3-Methyl-4-methoxyphenyl, 3-Brom-4-methoxyphenyl, 3-Ethyl-4-methoxyphenyl, 3,4-Dimethylphenyl, 3,5-Dichlor-4-Aminophenyl steht und B, Q und $R^1$ die obige Bedeutung haben.

**4.** Verbindungen nach Anspruch 3, worin $R^1$ Wasserstoff bedeutet.

**5.** Verbindungen nach Anspruch 1, 2, 3 oder 4, worin Q für $NR^8R^9$, worin $R^8$ und $R^9$ $C_1$-$C_4$-Alkyl ist, oder für die Morpholinogruppe steht.

**6.** Verbindungen nach Anspruch 1, 2, 3, 4 oder 5, worin
B für

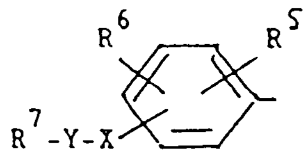

steht, worin -X-Y- eine Einfachbindung, Sauerstoff, Schwefel, $-CH_2O-$, $-OCH_2-$ oder -N=N- bedeutet.

**7.** Verbindungen nach Anspruch 6, worin $R^5$ und $R^6$ Wasserstoff bedeuten, -X-Y- eine Einfachbindung, Sauerstoff, Schwefel oder -N=N- darstellt und $R^7$ für ein gegebenenfalls substituiertes Phenyl, gegebenenfalls substituiertes $C_1$-$C_{12}$-Alkyl, gegebenenfalls durch Sauerstoff und/oder Schwefel und/oder $NR^{10}$ ein- oder mehrfach unterbrochenes gegebenenfalls hydroxy- oder $C_1$-$C_4$-alkylsubstituiertes $C_3$-$C_7$-Cycloalkyl oder bis zu zweifach durch $R^{10}$ und $R^{11}$ substituiertes Hetaryl steht.

**8.** 3-[4-(4-Chlorphenoxy)-phenyl]-3-(3,4-dimethoxyphenyl)-acrylsäuremorpholid.

**9.** 3-(4-n-Butylphenyl)-phenyl)-3-(3,4-dimethoxyphenyl)-acrylsäuremorpholid.

**10.** 3-[4-(4-Chlorophenyl)-phenyl]-3-(3,4-dimethoxyphenyl)-acrylsäure-methyl-ethylamid.

**11.** Fungizide Mittel, gekennzeichnet durch einen Gehalt an einer Verbindung nach Anspruch 1 bis 10 neben üblichen Hilfs- und/oder Trägerstoffen und gegebenenfalls einem oder mehreren zusätzlichen Wirkstoffen.

**12.** Verwendung von Verbindungen nach einem der Ansprüche 1 bis 10 gegebenenfalls in Kombination mit einem oder mehreren weiteren fungiziden Wirkstoffen zur Bekämpfung phytopathogener Pilze, insbesondere des falschen Mehltaus.

**13.** Verfahren zur Herstellung von Verbindungen nach Anspruch 1 bis 10 nach an sich bekannten Methoden, dadurch gekennzeichnet, dass man
a) eine Acrylsäure der Formel

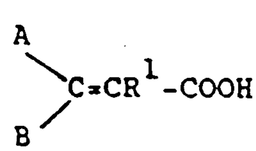

(II)

worin A, B und $R^1$ die obige Bedeutung haben, oder ein reaktionsfähiges Derivat, das gegebenenfalls in situ hergestellt wird, mit einem Amin der Formel

HQ III

worin Q die obige Bedeutung hat, umsetzt
oder daß man

b) ein Keton der Formel

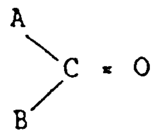

(IV),

worin A und B die obige Bedeutung haben, mit einem Phosphonessigsäurederivat der Formel

$(R'O)_2P(O)\text{-}CHR^1\text{-}COQ$ (V),

worin Q und $R^1$ die obige Bedeutung haben und R' für eine $C_1$-$C_4$-Alkylgruppe steht, umsetzt oder daß man

c) in einem Acrylsäureamid der Formel

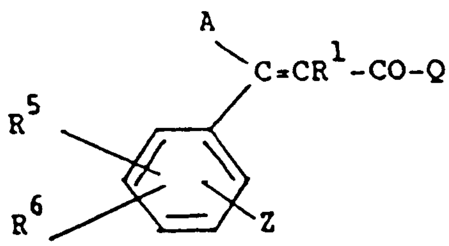

XI)

worin A, Q, $R^1$, $R^5$ und $R^6$ die obige Bedeutung haben und Z für Wasserstoff oder für einen Substituenten steht, der durch den Rest -XY-$R^7$ ersetzt oder in diesen Rest umgewandelt werden kann, eine solche Substitution oder Umwandlung vornimmt.

oder daß man

d) durch die Heck-Reaktion von Verbindungen der Formel

B-CH = CH-COQ (XIVa)

oder

A-CH = CH-COQ (XIVb),

worin A, B und Q die obige Bedeutung haben, jedoch nicht durch Brom oder Jod substituiert sind, mit Verbindungen der Formel

A - Hal (XVa)

oder

B - Hal (XVb),

worin Hal Brom oder Jod bedeutet und A und B die obige Bedeutung haben, jedoch keine zusätzlichen Brom- oder Jodatome enthalten, in Gegenwart von Palladium(O) den Rest A oder B einführt

und daß man nach (a) bis (d) erhaltene Verbindungen gegebenenfalls einer Isomerentrennung unterwirft und/oder solche erhaltene Verbindungen, die zur Salzbildung befähigt sind, gegebenenfalls in die Salze überführt.

**Patentansprüche für folgende Vertragsstaaten : AT, ES**

**1.** Verfahren zur Herstellung von Verbindungen der Formel

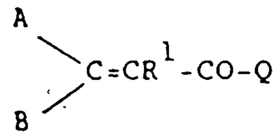

(I)

in der

A für

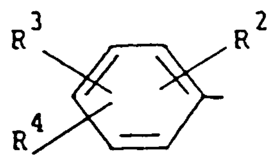

B für

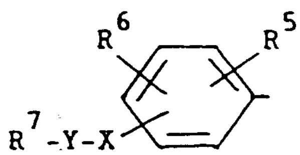
,
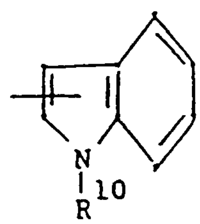
,
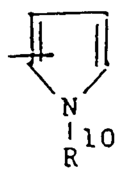

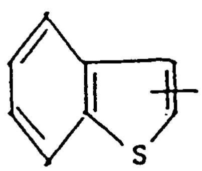
,
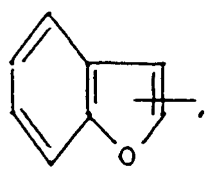
,
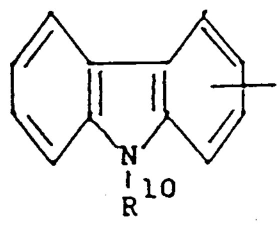

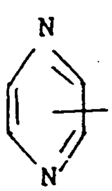
,
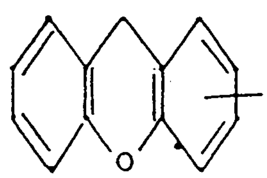
oder
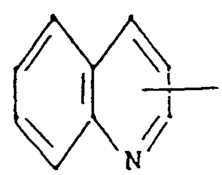

$R^1$ für Wasserstoff; Halogen, Cyano oder $C_1$-$C_4$-Alkyl gegebenenfalls ein oder mehrfach mit Hydroxy, Alkoxy, Mercapto, Halogen, Alkylthio, Nitro, Cyano, Amino oder Mono- oder Di-$C_1$-$C_4$-alkylamino substituiert, steht,

Q für

$NR^8R^9$ oder

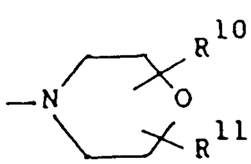

steht,

$R^2$, $R^3$, $R^4$, $R^5$ und $R^6$, die gleich oder verschieden sein können für Wasserstoff; Halogen, Nitro; Cyano; Carboxy; Hydroxy;
$C_1$-$C_4$-Alkoxycarbonyl; $CONR^{10}R^{11}$;
$NR^{10}R^{11}$; $NR^{10}$-CO-$R^{11}$;
oder für $C_1$-$C_6$-Alkyl, $C_1$-$C_4$-Alkoxy oder $C_1$-$C_4$-Alkyl-S(O)p (mit p = 0, 1, 2), gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl, Phenoxy oder Phenyl-S(O)p-(mit p = 0, 1, 2) gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^8$ und $R^9$, die gleich oder verschieden sein können für Wasserstoff;
$C_1$-$C_4$-Alkyl, $C_3$-$C_7$-Alkenyl, $C_3$-$C_7$-Alkinyl oder Alkoxyalkyl gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydrory, Oxo oder Amino substituiert, Phenyl oder Benzyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^{10}$ und $R^{11}$, die gleich oder verschieden sein können, für Wasserstoff, Benzyl, Phenyl oder $C_1$-$C_4$-Alkyl, als Substituenten in Hetaryl auch für Halogen, Amino oder Mono- oder Di-$C_1$-$C_4$alkylamino stehen, oder gemeinsam für $-(CH_2)_4-$ oder $-(CH_2)_5-$,

X - Y für eine Einfachbindung;
-O-; -S(O)p- (mit p = 0, 1, 2); -$CONR^{10}$-; -$NR^{10}$CO-; -$NR^{11}CONR^{10}$-; -N=N-; -$CHR^{10}$-S(O)p-(mit p = 0, 1, 2); -$CHR^{10}$O-; -$SO_2NR^{10}$-; -N=CH-; $-C_nH_{2n}-$ (mit n = 1 bis 10); -CH=CH-; -$NR^{10}CSNR^{11}$-; -$NR^{10}$-; -$R^{10}$N-$CHR^{11}$-; -$CHR^{10}NR^{11}$-; -O-$CHR^{10}$-; -S(O)p-$CHR^{10}$- (mit p = 0, 1, 2); -$NR^{10}SO_2$- oder -$OSO_2$-; -$O_2$SO-; -$O_2$S-$NR^{10}$-; -C≡C-; -S-S-; -CHOH-; -CO-;
$>C=CH_2$; $>C=CH$-COOH; -NH-NH-; -N(O)=N-;

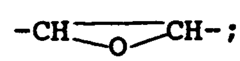

-N($R^{10}$)-N=CH-; -N($R^{10}$)-NH-CO-; -NH-CH=CH-; $>CH-C_6H_5$; -$COCH_2$O-; -CO-$CH_2$S-; -$COCH_2NR^{10}$-; -$OCH_2$CO; -$SCH_2$CO-; -CH=N-; -$NR^{10}CH_2$CO-; -CH=NO-; -$CH_2$O-$CONR^{10}$-; -$CH_2$O-CO-; -S-$CH_2$-; -$CH_2$CO-; -CO-O-; -O-CO-; -CO-S- oder -S-CO- steht;

$R^7$ für Wasserstoff; $NR^{10}R^{11}$; PO($OR^{10}$)($OR^{11}$); $(CH_2)$q-CO-O-$R^{10}$ (mit q = 0, 1, 2, 3); Cyano; $CONR^{10}R^{11}$; $SO_2NR^{10}R^{11}$;
Tri-$C_1$-$C_4$alkyl-silyl; bis zu zweifach durch $R^{10}$ und $R^{11}$ substituiertes Hetaryl worin Hetaryl für Pyrazolyl-, Pyrazinyl-, Imidazolyl-,1,2,4-Triazolyl-, Morpholinyl-, Piperidinyl- Pyrrolyl-, Pyrrolidinyl-,2,5-Dioxopyrrolidinyl-, 1,3-Isoindoldionyl- und Pyridinylradikale, sowie von Indol, Benzofuran, Chinolin oder Benzothiophen abgeleitete Reste, steht oder

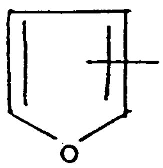 , 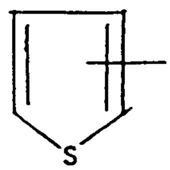 , 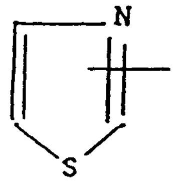 , 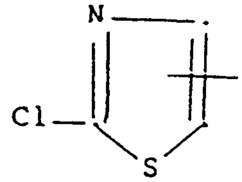

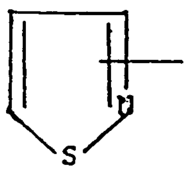 , 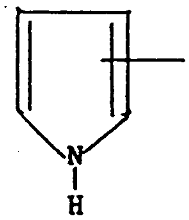 , 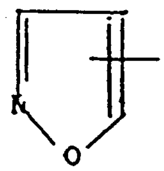 , 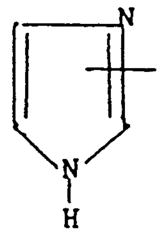

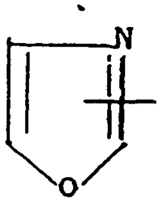 , 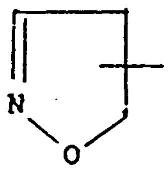 , 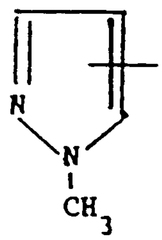 , 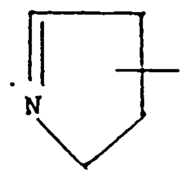

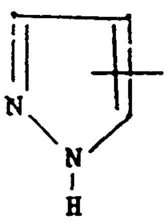 , 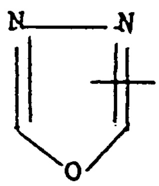 , 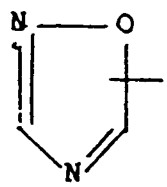 , 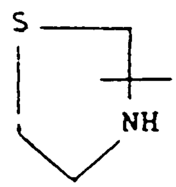

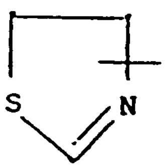 , 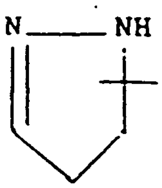 , 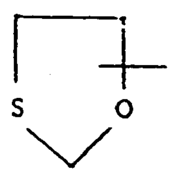 , 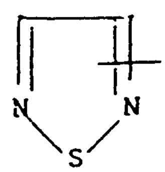

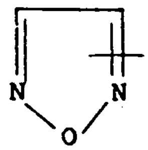 , 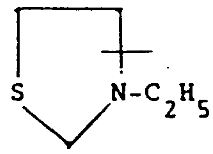 , 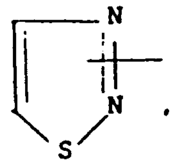 , 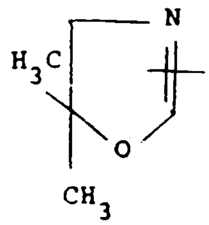

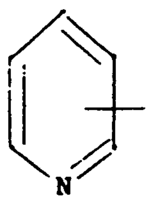 , 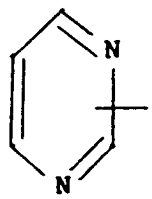 , 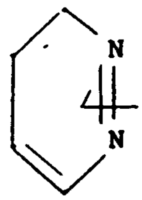 , 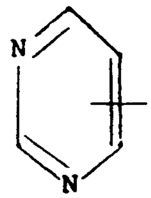

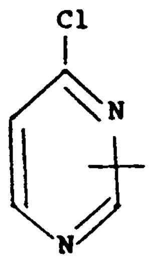 , 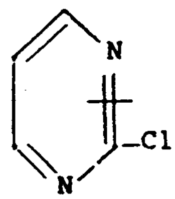 , 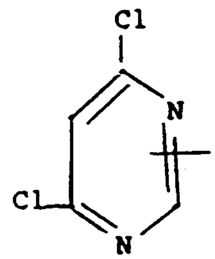 , 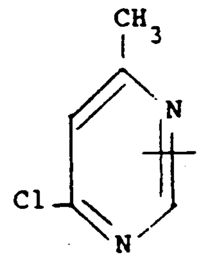

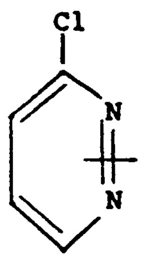 , 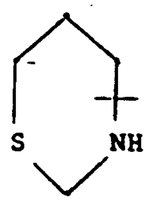 , 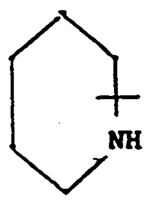 , 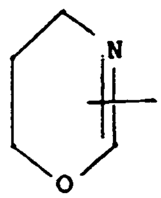

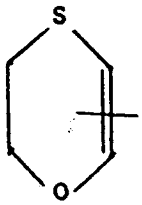 , 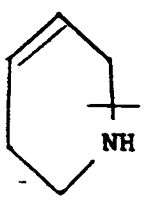 , 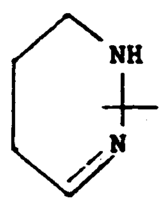 , 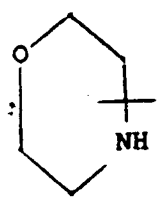

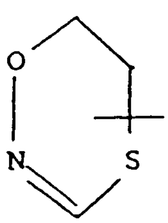 , 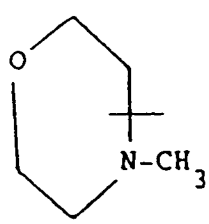 , 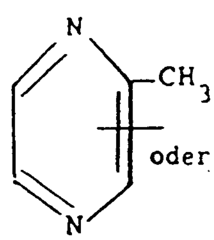 oder 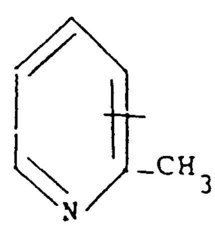

steht;

[(1-Formylamino-2,2,2-trichlor)ethyl]amino;

1-(3,4-Dimethoxyphenyl)-3-(morpholin-4-yl)-prop-1-en-3-on;

Phenyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert; Alkyl mit bis zu 12 C-Atomen gegebenenfalls durch Sauerstoff und/oder Schwefelatome unterbrochen und

gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, Cycloalkyl gegebenenfalls durch Sauerstoff und/oder Schwefelatome und/oder Stickstoff unterbrochen und gegebenenfalls bis zu dreifach durch Alkyl, Halogen, Hydroxy, Oxo oder Amino substituiert, phenylsubstituiertes $C_1$-$C_3$-Alkyl, $C_2$-$C_6$-Alkenyl, $C_2$-$C_6$-Alkinyl, $C_5$-$C_8$-Cycloalkenyl oder Naphthyl steht,

wenn X - Y für eine Einfachbindung steht, können $R^7$ and $R^6$ außerdem gemeinsam für eine vicinale Brücke der Formel

$-(CH_2)_n$-E- (mit n = 2, 3), -(CH = CH)-D oder

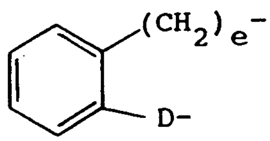

(mit e = 0 oder 1)

stehen;

wobei

E für $CH_2$; O; S oder $NR^{10}$

D für $CH_2$; O; S; $NR^{10}$; $-CH_2-CH_2-$ oder -CH = CH- steht, und gegebenenfalls ihre Salze; worin Alkyl $C_1$-$C_{12}$-C-Atome bedeutet;

und die Verbindungen:-

3-(3,4-Dimethoxyphenyl)-3-[4-(4,5-dihydrooxazol-2-yl)phenyl]-acrylsäuremorpholid,

3-(3,4-Dimethoxyphenyl)-3-(4-benzimidazol-2-yl-phenyl)-acrylsäuremorpholid,

3-(3,4-Dimethoxyphenyl)-3-[4-(1,2-dichlorvinyloxy)-phenyl]-acrylsäuremorpholid,

3-(3,4-Dimethoxyphenyl)-3-(4-benzothiazol-2-ylthiomethylphenyl)-acrylsäuremorpholid,

3-(3,4-Dimethoxyphenyl)-3-(4-benzothiazol-2-ylthiophenyl)-acrylsäuremorpholid,

3-(3,4-Dimethoxyphenyl)-3-[4-(3-p-toluol-sulfonylureido)-phenyl]-acrylsäuremorpholid,

3-(3,4-Dimethoxyphenyl)-3-[4-(4,6-dichlor-1,3,5-triazin-2-yloxy)-phenyl]-acrylsäuremorpholid,

3-(3,4-Dimethoxyphenyl)-3-[4-(2,5-dioxopyrrol-1-yl)-phenyl]-acrylsäuremorpholid,

3-(3,4-Dimethoxyphenyl)-3-[4-(6-chlor-2-ethylaminopyrimidin-4-yloxy)-phenyl]-acrylsäuremorpholid,

3-(3,4-Dimethoxyphenyl)-3-[4-(6-chlor-2-dimethylaminopyrimidin-4-yloxy)-phenyl]-acrylsäuremorpholid,

3-(3,4-Dimethoxyphenyl)-3-[4-(6-chlor-2-isopropylaminopyrimidin-4-yloxy)-phenyl]-acrylsäuremorpholid; mit Ausnahme von dem Gegenstand der in EP-A-0120321 offenbart ist und mit Ausnahme von den Verbindungen der Formel I in der A und B beide für einen Rest aus der Gruppe Phenyl, Monohalogenphenyl, Mononitrophenyl und Monoaminophenyl stehen, nach an sich bekannten Methoden, dadurch gekennzeichnet, dass man

a) eine Acrylsäure der Formel

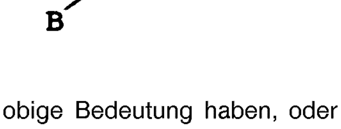

(II)

worin A, B und $R^1$ die obige Bedeutung haben, oder ein reaktionsfähiges Derivat, das gegebenenfalls in situ hergestellt wird, mit einem Amin der Formel

HQ III

worin Q die obige Bedeutung hat, umsetzt oder dass man
b) ein Keton der Formel

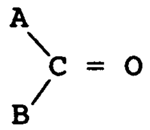

(IV)

worin A und B die obige Bedeutung haben, mit einem Phosphonessigsäurederivat der Formel

$(R'O)_2P(O)$-$CHR^1$-COQ (V)

worin Q und $R^1$ die obige Bedeutung haben und R' für eine $C_1$-$C_4$-Alkylgruppe steht, umsetzt oder dass man
c) in einem Acrylsäureamid der Formel

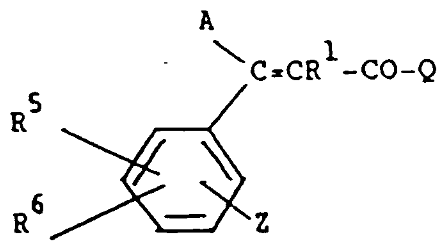

XI)

worin A, Q, $R^1$, $R^5$ und $R^6$ die obige Bedeutung haben und Z für Wasserstoff oder für einen Substituenten steht, der durch den Rest -XY-$R^7$ ersetzt oder in diesen Rest umgewandelt werden kann, eine solche Substitution oder Umwandlung vornimmt.
oder dass man
d) durch die Heck-Reaktion von Verbindungen der Formel

B-CH = CH-COQ (XIVa)

oder

A-CH = CH-COQ (XIVb)

worin A, B, und Q die obige Bedeutung haben, jedoch nicht durch Brom oder Jod substituiert sind, mit Verbindungen der Formel

A - Hal (XVa)

oder

B - Hal (XVb)

worin Hal Brom oder Jod bedeutet und A und B die obige Bedeutung haben, jedoch keine zusätzlichen Brom- oder Jodatome enthalten, in Gegenwart von Palladium(O) den Rest A oder B einführt
und dass man nach (a) bis (d) erhaltene Verbindungen gegebenenfalls einer Isomerentrennung unterwirft und/oder solche erhaltene Verbindungen, die zur Salzbildung befähigt sind, gegebenenfalls in die Salze überführt.

**2.** Verfahren zur Herstellung von Verbindungen der Formel I nach Anspruch 1

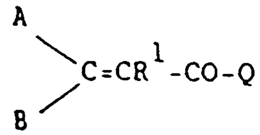 (I)

in der

A für

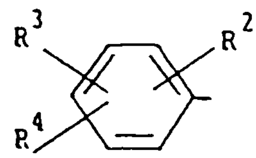

B für

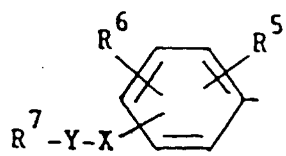 , 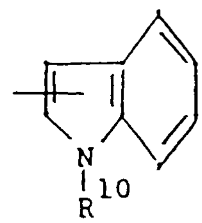 , 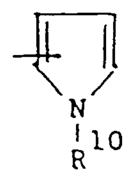 ,

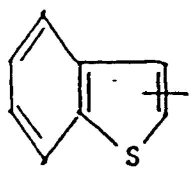 , 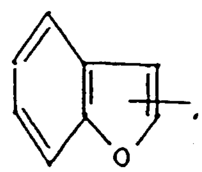 , 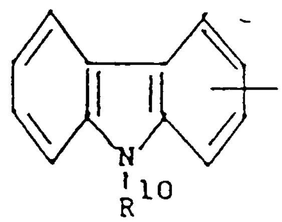

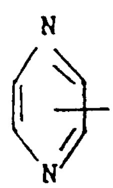 , 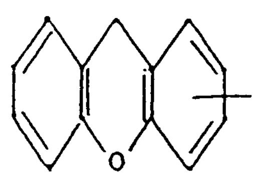 oder 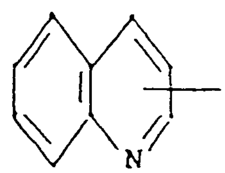

$R^1$ für Wasserstoff; Halogen, Cyano oder $C_1$-$C_4$-Alkyl gegebenenfalls ein oder mehrfach mit Hydroxy, Alkoxy, Mercapto, Halogen, Alkylthio, Nitro, Cyano, Amino oder Mono- oder Di-$C_1$-$C_4$-alkylamino substituiert, steht,

Q für

$NR^8R^9$ oder

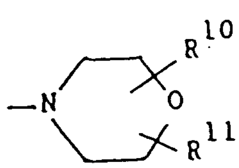

steht,

$R^2$, $R^3$, $R^4$, $R^5$ und $R^6$, die gleich oder verschieden sein können für Wasserstoff; Halogen, Nitro; Cyano; Carboxy; Hydroxy:
$C_1$-$C_4$-Alkoxycarbonyl; $CONR^{10}R^{11}$;
$NR^{10}R^{11}$; $NR^{10}$-CO-$R^{11}$;
oder für $C_1$-$C_6$-Alkyl, $C_1$-$C_4$-Alkoxy oder $C_1$-$C_4$-Alkyl-S(O)p (mit p = 0, 1, 2), gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl, Phenoxy oder Phenyl-S(O)p-(mit p = 0, 1, 2) gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^8$ und $R^9$, die gleich oder verschieden sein können für Wasserstoff;
$C_1$-$C_4$-Alkyl, $C_3$-$C_7$-Alkenyl, $C_3$-$C_7$-Alkinyl oder Alkoxyalkyl gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, $C_3$-$C_7$-Cycloalkyl gegebenenfalls bis zu dreifach durch Alkyl, Halogen, Hydroxy, Oxo oder Amino substituiert, Phenyl oder Benzyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert, stehen,

$R^{10}$ und $R^{11}$, die gleich oder verschieden sein können, für Wasserstoff, Benzyl, Phenyl oder $C_1$-$C_4$-Alkyl, als Substituenten in Hetaryl auch für Halogen, Amino oder Mono- oder Di-$C_1$-$C_4$-alkylamino stehen, oder gemeinsam für $-(CH_2)_4-$ oder $-(CH_2)_5-$,

X - Y für eine Einfachbndung;
-O-; -S(O)p- (mit p = 0, 1, 2); $-CONR^{10}-$; $-NR^{10}CO-$; $-NR^{11}CONR^{10}-$; -N=N-; $-CHR^{10}$-S(O)p-(mit p = 0, 1, 2); $-CHR^{10}O-$; $-SO_2NR^{10}-$; -N=CH-; $-C_nH_{2n}-$ (mit n = 1 bis 10); -CH=CH-; $-NR^{10}CSNR^{11}-$; $-NR^{10}-$; $-R^{10}N-CHR^{11}-$; $-CHR^{10}NR^{11}-$; $-O-CHR^{10}-$; -S(O)p-$CHR^{10}$- (mit p = 0, 1, 2); $NR^{10}SO_2-$ oder $-OSO_2-$; $-O_2SO-$; $-O_2S-NR^{10}-$; -C≡C-; -S-S-; -CHOH-; -CO-;
$>C=CH_2$; $>C=CH-COOH$; -NH-NH-; -N(O)=N-;

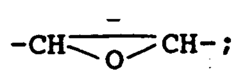

$-N(R^{10})-N=CH-$; $-N(R^{10})-NH-CO-$; -NH-CH=CH-; $>CH-C_6H_5$; $-COCH_2O-$; $-CO-CH_2S-$; $-COCH_2NR^{10}-$; $-OCH_2CO$; $-SCH_2CO-$; -CH=N-; $-NR^{10}CH_2CO-$;-CH=NO-; $-CH_2O-CONR^{10}-$; $-CH_2O-CO-$; $-S-CH_2-$; $-CH_2CO-$; -CO-O-; -O-CO-; -CO-S- oder -S-CO- steht;

$R^7$ für Wasserstoff; $NR^{10}R^{11}$; $PO(OR^{10})(OR^{11})$; $(CH_2)q-CO-O-R^{10}$ (mit q = 0, 1, 2, 3); Cyano; $CONR^{10}R^{11}$; $SO_2NR^{10}R^{11}$;
Tri-$C_1$-$C_4$-alkyl-silyl; bis zu zweifach durch $R^{10}$ und $R^{11}$ substituiertes Hetaryl worin Hetaryl für Pyrazolyl-, Pyrazinyl-, Imidazolyl-,1,2,4-Triazolyl-, Morpholinyl-, Piperidinyl- Pyrrolyl-, Pyrrolidinyl-,2,5-Dioxopyrrolidinyl-, 1,3-Isoindoldionyl- und Pyridinylradikale, sowie von Indol, Benzofuran, Chinolin oder Benzothiophen abgeleitete Reste, steht oder

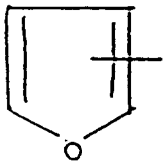 , 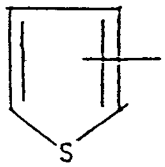 , 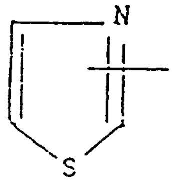 , 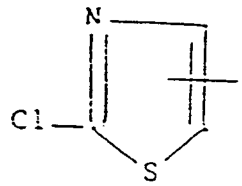

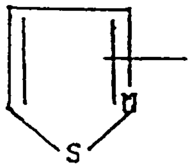 , 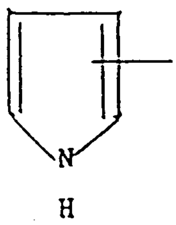 , 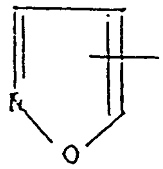 , 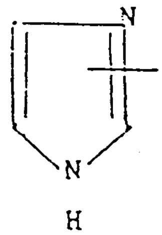

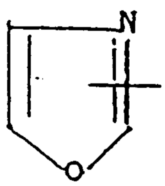 , 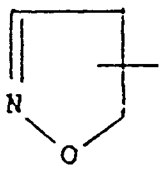 , 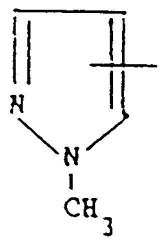 , 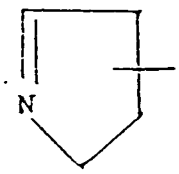

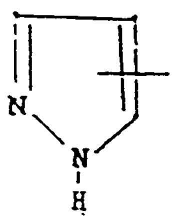 , 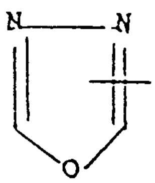 , 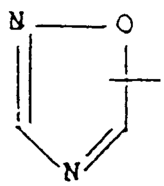 , 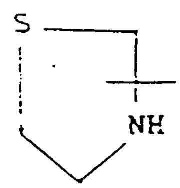

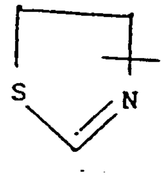 , 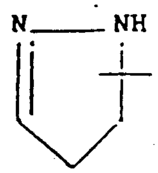 , 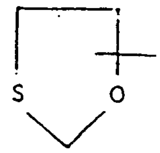 , 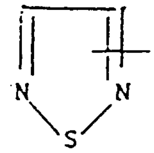

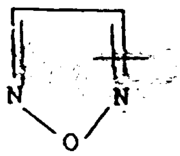 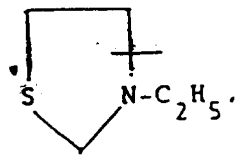 , 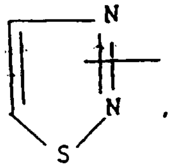 , 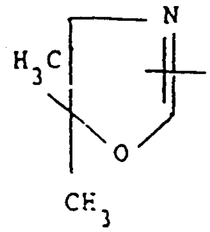

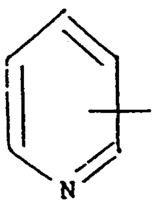 , 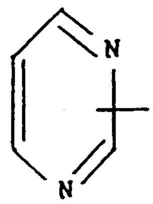 , 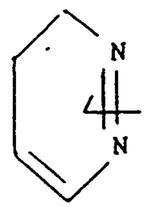 , 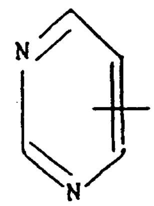

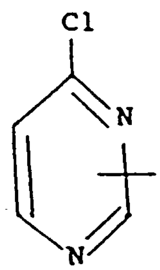 , 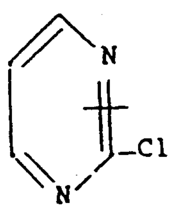 , 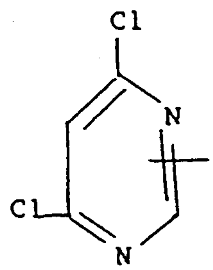 , 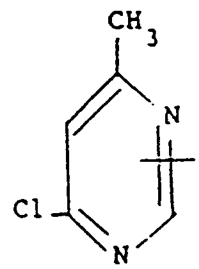

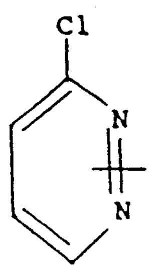 , 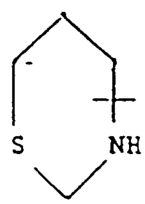 , 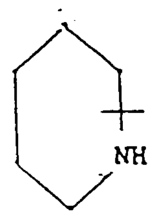 , 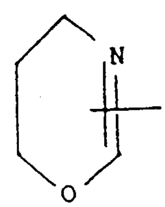

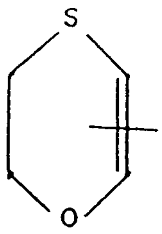 , 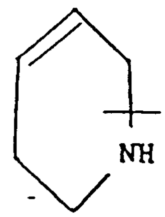 , 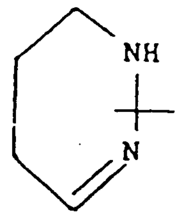 , 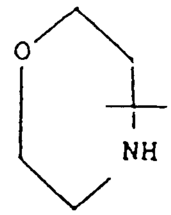

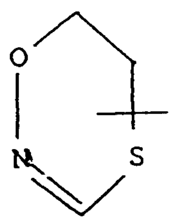 , 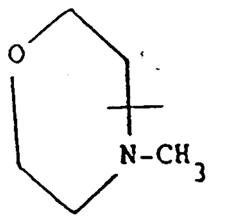 , 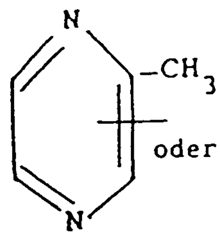 oder 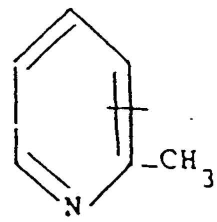

steht;

[(1-Formylamino-2,2,2-trichlor)ethyl]amino;

1-(3,4-Dimethoxyphenyl)-3-(morpholin-4-yl)-prop-1-en-3-on;

Phenyl gegebenenfalls bis zu dreifach durch Halogen, $C_{1-4}$ Alkyl, Alkylthio, Alkoxy, Nitro, Cyano, Amino, Monoalkylamino, Dialkylamino, Halogenalkyl, Halogenalkylthio, Halogenalkoxy, Carboxy oder Alkoxycarbonyl substituiert; Alkyl mit bis zu 12 C-Atomen gegebenenfalls durch Sauerstoff und/oder Schwefelatome unterbrochen und

gegebenenfalls ein oder mehrfach mit Hydroxy, Alkoxy, Mercapto, Halogen, Alkylthio, Nitro, Cyano, Amino oder Mono-oder Di-$C_1$-$C_4$-alkylamino substituiert, Cycloalkyl gegebenenfalls durch Sauerstoff und/oder Schwefelatome und/oder Stickstoff unterbrochen und gegebenenfalls bis zu dreifach durch Alkyl, Halogen, Hydroxy, Oxo oder Amino substituiert, phenylsubstituiertes $C_1$-$C_3$-Alkyl, $C_2$-$C_6$-Alkenyl, $C_2$-$C_6$-Alkinyl, $C_5$-$C_8$-Cycloalkenyl oder Naphthyl steht,

wenn X - Y für eine Einfachbindung steht, können $R^7$ and $R^6$ außerdem gemeinsam für eine vicinale Brücke der Formel

$-(CH_2)_n-E-$ (mit n = 2, 3), -(CH=CH)-D oder

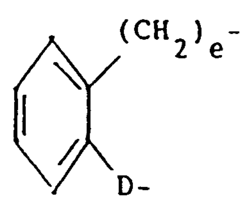

(mit e = O oder 1)

stehen;

wobei

E für $CH_2$; O; S oder $NR^{10}$

D für $CH_2$; O; S; $NR^{10}$; $-CH_2-CH_2-$ oder

-CH=CH- steht, und gegebenenfalls ihre Salze; worin Alkyl $C_1$-$C_{12}$-C-Atome bedeutet;

und die Verbindungen:-

3-(3,4-Dimethoxyphenyl)-3-[4-(4,5-dihydrooxazol-2-yl)phenyl]-acrylsäuremorpholid,

3-(3,4-Dimethoxyphenyl)-3-(4-benzimidazol-2-yl-phenyl)-acrylsäuremorpholid,

3-(3,4-Dimethoxphenyl)-3-[4-(1,2-dichlorvinyloxy)-phenyl]-acrylsäuremorpholid,

3-(3,4-Dimethoxyphenyl)-3-(4-benzothiazol-2-ylthiomethylphenyl)-acrylsäuremorpholid,

3-(3,4-Dimethoxyphenyl)-3-(4-benzothiazol-2-ylthiophenyl)-acrylsäuremorpholid,

3-(3,4-Dimethoxyphenyl)-3-[4-(3-p-toluol-sulfonylureido)-phenyl]-acrylsäuremorpholid,

3-(3,4-Dimethoxyphenyl)-3-[4-(4,6-dichlor-1,3,5-triazin-2-yloxy)-phenyl]-acrylsäuremorpholid,

3-(3,4-Dimethoxyphenyl)-3-[4-(2,5-dioxopyrrol-1-yl)-phenyl]-acrylsäuremorpholid,

3-(3,4-Dimethoxyphenyl)-3-[4-(6-chlor-2-ethylaminopyrimidin-4-yloxy)-phenyl]-acrylsäuremorpholid,

3-(3,4-Dimethoxyphenyl)-3-[4-(6-chlor-2-dimethylaminopyrimidin-4-yloxy)-phenyl]-acrylsäuremorpholid,

3-(3,4-Dimethoxyphenyl)-3-[4-(6-chlor-2-isopropylaminopyrimidin-4-yloxy)-phenyl]-acrylsäuremorpholid;

mit Ausnahme von den Verbindungen der Formel I in der

B für

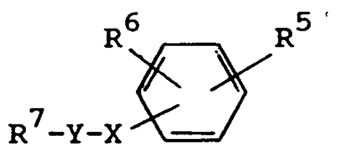

steht;

$R^1$ für Wasserstoff, $C_1$-$C_4$-Alkyl, Cyano, Chlor, Brom oder Jod steht;

$R^2$, $R^3$ und $R^4$, die gleich oder verschieden sein können, für Wasserstoff, Halogen, Nitro, gegebenenfalls ein- oder mehrfach durch Fluor oder Chlor substituiertes $C_1$-$C_4$-Alkyl oder -Alkoxy, Amino, NH($C_1$-$C_4$-Alkyl), N($C_1$-$C_4$-Alkyl)$_2$, Cyano, Phenyl, $C_3$-$C_6$-Cycloalkyl, Hydroxy ($C_1$-$C_4$-Alkyl), NHCOH, NHCO($C_1$-$C_4$ Alkyl), $CO_2H$, $CO_2$($C_1$-$C_4$-Alkyl), $CONR^{10}R^{11}$ in der $R^{10}$ und $R^{11}$, die gleich oder verschieden sein können, für $C_1$-$C_4$-Alkyl, Phenyl oder Benzyl, gemeinsam außerdem für $-(CH_2)_4-$ oder $-(CH_2)_5-$, und $R^{10}$ außerdem für Wasserstoff, stehen, ($C_1$-$C_4$-Alkyl)-S(O)$_p$ (mit p = 0,1,2) oder Hydroxy steht;

$R^5$ und $R^6$, die gleich oder verschieden sein können, für Wasserstoff, Halogen, $C_1$-$C_4$-Alkyl, $C_1$-$C_4$-Alkoxy, ($C_1$-$C_4$-Alkyl)-S(O)$_p$ (mit p = 0,1,2) oder Hydroxy stehen, und $R^5$ außerdem für Nitro, ein- oder mehrfach durch Fluor oder Chlor substituiertes $C_1$-$C_4$-Alkyl oder -Alkoxy, NHCOH oder NHCO($C_1$-$C_4$-Alkyl) steht;

X-Y für eine Einfachbindung steht;

$R^7$ für Wasserstoff, gegebenenfalls ein- oder mehrfach durch Fluor oder Chlor substituiertes $C_1$-$C_4$-Alkyl, Amino, NH($C_1$-$C_4$-Alkyl), N($C_1$-$C_4$-Alkyl)$_2$, Cyano, Phenyl, $C_3$-$C_6$-Cycloalkyl, Hydroxy($C_1$-$C_4$-Alkyl), $CO_2H$, $CO_2$($C_1$-$C_4$-Alkyl), $CONR^{10}R^{11}$ in der $R^{10}$ und $R^{11}$, die gleich oder verschieden sein können, für $C_1$-$C_4$-Alkyl, Phenyl oder Benzyl, gemeinsam außerdem für $-(CH_2)_4-$ oder $-(CH_2)_5-$, und $R^{10}$ außerdem für Wasserstoff, stehen, durch Sauerstoff unterbrochenes $C_2$-$C_8$-Alkyl steht; oder $R^7$ und $R^6$ außerdem gemeinsam für eine vicinale Brücke der Formel -(CH = CH)-D stehen können, wobei D für -CH = CH- steht;

Q für $NR^8R^9$ oder

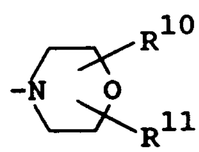

steht, in der

$R^8$ und $R^9$, die gleich oder verschieden sein können, für $C_1$-$C_4$-Alkyl, $C_3$-$C_7$-Cycloalkyl, Phenyl, Benzyl oder $C_3$-$C_4$-Alkenyl, und $R^8$ außerdem Wasserstoff, stehen, und

$R^{10}$ und $R^{11}$, die gleich oder verschieden sein können, für Wasserstoff oder $C_1$-$C_4$-Alkyl stehen;

und mit Ausnahme von den Verbindungen der Formel I in der A und B beide für einen Rest aus der Gruppe Phenyl, Monohalogenphenyl, Mononitrophenyl und Monoaminophenyl stehen.

**3.** Verfahren nach Anspruch 1 oder 2 worin A für 3,4-Dimethoxyphenyl, 3-Ethoxy-4-methoxyphenyl, 3-Chlor-4-methoxyphenyl, 3-Methyl-4-methoxyphenyl, 3-Brom-4-methoxyphenyl, 3-Ethyl-4-methoxyphenyl, 3,4-Dimethylphenyl, 3,5-Dichlor-4-Aminophenyl steht und B, Q und $R^1$ die obige Bedeutung haben.

**4.** Verfahren nach Anspruch 3, worin $R^1$ Wasserstoff bedeutet.

**5.** Verfahren nach Anspruch 1, 2, 3 oder 4, worin Q für $NR^8R^9$, worin $R^8$ und $R^9$ $C_1$-$C_4$-Alkyl ist, oder für die Morpholinogruppe steht.

**6.** Verfahren nach Anspruch 1, 2, 3, 4 oder 5, worin B für

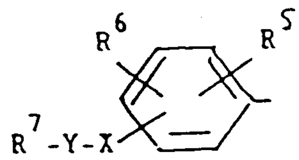

steht, worin -X-Y- eine Einfachbindung, Sauerstoff, Schwefel, $-CH_2O-$, $-OCH_2-$ oder -N = N- bedeutet.

**7.** Verfahren nach Anspruch 6, worin $R^5$ und $R^6$ Wasserstoff bedeuten, -X-Y- eine Einfachbindung, Sauerstoff, Schwefel oder -N = N- darstellt und $R^7$ für ein gegebenenfalls substituiertes Phenyl, gegebenenfalls substituiertes $C_1$-$C_{12}$-Alkyl, gegebenenfalls durch Sauerstoff und/oder Schwefel und/oder $NR^{10}$ ein- oder mehrfach unterbrochenes gegebenenfalls hydroxy- oder $C_1$-$C_4$-alkylsubstituiertes $C_3$-$C_7$-Cycloalkyl oder bis zu zweifach durch $R^{10}$ und $R^{11}$ substituiertes Hetaryl steht.

**8.** Verfahren nach Anspruch 1,2,3,4,5,6 oder 7, worin die Verbindung der Formel I ist 3-[4-(4-Chlorphenoxy)-phenyl]-3-(3,4-dimethoxyphenyl)-acrylsäuremorpholid.

**9.** Verfahren nach Anspruch 1,2,3,4,5,6 oder 7, worin die Verbindung der Formel I ist 3-(4-n-Butylphenyl)-phenyl)-3-(3,4-dimethoxyphenyl)-acrylsäuremorpholid.

**10.** Verfahren nach Anspruch 1,2,3,4,5,6 oder 7, worin die Verbindung der Formel I ist 3-[4-(4-Chlorophenyl)-phenyl]-3-(3,4-dimethoxyphenyl)-acrylsäure-methyl-ethylamid.

**11.** Verfahren zur Herstellung von fungiziden Mitteln, gekennzeichnet durch eine Verbindung nach Anspruch 1 bis 10 in Verbindung gebracht mit den üblichen Hilfs- und/oder Trägerstoffen und gegebenenfalls einem oder mehreren zusätzlichen Wirkstoffen.

**12.** Verwendung von Verbindungen nach einem der Ansprüche 1 bis 10 gegebenenfalls in Kombination mit einem oder mehreren weiteren fungiziden Wirkstoffen zur Bekämpfung phytopathogener Pilze, insbesondere des falschen Mehltaus.

**Claims**

**Claims for the following Contracting States : BE, CH, DE, FR, GB, GR, IT, LI, LU, NL, SE**

**1.** Compounds of the formula

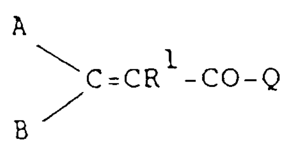

(I)

in which

A stands for

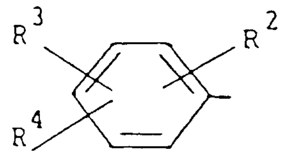

B for

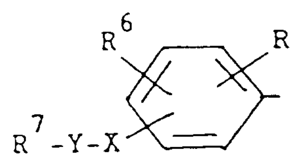 , 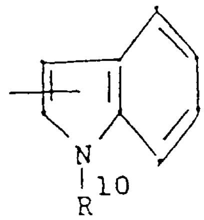 , 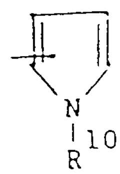

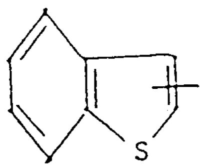 , 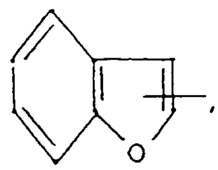 , 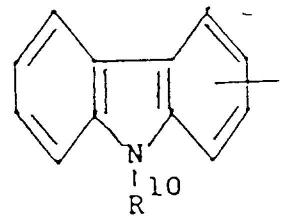

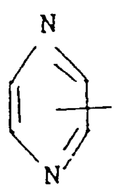 , 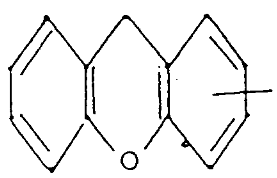 or 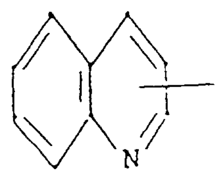

$R^1$ for hydrogen, halogen, cyano or $C_1$-$C_4$-alkyl optionally singly or multiply substituted with hydroxy, alkoxy, mercapto, halogen, alkylthio, nitro, cyano, amino or mono- or di-$C_1$-$C_4$-alkylamino,

Q stands for

$NR^8R^9$ or

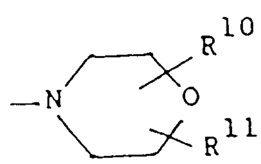

$R^2$, $R^3$, $R^4$, $R^5$ and $R^6$, which can be the same or different, stand for hydrogen, halogen, nitro, cyano, carboxy, hydroxy, $C_1$-$C_4$-alkoxycarbonyl, $CONR^{10}R^{11}$, $NR^{10}R^{11}$, $NR^{10}$-CO-$R^{11}$,

or for $C_1$-$C_6$-alkyl, $C_1$-$C_4$-alkoxy or $C_1$-$C_4$-alkyl-$S(O)_p$ (with p = 0, 1, 2), optionally singly or multiply substituted with hydroxy, alkoxy, mercapto, halogen, alkylthio, nitro, cyano, amino or mono- or di-$C_1$-$C_4$-alkylamino; $C_3$-$C_7$-cycloalkyl optionally up to triply substituted with alkyl, halogen, hydroxy, oxo or amino; phenyl, phenoxy or phenyl-$S(O)_p$- (with p = 0, 1, 2) optionally up to triply substituted with halogen, $C_1$-$C_4$-alkyl, alkylthio, alkoxy, nitro, cyano, amino, monoalkylamino, dialkylamino, haloalkyl,

haloalkylthio, haloalkoxy, carboxy or alkoxycarbonyl,

$R^8$ and $R^9$, which can be the same or different, stand for hydrogen, $C_1$-$C_4$-alkyl, $C_3$-$C_7$-alkenyl, $C_3$-$C_7$-alkynyl or alkoxyalkyl optionally singly or multiply substituted with hydroxy, alkoxy, mercapto, halogen, alkylthio, nitro, cyano, amino or mono- or di-$C_1$-$C_4$-alkylamino; $C_3$-$C_7$-cycloalkyl optionally up to triply substituted with alkyl, halogen, hydroxy, oxo or amino; phenyl or benzyl optionally up to triply substituted with halogen, $C_1$-$C_4$-alkyl, alkylthio, alkoxy, nitro, cyano, amino, monoalkylamino, dialkylamino, haloalkyl, haloalkylthio, haloalkoxy, carboxy or alkoxycarbonyl,

$R^{10}$ and $R^{11}$, which can be the same or different, stand for hydrogen, benzyl, phenyl or $C_1$-$C_4$-alkyl, as substituents in heteroaryl also stand for halogen, amino or mono- or di-$C_1$-$C_4$-alkylamino, or together for $-(CH_2)_4-$ or $-(CH_2)_5-$,

X - Y stands for a single bond, -O-, $-S(O)_p$ (with p = 0, 1, 2), $-CONR^{10}-$, $-NR^{10}CO-$, $-NR^{11}CONR^{10}-$, -N=N-, $-CHR^{10}-S(O)_p-$(with p = 0, 1, 2), $-CHR^{10}O-$, $-SO_2NR^{10}-$, -N=CH-, $-C_nH_{2n}-$ (with n = 1 to 10), -CH=CH-, $-NR^{10}CSNR^{11}-$, $-NR^{10}-$, $-R^{10}N-CHR^{11}-$, $-CHR^{10}NR^{11}-$, $-O-CHR^{10}-$, $-S(O)_p-CHR^{10}-$ (with p = 0, 1, 2), $-NR^{10}SO_2-$ or $-OSO_2-$; $-O_2SO-$; $-O_2S-NR^{10}-$; -C≡C-; -S-S-; -CHOH-; -CO-; $>C=CH_2$; $>C=CH-COOH$; -NH-NH-; -N(O)=N-;

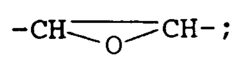

$-N(R^{10})-N=CH-$; $-N(R^{10})-NH-CO-$; -NH-CH=CH-; $>CH-C_6H_5$; $-COCH_2O-$; $-CO-CH_2S-$; $-COCH_2NR^{10}-$; $-OCH_2CO$; $-SCH_2CO-$; -CH=N-; $-NR^{10}CH_2CO-$; -CH=NO-; $-CH_2O-CONR^{10}-$; $-CH_2O-CO-$; $-S-CH_2-$, $-CH_2CO-$, -CO-O-, -O-CO-, -CO-S- or -S-CO-,

$R^7$ stands for hydrogen, $NR^{10}R^{11}$, $PO(OR^{10})(OR^{11})$, $(CH_2)q-CO-O-R^{10}$ (with q = 0, 1, 2, 3), cyano, $CONHR^{10}R^{11}$, $SO_2NR^{10}R^{11}$, tri-$C_1$-$C_4$-alkyl-silyl, heteroaryl up to doubly substituted with $R^{10}$ and $R^{11}$ where heteroaryl stands for pyrazolyl-, pyrazinyl-, imidazolyl-, 1,2,4-triazolyl-, morpholinyl-, piperidinyl-, pyrrolyl-, pyrrolidinyl-, 2,5-dioxopyrrolidinyl-, 1,3-isoindoldionyl- and pyridinyl radicals, and residues derived from indole, benzofuran, quinoline or benzothiophen or stands for

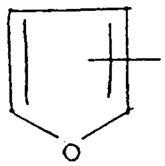 , 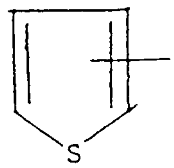 , 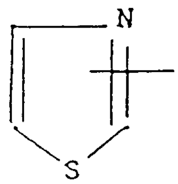 , 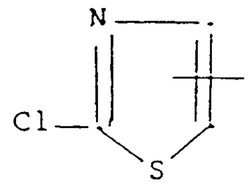

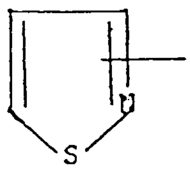 , 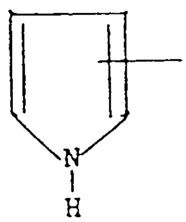 , 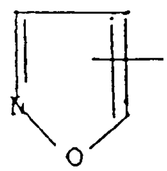 , 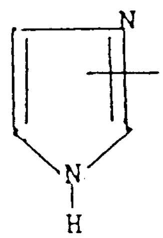

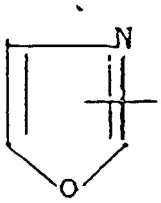 , 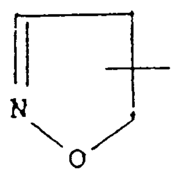 , 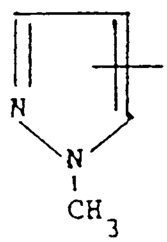 , 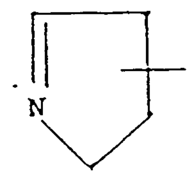

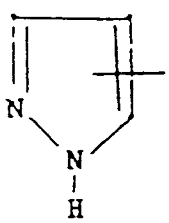 , 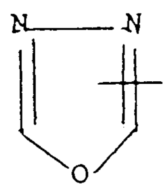 , 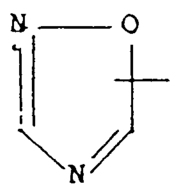 , 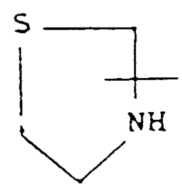

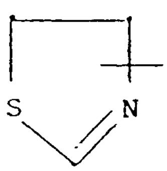 , 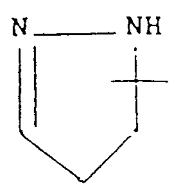 , 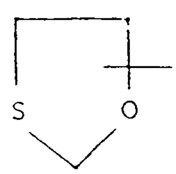 , 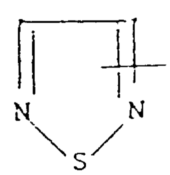

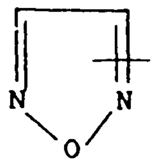 , 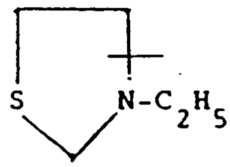 , 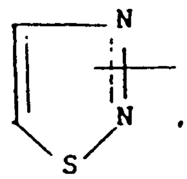 , 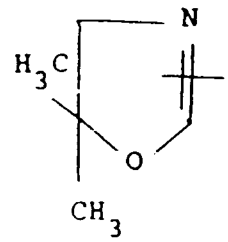

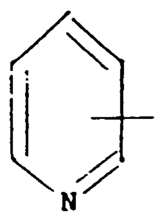 , 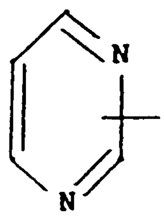 , 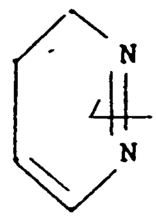 , 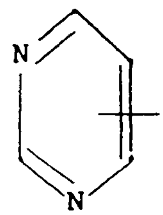

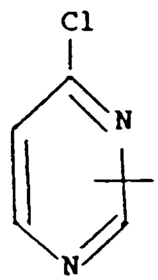 , 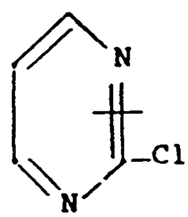 , 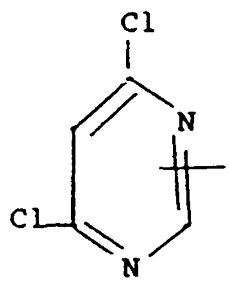 , 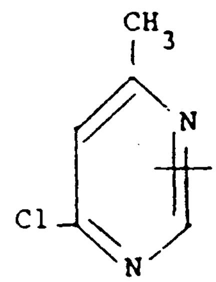

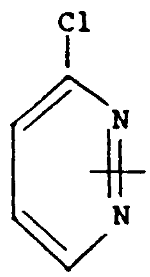 , 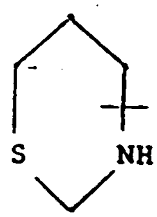 , 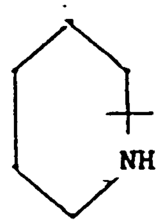 , 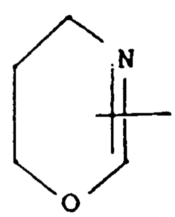

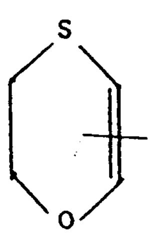 , 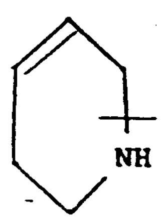 , 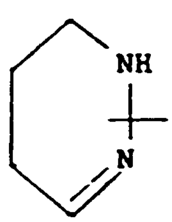 , 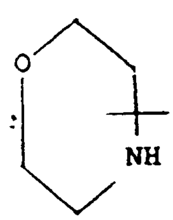

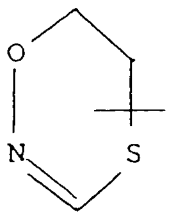 , 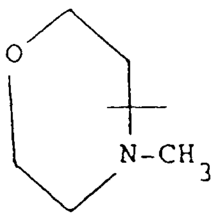 , 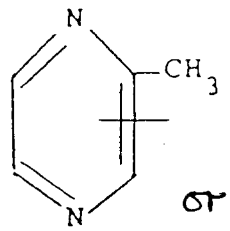 or 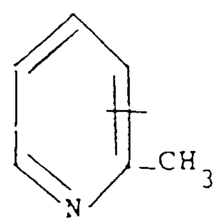

or

stands for [(1-formylamino-2,2,2-trichlor)ethyl]-amino, 1-(3,4-dimethoxyphenyl)-3-(morpholin-4-yl)-prop-1-en-3-one,

phenyl optionally up to triply substituted with halogen, $C_1$-$C_4$ alkyl, alkylthio, alkoxy, nitro, cyano, amino, monoalkylamino, dialkylamino, haloalkyl, haloalkylthio, haloalkoxy, carboxy or alkoxycarbonyl; alkyl with up to 12 C-atoms optionally interrupted with oxygen and/or sulphur atoms and optionally singly or multiply substituted with hydroxy, alkoxy, mercapto, halogen, alkylthio, nitro, cyano, amino or mono- or di-$C_1$-$C_4$-alkylamino; cycloalkyl optionally interrupted with oxygen and/or sulphur atoms and/or nitrogen and optionally up to triply substituted with alkyl, halogen, hydroxy, oxo or amino; phenyl-substituted $C_1$-$C_3$-alkyl, $C_2$-$C_6$-alkenyl, $C_2$-$C_6$-alkynyl, $C_5$-$C_8$-cycloalkenyl or naphthyl,

if X - Y stands for a single bond, $R^7$ and $R^6$ can also together stand for a vicinal bridge of the formula

$-(CH_2)_n$-E- (with n = 2, 3), -(CH=CH)-D or

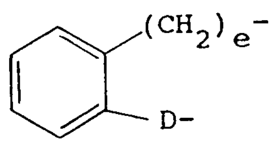

(with e = 0 or 1)

where

E stands for $CH_2$, O, S or $NR^{10}$

D stands for $CH_2$, O, S, $NR^{10}$, $-CH_2-CH_2-$ or -CH=CH-, and optionally salts thereof, wherein alkyl means $C_1$-$C_{12}$-C-atoms,

and the compounds:-

3-(3,4-dimethoxyphenyl)-3-[4-(4,5-dihydrooxazol-2-yl)phenyl]-acrylmorpholide,
3-(3,4-dimethoxyphenyl)-3-(4-benzimidazol-2-yl-phenyl)-acrylmorpholide,
3-(3,4-dimethoxyphenyl)-3-[4-(1,2-dichlorvinyloxy)-phenyl]-acrylmorpholide,
3-(3,4-dimethoxyphenyl)-3-(4-benzothiazol-2-yl-thiomethylphenyl)-acrylmorpholide,
3-(3,4-dimethoxyphenyl)-3-(4-benzothiazol-2-yl-thiophenyl)-acrylmorpholide,
3-(3,4-dimethoxyphenyl)-3-[4-(3-p-toluene-sulphonylureido)-phenyl]-acrylmorpholide,
3-(3,4-dimethoxyphenyl)-3-[4-(4,6-dichlor-1,3,5-triazin-2-yloxy)-phenyl]-acrylmorpholide,
3-(3,4-dimethoxyphenyl)-3-[4-(2,5-dioxopyrrol-1-yl)-phenyl]-acrylmorpholide,
3-(3,4-dimethoxyphenyl)-3-[4-(6-chlor-2-ethylaminopyrimidine-4-yloxy)-phenyl]-acrylmorpholide,
3-(3,4-dimethoxyphenyl)-3-[4-(6-chlor-2-dimethylaminopyrimidine-4-yloxy)-phenyl]-acrylmorpholide,
3-(3,4-dimethoxyphenyl)-3-[4-(6-chlor-2-isopropylaminopyrimidine-4-yloxy)-phenyl]-acrylmorpholide;

with the exception of the object which is disclosed in EP-A-0120321 and with the exception of the compounds of the formula I in which A and B both stand for a residue from the group phenyl, monohalophenyl, mononitrophenyl and monoaminophenyl.

**2.** Compounds of the formula I according to Claim 1

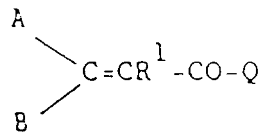

(I)

in which

A stands for

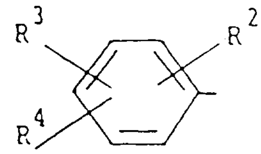

B for

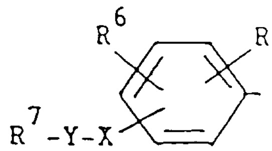 , 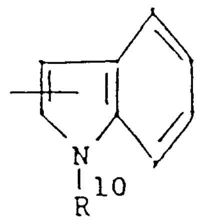 , 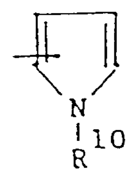 ,

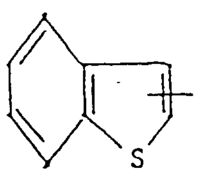 , 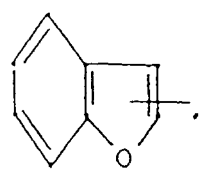 , 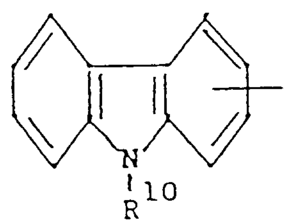

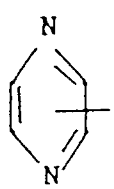 , 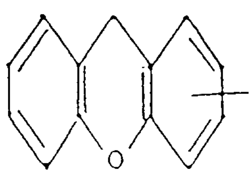 or 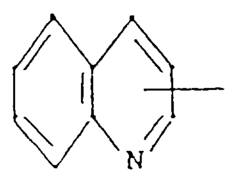

$R^1$ for hydrogen, halogen, cyano or $C_1$-$C_4$-alkyl optionally singly or multiply substituted with hydroxy, alkoxy, mercapto, halogen, alkylthio, nitro, cyano, amino or mono- or di-$C_1$-$C_4$-alkylamino,

Q stands for

$NR^8R^9$ or

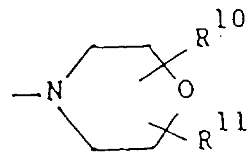

$R^2$, $R^3$, $R^4$, $R^5$ and $R^6$, which can be the same or different, stand for hydrogen, halogen, nitro, cyano, carboxy, hydroxy, $C_1$-$C_4$-alkoxycarbonyl, $CONR^{10}R^{11}$, $NR^{10}R^{11}$, $NR^{10}$-CO-$R^{11}$,
or for $C_1$-$C_6$-alkyl, $C_1$-$C_4$-alkoxy or $C_1$-$C_4$-alkyl-$S(O)_p$ (with p = 0, 1, 2), optionally singly or multiply substituted with hydroxy, alkoxy, mercapto, halogen, alkylthio, nitro, cyano, amino or mono- or di-$C_1$-$C_4$-alkylamino; $C_3$-$C_7$-cycloalkyl optionally up to triply substituted with alkyl, halogen, hydroxy, oxo or amino; phenyl, phenoxy or phenyl-$S(O)_p$- (with p = 0, 1, 2) optionally up to triply substituted with halogen, $C_1$-$C_4$-alkyl, alkylthio, alkoxy, nitro, cyano, amino, monoalkyl-amino, dialkylamino, haloalkyl, haloalkylthio, haloalkoxy, carboxy or alkoxycarbonyl,

$R^8$ and $R^9$, which can be the same or different, stand for hydrogen,
$C_1$-$C_4$-alkyl, $C_3$-$C_7$-alkenyl, $C_3$-$C_7$-alkynyl or alkoxyalkyl optionally singly or multiply substituted with hydroxy, alkoxy, mercapto, halogen, alkylthio, nitro, cyano, amino or mono- or di-$C_1$-$C_4$-alkylamino; $C_3$-$C_7$-cycloalkyl optionally up to triply substituted with alkyl, halogen, hydroxy, oxo or amino; phenyl or benzyl optionally up to triply substituted with halogen, $C_1$-$C_4$-alkyl, alkylthio, alkoxy, nitro, cyano, amino, monoalkylamino, dialkylamino, haloalkyl, haloalkylthio, haloalkoxy, carboxy or alkoxycarbonyl,

$R^{10}$ and $R^{11}$, which can be the same or different, stand for hydrogen, benzyl, phenyl or $C_1$-$C_4$-alkyl, as substituents in heteroaryl also stand for halogen, amino or mono- or di-$C_1$-$C_4$-alkylamino, or together for $-(CH_2)_4-$ or $-(CH_2)_5-$,

X - Y stands for a single bond,
-O-, $-S(O)_p$ (with p = 0, 1, 2), $-CONR^{10}-$, $-NR^{10}CO-$, $-NR^{11}CONR^{10}-$, -N=N-, $-CHR^{10}-S(O)_p-$(with p = 0, 1, 2), $-CHR^{10}O-$, $-SO_2NR^{10}-$, -N=CH-, $-C_nH_{2n}-$ (with n = 1 to 10), -CH=CH-, $-NR^{10}CSNR^{11}-$, $-NR^{10}-$, $-R^{10}N-CHR^{11}-$, $-CHR^{10}NR^{11}-$, $-O-CHR^{10}-$, $-S(O)_p-CHR^{10}-$ (with p = 0, 1, 2), $-NR^{10}SO_2-$ or $-OSO_2-$; $-O_2SO-$; $-O_2S-NR^{10}-$; -C≡C-; -S-S-; -CHOH-; -CO-; $>C=CH_2$; $>C=CH-COOH$; -NH-NH-; -N(O)=N-;

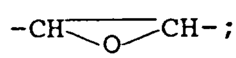

$-N(R^{10})-N=CH-$; $-N(R^{10})-NH-CO-$; -NH-CH=CH-; $>CH-C_6H_5$; $-COCH_2O-$; $-CO-CH_2S-$; $-COCH_2NR^{10}-$; $-OCH_2CO$; $-SCH_2CO-$; -CH=N-; $-NR^{10}CH_2CO-$; -CH=NO-; $-CH_2O-CONR^{10}-$; $-CH_2O-CO-$; $-S-CH_2-$, $-CH_2CO-$, -CO-O-, -O-CO-, -CO-S- or -S-CO-,

$R^7$ stands for hydrogen, $NR^{10}R^{11}$, $PO(OR^{10})(OR^{11})$, $(CH_2)_q-CO-O-R^{10}$ (with q = 0, 1, 2, 3), cyano, $CONHR^{10}R^{11}$, $SO_2NR^{10}R^{11}$,
tri-$C_1$-$C_4$-alkyl-silyl, heteroaryl up to doubly substituted with $R^{10}$ and $R^{11}$ where heteroaryl stands for pyrazolyl-, pyrazinyl-, imidazolyl-, 1,2,4-triazolyl-, morpholinyl-, piperidinyl-, pyrrolyl-, pyrrolidinyl-, 2,5-dioxopyrrolidinyl-, 1,3-isoindoldionyl- and pyridinyl radicals, and residues derived from indole, benzofuran, quinoline or benzothiophen or stands for

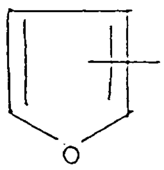 , 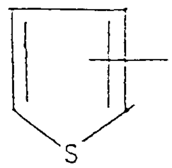 , 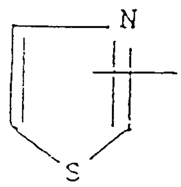 , 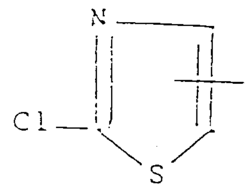

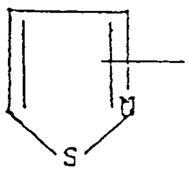 , 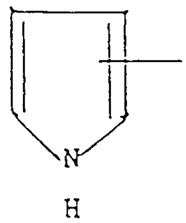 , 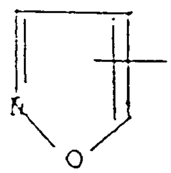 , 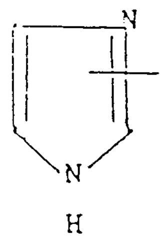

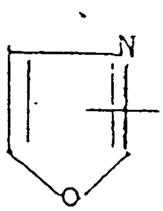 , 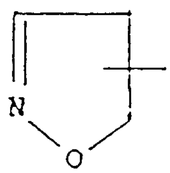 , 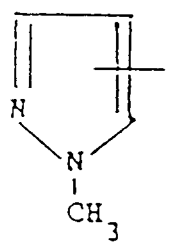 , 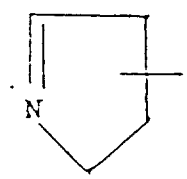

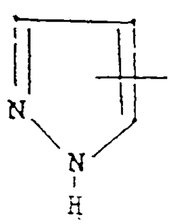 , 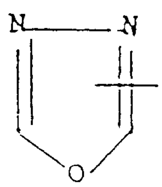 , 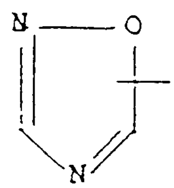 , 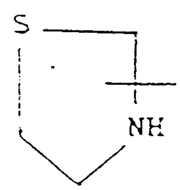

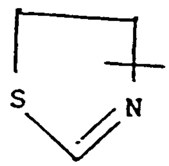 , 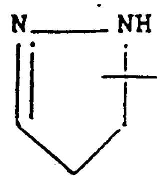 , 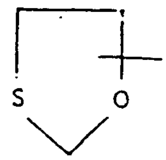 , 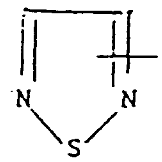

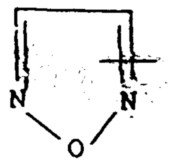 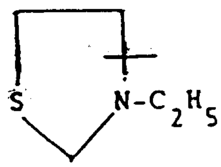 . 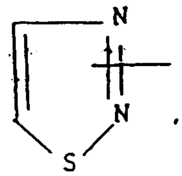 , 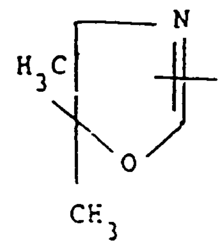

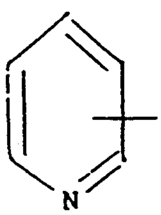 , 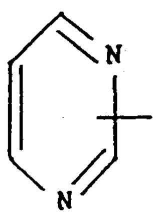 , 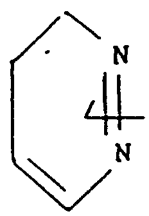 , 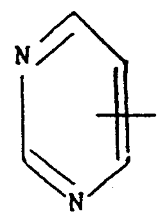

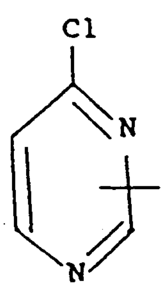 , 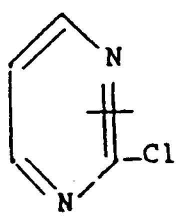 , 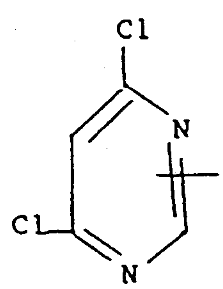 , 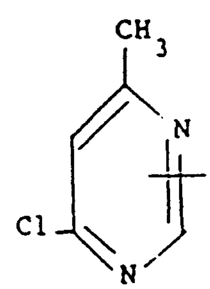

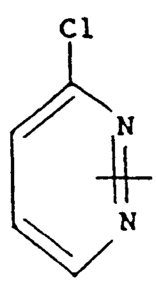 , 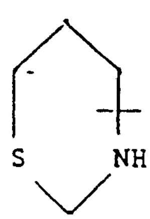 , 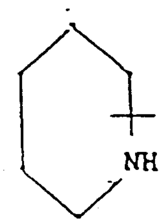 , 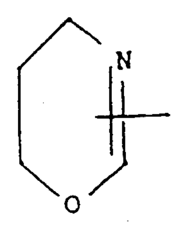

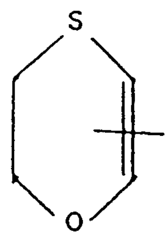 , 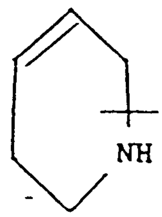 , 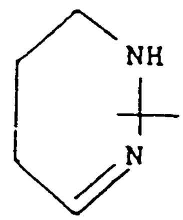 , 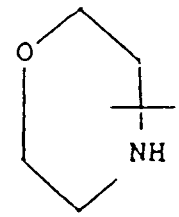

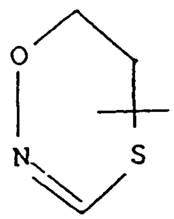 , 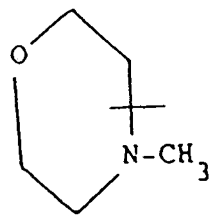 , 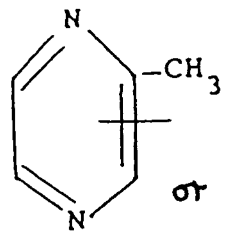 or 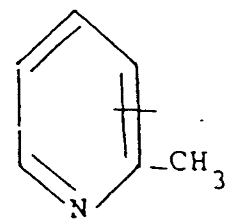

or

stands for [(1-formylamino-2,2,2-trichlor)ethyl]-amino, 1-(3,4-dimethoxyphenyl)-3-(morpholin-4-yl)-prop-1-en-3-one,

phenyl optionally up to triply substituted with halogen, $C_1$-$C_4$ alkyl, alkylthio, alkoxy, nitro, cyano, amino, monoalkylamino, dialkylamino, haloalkyl, haloalkylthio, haloalkoxy, carboxy or alkoxycarbonyl; alkyl with up to 12 C-atoms optionally interrupted with oxygen and/or sulphur atoms and optionally singly or multiply substituted with hydroxy, alkoxy, mercapto, halogen, alkylthio, nitro, cyano, amino or mono- or di-$C_1$-$C_4$-alkylamino; cycloalkyl optionally interrupted with oxygen and/or sulphur atoms and/or nitrogen and optionally up to triply substituted with alkyl, halogen, hydroxy, oxo or amino; phenyl-substituted $C_1$-$C_3$-alkyl, $C_2$-$C_6$-alkenyl, $C_2$-$C_6$-alkynyl, $C_5$-$C_8$-cycloalkenyl or naphthyl,

if X - Y stands for a single bond, $R^7$ and $R^6$ can also together stand for a vicinal bridge of the formula

$-(CH_2)_n$-E- (with n = 2, 3), -(CH=CH)-D or

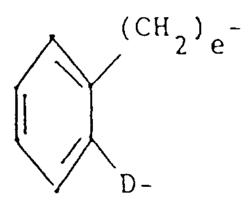

(with e = 0 or 1)

wherein

E stands for $CH_2$, O, S or $NR^{10}$

D stands for $CH_2$, O, S, $NR^{10}$, $-CH_2-CH_2-$ or -CH=CH-, and optionally salts thereof, wherein alkyl means $C_1$-$C_{12}$-C-atoms;

and the compounds:-

3-(3,4-dimethoxyphenyl)-3-[4-(4,5-dihydrooxazol-2-yl)phenyl]-acrylmorpholide,

3-(3,4-dimethoxyphenyl)-3-(4-benzimidazol-2-yl-phenyl)-acrylmorpholide,

3-(3,4-dimethoxyphenyl)-3-[4-(1,2-dichlorvinyloxy)-phenyl]-acrylmorpholide,

3-(3,4-dimethoxyphenyl)-3-(4-benzothiazol-2-yl-thiomethylphenyl]-acrylmorpholide,

3-(3,4-dimethoxyphenyl)-3-(4-benzothiazol-2-yl-thiophenyl)-acrylmorpholide,

3-(3,4-dimethoxyphenyl)-3-[4-(3-p-toluene-sulphonylureido)-phenyl]-acrylmorpholide,

3-(3,4-dimethoxyphenyl)-3-[4-(4,6-dichlor-1,3,5-triazine-2-yloxy)-phenyl]-acrylmorpholide,

3-(3,4-dimethoxyphenyl)-3-[4-(2,5-dioxopyrrol-1-yl)-phenyl]-acrylmorpholide,

3-(3,4-dimethoxyphenyl)-3-[4-(6-chlor-2-ethylaminopyrimidine-4-yloxy)-phenyl]-acrylmorpholide,

3-(3,4-dimethoxyphenyl)-3-[4-(6-chlor-2-dimethylaminopyrimidine-4-yloxy)-phenyl]-acrylmorpholide,

3-(3,4-dimethoxyphenyl)-3-[4-(6-chlor-2-isopropylaminopyrimidine-4-yloxy)-phenyl]-acrylmorpholide;

with the exception of the compounds of the formula I in which

B stands for

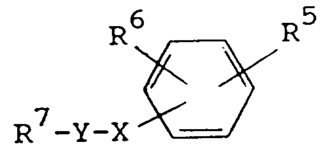

$R^1$ stands for hydrogen, $C_1$-$C_4$-alkyl, cyano, chlorine, bromine or iodine,

$R^2$, $R^3$, and $R^4$, which can be the same or different, stand for hydrogen, halogen, nitro, $C_1$-$C_4$-alkyl or alkoxy optionally singly or multiply substituted with fluorine or chlorine, amino, $NH(C_1$-$C_4$-alkyl), $N(C_1$-$C_4$-alkyl$)_2$, cyano, phenyl, $C_3$-$C_6$-cycloalkyl, hydroxy ($C_1$-$C_4$-alkyl), NHCOH, NHCO-($C_1$-$C_4$-alkyl), $CO_2H$, $CO_2$-($C_1$-$C_4$-alkyl), $CONR^{10}R^{11}$ in which $R^{10}$ and $R^{11}$, which can be the same or different, stand for $C_1$-$C_4$-alkyl, phenyl or benzyl, and also together for $-(CH_2)_4-$ or $-(CH_2)_5-$, and $R^{10}$ also for hydrogen; ($C_1$-$C_4$-alkyl)-$S(O)_p$ - (with p = 0, 1, 2) or hydroxy,

$R^5$ and $R^6$, which can be the same or different, stand for hydrogen, halogen, $C_1$-$C_4$-alkyl, $C_1$-$C_4$-alkoxy, ($C_1$-$C_4$-alkyl)-$S(O)_p$ (with p = 0, 1, 2) or hydroxy and $R^5$ also stands for nitro, $C_1$-$C_4$-alkyl or alkoxy optionally singly or multiply substituted with fluorine or chlorine, NHCOH or NHCO($C_1$-$C_4$-alkyl),

X - Y stands for a single bond,

$R^7$ stands for hydrogen, $C_1$-$C_4$-alkyl optionally singly or multiply substituted with fluorine or chlorine, amino, $NH(C_1$-$C_4$-alkyl), $N(C_1$-$C_4$-alkyl$)_2$, cyano, phenyl, $C_3$-$C_6$-cycloalkyl, hydroxy ($C_1$-$C_4$-alkyl), $CO_2H$, $CO_2(C_1$-$C_4$-alkyl), $CONR^{10}R^{11}$ in which $R^{10}$ and $R^{11}$, which can be the same or different, stand for $C_1$-$C_4$-alkyl, phenyl or benzyl, together also for $-(CH_2)_4-$ or $-(CH_2)_5-$, and $R^{10}$ also for hydrogen, $C_2$-$C_8$-alkyl interrupted with oxygen; or $R^7$ and $R^6$ also may together stand for a vicinal bridge of the formula -(CH = CH)-D, where D stands for -CH = CH-,

Q stands for $NR^8R^9$ or

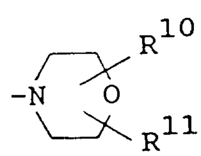 ,

in which

$R^8$ and $R^9$, which can be the same or different, stand for $C_1$-$C_4$-alkyl, $C_3$-$C_7$-cycloalkyl, phenyl, benzyl or $C_3$-$C_4$-alkenyl, and $R^8$ also for hydrogen, and

$R^{10}$ and $R^{11}$, which can be the same or different, stand for hydrogen or $C_1$-$C_4$-alkyl,

and with the exception of the compounds of the formula I in which both A and B stand for a residue from the group phenyl, monohalophenyl, mononitrophenyl and monoaminophenyl.

3. Compounds of the formula I, according to Claim 1 or 2 wherein A stands for 3,4-dimethoxyphenyl, 3-ethoxy-4-methoxyphenyl, 3-chloro-4-methoxyphenyl, 3-methyl-4-methoxyphenyl, 3-bromo-4-methoxyphenyl, 3-ethyl-4-methoxyphenyl, 3,4-dimethylphenyl and 3,5-dichlor-4-aminophenyl and B, Q and $R^1$ have the above meaning.

4. Compounds according to Claim 3, wherein $R^1$ means hydrogen.

5. Compounds according to Claim 1, 2, 3 or 4, wherein Q stands for $NR^8R^9$, wherein $R^8$ and $R^9$ are $C_1$-$C_4$-alkyl, or stand for the morpholino group.

**6.** Compounds according to Claim 1, 2, 3, 4 or 5 wherein B stands for

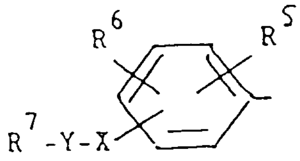

where -X-Y- means a single bond, oxygen, sulphur, $-CH_2O-$, $-OCH_2-$ or -N = N-.

**7.** Compounds according to Claim 6, wherein $R^5$ and $R^6$ mean hydrogen, -X-Y- represents a single bond, oxygen, sulphur or -N = N- and $R^7$ stands for an optionally substituted phenyl, optionally substituted $C_1$-$C_{12}$-alkyl, optionally singly or multiply interrupted with oxygen and/or sulphur and/or $NR^{10}$, optionally hydroxy- or $C_1$-$C_4$-alkyl-substituted $C_3$-$C_7$-cycloalkyl or up to doubly substituted with $R^{10}$ and $R^{11}$ heteroaryl.

**8.** 3-[4-(4-chlorphenoxy)-phenyl]-3-(3,4-dimethoxyphenyl)-acrylmorpholide.

**9.** 3-(4-n-butylphenyl)-phenyl)-3-(3,4-dimethoxyphenyl)-acrylmorpholide.

**10.** 3-[4-(4-chlorophenyl)-phenyl]-3-(3,4-dimethoxyphenyl)-acryl-methyl-ethylamide.

**11.** Fungicidal agents, characterised by a content of a compound according to Claim 1 to 10 together with normal auxiliary and/or carrier substances and optionally with one or more additional active substances.

**12.** Use of compounds according to one of the Claims 1 to 10 optionally in combination with one or more further fungicidal active substances for the control of phytopathogenic fungi, especially false mildew.

**13.** Process for the preparation of compounds according to Claims 1 to 10 by methods known in themselves, characterised in that

a) an acrylic acid of the formula

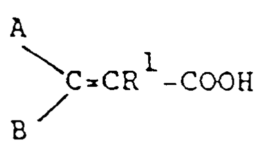

(II)

wherein A, B and $R^1$ have the above meaning, or a reactive derivative, which is optionally prepared in situ, is reacted with an amine of the formula

HQ III

in which Q has the above meaning, or that

b) a ketone of the formula

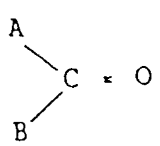

(IV),

wherein A and B have the above meaning, is reacted with a phosphonoacetic acid derivative of the formula

$(R'O)_2P(O)-CHR^1-COQ$ (V),

wherein Q and $R^1$ have the above meaning and R' stands for a $C_1$-$C_4$-alkyl group, or that
c) in an acrylamide of the formula

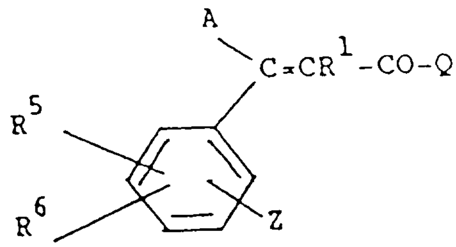

XI)

where A, Q, $R^1$, $R^5$ and $R^6$ have the above meaning and Z stands for hydrogen or for a substituent which can be replaced by the residue $-XY-R^7$ or converted into this residue, such a substitution or conversion is performed.
or that
d) the residue A or B is introduced by the Heck reaction of compounds of the formula

B-CH=CH-COQ (XIVa)

or

A-CH=CH-COQ (XIVb),

wherein A, B and Q have the above meaning, but are not substituted by bromine or iodine, with compounds of the formula

A - Hal (XVa)

or

B - Hal (XVb),

wherein Hal means bromine or iodine and A and B have the above meaning, but contain no additional bromine or iodine atoms, in presence of palladium(0)
and that compounds obtained according to (a) to (d) are optionally subjected to isomer separation and/or such compounds obtained which are capable of salt formation are optionally converted into the salts.

**Claims for the following Contracting States : AT, ES**

**1.** Process for the preparation of compounds of the formula

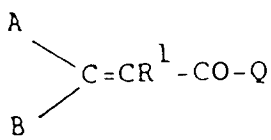

(I)

in which
A stands for

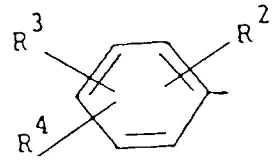

B for

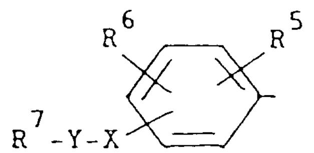 , 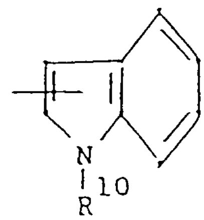 , 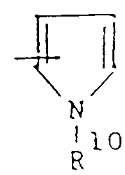

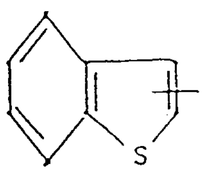 , 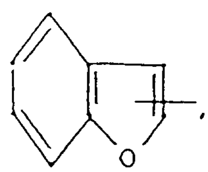 , 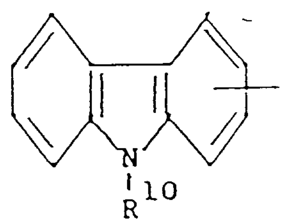

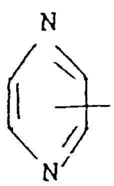 , 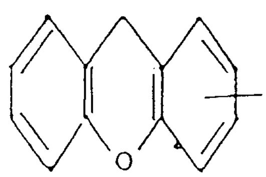 or 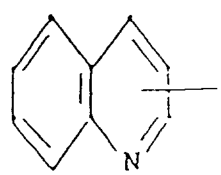

$R^1$ for hydrogen, halogen, cyano or $C_1$-$C_4$-alkyl optionally singly or multiply substituted with hydroxy, alkoxy, mercapto, halogen, alkylthio, nitro, cyano, amino or mono- or di-$C_1$-$C_4$-alkylamino,

Q stands for

$NR^8R^9$ or

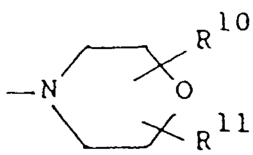

$R^2$, $R^3$, $R^4$, $R^5$ and $R^6$, which can be the same or different, stand for hydrogen, halogen, nitro, cyano, carboxy, hydroxy, $C_1$-$C_4$-alkoxycarbonyl, $CONR^{10}R^{11}$, $NR^{10}R^{11}$, $NR^{10}$-CO-$R^{11}$,
or for $C_1$-$C_6$-alkyl, $C_1$-$C_4$-alkoxy or $C_1$-$C_4$-alkyl-$S(O)_p$ (with p = 0, 1, 2), optionally singly or multiply substituted with hydroxy, alkoxy, mercapto, halogen, alkylthio, nitro, cyano, amino or mono- or di-$C_1$-$C_4$-alkylamino; $C_3$-$C_7$-cycloalkyl optionally up to triply substituted with alkyl, halogen, hydroxy, oxo or amino, phenyl, phenoxy or phenyl-$S(O)_p$- (with p = 0, 1, 2) optionally up to triply substituted with halogen, $C_1$-$C_4$-alkyl, alkylthio, alkoxy, nitro, cyano, amino, monoalkylamino, dialkylamino, haloalkyl, haloalkylthio, haloalkoxy, carboxy or alkoxycarbonyl,

$R^8$ and $R^9$, which can be the same or different, stand for hydrogen, $C_1$-$C_4$-alkyl, $C_3$-$C_7$-alkenyl, $C_3$-$C_7$-alkynyl or alkoxyalkyl optionally singly or multiply substituted with hydroxy, alkoxy, mercapto, halogen, alkylthio, nitro, cyano, amino or mono- or di-$C_1$-$C_4$-alkylamino; $C_3$-$C_7$-cycloalkyl optionally up to triply substituted with alkyl, halogen, hydroxy, oxo or amino; phenyl or benzyl optionally up to triply substituted with halogen, $C_1$-$C_4$-alkyl, alkylthio, alkoxy, nitro, cyano, amino, monoalkylamino, dialkylamino, haloalkyl, haloalkylthio, haloalkoxy, carboxy or alkoxycarbonyl,

$R^{10}$ and $R^{11}$, which can be the same or different, stand for hydrogen, benzyl, phenyl or $C_1$-$C_4$-alkyl, as substituents in heteroaryl also for halogen, amino or mono- or di-$C_1$-$C_4$-alkylamino, or together for $-(CH_2)_4-$ or $-(CH_2)_5-$,

X - Y stands for a single bond, -O-, $-S(O)_p$ (with p = 0, 1, 2), $-CONR^{10}-$, $-NR^{10}CO-$, $-NR^{11}CONR^{10}-$, -N=N-, $-CHR^{10}-S(O)_p-$(with p = 0, 1, 2), $-CHR^{10}O-$, $SO_2NR^{10}-$, -N=CH-, $-C_nH_{2n}-$ (with n = 1 to 10), -CH=CH-, $-NR^{10}CSNR^{11}-$, $-NR^{10}-$, $-R^{10}N-CHR^{11}-$, $-CHR^{10}NR^{11}-$, $-O-CHR^{10}-$, $-S(O)_p-CHR^{10}-$ (with p = 0, 1, 2), $-NR^{10}SO_2-$ or $-OSO_2-$; $-O_2SO-$; $-O_2S-NR^{10}-$; -C≡C-; -S-S-; -CHOH-; -CO-; $>C=CH_2$; $>C=CH-COOH$; -NH-NH-; -N(O)=N-;

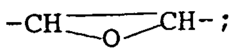

$-N(R^{10})-N=CH-$; $-N(R^{10})-NH-CO-$; -NH-CH=CH-; $>CH-C_6H_5$; $-COCH_2O-$; $-CO-CH_2S-$; $-COCH_2NR^{10}-$; $-OCH_2CO$; $-SCH_2CO-$; -CH=N-; $-NR^{10}CH_2CO-$; -CH=NO-; $-CH_2O-CONR^{10}-$; $-CH_2O-CO-$; $-S-CH_2-$, $-CH_2CO-$, -CO-O-, -O-CO-, -CO-S- or -S-CO-,

$R^7$ stands for hydrogen, $NR^{10}R^{11}$, $PO(OR^{10})(OR^{11})$, $(CH_2)_q-CO-O-R^{10}$ (with q = 0, 1, 2, 3), cyano, $CONHR^{10}R^{11}$, $SO_2NR^{10}R^{11}$, tri-$C_1$-$C_4$-alkyl-silyl, heteroaryl up to doubly substituted with $R^{10}$ and $R^{11}$ where heteroaryl stands for pyrazolyl-, pyrazinyl-, imidazolyl-, 1,2,4-triazolyl-, morpholinyl-, piperidinyl-, pyrrolyl-, pyrrolidinyl-, 2,5-dioxopyrrolidinyl-, 1,3-isoindoldionyl- and pyridinyl radicals, and residues derived from indole, benzofuran, quinoline or benzothiophen or stands for

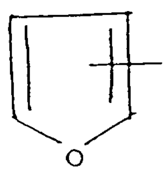 , 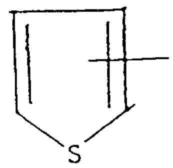 , 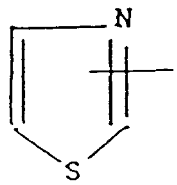 , 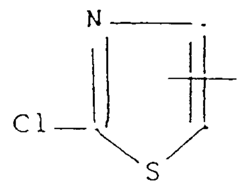

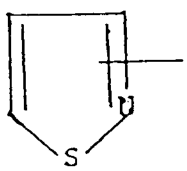 , 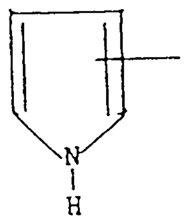 , 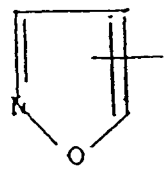 , 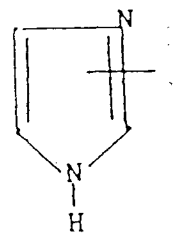

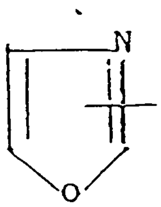 , 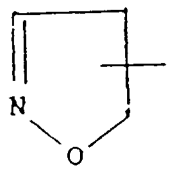 , 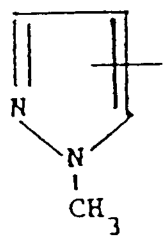 , 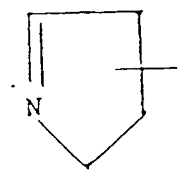

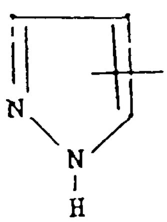 , 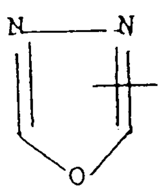 , 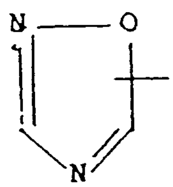 , 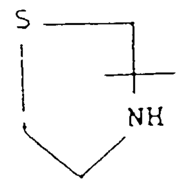

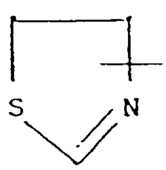 , 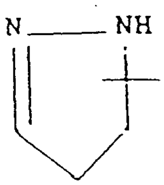 , 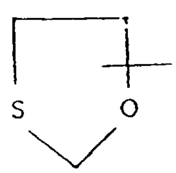 , 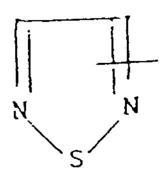

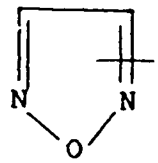 , 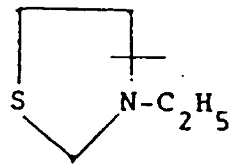 , 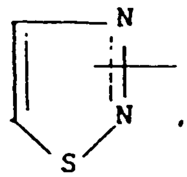 , 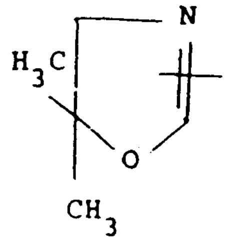

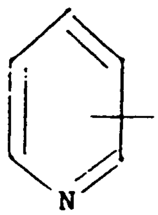 , 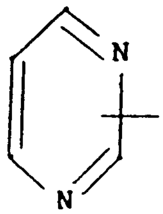 , 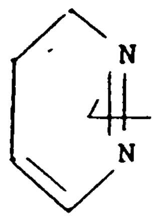 , 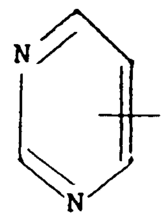

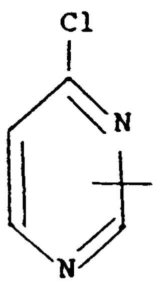 , 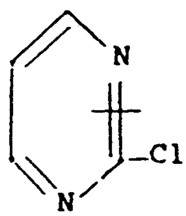 , 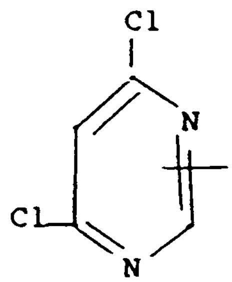 , 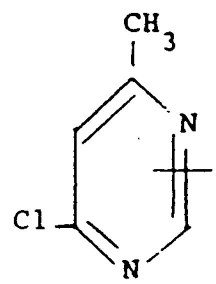

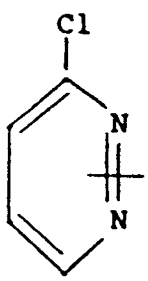 , 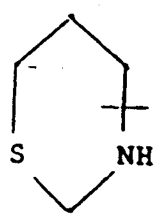 , 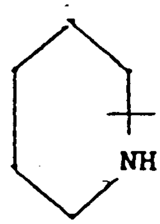 , 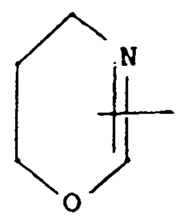

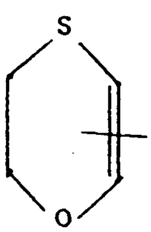 , 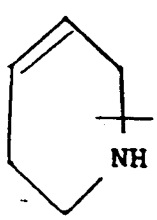 , 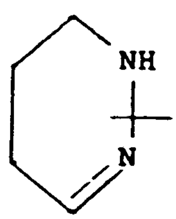 , 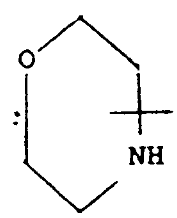

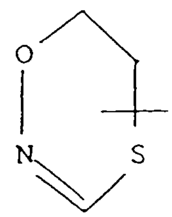 , 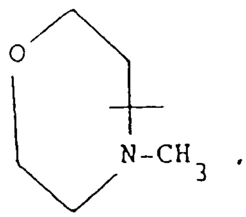 , 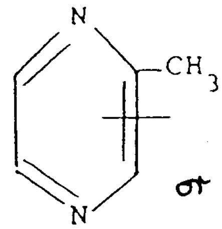 or 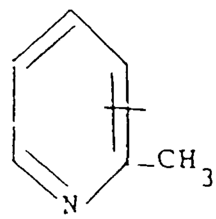

or

stand for [(1-formylamino-2,2,2-trichlor)ethyl]-amino, 1-(3,4-dimethoxyphenyl)-3-(morpholin-4-yl)-prop-1-en-3-one,

phenyl optionally up to triply substituted with halogen, $C_1$-$C_4$ alkyl, alkylthio, alkoxy, nitro, cyano, amino, monoalkylamino, dialkylamino, haloalkyl, haloalkylthio, haloalkoxy, carboxy or alkoxycarbonyl; alkyl with up to 12 C-atoms optionally interrupted with oxygen and/or sulphur atoms and optionally singly or multiply substituted with hydroxy, alkoxy, mercapto, halogen, alkylthio, nitro, cyano, amino or mono- or di-$C_1$-$C_4$-alkylamino; cycloalkyl optionally interrupted with oxygen and/or sulphur atoms and/or nitrogen and optionally up to triply substituted with alkyl, halogen, hydroxy, oxo or amino; phenyl-substituted $C_1$-$C_3$-alkyl, $C_2$-$C_6$-alkenyl, $C_2$-$C_6$-alkynyl, $C_5$-$C_8$-cycloalkenyl or naphthyl,

if X - Y stands for a single bond, $R^7$ and $R^6$ can also together stand for a vicinal bridge of the formula

$-(CH_2)_n$-E- (with n = 2, 3), -(CH=CH)-D or

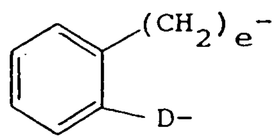

(with e = 0 or 1)

where

E stands for $CH_2$, O, S or $NR^{10}$

D stands for $CH_2$, O, S, $NR^{10}$, $-CH_2-CH_2-$ or -CH=CH-, and optionally salts thereof, wherein alkyl means $C_1$-$C_{12}$-C-atoms,

and the compounds:-

3-(3,4-dimethoxyphenyl)-3-[4-(4,5-dihydrooxazol-2-yl)phenyl]-acrylmorpholide,

3-(3,4-dimethoxyphenyl)-3-(4-benzimidazol-2-yl-phenyl)-acrylmorpholide,

3-(3,4-dimethoxyphenyl)-3-[4-(1,2-dichlorvinyloxy)-phenyl]-acrylmorpholide,

3-(3,4-dimethoxyphenyl)-3-(4-benzothiazol-2-yl-thiomethylphenyl)-acrylmorpholide,

3-(3,4-dimethoxyphenyl)-3-(4-benzothiazol-2-yl-thiophenyl)-acrylmorpholide,

3-(3,4-dimethoxyphenyl)-3-[4-(3-p-toluene-sulphonylureido)-phenyl]-acrylmorpholide,

3-(3,4-dimethoxyphenyl)-3-[4-(4,6-dichlor-1,3,5-triazin-2-yloxy)-phenyl]-acrylmorpholide,

3-(3,4-dimethoxyphenyl)-3-[4-(2,5-dioxopyrrol-1-yl)-phenyl]-acrylmorpholide,

3-(3,4-dimethoxyphenyl)-3-[4-(6-chlor-2-ethylaminopyrimidine-4-yloxy)-phenyl]-acrylmorpholide,

3-(3,4-dimethoxyphenyl)-3-[4-(6-chlor-2-dimethylaminopyrimidine-4-yloxy)-phenyl]-acrylmorpholide,

3-(3,4-dimethoxyphenyl)-3-[4-(6-chlor-2-isopropylaminopyrimidine-4-yloxy)-phenyl]-acrylmorpholide;

with the exception of the object which is disclosed in EP-A-0120321 and with the exception of the compounds of the formula I in which A and B both stand for a residue from the group phenyl, monohalophenyl, mononitrophenyl and monoaminophenyl, by methods known in themselves, characterised in that

a) an acrylic acid of the formula

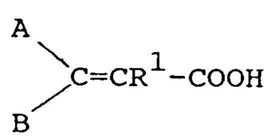 (II)

wherein A, B and $R^1$ have the above meaning, or a reactive derivative, which is optionally prepared in situ, is reacted with an amine of the formula

HQ III

wherein Q has the above meaning, or that
b) a ketone of the formula

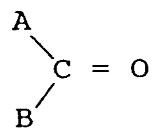

(IV)

wherein A and B have the above meaning, is reacted with a phosphonoacetic acid derivative of the formula

$(R'O)_2P(O)-CHR^1-COQ$ (V)

wherein Q and $R^1$ have the above meaning and R' stands for a $C_1$-$C_4$-alkyl group, or that
c) in an acrylamide of the formula

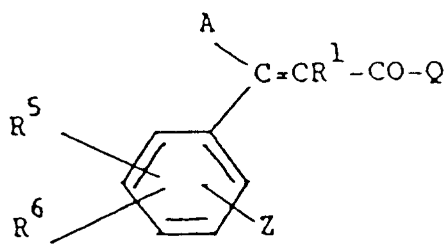

XI)

wherein A, Q, $R^1$, $R^5$ and $R^6$ have the above meaning and Z stands for hydrogen or for a substituent which can be replaced by the residue $-XY-R^7$ or converted into this residue, such a substitution or conversion is performed.
or that
d) the residue A or B is introduced by the Heck reaction of compounds of the formula

B-CH=CH-COQ (XIVa)

or

A-CH=CH-COQ (XIVb)

wherein A, B and Q have the above meaning, but are not substituted by bromine or iodine, with compounds of the formula

A - Hal (XVa)

or

B - Hal (XVb)

wherein Hal means bromine or iodine and A and B have the above meaning, but contain no additional bromine or iodine atoms, in presence of palladium(0)
and that compounds obtained according to (a) to (d) are optionally subjected to isomer separation and/or such compounds obtained which are capable of salt formation are optionally converted into

the salts.

**2.** Process for the preparation of compounds of the formula I according to Claim 1

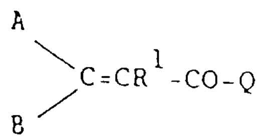

(I)

in which

A stands for

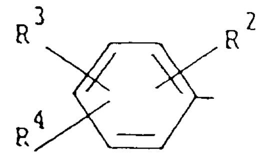

B for

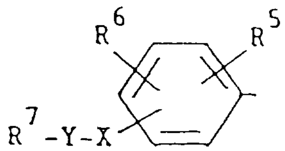
,
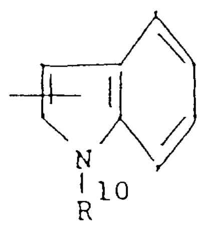
,
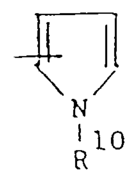

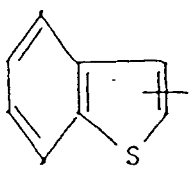
,
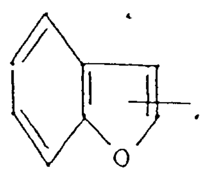
,
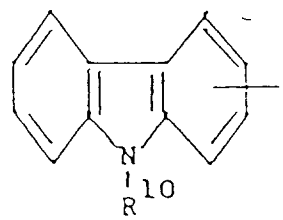

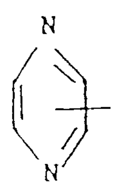
,
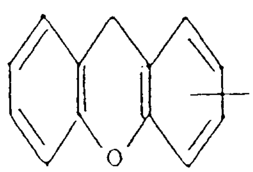
or
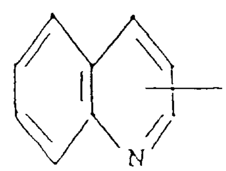

$R^1$ for hydrogen, halogen, cyano or $C_1$-$C_4$-alkyl optionally singly or multiply substituted with hydroxy, alkoxy, mercapto, halogen, alkylthio, nitro, cyano, amino or mono- or di-$C_1$-$C_4$-alkylamino,

Q stands for

$NR^8R^9$ or

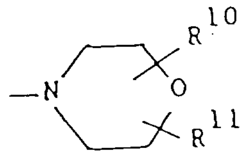

$R^2$, $R^3$, $R^4$, $R^5$ and $R^6$, which can be the same or different, stand for hydrogen, halogen, nitro, cyano, carboxy, hydroxy, $C_1$-$C_4$-alkoxycarbonyl, $CONR^{10}R^{11}$, $NR^{10}R^{11}$, $NR^{10}$-CO-$R^{11}$,
or for $C_1$-$C_6$-alkyl, $C_1$-$C_4$-alkoxy or $C_1$-$C_4$-alkyl-$S(O)_p$ (with p = 0, 1, 2), optionally singly or multiply substituted with hydroxy, alkoxy, mercapto, halogen, alkylthio, nitro, cyano, amino or mono- or di-$C_1$-$C_4$-alkylamino; $C_3$-$C_7$-cycloalkyl optionally up to triply substituted with alkyl, halogen, hydroxy, oxo or amino; phenyl, phenoxy or phenyl-$S(O)_p$- (with p = 0, 1, 2) optionally up to triply substituted with halogen, $C_1$-$C_4$-alkyl, alkylthio, alkoxy, nitro, cyano, amino, monoalkylamino, dialkylamino, haloalkyl, haloalkylthio, haloalkoxy, carboxy or alkoxycarbonyl,

$R^8$ and $R^9$, which can be the same or different, stand for hydrogen,
$C_1$-$C_4$-alkyl, $C_3$-$C_7$-alkenyl, $C_3$-$C_7$-alkynyl or alkoxyalkyl optionally singly or multiply substituted with hydroxy, alkoxy, mercapto, halogen, alkylthio, nitro, cyano, amino or mono- or di-$C_1$-$C_4$-alkylamino; $C_3$-$C_7$-cycloalkyl optionally up to triply substituted with alkyl, halogen, hydroxy, oxo or amino; phenyl or benzyl optionally up to triply substituted with halogen, $C_1$-$C_4$-alkyl, alkylthio, alkoxy, nitro, cyano, amino, monoalkylamino, dialkylamino, haloalkyl, haloalkylthio, haloalkoxy, carboxy or alkoxycarbonyl,

$R^{10}$ and $R^{11}$, which can be the same or different, stand for hydrogen, benzyl, phenyl or $C_1$-$C_4$-alkyl, as substituents in heteroaryl also for halogen, amino or mono- or di-$C_1$-$C_4$-alkylamino, or together for $-(CH_2)_4-$ or $-(CH_2)_5-$,

X - Y stands for a single bond,
-O-, $-S(O)_p$ (with p = 0, 1, 2), $-CONR^{10}-$, $-NR^{10}CO-$, $-NR^{11}CONR^{10}-$, -N=N-, $-CHR^{10}-S(O)_p-$(with p = 0, 1, 2), $-CHR^{10}O-$, $-SO_2NR^{10}-$, -N=CH-, $-C_nH_{2n}-$ (with n = 1 to 10), -CH=CH-, $-NR^{10}CSNR^{11}-$, $-NR^{10}-$, $-R^{10}N-CHR^{11}-$, $-CHR^{10}NR^{11}-$, $-O-CHR^{10}-$, $-S(O)_p-CHR^{10}-$ (with p = 0, 1, 2), $-NR^{10}SO_2-$ or $-OSO_2-$; $-O_2SO-$; $-O_2S-NR^{10}-$; -C≡C-; -S-S-; -CHOH-; -CO-; $>C=CH_2$; $>C=CH-COOH$; -NH-NH-; -N(O)=N-;

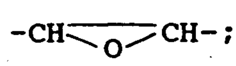

$-N(R^{10})-N=CH-$; $-N(R^{10})-NH-CO-$; -NH-CH=CH-; $>CH-C_6H_5$; $-COCH_2O-$; $-CO-CH_2S-$; $-COCH_2NR^{10}-$; $-OCH_2CO$; $-SCH_2CO-$; -CH=N-; $-NR^{10}CH_2CO-$;-CH=NO-; $-CH_2O-CONR^{10}-$; $-CH_2O-CO-$; $-S-CH_2-$, $-CH_2CO-$, -CO-O-, -O-CO-, -CO-S- or -S-CO-,

$R^7$ stands for hydrogen, $NR^{10}R^{11}$, $PO(OR^{10})(OR^{11})$, $(CH_2)_q-CO-O-R^{10}$ (with q = 0, 1, 2, 3), cyano, $CONHR^{10}R^{11}$, $SO_2NR^{10}R^{11}$,
tri-$C_1$-$C_4$-alkyl-silyl, heteroaryl up to doubly substituted with $R^{10}$ and $R^{11}$ wherein heteroaryl stands for pyrazolyl-, pyrazinyl-, imidazolyl-, 1,2,4-triazolyl-, morpholinyl-, piperidinyl-, pyrrolyl-, pyrrolidinyl-, 2,5-dioxopyrrolidinyl-, 1,3-isoindoldionyl- and pyridinyl radicals, and residues derived from indole, benzofuran, quinoline or benzothiophen or stands for

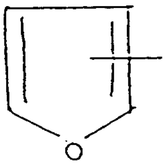 , 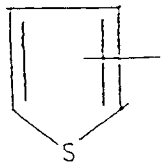 , 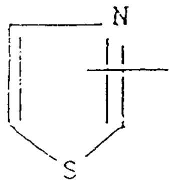 , 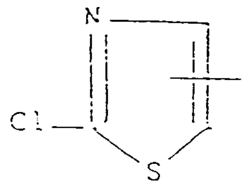

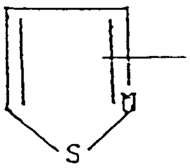 , 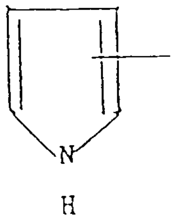 , 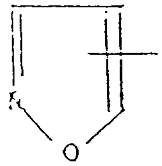 , 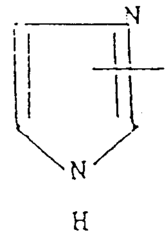

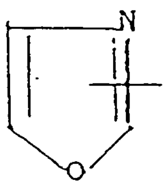 , 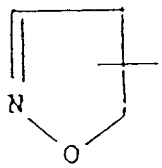 , 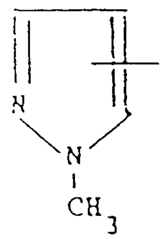 , 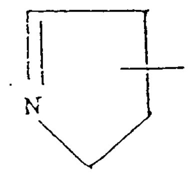

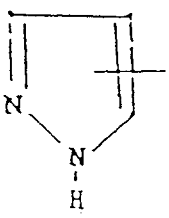 , 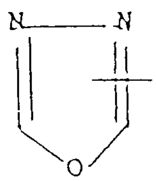 , 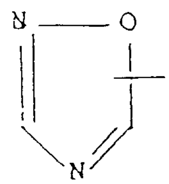 , 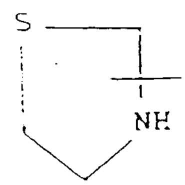

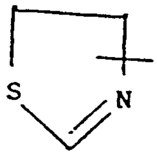 , 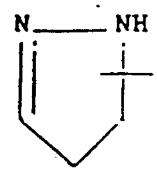 , 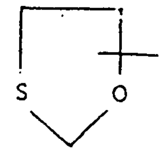 , 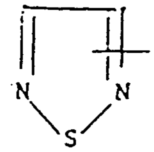

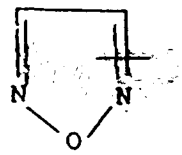 , 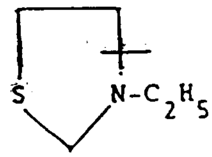 , 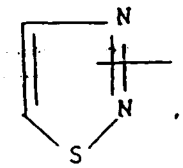 , 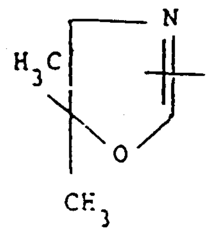

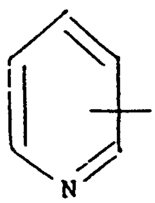 , 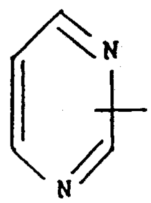 , 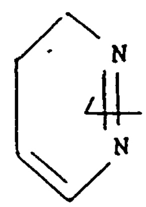 , 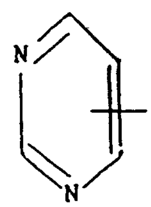

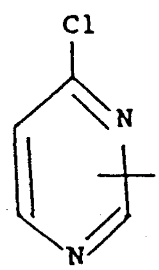 , 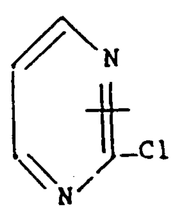 , 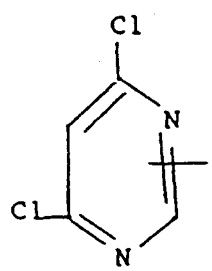 , 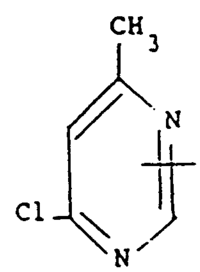

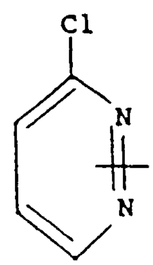 , 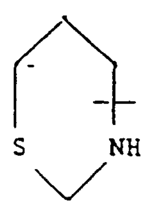 , 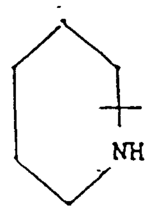 , 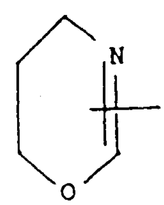

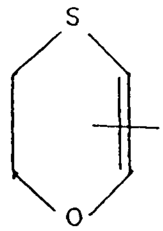 , 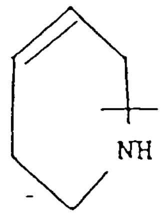 , 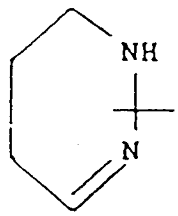 , 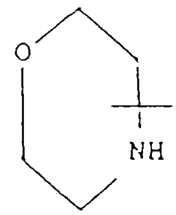

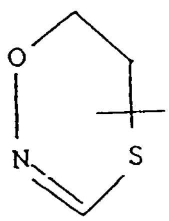 , 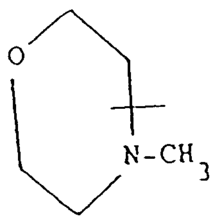 , 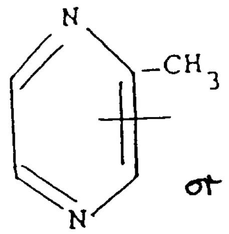 or 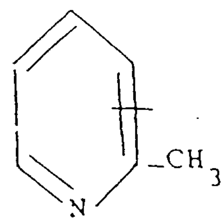

or

stands for [(1-formylamino-2,2,2-trichlor)ethyl]-amino, 1-(3,4-dimethoxyphenyl)-3-(morpholin-4-yl)-prop-1-en-3-one,

phenyl optionally up to triply substituted with halogen, $C_1$-$C_4$ alkyl, alkylthio, alkoxy, nitro, cyano, amino, monoalkylamino, dialkylamino, haloalkyl, haloalkylthio, haloalkoxy, carboxy or alkoxycarbonyl; alkyl with up to 12 C-atoms optionally interrupted with oxygen and/or sulphur atoms and optionally singly or multiply substituted with hydroxy, alkoxy, mercapto, halogen, alkylthio, nitro, cyano, amino or mono- or di-$C_1$-$C_4$-alkylamino; cycloalkyl optionally interrupted with oxygen and/or sulphur atoms and/or nitrogen and optionally up to triply substituted with alkyl, halogen, hydroxy, oxo or amino; phenyl-substituted $C_1$-$C_3$-alkyl, $C_2$-$C_6$-alkenyl, $C_2$-$C_6$-alkynyl, $C_5$-$C_8$-cycloalkenyl or naphthyl,

if X - Y stands for a single bond, $R^7$ and $R^6$ can also together stand for a vicinal bridge of the formula

$-(CH_2)_n-E-$ (with n = 2, 3), -(CH=CH)-D or

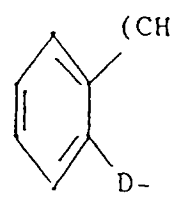

(with e = 0 or 1)

wherein

E stands for $CH_2$, O, S or $NR^{10}$

D stands for $CH_2$, O, S, $NR^{10}$, $-CH_2-CH_2-$ or -CH=CH-, and optionally salts thereof, wherein alkyl means $C_1$-$C_{12}$-C-atoms;

and the compounds:-

3-(3,4-dimethoxyphenyl)-3-[4-(4,5-dihydrooxazol-2-yl)phenyl]-acrylmorpholide,
3-(3,4-dimethoxyphenyl)-3-(4-benzimidazol-2-yl-phenyl)-acrylmorpholide,
3-(3,4-dimethoxyphenyl)-3-[4-(1,2-dichlorvinyloxy)-phenyl]-acrylmorpholide,
3-(3,4-dimethoxyphenyl)-3-(4-benzothiazol-2-yl-thiomethylphenyl)-acrylmorpholide,
3-(3,4-dimethoxyphenyl)-3-(4-benzothiazol-2-yl-thiophenyl)-acrylmorpholide,
3-(3,4-dimethoxyphenyl)-3-[4-(3-p-toluene-sulphonylureido)-phenyl]-acrylmorpholide,
3-(3,4-dimethoxyphenyl)-3-[4-(4,6-dichlor-1,3,5-triazine-2-yloxy)-phenyl]-acrylmorpholide,
3-(3,4-dimethoxyphenyl)-3-[4-(2,5-dioxopyrrol-1-yl)-phenyl]-acrylmorpholide,
3-(3,4-dimethoxyphenyl)-3-[4-(6-chlor-2-ethylaminopyrimidine-4-yloxy)-phenyl]-acrylmorpholide,
3-(3,4-dimethoxyphenyl)-3-[4-(6-chlor-2-dimethylaminopyrimidine-4-yloxy)-phenyl]-acrylmorpholide,
3-(3,4-dimethoxyphenyl)-3-[4-(6-chlor-2-isopropylaminopyrimidine-4-yloxy)-phenyl]-acrylmorpholide;

with the exception of the compounds of the formula I in which

B stands for

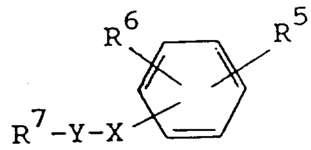

$R^1$ stands for hydrogen, $C_1$-$C_4$-alkyl, cyano, chlorine, bromine or iodine,

$R^2$, $R^3$, and $R^4$, which can be the same or different, stand for hydrogen, halogen, nitro, $C_1$-$C_4$-alkyl or alkoxy optionally singly or multiply substituted with fluorine or chlorine, amino, NH($C_1$-$C_4$-alkyl), N($C_1$-$C_4$-alkyl)$_2$, cyano, phenyl, $C_3$-$C_6$-cycloalkyl, hydroxy ($C_1$-$C_4$-alkyl), NHCOH, NHCO-($C_1$-$C_4$-alkyl), $CO_2H$, $CO_2$-($C_1$-$C_4$-alkyl), $CONR^{10}R^{11}$ in which $R^{10}$ and $R^{11}$, which can be the same or different, stand for $C_1$-$C_4$-alkyl, phenyl or benzyl, and also together for $-(CH_2)_4-$ or $-(CH_2)_5-$, and $R^{10}$ also for hydrogen, ($C_1$-$C_4$-alkyl)-S(O)$_p$ - (with p = 0, 1, 2) or hydroxy,

$R^5$ and $R^6$, which can be the same or different, stand for hydrogen, halogen, $C_1$-$C_4$-alkyl, $C_1$-$C_4$-alkoxy, ($C_1$-$C_4$-alkyl)-S(O)$_p$ (with p = 0, 1, 2) or hydroxy and $R^5$ also for nitro, $C_1$-$C_4$-alkyl or alkoxy optionally singly or multiply substituted with fluorine or chlorine, NHCOH or NHCO($C_1$-$C_4$-alkyl),

X - Y stands for a single bond,

$R^7$ stands for hydrogen, $C_1$-$C_4$-alkyl optionally singly or multiply substituted with fluorine or chlorine, amino, NH($C_1$-$C_4$-alkyl), N($C_1$-$C_4$-alkyl)$_2$, cyano, phenyl, $C_3$-$C_6$-cycloalkyl, hydroxy($C_1$-$C_4$-alkyl), $CO_2H$, $CO_2$($C_1$-$C_4$-alkyl), $CONR^{10}R^{11}$ in which $R^{10}$ and $R^{11}$, which can be the same or different, stand for $C_1$-$C_4$-alkyl, phenyl or benzyl, together also for $-(CH_2)_4-$ or $-(CH_2)_5-$, and $R^{10}$ also for hydrogen, $C_2$-$C_8$-alkyl interrupted with oxygen; or $R^7$ and $R^6$ also may together stand for a vicinal bridge of the formula -(CH = CH)-D, where D stands for -CH = CH-;

Q stands for $NR^8R^9$ or

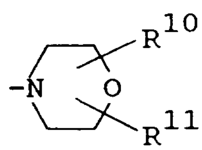 ,

in which

$R^8$ and $R^9$, which can be the same or different, stand for $C_1$-$C_4$-alkyl, $C_3$-$C_7$-cycloalkyl, phenyl, benzyl or $C_3$-$C_4$-alkenyl, and $R^8$ also for hydrogen, and

$R^{10}$ and $R^{11}$, which can be the same or different, stand for hydrogen or $C_1$-$C_4$-alkyl;

and with the exception of the compounds of the formula I in which both A and B stand for a residue from the group phenyl, monohalophenyl, mononitrophenyl and monoaminophenyl.

**3.** Process according to Claim 1 or 2 wherein A stands for 3,4-dimethoxyphenyl, 3-ethoxy-4-methoxyphenyl, 3-chloro-4-methoxyphenyl, 3-methyl-4-methoxyphenyl, 3-bromo-4-methoxyphenyl, 3-ethyl-4-methoxyphenyl, 3,4-dimethylphenyl and 3,5-dichlor-4-aminophenyl and B, Q and $R^1$ have the above meaning.

**4.** Process according to Claim 3, wherein $R^1$ means hydrogen.

**5.** Process according to Claim 1, 2, 3 or 4, wherein Q stands for $NR^8R^9$, where $R^8$ and $R^9$ are $C_1$-$C_4$-alkyl, or stand for the morpholino group.

**6.** Process according to Claim 1, 2, 3, 4 or 5, wherein B stands for

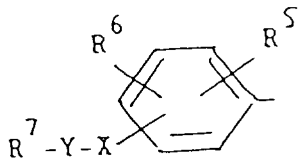

where -X-Y- means a single bond, oxygen, sulphur, $-CH_2O-$, $-OCH_2-$ or -N = N-.

**7.** Process according to Claim 6, wherein $R^5$ and $R^6$ mean hydrogen, -X-Y- represents a single bond, oxygen, sulphur or -N = N- and $R^7$ stands for an optionally substituted phenyl, optionally substituted $C_1$-$C_{12}$-alkyl, optionally singly or multiply interrupted with oxygen and/or sulphur and/or $NR^{10}$, optionally hydroxy- or $C_1$-$C_4$-alkyl-substituted $C_3$-$C_7$-cycloalkyl or up to doubly substituted with $R^{10}$ and $R^{11}$ heteroaryl.

**8.** Process according to Claim 1, 2, 3, 4, 5, 6 or 7, wherein the compound of the formula I is 3-[4-(4-chlorphenoxy)-phenyl]-3-(3,4-dimethoxyphenyl)-acrylmorpholide.

**9.** Process according to Claim 1, 2, 3, 4, 5, 6 or 7, wherein the compound of the formula I is 3-(4-n-butylphenyl)-phenyl)-3-(3,4-dimethoxyphenyl)-acrylmorpholide.

**10.** Process according to Claim 1, 2, 3, 4, 5, 6 or 7, wherein the compound of the formula I is 3-[4-(4-chlorophenyl)-phenyl]-3-(3,4-dimethoxyphenyl)-acryl-methyl-ethylamide.

**11.** Process for the preparation of fungicidal agents, characterised by a compound according to Claim 1 to 10 brought into combination with the normal auxiliary and/or carrier substances and optionally one or more additional active substances.

**12.** Use of compounds according to one of the Claims 1 to 10 optionally in combination with one or more further fungicidal active substances for the control of phytopathogenic fungi, especially false mildew.

**Revendications**
**Revendications pour les Etats contractants suivants : BE, CH, DE, FR, GB, GR, IT, LI, LU, NL, SE**

**1.** Composés de formule

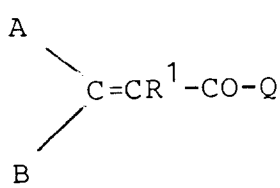

(I)

dans laquelle
A représente

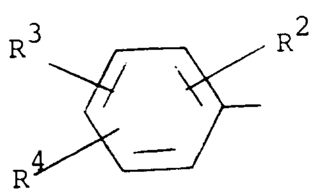

B représente

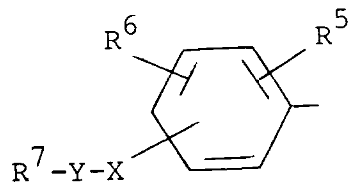
,
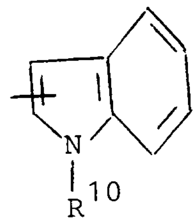
,
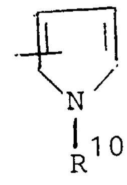

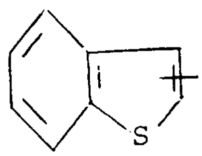
,
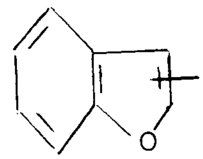
,
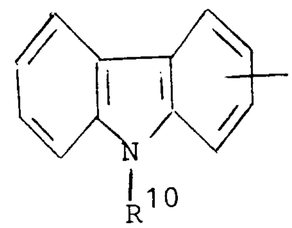

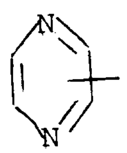
,
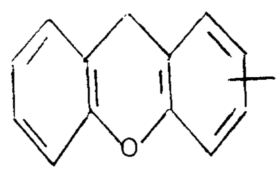
ou
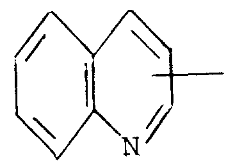

$R^1$ représente l'hydrogène; un halogène, un groupe cyano ou un groupe alkyle en C1-C4 portant éventuellement un ou plusieurs substituants hydroxy, alcoxy, mercapto, halogéno, alkylthio, nitro, cyano, amino ou mono- ou di-(alkyle en C1-C4)-amino,

Q représente
$NR^8R^9$ ou

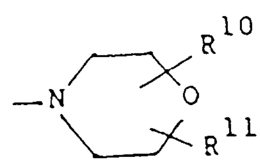

$R^2$, $R^3$, $R^4$, $R^5$ et $R^6$, qui peuvent avoir des significations identiques ou différentes, représentent chacun l'hydrogène; un halogène; un groupe nitro; un groupe cyano; un groupe carboxy; un groupe hydroxy; un groupe alcoxycarbonyle en C1-C4; un groupe $CONR^{10}R^{11}$; un groupe $NR^{10}R^{11}$; un groupe $NR^{10}$-CO-$R^{11}$; ou bien un groupe alkyle en C1-C6, alcoxy en C1-C4 ou (alkyle en C1-C4)-S-(O)p (avec p = 0, 1, 2), portant éventuellement un ou plusieurs substituants hydroxy, alcoxy, mercapto, halogéno, alkylthio, nitro, cyano, amino ou mono- ou di-(alkyle en C1-C4)-amino,
un groupe cycloalkyle en C3-C7 portant éventuellement jusqu'à trois substituants alkyle, halogéno, hydroxy, oxo ou amino, un groupe phényle, phénoxy ou phényl-S(O)p (avec p = 0, 1, 2) portant éventuellement jusqu'à trois substituants halogéno, alkyle en C1-C4, alkylthio, alcoxy, nitro, cyano,amino, monoalkylamino, dialkylamino, halogénoalkyle, halogénoalkylthio, halogénoalcoxy, carboxy ou alcoxycarbonyle,

$R^8$ et $R^9$, qui peuvent avoir des significations identiques ou différentes, représentent chacun l'hydrogène;
un groupe alkyle en C1-C3, alcényle en C3-C7, alcynyle en C3-C7 ou alcoxyalkyle portant éventuellement un ou plusieurs substituants hydroxy, alcoxy, mercapto, halogéno, alkylthio, nitro, cyano, amino ou mono- ou di-(alkyle en C1-C4)-amino, un groupe cyanoalkyle en C3-C7 portant éventuellement jusqu'à trois substituants alkyle, halogéno, hydroxy, oxo ou amino, un groupe phényle ou benzyle portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle,

$R^{10}$ et $R^{11}$, qui peuvent avoir des significations identiques ou différentes, représentent chacun l'hydrogène, un groupe benzyle, phényle ou alkyle en C1-C4, ou encore, en tant que substituants d'un noyau hétéroaryle, un halogène, un groupe amino, ou mono- ou di-(alkyle en C1-C4)-amino,
ou bien forment ensemble un groupe $-(CH_2)_4-$ ou $-(CH_2)_5-$,

X-Y représentent une liaison simple;
-O-; -S(O)p- (avec p = 0, 1, 2); $-CONR^{10}-$; $-NR^{10}CO-$; $-NR^{11}CONR^{10}-$; -N=N-; $-CHR^{10}$-S(O)p-(avec p = 0, 1, 2); $-CHR^{10}O-$; $-SO_2NR^{10}-$; -N=CH-; $-C_nH_{2n}-$ (avec n = 1 à 10); -CH=CH-; $-NR^{10}CSNR^{11}-$; $-NR^{10}-$; $-R^{10}N-CHR^{11}-$; $-CHR^{10}NR^{11}-$; $-O-CHR^{10}-$; -S(O)p-$CHR^{10}$- (avec p = 0, 1, 2); $-NR^{10}SO_2-$ ou $-OSO_2-$; $-O_2SO-$; $-O_2S-NR^{10}-$; -C≡C-; -S-S-; -CHOH-; -CO-;
$>C=CH_2$; $>C=CH-COOH$; -NH-NH-; -N(O)=N-;

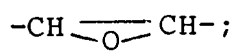

$-N(R^{10})-N=CH-$; $-N(R^{10})-NH-CO-$; -NH-CH=CH-; $>CH-C_6H_5$; $-COCH_2O-$; $-CO-CH_2S-$; $-COCH_2NR^{10}-$; $-OCH_2CO$; $-SCH_2CO-$; -CH=N-; $-NR^{10}CH_2CO-$; -CH=NO-; $-CH_2O-CONR^{10}-$; $-CH_2O-CO-$; $-S-CH_2-$; $-CH_2CO-$; -CO-O-; -O-CO-; -CO-S- ou -S-CO-;

$R^7$ représente l'hydrogène; $NR^{10}R^{11}$; $PO(OR^{10})(OR^{11})$; $(CH_2)q-CO-O-R^{10}$ (avec q = 0, 1, 2, 3); cyano; $CONR^{10}R^{11}$; $SO_2NR^{10}R^{11}$;
un groupe tri-(alkyle en C1-C4)-silyle; un noyau hétéroaryle portant jusqu'à deux substituants $R^{10}$ et $R^{11}$, le noyau hétéroaryle consistant en un noyau pyrazolyle, pyrazinyle, imidazolyle, 1,2,4-triazolyle, morpholinyle, pipéridinyle, pyrrolyle, pyrrolidinyle, 2,5-dioxopyrrolidinyle, 1,3-isoindole-dionyle, et pyridinyle ou en un noyau dérivé de l'indole, du benzofuranne, de la quinoléine ou du benzothiophène, ou en un noyau

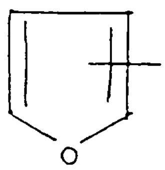 , 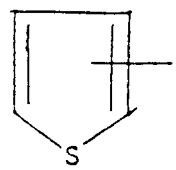 , 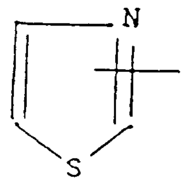 , 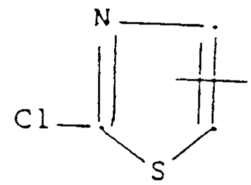

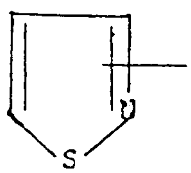 , 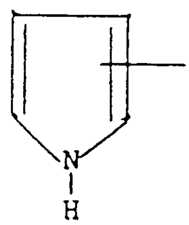 , 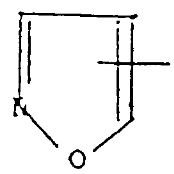 , 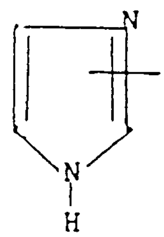

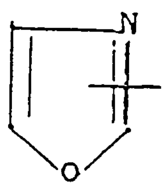 , 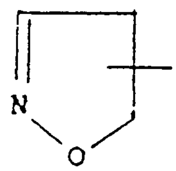 , 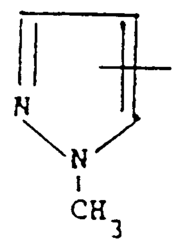 , 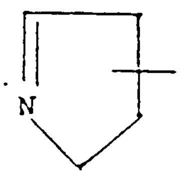

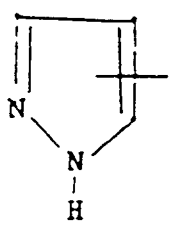 , 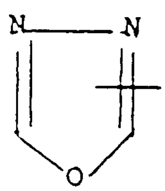 , 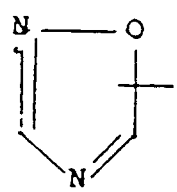 , 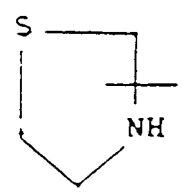

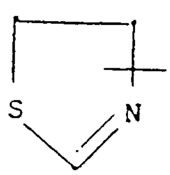 , 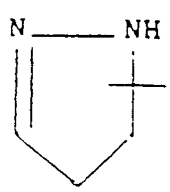 , 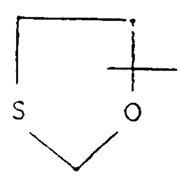 , 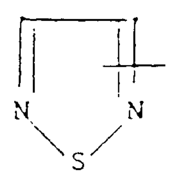

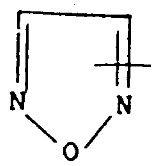 , 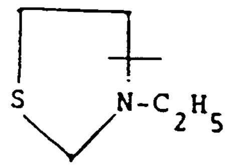 , 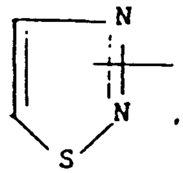 , 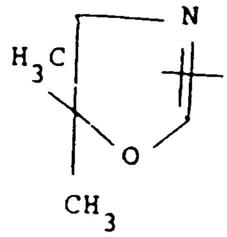

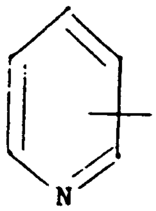 , 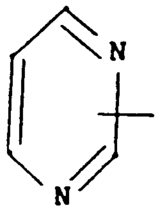 , 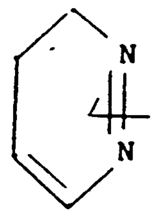 , 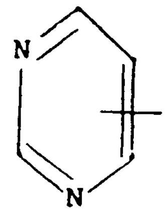

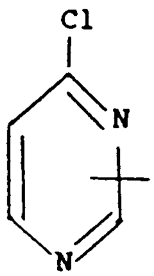 , 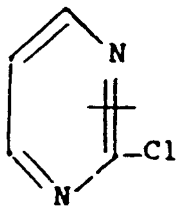 , 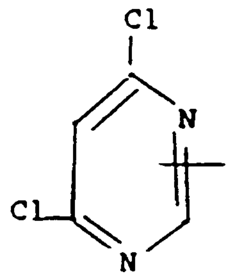 , 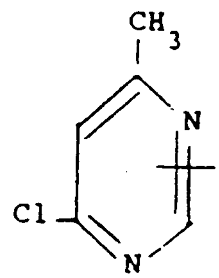

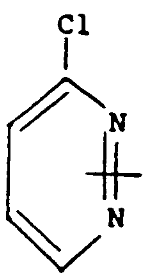 , 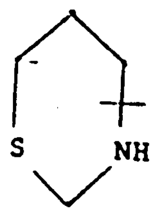 , 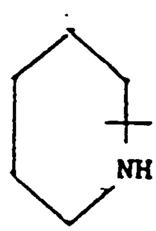 , 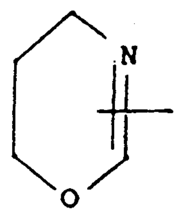

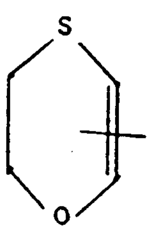 , 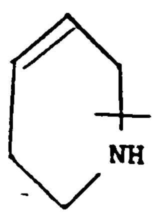 , 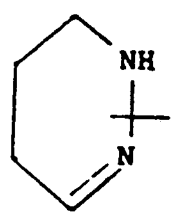 , 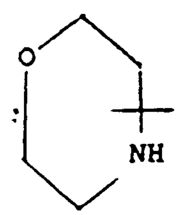

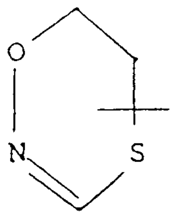 , 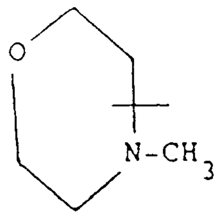 , 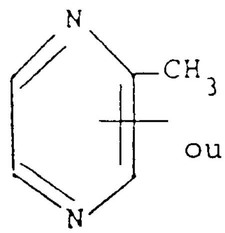 ou 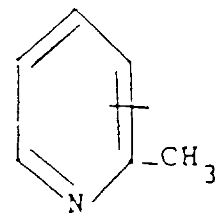

un groupe [(1-formylamino-2,2,2-trichloro)-éthyl]-amino;

un groupe 1-(3,4-diméthoxyphényl)-3-(morpholino-4-yl)-propa-1-ène-3-one;

un groupe phényle portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle; un

groupe alkyle contenant jusqu'à 12 atomes de carbone, éventuellement interrompu par des atomes d'oxygène et/ou de soufre et portant éventuellement un ou plusieurs substituants hydroxy, alcoxy, mercapto, halogéno, alkylthio, nitro, cyano, amino, mono- ou di-(alkyle en C1-C4)-amino, un groupe cycloalkyle éventuellement interrompu par des atomes d'oxygène et/ou de soufre et/ou d'azote et portant éventuellement jusqu'à trois substituants alkyle, halogéno, hydroxy, oxo ou amino, un groupe alkyle en C1-C3, alcényle en C2-C6, alcynyle en C2-C6, cycloalcényle en C5-C8 à substituant phényle ou un groupe naphtyle et lorsque X - Y représentent une liaison simple,

$R^7$ et $R^6$ peuvent encore former ensemble un pont vicinal de formule

$-(CH_2)_n-E-$ (avec n = 2, 3), -(CH = CH)-D ou

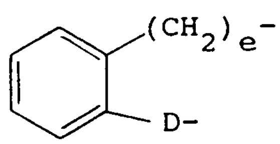

(avec e = 0 ou 1)

dans lesquels

E représente $CH_2$; O; S ou $NR^{10}$,

D représente $CH_2$; O; S; $NR^{10}$; $-CH_2-CH_2-$ ou -CH = CH-, et le cas échéant leurs sels;

dans lesquels les groupes alkyle contiennent de 1 à 12 atomes de carbone;

et les composés suivants :

3-(3,4-diméthoxyphényl)-3-[4-(4,5-dihydrooxazole-2-yl)phényl]-acrylomorpholide,

3-(3,4-diméthoxyphényl)-3-(4-benzimidazole-2-yl-phényl)-acrylomorpholide,

3-(3,4-diméthoxyphényl)-3-[4-(1,2-dichlorovinyloxy)-phényl]-acrylomorpholide,

3-(3,4-diméthoxyphényl)-3-(4-benzothiazole-2-yl-thiométhylphényl)-acrylomorpholide,

3-(3,4-diméthoxyphényl)-3-(4-benzothiazole-2-ylthio-phényl)-acrylomorpholide,

3-(3,4-diméthoxyphényl)-3-[4-(3-p-toluène-sulfonyluréido)-phényl]-acrylomorpholide,

3-(3,4-diméthoxyphényl)-3-[4-(4,6-dichloro-1,3,5-triazine-2-yloxy)-phényl]-acrylomorpholide,

3-(3,4-diméthoxyphényl)-3-[4-(2,5-dioxopyrrole-1-yl)-phényl]-acrylomorpholide,

3-(3,4-diméthoxyphényl)-3-[4-(6-chloro-2-éthylaminopyrimidine-4-yloxy)-phényl]-acrylomorpholide,

3-(3,4-diméthoxyphényl)-3-[4-(6-chloro-2-diméthylaminopyrimidine-4-yloxy)-phényl]-acrylomorpholide,

3-(3,4-diméthoxyphényl)-3-[4-(6-chloro-2-isopropylaminopyrimidine-4-yloxy)-phényl]-acrylomorpholide;

à l'exception de l'objet décrit dans le brevet européen n° 0 120 321 et à l'exception des composés de formule I dans laquelle A et B représentent tous deux un groupe choisi parmi les groupes phényle, monohalogénophényle, mononitrophényle et monoaminophényle.

**2.** Composés de formule I de la revendication 1

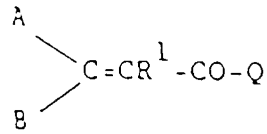

(I)

dans laquelle

A représente

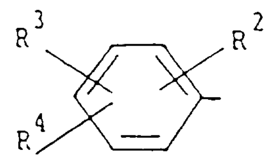

B représente

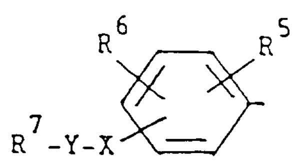

,

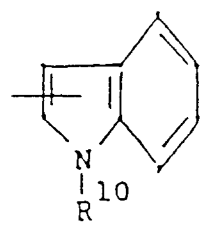

,

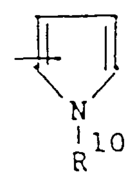

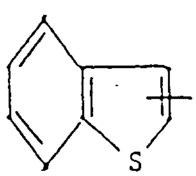

,

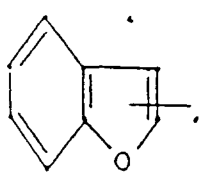

,

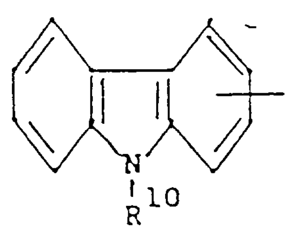

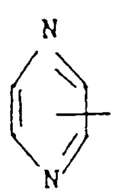

,

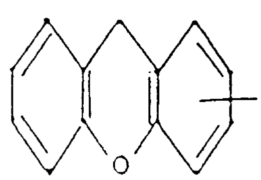

ou

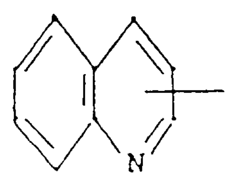

$R^1$ représente l'hydrogène; un halogène, un groupe cyano ou alkyle en C1-C4 portant éventuellement un ou plusieurs substituants hydroxy, alcoxy, mercapto, halogéno, alkylthio, nitro, cyano, amino ou mono- ou di-(alkyle en C1-C4)-amino,

Q représente

$NR^8R^9$ ou

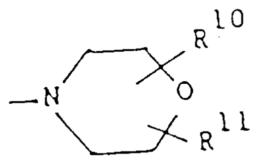

$R^2$, $R^3$, $R^4$, $R^5$ et $R^6$, qui peuvent avoir des significations identiques ou différentes, représentent chacun l'hydrogène, un halogène, un groupe nitro, cyano, carboxy, hydroxy, (alcoxy en C1-C4)-carbonyle, $CONR^{10}R^{11}$, $NR^{10}R^{11}$, $NR^{10}$-CO-$R^{11}$, ou un groupe alkyle en C1-C7, alcoxy en C1-C4 ou (alkyle en C1-C4)-S(O)p (avec p = 0, 1, 2) portant éventuellement un ou plusieurs substituants hydroxy, alcoxy, mercapto, halogéno, alkylthio, nitro, cyano, amino ou mono- ou di-(alkyle en C1-C4)-amino, un groupe cycloalkyle en C3-C7 portant éventuellement jusqu'à trois substituants alkyle, halogéno, hydroxy, oxo ou amino, un groupe phényle, phénoxy ou phényl-S(O)p (avec p = 0, 1, 2) portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle,

$R^8$ et $R^9$, qui peuvent avoir des significations identiques ou différentes, représentent chacun l'hydrogène;

un groupe alkyle en C1-C4, alcényle en C3-C7 ou alcoxyalkyle portant éventuellement un ou plusieurs substituants hydroxy, alcoxy, mercapto, halogéno, alkylthio, nitro, cyano, amino ou mono- ou di-(alkyle en C1-C4)-amino, un groupe cycloalkyle en C3-C7 portant éventuellement jusqu'à trois substituants alkyle, halogéno, hydroxy, oxo ou amino, un groupe phényle ou benzyle portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle,

$R^{10}$ et $R^{11}$, qui peuvent avoir des significations identiques ou différentes, représentent chacun l'hydrogène, un groupe benzyle, phényle ou alkyle en C1-C4 ou bien encore, en tant que substituants d'un noyau hétéroaryle, un halogène, un groupe amino ou mono- ou di-(alkyle en C1-C4)-amino,

ou forment ensemble un groupe $-(CH_2)_4-$ ou $-(CH_2)_5-$,

X - Y représentent une liaison simple;

-O-; -S(O)p- (avec p = 0, 1, 2); $-CONR^{10}-$; $-NR^{10}CO-$; $-NR^{11}CONR^{10}-$; -N=N-; $-CHR^{10}$-S(O)p-(avec p = 0, 1, 2); $-CHR^{10}O-$; $-SO_2NR^{10}-$; -N=CH-; $-C_nH_{2n}-$ (avec n = 1 à 10); -CH=CH-; $-NR^{10}CSNR^{11}-$; $-NR^{10}-$; $-R^{10}N-CHR^{11}-$; $-CHR^{10}NR^{11}-$; $-O-CHR^{10}-$; -S(O)p-$CHR^{10}$- (avec p = 0, 1, 2); $-NR^{10}SO_2-$ ou $-OSO_2-$; $-O_2SO-$; $-O_2S-NR^{10}-$; -C≡C-; -S-S-; -CHOH-; -CO-;

>C=$CH_2$; >C=CH-COOH; -NH-NH-; -N(O)=N-;

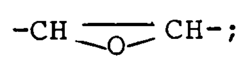

$-N(R^{10})$-N=CH-; $-N(R^{10})$-NH-CO-; -NH-CH=CH-; >CH-$C_6H_5$; $-COCH_2O-$; $-CO-CH_2S-$; $-COCH_2NR^{10}-$; $-OCH_2CO$; $-SCH_2CO-$; -CH=N-; $-NR^{10}CH_2CO-$; -CH=NO-; $-CH_2O-CONR^{10}-$; $-CH_2O-CO-$; $-S-CH_2-$; $-CH_2CO-$; -CO-O-; -O-CO-; -CO-S- ou -S-CO-;

$R^7$ représente l'hydrogène; $NR^{10}R^{11}$; $PO(OR^{10})(OR^{11})$; $(CH_2)q-CO-O-R^{10}$ (avec q = 0, 1, 2, 3); cyano; $CONR^{10}R^{11}$; $SO_2NR^{10}R^{11}$;

un groupe tri-(alkyle en C1-C4)-silyle; un noyau hétéroaryle portant jusqu'à deux substituants $R^{10}$ et $R^{11}$, ce noyau hétéroaryle consistant en un noyau pyrazolyle, pyrazinyle, imidazolyle, 1,2,4-triazolyle, morpholinyle, pipéridinyle, pyrrolyle, pyrrolidinyle, 2,5-dioxopyrrolidinyle, 1,3-isoindole-dionyle ou pyridinyle, ou en un noyau dérivé de l'indole, du benzofuranne, de la

quinoléine ou du benzothiophène, ou en un noyau

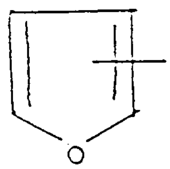 , 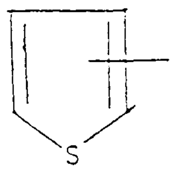 , 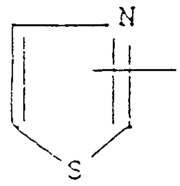 , 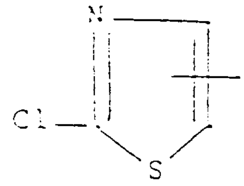

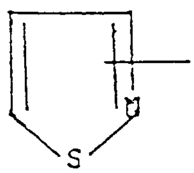 , 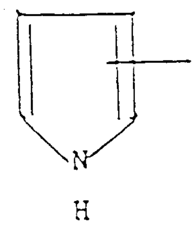 , 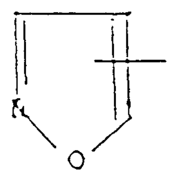 , 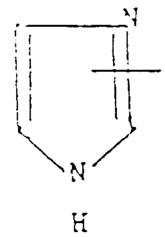

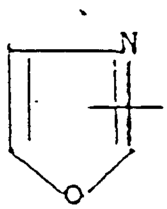 , 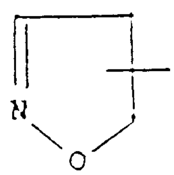 , 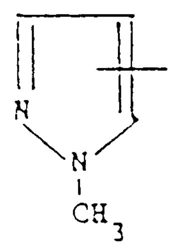 , 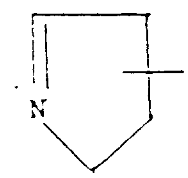

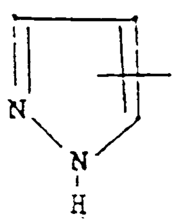 , 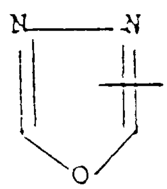 , 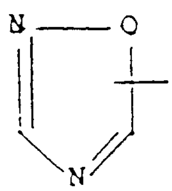 , 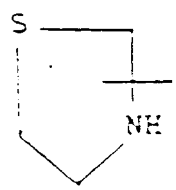

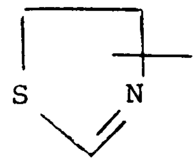 , 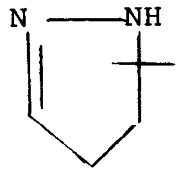 , 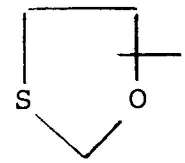 , 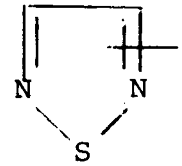

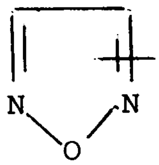 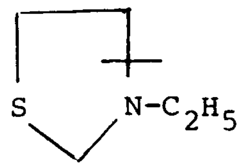, 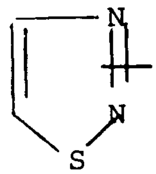 , 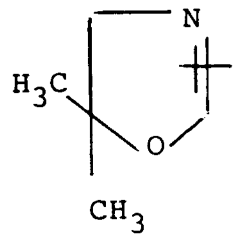

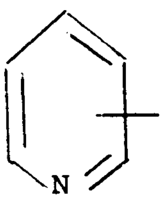 , 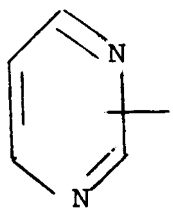 , 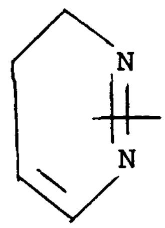 , 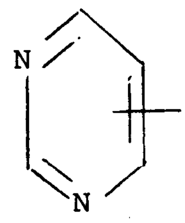

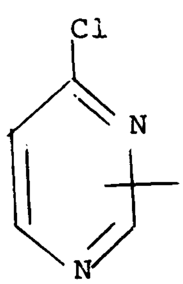 , 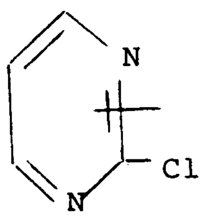 , 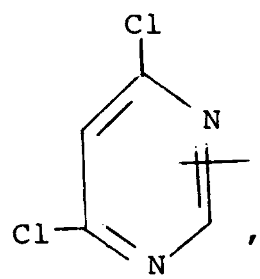 , 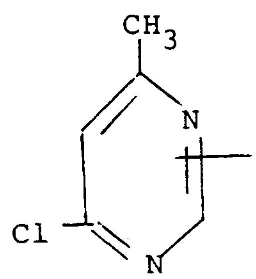

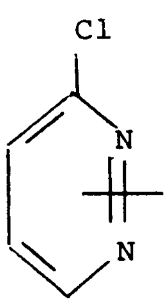 , 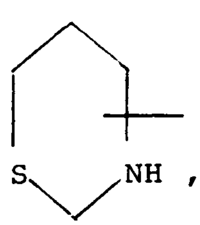 , 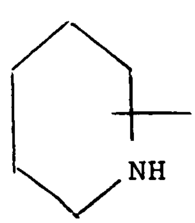 , 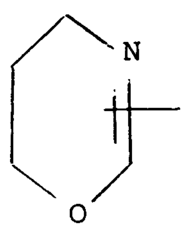

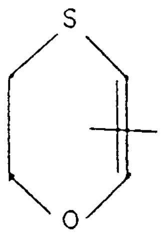 , 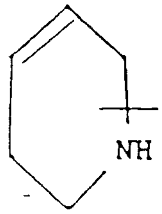 , 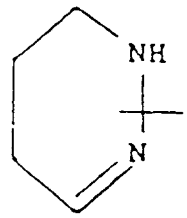 , 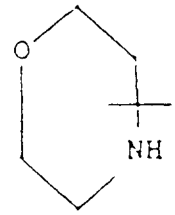

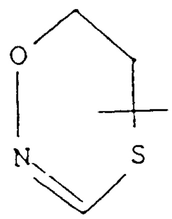 , 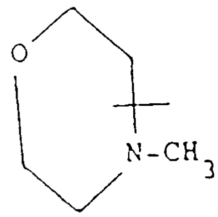 , 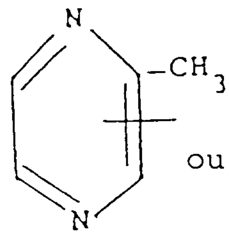 ou 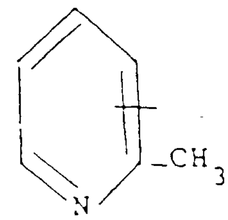

un groupe [(1-formylamino-2,2,2-trichloro)-éthyl]-amino;

un groupe 1-(3,4-diméthoxyphényl)-3-(morpholine-4-yl)-propa-1-ène-3-one;

un groupe phényle portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle; un groupe alkyle contenant jusqu'à 12 atomes de carbone, éventuellement interrompu par des atomes d'oxygène et/ou de soufre et portant éventuellement un ou plusieurs substituants hydroxy, alcoxy, mercapto, halogéno, alkylthio, nitro, cyano, amino ou mono- ou di-(alkyle en C1-C4)-amino, un groupe cycloalkyle éventuellement interrompu par des atomes d'oxygène et/ou de soufre et/ou d'azote et portant le cas échéant jusqu'à trois substituants alkyle, halogéno, hydroxy, oxo ou amino, un groupe alkyle en C1-C3, alcényle en C2-C6, alcynyle en C2-C6, cycloalcényle en C5-C8 à substituant phényle ou un groupe naphtyle, et lorsque X-Y représentent une liaison simple, $R^7$ et $R^6$ peuvent encore former ensemble un pont vicinal de formule

$-(CH_2)_n-E-$ (avec n = 2, 3), -(CH = CH)-D ou

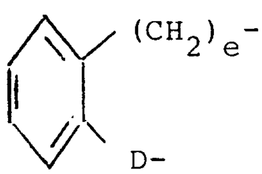

(avec e = 0 ou 1)

dans lesquels

E représente $CH_2$; O; S ou $NR^{10}$

D représente $CH_2$; O; S; $NR^{10}$; $-CH_2-CH_2-$ ou -CH = CH-, et le cas échéant leurs sels

les groupes alkyle contenant de 1 à 12 atomes de carbone;

et les composés suivants :

3-(3,4-diméthoxyphényl)-3-[4-(4,5-dihydrooxazole-2-yl)phényl]-acrylomorpholide,

3-(3,4-diméthoxyphényl)-3-(4-benzimidazole-2-yl)-phényl)-acrylomorpholide,

3-(3,4-diméthoxyphényl)-3-[4-(1,2-dichlorovinyloxy)-phényl]-acrylomorpholide,

3-(3,4-diméthoxyphényl)-3-(4-benzothiazole-2-yl)-thiométhylphényl)-acrylomorpholide,

3-(3,4-diméthoxyphényl)-3-(4-benzothiazole-2-ylthio-phényl)-acrylomorpholide,

3-(3,4-diméthoxyphényl)-3-[4-(3-p-toluène-sulfonyluréido)-phényl]-acrylomorpholide,

3-(3,4-diméthoxyphényl)-3-[4-(4,6-dichloro-1,3,5-triazine-2-yloxy)-phényl]-acrylomorpholide,

3-(3,4-diméthoxyphényl)-3-[4-(2,5-dioxopyrrole-1-yl)-phényl]-acrylomorpholide,

3-(3,4-diméthoxyphényl)-3-[4-(6-chloro-2-éthylaminopyrimidine-4-yloxy)-phényl]-acrylomorpholide,

3-(3,4-diméthoxyphényl)-3-[4-(6-chloro-2-diméthylaminopyrimidine-4-yloxy)-phényl]-acrylomorpholide 3-(3,4-diméthoxyphényl)-3-[4-(6-chloro-2-isopropylaminopyrimidine-4-yloxy)-phényl]-acrylomorpholide;

à l'exception des composés de formule I dans laquelle

B représente

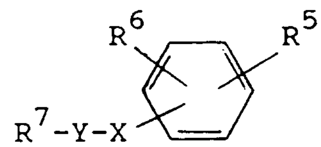

$R^1$ représente l'hydrogène, un groupe alkyle en C1-C4, cyano, chlore, brome ou l'iode;

$R^2$, $R^3$ et $R^4$, qui peuvent avoir des significations identiques ou différentes, représentent chacun l'hydrogène, un halogène, un groupe nitro, un groupe alkyle en C1-C4 ou alcoxy en C1-C4 portant éventuellement un ou plusieurs substituants fluoro ou chloro, un groupe amino, NH(alkyle en C1-C4), N(alkyle en C1-C4)$_2$, cyano, phényle, cycloalkyle en C3-C6, hydroxyalkyle en C1-C4, NHCOH, NHCO(alkyle en C1-C4), $CO_2H$, $CO_2$(alkyle en C1-C4), $CONR^{10}R^{11}$ dans lequel $R^{10}$ et $R^{11}$, qui peuvent avoir des significations identiques ou différentes, représentent chacun un groupe alkyle en C1-C4, phényle ou benzyle, et peuvent en outre former ensemble un groupe $-(CH_2)_4-$ ou $-(CH_2)_5-$, et $R^{10}$ peut en outre représenter l'hydrogène, un groupe (alkyle en C1-C4)-$S(O)_p$ (avec p = 0, 1, 2) ou hydroxy;

$R^5$ et $R^6$ qui peuvent avoir des significations identiques ou différentes, représentent chacun l'hydrogène, un halogène, un groupe alkyle en C1-C4, alcoxy en C1-C4, (alkyle en C1-C4)-$S(O)_p$ (avec p = 0, 1, 2) ou hydroxy, $R^5$ pouvant en outre représenter un groupe nitro, un groupe alkyle ou alcoxy en C1-C4 portant un ou plusieurs substituants fluoro ou chloro, un groupe NHCOH ou NHCO(alkyle en C1-C4);

X-Y représentent une liaison simple;

$R^7$ représente l'hydrogène, un groupe alkyle en C1-C4 portant éventuellement un ou plusieurs substituants fluoro ou chloro, un groupe amino, NH(alkyle en C1-C4), N-(alkyle en C1-C4)$_2$, cyano, phényle, cycloalkyle en C3-C6, hydroxyalkyle en C1-C4, $CO_2H$, $CO_2$(alkyle en C1-C4), $CONR^{10}R^{11}$ dans lequel $R^{10}$ et $R^{11}$, qui peuvent avoir des significations identiques ou différentes, représentent chacun un groupe alkyle en C1-C4, phényle ou benzyle, ou bien forment ensemble un groupe $-(CH_2)_4-$ ou $-(CH_2)_5-$, $R^{10}$ pouvant en outre représenter l'hydrogène, un groupe alkyle en C2-C8 interrompu par l'oxygène; ou bien $R^7$ et $R^6$ peuvent en outre former ensemble un pont vicinal de formule -(CH=CH)-D dans lequel D représente -CH=CH-;

Q représente $NR^8R^9$ ou

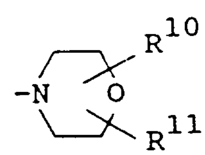

dans lesquels

$R^8$ et $R^9$ qui peuvent avoir des significations identiques ou différentes, représentent un groupe alkyle en C1-C4, cycloalkyle en C3-C7, phényle, benzyle ou alcényle en C3-C4, et $R^8$ pouvant en outre représenter l'hydrogène, et

$R^{10}$ et $R^{11}$, qui peuvent avoir des significations identiques ou différentes, représentent l'hydrogène ou un groupe alkyle en C1-C4;

et à l'exception des composés de formule I dans laquelle A et B représentent tous deux des groupes choisis parmi les groupes phényle, monohalogénophényle, mononitrophényle et monoaminophényle.

**3.** Composés de formule I selon revendication 1 ou 2, dans laquelle A représente un groupe 3,4-diméthoxyphényle, 3-éthoxy-4-méthoxyphényle, 3-chloro-4-méthoxyphényle, 3-méthyl-4-méthoxyphényle, 3-bromo-4-méthoxyphényle, 3-éthyl-4-méthoxyphényle, 3,4-diméthylphényle, 3,5-dichloro-4-aminophényle et B, Q et $R^1$ ont les significations indiquées ci-dessus.

**4.** Composés selon revendication 3, pour lesquels $R^1$ représente l'hydrogène.

**5.** Composés selon la revendication 1, 2, 3 ou 4, pour lesquels Q représente $NR^8R^9$ dans lequel $R^8$ et $R^9$ sont des groupes alkyle en C1-C4, ou le groupe morpholino.

**6.** Composés selon la revendication 1, 2, 3, 4 ou 5, pour lesquels B représente

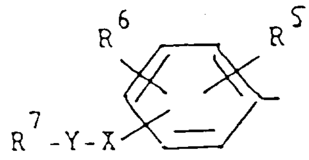

-X-Y- représentant une liaison simple, l'oxygène, le soufre, un groupe $-CH_2O-$, $-OCH_2-$ ou -N = N-.

**7.** Composés selon revendication 6, pour lesquels $R^5$ et $R^6$ représentent l'hydrogène, -X-Y- représentent une liaison simple, l'oxygène, le soufre ou -N = N- et $R^7$ représente un groupe phényle éventuellement substitué, un groupe alkyle en C1-C12 éventuellement substitué, un groupe cycloalkyle en C3-C7 éventuellement interrompu une ou plusieurs fois par l'oxygène et/ou le soufre et/ou $NR^{10}$ et portant éventuellement des substituants hydroxy ou alkyle en C1-C4, ou un cycle hétéroaryle portant jusqu'à deux substituants $R^{10}$ et $R^{11}$.

**8.** Le 3-[4-(4-chlorophénoxy)-phényl]-3-(3,4-diméthoxyphényl)-acrylomorpholide.

**9.** Le 3-(4-n-butylphényl)-phényl)-3-(3,4-diméthoxyphényl)-acrylomorpholide.

**10.** Le 3-[4-(4-chlorophényl)-phényl]-3-(3,4-diméthoxyphényl)-acrylométhyl-éthylamide.

**11.** Produits fongicides, caractérisés en ce qu'ils contiennent un composé selon les revendications 1 à 10 avec des produits auxiliaires et/ou véhicules usuels et le cas échéant une ou plusieurs autres substances actives.

**12.** Utilisation des composés selon une des revendications 1 à 10, éventuellement en combinaison avec une ou plusieurs autres substances actives fongicides, pour la lutte contre les mycètes phytopathogènes, en particulier contre le faux blanc.

**13.** Procédé de préparation des composés selon les revendications 1 à 10 par des modes opératoires connus en soi, caractérisé en ce que :

a) on fait réagir un acide acrylique de formule

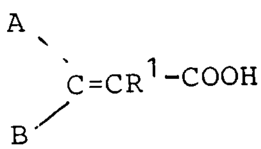

(II)

dans laquelle A, B et $R^1$ ont les significations indiquées ci-dessus, ou un dérivé réactif éventuellement préparé in situ, avec une amine de formule

HQ (III)

dans laquelle Q a les significations indiquées ci-dessus,
ou bien

b) on fait réagir une cétone de formule

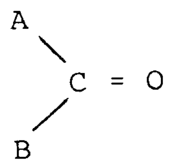

(IV)

dans laquelle A et B ont les significations indiquées ci-dessus, avec un dérivé de l'acide phosphonoacétique de formule

$(R'O)_2P(O)-CHR^1-COQ$ (V)

dans laquelle Q et $R^1$ ont les significations indiquées ci-dessus et R' représente un groupe alkyle en C1-C4,
ou bien
c) dans un acrylamide de formule

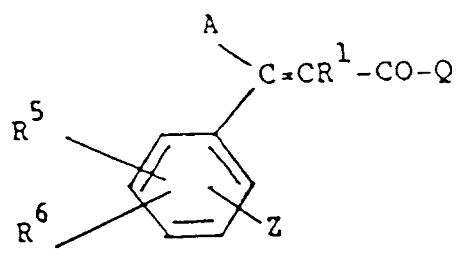

XI)

dans laquelle A, Q, $R^1$, $R^5$ et $R^6$ ont les significations indiquées ci-dessus, et Z représente l'hydrogène ou un substituant qui peut être remplacé par le groupe $-XY-R^7$ ou converti en ce groupe, on procède à ce remplacement ou à cette conversion,
ou bien
d) par la réaction de Heck appliquée à des composés de formule

B-CH = CH-COQ (XIVa)

ou

A-CH = CH-COQ (XIVb)

dans laquelle A, B et Q ont les significations indiquées ci-dessus, mais qui ne portent pas de substituants bromo ou iodo, on introduit le groupe A ou B en présence de palladium-O à l'aide de composés de formule

A - Hal (XVa)

ou

B - Hal (XVb)

dans lesquelles Hal représente le brome ou l'iode et A et B ont les significations indiquées ci-dessus, mais ne contiennent pas d'autres atomes de brome ou d'iode, et le cas échéant on soumet les composés obtenus en (a) à (d) ci-dessus à une séparation des isomères et/ou le cas échéant on convertit en sels les composés ainsi obtenus qui sont salifiables.

**Revendications pour les Etats contractants suivants : AT, ES**

**1.** Procédé de préparation des composés de formule

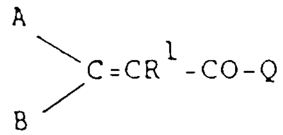

(I)

dans laquelle

A représente

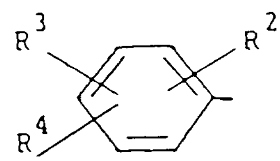

B représente

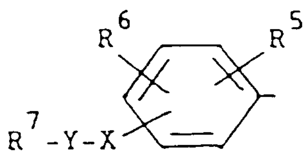 , 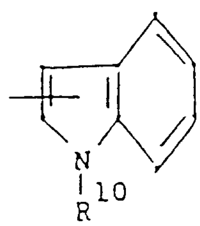 , 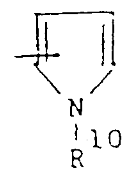

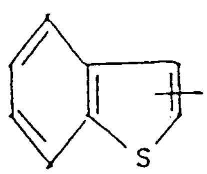 , 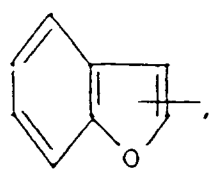 , 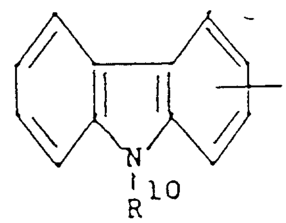

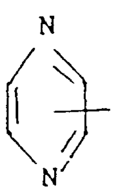 , 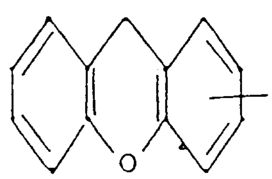 ou 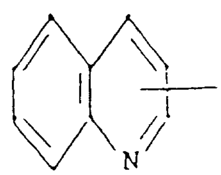

$R^1$ représente l'hydrogène; un halogène, un groupe cyano ou un groupe alkyle en C1-C4 portant éventuellement un ou plusieurs substituants hydroxy, alcoxy, mercapto, halogéno, alkylthio, nitro, cyano, amino ou mono- ou di-(alkyle en C1-C4)-amino,

Q représente

$NR^8R^9$ ou

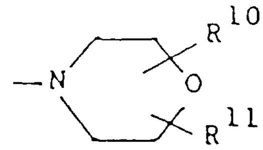

$R^2$, $R^3$, $R^4$, $R^5$ et $R^6$, qui peuvent avoir des significations identiques ou différentes, représentent chacun l'hydrogène; un halogène; un groupe nitro; un groupe cyano; un groupe carboxy; un groupe hydroxy; un groupe alcoxycarbonyle en C1-C4; un groupe $CONR^{10}R^{11}$ un groupe $NR^{10}R^{11}$; un groupe $NR^{10}$-CO-$R^{11}$; ou bien un groupe alkyle en C1-C6, alcoxy en C1-C4 ou (alkyle en C1-C4)-S(O)p (avec p = 0, 1, 2), portant éventuellement un ou plusieurs substituants hydroxy, alcoxy, mercapto, halogéno, alkylthio, nitro, cyano, amino ou mono- ou di-(alkyle en C1-C4)-amino,

un groupe cycloalkyle en C3-C7 portant éventuellement jusqu'à trois substituants alkyle, halogéno, hydroxy, oxo ou amino, un groupe phényle, phénoxy ou phénylS(O)p (avec p = 0, 1, 2) portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle,

$R^8$ et $R^9$, qui peuvent avoir des significations identiques ou différentes, représentent chacun l'hydrogène;

un groupe alkyle en C1-C3, alcényle en C3-C7, alcynyle en C3-C7 ou alcoxyalkyle portant éventuellement un ou plusieurs substituants hydroxy, alcoxy, mercapto, halogéno, alkylthio, nitro, cyano, amino ou mono- ou di-(alkyle en C1-C4)-amino, un groupe cyanoalkyle en C3-C7 portant éventuellement jusqu'à trois substituants alkyle, halogéno, hydroxy, oxo ou amino, un groupe phényle ou benzyle portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle,

$R^{10}$ et $R^{11}$, qui peuvent avoir des significations identiques ou différentes, représentent chacun l'hydrogène, un groupe benzyle, phényle ou alkyle en C1-C4, ou encore, en tant que substituants d'un noyau hétéroaryle, un halogène, un groupe amino, ou mono-ou di-(alkyle en C1-C4)-amino,

ou bien forment ensemble un groupe $-(CH_2)_4-$ ou $-(CH_2)_5-$,

X-Y représentent une liaison simple;

-O-; -S(O)p- (avec p = 0, 1, 2); -$CONR^{10}$-; -$NR^{10}$CO-; -$NR^{11}CONR^{10}$-; -N=N-; -$CHR^{10}$-S(O)p-(avec p = 0, 1, 2); -$CHR^{10}$O-; -$SO_2NR^{10}$-; -N=CH-; -$C_nH_{2n}$- (avec n = 1 à 10); -CH=CH-; -$NR^{10}CSNR^{11}$-; -$NR^{10}$-; -$R^{10}$N-$CHR^{11}$-; -$CHR^{10}NR^{11}$-; -O-$CHR^{10}$-; -S(O)p-$CHR^{10}$- (avec p = 0, 1, 2); -$R^{10}SO_2$- ou -$OSO_2$-; -$O_2$SO-; -$O_2$S-$NR^{10}$-; -C≡C-; -S-S-; -CHOH-; -CO-;
>C=$CH_2$; >C=CH-COOH; -NH-NH-; -N(O)=N-;

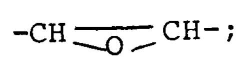

-N($R^{10}$)-N=CH-; -N($R^{10}$)-NH-CO-; -NH-CH=CH-; >CH-$C_6H_5$; -$COCH_2$O-; -CO-$CH_2$S-; -$COCH_2NR^{10}$-; -$OCH_2$CO; -$SCH_2$CO-; -CH=N-; -$NR^{10}CH_2$CO-; -CH=NO-; -$CH_2$O-$CONR^{10}$-; -$CH_2$O-CO-; -S-$CH_2$-; -$CH_2$CO-; -CO-O-; -O-CO-; -CO-S- ou -S-CO-;

$R^7$ représente l'hydrogène; $NR^{10}R^{11}$; PO($OR^{10}$)($OR^{11}$); $(CH_2)$q-CO-O-$R^{10}$ (avec q = 0, 1, 2, 3); cyano; $CONR^{10}R^{11}$; $SO_2NR^{10}R^{11}$;

un groupe tri-(alkyle en C1-C4)-silyle; un noyau hétéroaryle portant jusqu'à deux substituants $R^{10}$ et $R^{11}$, le noyau hétéroaryle consistant en un noyau pyrazolyle, pyrazinyle, imidazolyle, 1,2,4-triazolyle, morpholinyle, pipéridinyle, pyrrolyle, pyrrolidinyle, 2,5-dioxopyrrolidinyle, 1,3-isoindole-dionyle, et

pyridinyle ou en un noyau dérivé de l'indole, du benzofuranne, de la quinoléine ou du benzothiophène, ou en un noyau

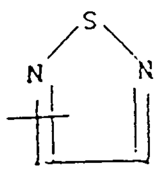 , 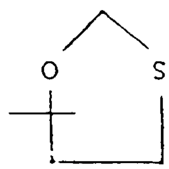 , 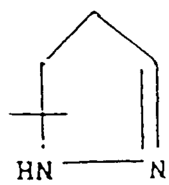 , 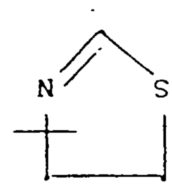

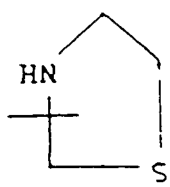 , 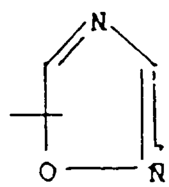 , 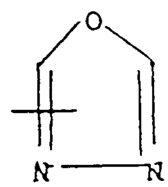 , 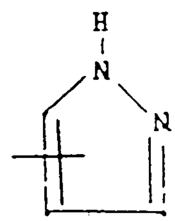

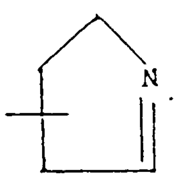 , 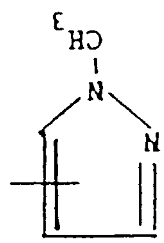 , 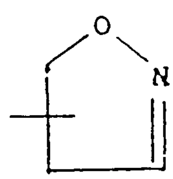 , 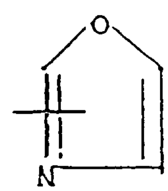

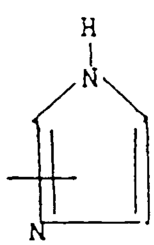 , 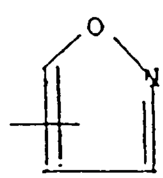 , 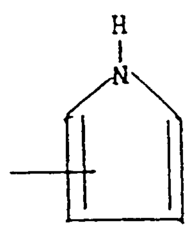 , 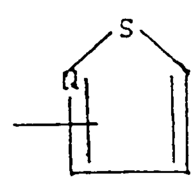

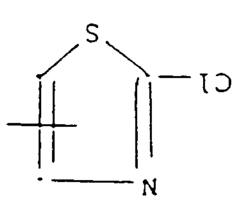 , 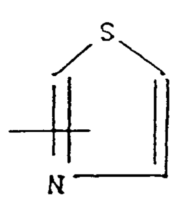 , 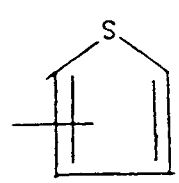 , 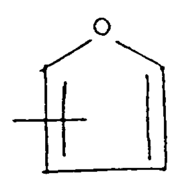

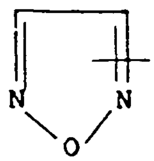 , 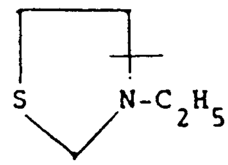 , 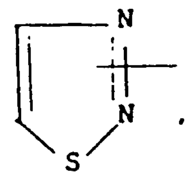 , 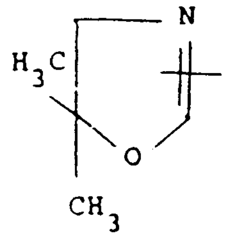

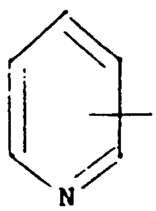 , 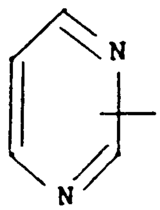 , 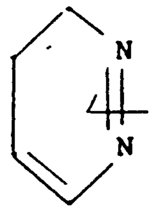 , 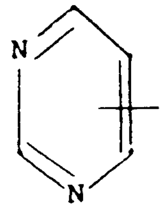

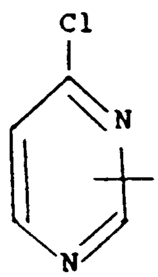 , 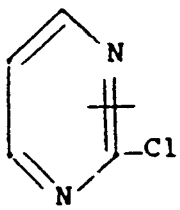 , 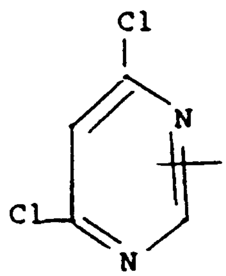 , 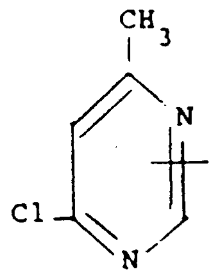

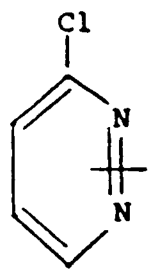 , 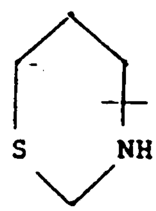 , 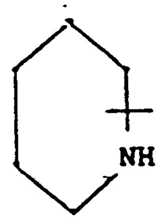 , 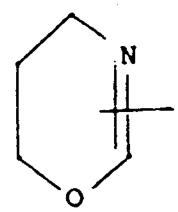

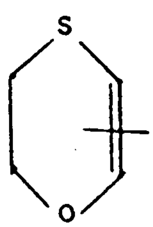 , 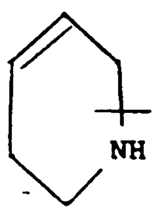 , 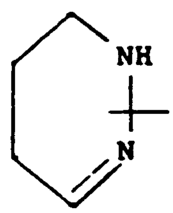 , 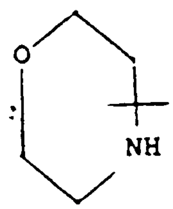

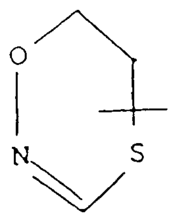 , 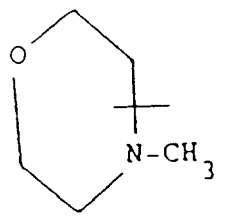 , 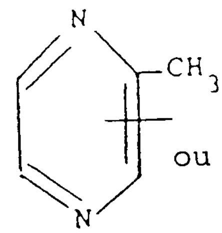 ou 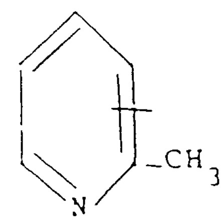

un groupe [(1-formylamino-2,2,2-trichloro)-éthyl]-amino,

un groupe 1-(3,4-diméthoxyphényl)-3-(morpholino-4-yl)-propa-1-ène-3-one;

un groupe phényle portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle; un groupe alkyle contenant jusqu'à 12 atomes de carbone, éventuellement interrompu par des atomes d'oxygène et/ou de soufre et portant éventuellement un ou plusieurs substituants hydroxy, alcoxy, mercapto, halogéno, alkylthio, nitro, cyano, amino, mono- ou di-(alkyle en C1-C4)-amino, un groupe cycloalkyle éventuellement interrompu par des atomes d'oxygène et/ou de soufre et/ou d'azote et portant éventuellement jusqu'à trois substituants alkyle, halogéno, hydroxy, oxo ou amino, un groupe alkyle en C1-C3, alcényle en C2-C6, alcynyle en C2-C6, cycloalcényle en C5-C8 à substituant phényle ou un groupe naphtyle et lorsque X - Y représentent une liaison simple,

$R^7$ et $R^6$ peuvent encore former ensemble un pont vicinal de formule

$-(CH_2)_n-E-$ (avec n = 2, 3), -(CH = CH)-D ou

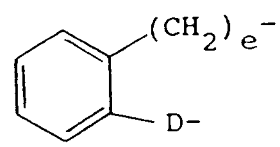

(avec e = 0 ou 1)

dans lesquels

E représente $CH_2$; O; S ou $NR^{10}$;

D représente $CH_2$; O; S; $NR^{10}$; $-CH_2-CH_2-$ ou -CH = CH-, et le cas échéant leurs sels;

dans lesquels les groupes alkyle contiennent de 1 à 12 atomes de carbone;

et les composés suivants :

3-(3,4-diméthoxyphényl)-3-[4-(4,5-dihydrooxazole-2-yl)phényl]-acrylomorpholide,

3-(3,4-diméthoxyphényl)-3-(4-benzimidazole-2-yl-phényl)-acrylomorpholide,

3-(3,4-diméthoxyphényl)-3-[4-(1,2-dichlorovinyloxy)-phényl]-acrylomorpholide,

3-(3,4-diméthoxyphényl)-3-(4-benzothiazole-2-yl-thiométhylphényl)-acrylomorpholide,

3-(3,4-diméthoxyphényl)-3-(4-benzothiazole-2-ylthio-phényl)-acrylomorpholide,

3-(3,4-diméthoxyphényl)-3-[4-(3-p-toluène-sulfonyluréido)-phényl]-acrylomorpholide,

3-(3,4-diméthoxyphényl)-3-[4-(4,6-dichloro-1,3,5-triazine-2-yloxy)-phényl]-acrylomorpholide,

3-(3,4-diméthoxyphényl)-3-[4-(2,5-dioxopyrrole-1-yl)-phényl]-acrylomorpholide,

3-(3,4-diméthoxyphényl)-3-[4-(6-chloro-2-éthylaminopyrimidine-4-yloxy)-phényl]-acrylomorpholide,

3-(3,4-diméthoxyphényl)-3-[4-(6-chloro-2-diméthylaminopyrimidine-4-yloxy)-phényl]-acrylomorpholide,

3-(3,4-diméthoxyphényl)-3-[4-(6-chloro-2-isopropylaminopyrimidine-4⁻yloxy)-phényl]-acrylomorpholide;

à l'exception de l'objet décrit dans le brevet européen n° 0 120 321 et à l'exception des composés de formule I dans laquelle A et B repréentent tous deux un groupe choisi parmi les groupes phényle, monohalogénophényle, mononitrophényle et monoaminophényle,

selon des modes opératoires connus en soi, caractérisé en ce que :

(a) on fait réagir un acide acrylique

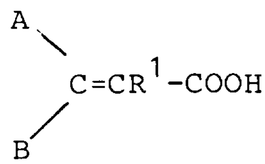

(II)

dans laquelle A, B et $R^1$ ont les significations indiquées ci-dessus, ou un dérivé réactif éventuellement préparé in situ, avec une amine de formule

HQ (III)

dans laquelle Q a les significations indiquées ci-dessus,
ou bien
b) on fait réagir une cétone de formule

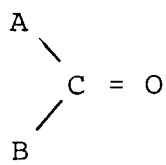

(IV)

dans laquelle A et B ont les significations indiquées ci-dessus, avec un dérivé de l'acide phosphonoacétique de formule

$(R'O)_2P(O)-CHR^1-COQ$ (V)

dans laquelle Q et $R^1$ ont les significations indiquées ci-dessus et R' représente un groupe alkyle en C1-C4,
ou bien
c) dans un acrylamide de formule

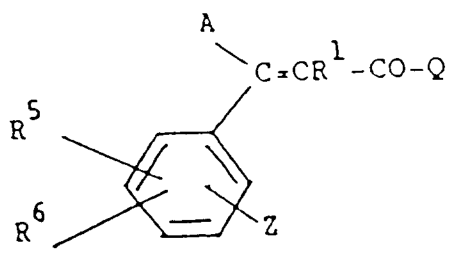

XI)

dans laquelle A, Q, $R^1$, $R^5$ et $R^6$ ont les significations indiquées ci-dessus, et Z représente l'hydrogène ou un substituant qui peut être remplacé par le groupe $-XY-R^7$ ou converti en ce groupe, on procède à ce remplacement ou à cette conversion,
ou bien
d) par la réaction de Heck appliquée à des composés de formule

B-CH = CH-COQ (XIVa)

ou

A-CH = CH-COQ (XIVb)

dans laquelle A, B et Q ont les significations indiquées ci-dessus, mais qui ne portent pas de

substituants bromo ou iodo, on introduit le groupe A ou B en présence de palladium-O à l'aide de composés de formule

A - Hal (XVa)

ou

B - Hal (XVb)

dans lesquelles Hal représente le brome ou l'iode et A et B ont les significations indiquées ci-dessus, mais ne contiennent pas d'autres atomes de brome ou d'iode, et le cas échéant on soumet les composés obtenus en (a) à (d) ci-dessus à une séparation des isomères et/ou le cas échéant on convertit en sels les composés ainsi obtenus qui sont salifiables.

**2.** Procédé de préparation des composés de formule I de la revendication 1

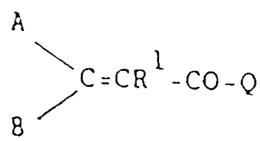

(I)

dans laquelle

A représente

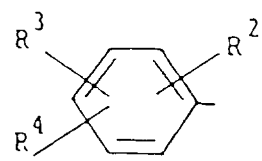

B représente

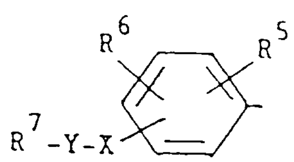
,
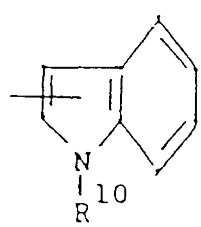
,
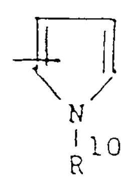

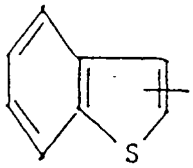
,
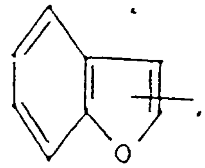
,
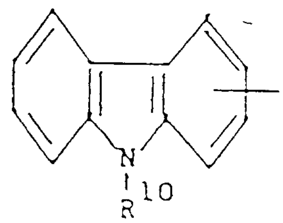

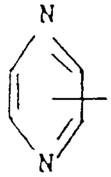
,
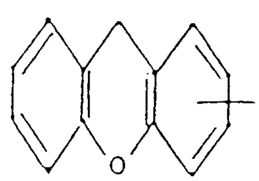
ou
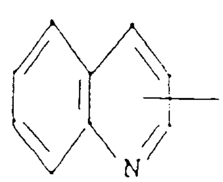

$R^1$ représente l'hydrogène; un halogène, un groupe cyano ou alkyle en C1-C4 portant éventuellement un ou plusieurs substituants hydroxy, alcoxy, mercapto, halogéno, alkylthio, nitro, cyano, amino ou mono- ou di-(alkyle en C1-C4)-amino,

Q représente

$NR^8R^9$ ou

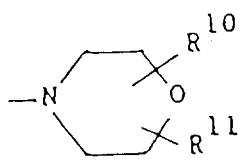

$R^2$, $R^3$, $R^4$, $R^5$ et $R^6$, qui peuvent avoir des significations identiques ou différentes, représentent chacun l'hydrogène, un halogène, un groupe nitro, cyano, carboxy, hydroxy, (alcoxy en C1-C4)-carbonyle, $CONR^{10}R^{11}$, $NR^{10}R^{11}$, $NR^{10}$-CO-$R^{11}$, ou un groupe alkyle en C1-C7, alcoxy en C1-C4 ou (alkyle en C1-C4)-S(O)p (avec p = 0, 1, 2) portant éventuellement un ou plusieurs substituants hydroxy, alcoxy, mercapto, halogéno, alkylthio, nitro, cyano, amino ou mono- ou di-(alkyle en C1-C4)-amino, un groupe cycloalkyle en C3-C7 portant éventuellement jusqu'à trois substituants alkyle, halogéno, hydroxy, oxo ou amino,

un groupe phényle, phénoxy ou phényl-S(O)p (avec p = 0, 1, 2) portant éventuellement jusqu'à trois substituants halogéno, alkyle en C1-C4, alkylthio, alcoxy, nitro, cyano, amino, monoalkylamino, dialkylamine, halogénoalkyle, halogénoalkylthio, halogénoalcoxy, carboxy ou alcoxycarbonyle,

$R^8$ et $R^9$, qui peuvent avoir des significations identiques ou différentes, représentent chacun l'hydrogène;

un groupe alkyle en C1-C4, alcényle en C3-C7 ou alcoxyalkyle portant éventuellement un ou plusieurs substituants hydroxy, alcoxy, mercapto, halogéno, alkylthio, nitro, cyano, amino ou mono- ou di-(alkyle en C1-C4)-amino, un groupe cycloalkyle en C3-C7 portant éventuellement jusqu'à trois substituants alkyle, halogéno, hydroxy, oxo ou amino, un groupe phényle ou benzyle portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle,

$R^{10}$ et $R^{11}$, qui peuvent avoir des significations identiques ou différentes, représentent chacun l'hydrogène, un groupe benzyle, phényle ou alkyle en C1-C4 ou bien encore, en tant que substituants d'un noyau hétéroaryle, un halogène, un groupe amino ou mono-ou di-(alkyle en C1-C4)-amino,

ou forment ensemble un groupe $-(CH_2)_4-$ ou $-(CH_2)_5-$,

X-Y représentent une liaison simple;

-O-; -S(O)p- (avec p = 0, 1, 2); $-CONR^{10}-$; $-NR^{10}CO-$; $-NR^{11}CONR^{10}-$; -N=N-; $-CHR^{10}$-S(O)p-(avec p = 0, 1, 2); $-CHR^{10}O-$; $-SO_2NR^{10}-$; -N=CH-; $-C_nH_{2n}-$ (avec n = 1 à 10); -CH=CH-; $-NR^{10}CSNR^{11}-$; $-NR^{10}-$; $-R^{10}N-CHR^{11}-$; $-CHR^{10}NR^{11}-$; $-O-CHR^{10}-$; $-S(O)p-CHR^{10}-$ (avec p = 0, 1, 2); $-NR^{10}SO_2-$ ou $-OSO_2-$; $-O_2SO-$; $-O_2S-NR^{10}-$; -C≡C-; -S-S-; -CHOH-; -CO-;

$>C=CH_2$; $>C=CH-COOH$; -NH-NH-; -N(O)=N-;

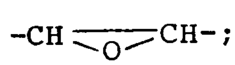

$-N(R^{10})-N=CH-$; $-N(R^{10})-NH-CO-$; -NH-CH=CH-; $>CH-C_6H_5$; $-COCH_2O-$; $-CO-CH_2S-$; $-COCH_2NR^{10}-$; $-OCH_2CO$; $-SCH_2CO-$; -CH=N-; $-NR^{10}CH_2CO-$; -CH=NO-; $-CH_2O-CONR^{10}-$; $-CH_2O-CO-$; $-S-CH_2-$; $-CH_2CO-$; -CO-O-; -O-CO-; -CO-S- ou -S-CO-;

$R^7$ représente l'hydrogène; $NR^{10}R^{11}$; $PO(OR^{10})(OR^{11})$; $(CH_2)q$-CO-O-$R^{10}$ (avec q = 0, 1, 2, 3); cyano; $CONR^{10}R^{11}$; $SO_2NR^{10}R^{11}$;
un groupe tri-(alkyle en C1-C4)-silyle; un noyau hétéroaryle portant jusqu'à deux substituants $R^{10}$ et $R^{11}$, ce noyau hétéroaryle consistant en un noyau pyrazolyle, pyrazinyle, imidazolyle, 1,2,4-triazolyle, morpholinyle, pipéridinyle, pyrrolyle, pyrrolidinyle, 2,5-dioxopyrrolidinyle, 1,3-isoindole-dionyle ou pyridinyle, ou en un noyau dérivé de l'indole, du benzofuranne, de la quinoléine ou du benzothiophène, ou en un noyau

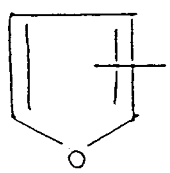 , 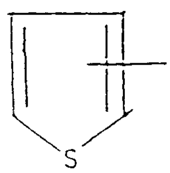 , 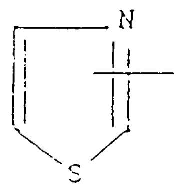 , 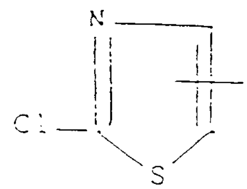

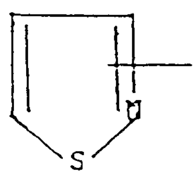 , 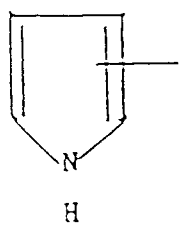 , 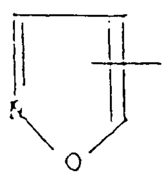 , 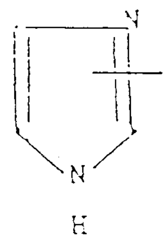

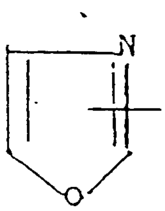 , 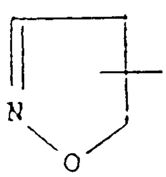 , 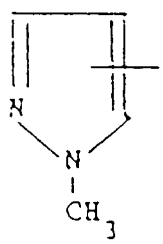 , 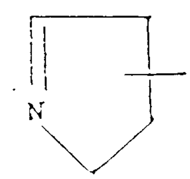

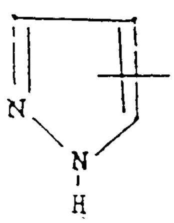 , 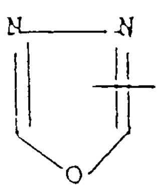 , 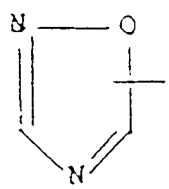 , 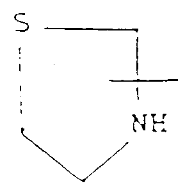

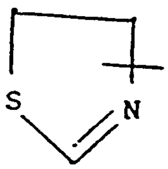 , 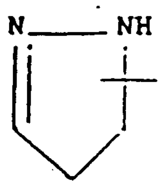 , 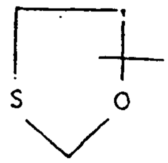 , 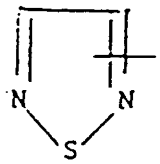

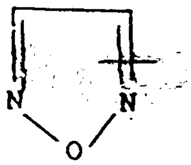 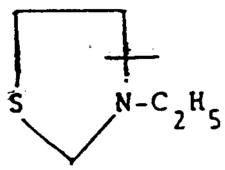 . 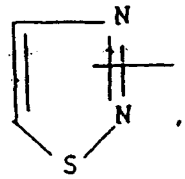 , 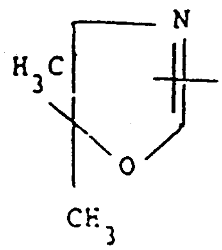

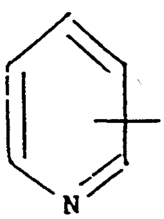 , 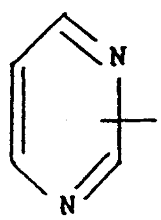 , 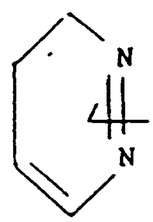 , 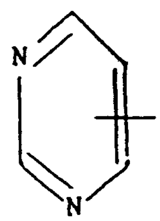

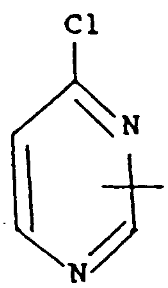 , 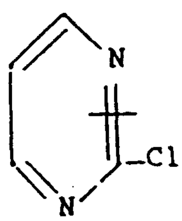 , 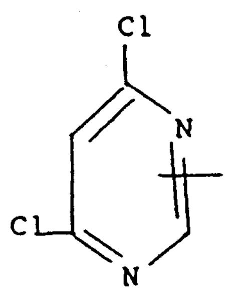 , 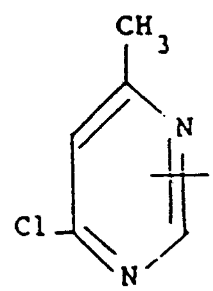

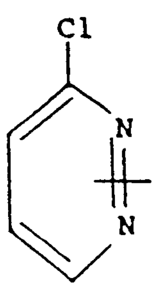 , 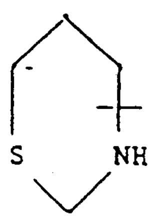 , 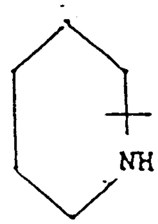 , 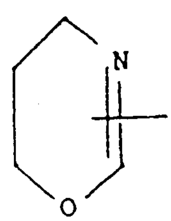

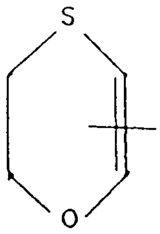, 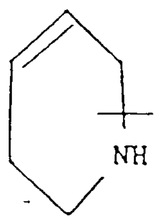, 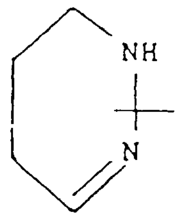, 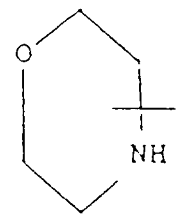

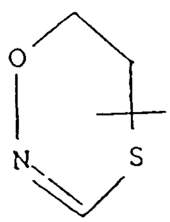, 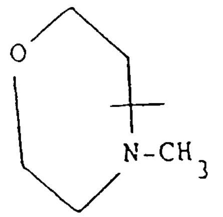, 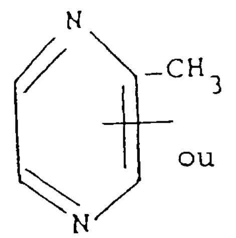 ou 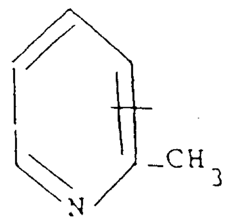

un groupe [(1-formylamino-2,2,2-trichloro)-éthyl]-amino;

un groupe 1-(3,4-diméthoxyphényl)-3-(morpholine-4-yl)-propa-1-ène-3-one;

un groupe phényle portant éventuellement jusqu'à trois substituants halogéno, alkyle en C1-C4, alkylthio, alcoxy, nitro, cyano, amino, monoalkylamino, dialkylamino, halogénoalkyle, halogénoalkylthio, halogénoalcoxy, carboxy ou alcoxycarbonyle; un groupe alkyle contenant jusqu'à 12 atomes de carbone, éventuellement interrompu par des atomes d'oxygène et/ou de soufre et portant éventuellement un ou plusieurs substituants hydroxy, alcoxy, mercapto, halogéno, alkylthio, nitro, cyano, amino ou mono- ou di-(alkyle en C1-C4)-amino, un groupe cycloalkyle éventuellement interrompu par des atomes d'oxygène et/ou de soufre et/ou d'azote et portant le cas échéant jusqu'à trois substituants alkyle, halogéno, hydroxy, oxo ou amino, un groupe alkyle en C1-C3, alcényle en C2-C6, alcynyle en C2-C6, cycloalcényle en C5-C8 à substituant phényle ou un groupe naphtyle, et lorsque X-Y représentent une liaison simple, $R^7$ et $R^6$ peuvent encore former ensemble un pont vicinal de formule

$-(CH_2)_n-E-$ (avec n = 2, 3), $-(CH=CH)-D$ ou

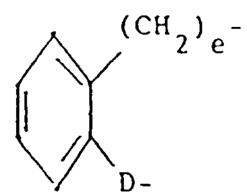

(avec e = 0 ou 1)

dans lesquels

E représente $CH_2$; O; S ou $NR^{10}$

D représente $CH_2$; O; S; $NR^{10}$; $-CH_2-CH_2-$ ou $-CH=CH-$, et le cas échéant leurs sels

les groupes alkyle contenant de 1 à 12 atomes de carbone;

et les composés suivants :

3-(3,4-diméthoxyphényl)-3-[4-(4,5-dihydrooxazole-2-yl)phényl]-acrylomorpholide,

3-(3,4-diméthoxyphényl)-3-(4-benzimidazole-2-yl)-phényl)-acrylomorpholide,

3-(3,4-diméthoxyphényl)-3-[4-(1,2-dichlorovinyloxy)-phényl]-acrylomorpholide,

3-(3,4-diméthoxyphényl)-3-(4-benzothiazole-2-yl)-thiométhylphényl)-acrylomorpholide,

3-(3,4-diméthoxyphényl)-3-(4-benzothiazole-2-ylthio-phényl)-acrylomorpholide,

3-(3,4-diméthoxyphényl)-3-[4-(3-p-toluène-sulfonyluréido)-phényl]-acrylomorpholide,

3-(3,4-diméthoxyphényl)-3-[4-(4,6-dichloro-1,3,5-triazine-2-yloxy)-phényl]-acrylomorpholide,

3-(3,4-diméthoxyphényl)-3-[4-(2,5-dioxopyrrole-1-yl)-phényl]-acrylomorpholide,

3-(3,4-diméthoxyphényl)-3-[4-(6-chloro-2-éthylaminopyrimidine-4-yloxy)-phényl]-acrylomorpholide,

3-(3,4-diméthoxyphényl)-3-[4-(6-chloro-2-diméthylaminopyrimidine-4-yloxy)-phényl]-acrylomorpholide 3-(3,4-diméthoxyphényl)-3-[4-(6-chloro-2-isopropylaminopyrimidine-4-yloxy)-phényl]-acrylomorpholide;
à l'exception des composés de formule I dans laquelle

B représente

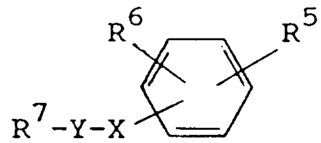

$R^1$ représente l'hydrogène, un groupe alkyle en C1-C4, cyano, chlore, brome ou l'iode;

$R^2$, $R^3$ et $R^4$, qui peuvent avoir des significations identiques ou différentes, représentent chacun l'hydrogène, un halogène, un groupe nitro, un groupe alkyle en C1-C4 ou alcoxy en C1-C4 portant éventuellement un ou plusieurs substituants fluoro ou chloro, un groupe amino, NH(alkyle en C1-C4), N(alkyle en C1-C4)$_2$, cyano, phényle, cycloalkyle en C3-C6, hydroxyalkyle en C1-C4, NHCOH, NHCO(alkyle en C1-C4), $CO_2H$, $CO_2$(alkyle en C1-C4), $CONR^{10}R^{11}$ dans lequel $R^{10}$ et $R^{11}$, qui peuvent avoir des significations identiques ou différentes, représentent chacun un groupe alkyle en C1-C4, phényle ou benzyle, et peuvent en outre former ensemble un groupe $-(CH_2)_4-$ou $-(CH_2)_5-$, et $R^{10}$ peut en outre représenter l'hydrogène, un groupe (alkyle en C1-C4)-$S(O)_p$ (avec p = 0, 1, 2) ou hydroxy;

$R^5$ et $R^6$ qui peuvent avoir des significations identiques ou différentes, représentent chacun l'hydrogène, un halogène, un groupe alkyle en C1-C4, alcoxy en C1-C4, (alkyle en C1-C4)-$S(O)_p$ (avec p = 0, 1, 2) ou hydroxy, $R^5$ pouvant en outre représenter un groupe nitro, un groupe alkyle ou alcoxy en C1-C4 portant un ou plusieurs substituants fluoro ou chloro, un groupe NHCOH ou NHCO(alkyle en C1-C4);

X-Y représentent une liaison simple;

$R^7$ représente l'hydrogène, un groupe alkyle en C1-C4 portant éventuellement un ou plusieurs substituants fluoro ou chloro, un groupe amino, NH(alkyle en C1-C4), N-(alkyle en C1-C4)$_2$, cyano, phényle, cycloalkyle en C3-C6, hydroxyalkyle en C1-C4, $CO_2H$, $CO_2$(alkyle en C1-C4), $CONR^{10}R^{11}$ dans lequel $R^{10}$ et $R^{11}$, qui peuvent avoir des significations identiques ou différentes, représentent chacun un groupe alkyle en C1-C4, phényle ou benzyle, ou bien forment ensemble un groupe $-(CH_2)_4-$ ou $-(CH_2)_5-$, $R^{10}$ pouvant en outre représenter l'hydrogène, un groupe alkyle en C2-C8 interrompu par l'oxygène; ou bien $R^7$ et $R^6$ peuvent en outre former ensemble un pont vicinal de formule -(CH=CH)-D dans lequel D représente -CH=CH-;

Q représente $NR^8R^9$ ou

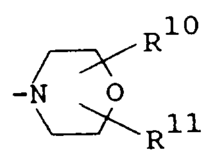

dans lesquels

$R^8$ et $R^9$ qui peuvent avoir des significations identiques ou différentes, représentent un groupe alkyle en C1-C4, cycloalkyle en C3-C7, phényle, benzyle ou alcényle en C3-C4, et $R^8$ pouvant en outre représenter l'hydrogène, et

$R^{10}$ et $R^{11}$, qui peuvent avoir des significations identiques ou différentes, représentent l'hydrogène ou un groupe alkyle en C1-C4;

et à l'exception des composés de formule I dans laquelle A et B représentent tous deux des groupes choisis parmi les groupes phényle, monohalogénophényle, mononitrophényle et monoaminophényle.

**3.** Procédé selon revendication 1 ou 2, dans lequel A représente un groupe 3,4-diméthoxyphényle, 3-éthoxy-4-méthoxyphényle, 3-chloro-4-méthoxyphényle, 3-méthyl-4-méthoxyphényle, 3-bromo-4-méthoxyphényle, 3-éthyl-4-méthoxyphényle, 3,4-diméthylphényle, 3,5-dichloro-4-aminophényle et B, Q et $R^1$ ont les significations indiquées ci-dessus.

**4.** Procédé selon revendication 3, dans lequel $R^1$ représente l'hydrogène.

**5.** Procédé selon la revendication 1, 2, 3 ou 4, dans lequel Q représente $NR^8R^9$ et $R^8$ et $R^9$ des groupes alkyle en C1-C4, ou le groupe morpholino.

**6.** Procédé selon revendication 1, 2, 3, 4 ou 5, dans lequel B représente

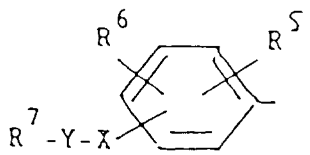

dans lequel -X-Y- représentent une liaison simple, l'oxygène, le soufre, $-CH_2O-$, $-OCH_2-$ ou -N = N-.

**7.** Procédé selon revendication 6, dans lequel $R^5$ et $R^6$ représentent l'hydrogène, -X-Y- représentent une liaison simple, l'oxygène, le soufre ou -N = N- et $R^7$ représente un groupe phényle éventuellement substitué, un groupe alkyle en C1-C12 éventuellement substitué, un groupe cycloalkyle en C3-C7 éventuellement interrompu une ou plusieurs fois par l'oxygène et/ou le soufre et/ou $NR^{10}$ et portant éventuellement des substituants hydroxy ou alkyle en C1-C4, ou un cycle hétéroaryle portant jusqu'à deux substituants $R^{10}$ et $R^{11}$.

**8.** Procédé selon revendication 1, 2, 3, 4, 5, 6 ou 7, dans lequel le composé de formule I est le 3-[4-(4-chlorophénoxy)-phényl]-3-(3,4-diméthoxyphényl)-acrylomorpholide.

**9.** Procédé selon revendication 1, 2, 3, 4, 5, 6 ou 7, dans lequel le composé de formule I est le 3-[(4-n-butylphényl)-phényl]-3-(3,4-diméthoxyphényl)-acrylomorpholide.

**10.** Procédé selon revendication 1, 2, 3, 4, 5, 6 ou 7, dans lequel le composé de formule I est le 3-[4-(4-chlorophényl)-phényl]-3-(3,4-diméthoxyphényl)-acrylo-méthyl-éthylamide.

**11.** Procédé de préparation de produits fongicides, caractérisé en ce que l'on combine un composé selon les revendications 1 à 10 avec les produits auxiliaires et/ou véhicules usuels et le cas échéant une ou plusieurs autres substances actives.

**12.** Utilisation de composés selon une des revendications 1 à 10, éventuellement en combinaison avec une ou plusieurs autres substances actives fongicides, pour la lutte contre les mycètes phytopathogènes et en particulier contre le faux blanc.